(19) Europäisches Patentamt
European Patent Office
Office européen des brevets

(11) EP 4 362 119 A1

(12) **EUROPEAN PATENT APPLICATION**
published in accordance with Art. 153(4) EPC

(43) Date of publication:
**01.05.2024 Bulletin 2024/18**

(21) Application number: **22828516.9**

(22) Date of filing: **23.06.2022**

(51) International Patent Classification (IPC):
***H01L 51/50*** (2006.01) ***C07D 209/86*** (2006.01)
***C07F 5/02*** (2006.01) ***C09K 11/06*** (2006.01)

(52) Cooperative Patent Classification (CPC):
**C07D 209/86; C07F 5/02; C09K 11/06**

(86) International application number:
**PCT/JP2022/025188**

(87) International publication number:
**WO 2022/270600 (29.12.2022 Gazette 2022/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**23.06.2021 JP 2021103702**
**17.09.2021 JP 2021151805**
**17.12.2021 JP 2021204983**
**19.04.2022 JP 2022068628**
**28.04.2022 JP 2022073952**
**06.06.2022 JP 2022091795**

(71) Applicant: **Kyulux, Inc.**
**Fukuoka-shi, Fukuoka 819-0388 (JP)**

(72) Inventors:
- **YOSHIZAKI, Makoto**
**Fukuoka-shi, Fukuoka 819-0388 (JP)**
- **HWANG, Songhye**
**Fukuoka-shi, Fukuoka 819-0388 (JP)**
- **MORIMOTO, Kei**
**Fukuoka-shi, Fukuoka 819-0388 (JP)**
- **ENDO, Ayataka**
**Fukuoka-shi, Fukuoka 819-0388 (JP)**
- **KUROTAKI, Masayuki**
**Fukuoka-shi, Fukuoka 819-0388 (JP)**
- **KANAHARA, Kousei**
**Fukuoka-shi, Fukuoka 819-0388 (JP)**

(74) Representative: **Zimmermann & Partner Patentanwälte mbB**
**Postfach 330 920**
**80069 München (DE)**

(54) **ORGANIC LIGHT EMITTING ELEMENT AND FILM**

(57) An organic light-emitting device including a compound represented by each of the following formulas has excellent luminescence characteristics. One of $X^1$ and $X^2$ is N, and the other is B; $R^1$ to $R^{26}$, $A^1$, and $A^2$ are H, D, or a substituent; one of $R^{112}$ and $R^{113}$ is CN or a triazinyl group; and the other one, $R^{111}$, $R^{114}$, and $R^{115}$ are H, D, a donor group having a substituted amino group, or an aryl group, but at least one thereof is a substituted or ring-condensed carbazole-9-yl group.

EP 4 362 119 A1

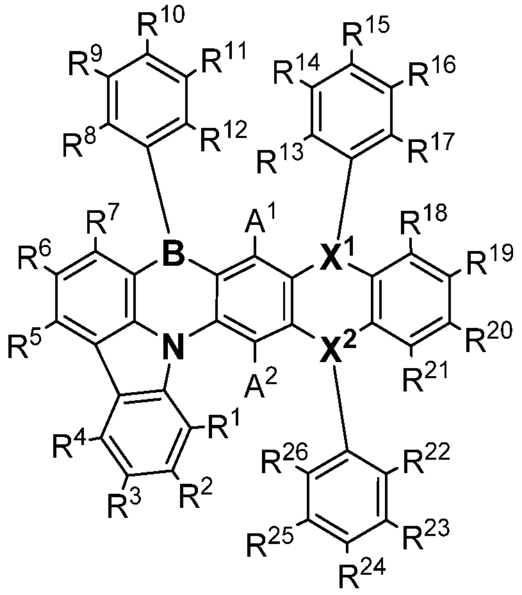

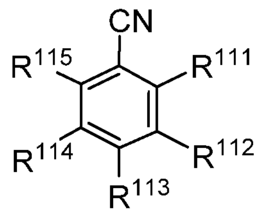

## Description

Background Art

The present invention relates to an organic light-emitting device and a film having good luminescence characteristics.

Researches have been actively conducted to improve the luminous efficiency of organic light-emitting devices such as organic light emitting diodes (OLEDs).

For example, Non-Patent Document 1 describes that when a compound that exhibits a multiple resonance effect, such as 5,9-diphenyl-5H,9H-[1,4]benzazaborino[2,3,4-kl]phenazaborine(DABNA-1), is used, thermal activation-type delayed fluorescence is expressed by an inverse intersystem crossing process, and then light emission with narrow half width and high color purity is realized. Such light emission is useful in display-oriented purposes because high luminous efficiency can be achieved.

Further, Non-Patent Documents 1 and 2 describe that through modification of DABNA-1, energy levels such as the highest occupied molecular orbital (HOMO) and the lowest unoccupied molecular orbital (LUMO) are adjusted, and a fluorescence radiation process or an inverse intersystem crossing process which contributes to light emission is promoted, thereby improving the electroluminescence quantum efficiency.

Non-Patent Document 1 Adv. Mater. 2016, 28, 2777-2781
Non-Patent Document 2 Angew. Chem. Int. Ed. 2018, 57, 11316-11320

In this manner, although various studies have been conducted on compounds that exhibit the multiple resonance effect, there are also many unknown points about the relationship between the structure and the luminescence characteristics. In order to manufacture a practical light emitting device, it is required to provide a material having excellent luminescence characteristics as much as possible. Further, it is desirable not only to provide a material having excellent luminescence characteristics, but also to provide an organic light-emitting device having more excellent luminescence characteristics by selecting materials to be used in combination therewith.

Therefore, the present inventors have conducted intensive studies for the purpose of developing derivatives of a compound exhibiting a multiple resonance effect and providing an organic light-emitting device having more excellent luminescence characteristics by selecting materials to be used in combination therewith.

Summary of Invention

The present inventors have conducted the intensive studies, and as a result, have found that among compounds exhibiting the multiple resonance effect, those having a specific structure have excellent luminescence characteristics. It has been found that the luminescence characteristics become more excellent by using the compounds in combination with a material having a specific structure. The invention is suggested on the basis of such findings, and has the following configurations.

[1] An organic light-emitting device including a compound represented by the following formula (1) and a compound represented by the following formula (2).

Formula (1)

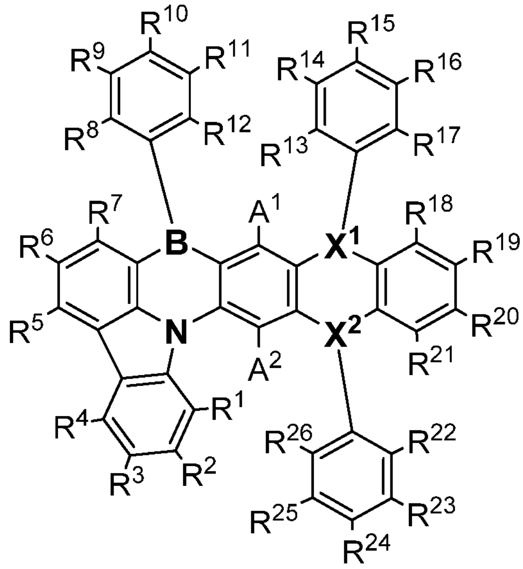

[In the formula (1), one of $X^1$ and $X^2$ is a nitrogen atom, and the other is a boron atom. Each of $R^1$ to $R^{26}$, $A^1$, and $A^2$ independently represents a hydrogen atom, a deuterium atom, or a substituent. $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ may be bonded to each other to form ring structures. Here, when $X^1$ is a nitrogen atom, $R^{17}$ and $R^{18}$ are bonded to each other to form a single bond and to form a pyrrole ring, and when $X^2$ is a nitrogen atom, $R^{21}$ and $R^{22}$ are bonded to each other to form a single bond and to form a pyrrole ring. Here, when $X^1$ is a nitrogen atom, $R^7$ and $R^8$, and $R^{21}$ and $R^{22}$ are bonded via nitrogen atoms to form 6-membered rings, and $R^{17}$ and $R^{18}$ are bonded to each other to form a single bond, at least one of $R^1$ to $R^6$ is a substituted or unsubstituted aryl group, or an aromatic ring or a heteroaromatic ring is formed through bonding in any of $R^1$ and $R^2$, $R^2$ and $R^3$, $R^3$ and $R^4$, $R^4$ and $R^5$, and $R^5$ and $R^6$.]

Formula (2)

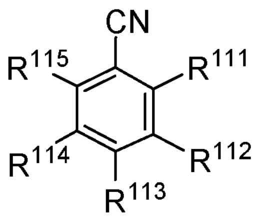

[In the formula (2), one of $R^{112}$ and $R^{113}$ represents a cyano group or a substituted or unsubstituted triazinyl group. Each of the other of $R^{112}$ and $R^{113}$, and $R^{111}$, $R^{114}$, and $R^{115}$ independently represents a hydrogen atom, a deuterium atom, a donor group having a substituted amino group, or a substituted or unsubstituted aryl group (excluding the donor group having a substituted amino group). Here, each of the other of $R^{112}$ and $R^{113}$, and at least one of $R^{111}$, $R^{114}$, and $R^{115}$ is independently a carbazole-9-yl group (the carbazole-9-yl group is at least substituted, or has a ring further condensed).]

[2] The organic light-emitting device described in [1], in which both $R^3$ and $R^6$ in the formula (1) are substituents.

[3] The organic light-emitting device described in [1] or [2], in which both $R^8$ and $R^{12}$ in the formula (1) are substituents. In particular, in the compound, $R^8$ and $R^{12}$ are alkyl groups having 2 or more carbon atoms, preferably alkyl groups having 3 or more carbon atoms, more preferably alkyl groups having 3 to 8 carbon atoms, further preferably alkyl groups having 3 or 4 carbon atoms.

[4] The organic light-emitting device described in any one of [1] to [3], in which in the formula (1), $X^1$ is a nitrogen atom, and $X^2$ is a boron atom.

[5] The organic light-emitting device described in any one of [1] to [4], in which in the formula (1), $R^7$ and $R^8$, and

$R^{17}$ and $R^{18}$ are bonded to each other to form -B($R^{32}$)-, and each of $R^{32}$'s independently represents a hydrogen atom, a deuterium atom, or a substituent.

[6] The organic light-emitting device described in any one of [1] to [4], in which $R^7$ and $R^8$, and $R^{17}$ and $R^{18}$ in the formula (1) are bonded to each other to form -CO-.

[7] The organic light-emitting device described in any one of [1] to [4], in which $R^7$ and $R^8$, and $R^{17}$ and $R^{18}$ in the formula (1) are bonded to form -CS-.

[8] The organic light-emitting device described in any one of [1] to [4], in which in the formula (1), $R^7$ and $R^8$, and $R^{17}$ and $R^{18}$ are bonded to form -N($R^{27}$)-, and each of $R^{27}$'s independently represents a hydrogen atom, a deuterium atom, or a substituent.

[9] The organic light-emitting device described in any one of [1] to [8], in which 1 to 6 sets among $R^1$ and $R^2$, $R^2$ and $R^3$, $R^3$ and $R^4$, $R^4$ and $R^5$, $R^5$ and $R^6$, $R^6$ and $R^7$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{18}$ and $R^{19}$, $R^{19}$ and $R^{20}$, $R^{20}$ and $R^{21}$, $R^{22}$ and $R^{23}$, $R^{23}$ and $R^{24}$, $R^{24}$ and $R^{25}$, and $R^{25}$ and $R^{26}$ in the formula (1) are bonded to each other to form benzofuran rings or benzothiophene rings.

[10] The organic light-emitting device described in any one of [1] to [9], in which the compound represented by the formula (1) has a rotationally symmetric structure.

[11] The organic light-emitting device described in [1], in which the compound represented by the formula (1) has any of following structures. For example, the compound may be selected from the group consisting of 9 compounds other than a compound on the top left.

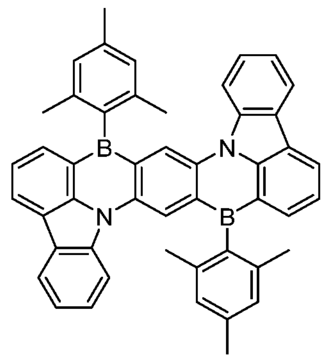

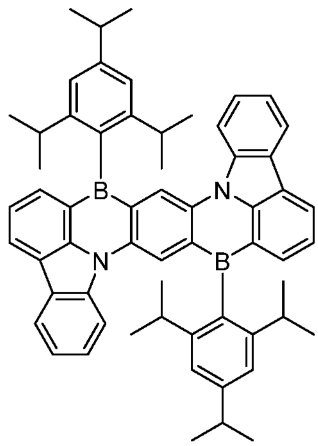

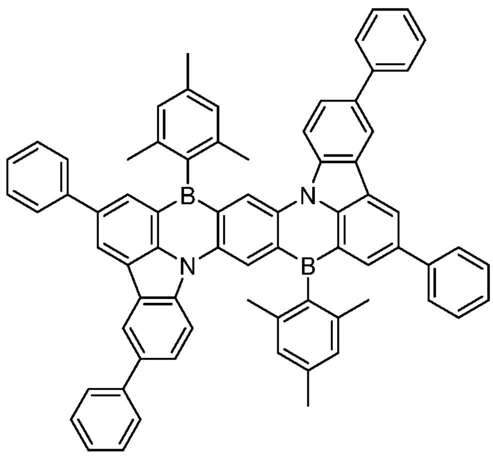

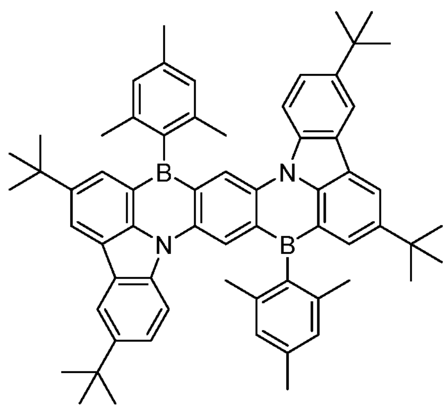

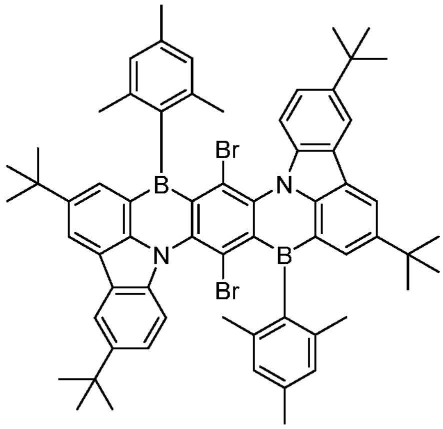
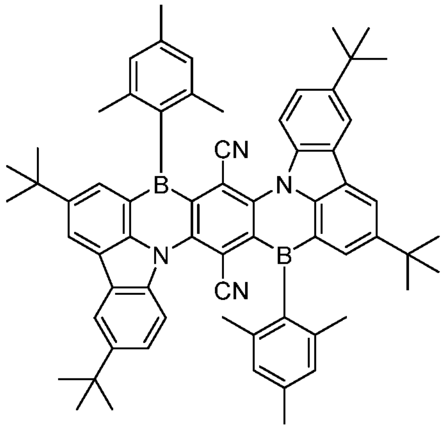
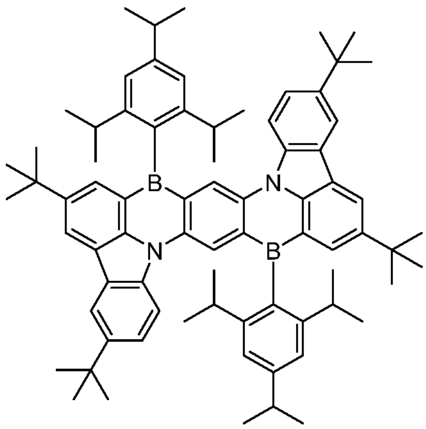
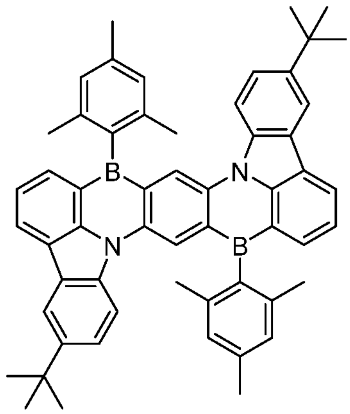
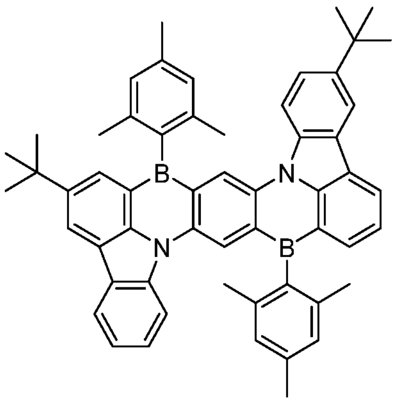
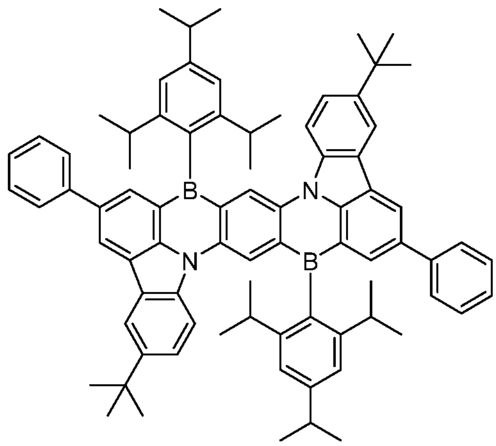

[12] The organic light-emitting device described in any one of [1] to [11], in which $R^{112}$ in the formula (2) is a cyano group or a substituted or unsubstituted triazinyl group.

[13] The organic light-emitting device described in any one of [1] to [12], in which the donor group in the formula (2) has a carbazole-9-yl structure.

[14] A film including the compound represented by the formula (1) and the compound represented by the formula (2).

[15] The film described in [14], in which a content of the compound represented by the formula (1) is smaller than that of the compound represented by the formula (2).

[16] The film described in [14] or [15], further including a host material having lowest excited singlet energy higher than that of the compound represented by the formula (1) and the compound represented by the formula (2).

In the film of the invention, the light-emitting material exhibits excellent orientation, and thus, the film can be preferably used in the organic light-emitting device. Further, the organic light-emitting device of the invention exhibits

excellent luminescence characteristics. In particular, the organic light-emitting device of the invention has high luminous efficiency, long device lifetime, and excellent durability.

Description of Embodiments

Hereinafter, the contents of the invention will be described in detail. The descriptions on constituent elements to be described below may be made on the basis of representative embodiments or specific examples of the invention, but the invention is not limited to such embodiments or specific examples. The numerical value range represented by using "to" in the present specification means a range including numerical values described before and after "to", as the lower limit value and the upper limit value. Further, a part or all of hydrogen atoms present in the molecule of the compound used in the invention may be replaced with deuterium atoms ($^2H$, deuterium D). In the chemical structural formula of the present specification, the hydrogen atom is indicated by H, or the indication thereof is omitted. For example, when the indication of an atom bonded to a ring skeleton forming carbon atom of a benzene ring is omitted, it is assumed that, at a location where the indication is omitted, H is bonded to the ring skeleton forming carbon atom. In the present specification, the term of "substituent" means an atom or a group of atoms other than a hydrogen atom and a deuterium atom. Meanwhile, the term of "substituted or unsubstituted" means that a hydrogen atom may be substituted with a deuterium atom or a substituent.

[Compound represented by formula (1)]

A compound represented by the following formula (1) will be described.

Formula (1)

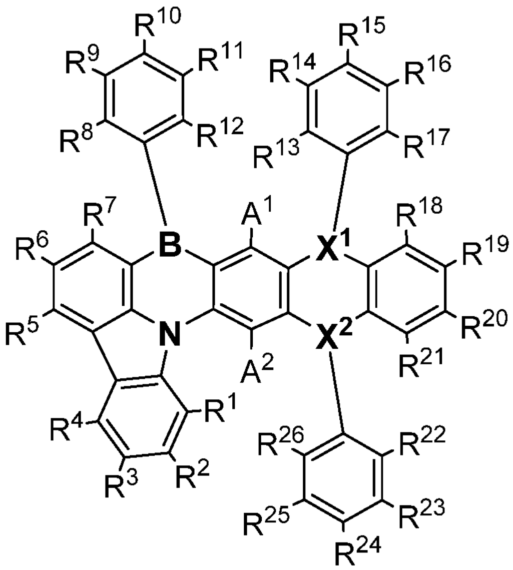

In the formula (1), one of $X^1$ and $X^2$ is a nitrogen atom, and the other is a boron atom. In one aspect of the invention, $X^1$ is a nitrogen atom, and $X^2$ is a boron atom. Here, $R^{17}$ and $R^{18}$ are bonded to each other to form a single bond so as to form a pyrrole ring. In another aspect of the invention, $X^1$ is a boron atom, and $X^2$ is a nitrogen atom. Here, $R^{21}$ and $R^{22}$ are bonded to each other to form a single bond so as to form a pyrrole ring.

In the formula (1), each of $R^1$ to $R^{26}$, $A^1$, and $A^2$ independently represents a hydrogen atom, a deuterium atom, or a substituent.

$R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ may be bonded to each other to form ring structures.

The ring structure formed by combining $R^7$ and $R^8$ includes a boron atom and four carbon atoms as ring skeleton forming atoms. The ring structure formed by combining $R^{17}$ and $R^{18}$ includes a boron atom and four carbon atoms as ring skeleton forming atoms when $X^1$ is a boron atom. When $X^1$ is a nitrogen atom, the ring structure is limited to a pyrrole ring. The ring structure formed by combining $R^{21}$ and $R^{22}$ includes a boron atom and four carbon atoms as ring

skeleton forming atoms when $X^2$ is a boron atom. When $X^2$ is a nitrogen atom, the ring structure is limited to a pyrrole ring. When $R^7$ and $R^8$, $R^{17}$ and $R^{18}$, and $R^{21}$ and $R^{22}$ are bonded to each other to form boron atom-containing ring structures, the ring structure is preferably a 5 to 7-membered ring, more preferably a 5 or 6-membered ring, further preferably a 6-membered ring. When $R^7$ and $R^8$, $R^{17}$ and $R^{18}$, and $R^{21}$ and $R^{22}$ are bonded to each other, these preferably form a single bond, -O-, -S-, -N($R^{27}$)-, -C($R^{28}$)($R^{29}$)-, -Si($R^{30}$)($R^{31}$)-, -B($R^{32}$)-, -CO-, or -CS- by combining with each other, more preferably form -O-, -S- or -N($R^{27}$)-, further preferably form -N($R^{27}$)-. Here, each of $R^{27}$ to $R^{32}$ independently represents a hydrogen atom, a deuterium atom, or a substituent. As the substituent, a group selected from any of substituent groups A to E to be described below may be employed, but a substituted or unsubstituted alkyl group, a substituted or unsubstituted aryl group, or a substituted or unsubstituted heteroaryl group is preferable. In particular, $R^{27}$ is preferably a substituted or unsubstituted aryl group. When $R^{27}$ to $R^{32}$ are substituents, $R^{27}$ to $R^{32}$ in the ring formed by bonding $R^7$ and $R^8$ to each other may further form a ring structure by bonding to at least one of $R^6$ and $R^9$, $R^{27}$ to $R^{32}$ in the ring formed by bonding $R^{17}$ and $R^{18}$ to each other may further form a ring structure by bonding to at least one of $R^{16}$ and $R^{19}$, and $R^{27}$ to $R^{32}$ in the ring formed by bonding $R^{21}$ and $R^{22}$ to each other may further form a ring structure by bonding to at least one of $R^{20}$ and $R^{23}$. In one aspect of the invention, in only one set among $R^7$ and $R^8$, $R^{17}$ and $R^{18}$, and $R^{21}$ and $R^{22}$, these are bonded to each other. In one aspect of the invention, in only two sets among $R^7$ and $R^8$, $R^{17}$ and $R^{18}$, and $R^{21}$ and $R^{22}$, these are bonded to each other. In one aspect of the invention, all of $R^7$ and $R^8$, $R^{17}$ and $R^{18}$, and $R^{21}$ and $R^{22}$ are bonded to each other.

The ring structure formed by bonding $R^1$ and $R^2$, $R^2$ and $R^3$, $R^3$ and $R^4$, $R^4$ and $R^5$, $R^5$ and $R^6$, $R^6$ and $R^7$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{18}$ and $R^{19}$, $R^{19}$ and $R^{20}$, $R^{20}$ and $R^{21}$, $R^{22}$ and $R^{23}$, $R^{23}$ and $R^{24}$, $R^{24}$ and $R^{25}$, and $R^{25}$ and $R^{26}$ to each other may be an aromatic ring or an adipose ring, or may include a hetero atom. Further, one or more rings, as other rings, may be condensed. The hetero atom mentioned herein is preferably one selected from the group consisting of a nitrogen atom, an oxygen atom, and a sulfur atom. Examples of the ring structure to be formed include a benzene ring, a pyridine ring, a pyridazine ring, a pyrimidine ring, a pyrazine ring, a pyrrole ring, an imidazole ring, a pyrazole ring, a triazole ring, an imidazoline ring, a furan ring, a thiophene ring, an oxazole ring, an isooxazole ring, a thiazole ring, an isothiazole ring, a cyclohexadiene ring, a cyclohexene ring, a cyclopentene ring, a cycloheptatriene ring, a cycloheptadiene ring, a cycloheptene ring, and a ring in which one or more rings selected from the group consisting of these rings are further condensed. In a preferred aspect of the invention, the ring structure is a substituted or unsubstituted benzene ring (further, a ring may be condensed), and is for example, a benzene ring which may be substituted with an alkyl group or an aryl group. In a preferred aspect of the invention, the ring structure is a substituted or unsubstituted heteroaromatic ring, preferably a furan ring of benzofuran, or a thiophene ring of benzothiophene. Among $R^1$ and $R^2$, $R^2$ and $R^3$, $R^3$ and $R^4$, $R^4$ and $R^5$, $R^5$ and $R^6$, $R^6$ and $R^7$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{18}$ and $R^{19}$, $R^{19}$ and $R^{20}$, $R^{20}$ and $R^{21}$, $R^{22}$ and $R^{23}$, $R^{23}$ and $R^{24}$, $R^{24}$ and $R^{25}$, and $R^{25}$ and $R^{26}$, the number of combinations that are bonded to each other to form ring structures may be 0, or may be, for example, any one of 1 to 6. For example, it may be any one of 1 to 4, 1 may be selected, 2 may be selected, or 3 or 4 may be selected. In one aspect of the invention, in one set selected from $R^1$ and $R^2$, $R^2$ and $R^3$, and $R^3$ and $R^4$, a ring structure is formed through bonding. In one aspect of the invention, $R^5$ and $R^6$ are bonded to each other to form a ring structure. In one aspect of the invention, in one set selected from $R^9$ and $R^{10}$, $R^{10}$ and $R^{11}$, and $R^{11}$ and $R^{12}$, a ring structure is formed through bonding. In one aspect of the invention, in both of $R^1$ and $R^2$, and $R^{13}$ and $R^{14}$, ring structures are formed through bonding. In one aspect of the invention, in one set selected from $R^1$ and $R^2$, $R^2$ and $R^3$, and $R^3$ and $R^4$, a ring structure is formed through bonding, and moreover $R^5$ and $R^6$ are bonded to each other to form a ring structure. In one aspect of the invention, in both of $R^5$ and $R^6$, and $R^{19}$ and $R^{20}$, ring structures are formed through bonding.

$R^1$ to $R^{26}$ which are not bonded to adjacent $R^n$(n=1 to 26) together are hydrogen atoms, deuterium atoms, or substituents. As the substituent, a group selected from any of substituent groups A to E to be described below may be employed.

Preferable substituents that may be possessed by $R^1$ to $R^{26}$ are a substituted or unsubstituted alkyl group, a substituted or unsubstituted aryl group, and a substituted or unsubstituted heteroaryl group. For example, the substituent may be a substituted or unsubstituted aryl group, and for example the substituent may be a substituted or unsubstituted alkyl group. As the substituent of the alkyl group, the aryl group, or the heteroaryl group mentioned herein, a group selected from any of substituent groups A to E may also be employed. Meanwhile, one or more groups selected from the group consisting of an alkyl group, an aryl group, and a heteroaryl group are preferred, and a group of a substituent group E is more preferred, and it may be unsubstituted. In a preferred aspect of the invention, at least one of $R^1$ to $R^6$ is a substituent, preferably a group of a substituent group E. For example, at least one of $R^2$ to $R^6$ is a substituent, preferably a group of a substituent group E. For example, at least one of $R^5$ and $R^6$ is a substituent, preferably a group of a substituent group E. In a preferred aspect of the invention, at least one of $R^3$ and $R^6$ is a substituent, more preferably both are substituents, and a group of a substituent group E is preferred. In a preferred aspect of the invention, when $X^1$ is a nitrogen atom, at least one of $R^{15}$ and $R^{20}$ is a substituent, more preferably both are substituents, and a group of a substituent group E is preferred. Here, $R^{17}$ and $R^{18}$ are bonded to each other to form a single bond. In a preferred aspect

of the invention, when $X^2$ is a nitrogen atom, at least one of $R^{19}$ and $R^{24}$ is a substituent, more preferably both are substituents, and a group of a substituent group E is preferred. Here, $R^{21}$ and $R^{22}$ are bonded to each other to form a single bond. In one aspect of the invention, at least one of $R^8$ and $R^{12}$ is a substituent, and preferably both are substituents. In one aspect of the invention, $R^8$, $R^{10}$ and $R^{12}$ are substituents. As for the substituent of $R^8$ to $R^{12}$, an unsubstituted alkyl group is preferable. In particular, the case where $R^8$ and $R^{12}$ are alkyl groups having 2 or more carbon atoms (preferably alkyl groups having 3 or more carbon atoms, more preferably alkyl groups having 3 to 8 carbon atoms, further preferably alkyl groups having 3 to 4 carbon atoms) is preferable because orientation becomes high when a film is formed. Among them, particularly preferred is a case where $R^8$ and $R^{12}$ are substituents (preferably alkyl groups, more preferably alkyl groups having 2 or more carbon atoms, further preferably alkyl groups having 3 or more carbon atoms, still further preferably alkyl groups having 3 to 8 carbon atoms, particularly preferably alkyl groups having 3 or 4 carbon atoms), and moreover, at least one of $R^1$ to $R^6$ is a substituent (preferably a group of a substituent group E). When $X^1$ is a boron atom, at least one of $R^{13}$ and $R^{17}$ is a substituent, and preferably both are substituents. In one aspect of the invention, when $X^1$ is a boron atom, $R^{13}$, $R^{15}$ and $R^{17}$ are substituents. When $X^1$ is a boron atom, as for the substituent of $R^{13}$ to $R^{17}$, an unsubstituted alkyl group is preferable. When $X^2$ is a boron atom, at least one of $R^{22}$ and $R^{26}$ is a substituent, and preferably both are substituents. In one aspect of the invention, when $X^2$ is a boron atom, $R^{22}$, $R^{24}$ and $R^{26}$ are substituents. When $X^2$ is a boron atom, as for the substituent of $R^{22}$ to $R^{26}$, an unsubstituted alkyl group is preferable. Specific examples of the group that is bonded to the boron atom represented by B in the formula (1) or the boron atom represented by $X^1$ or $X^2$ will be given below. Meanwhile, groups bonded to the boron atom, which may be adopted in the invention, are not construed as limiting to the following specific examples. In the present specification, indication of $CH_3$ is omitted for a methyl group. * represents a bond position.

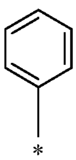
Ar1

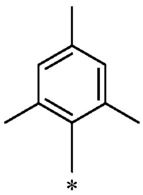
Ar2

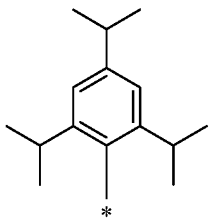
Ar3

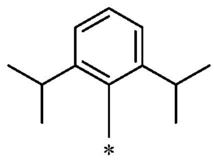
Ar4

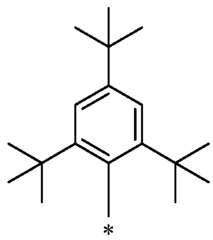
Ar5

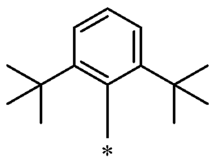
Ar6

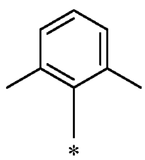
Ar7

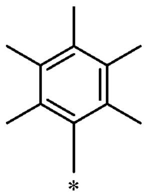
Ar8

Hereinafter, specific examples of $R^1$ to $R^{26}$ in the formula (1) will be given. Z1 to Z9 are preferable as $R^1$ to $R^7$, as $R^{13}$ to $R^{21}$ when $X^1$ is a nitrogen atom, and as $R^{18}$ to $R^{26}$ when $X^2$ is a nitrogen atom. Z1 to Z7 are preferable as $R^8$ to $R^{12}$, as $R^{22}$ to $R^{26}$ when $X^1$ is a nitrogen atom, and as $R^{13}$ to $R^{17}$ when $X^2$ is a nitrogen atom. Meanwhile, groups bonded to the boron atom, which may be adopted in the invention, are not construed as limiting to the following specific examples. D represents a deuterium atom. * represents a bond position.

*−H
Z1

*−D
Z2

$*-CH_3$
Z3

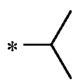
Z4

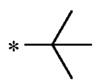
Z5

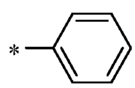
Z6

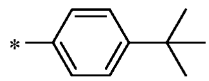 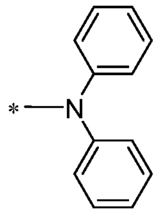 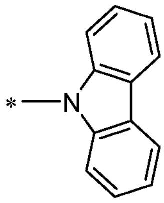

Z7 Z8 Z9

$A^1$ and $A^2$ are hydrogen atoms, deuterium atoms, or substituents. As for the substituent, a group selected from any of substituent groups A to E to be described below may be adopted.

In a preferred aspect of the invention, each of $A^1$ and $A^2$ is independently a hydrogen atom or a deuterium atom. For example, $A^1$ and $A^2$ are hydrogen atoms. For example, $A^1$ and $A^2$ are deuterium atoms.

One of $A^1$ and $A^2$ may be a substituent. Further, each of $A^1$ and $A^2$ may be independently a substituent.

Apreferable substituent that may be possessed by $A^1$ and $A^2$ is an acceptor group. The acceptor group is a group having a positive Hammett $\sigma p$ value. Here, the "Hammett $\sigma p$ value", which is proposed by L.P. Hammett, indicates the quantified effect of a substituent on the reaction rate or equilibrium of a para-substituted benzene derivative. Specifically, it is a constant ($\alpha p$) peculiar to the substituent in the following equation, which is established between the substituent in the para-substituted benzene derivative and the reaction rate constant or the equilibrium constant:

$$\log(k/k_0)=\rho\sigma p$$

or

$$\log(K/K_0)=\rho\sigma p$$

In the equation, $k_0$ represents a rate constant of a benzene derivative having no substituent, k represents a rate constant of a benzene derivative substituted with a substituent, $K_0$ represents an equilibrium constant of a benzene derivative having no substituent, K represents an equilibrium constant of a benzene derivative substituted with a substituent, and $\rho$ represents a reaction constant determined by the type and condition of the reaction. In relation to descriptions on "the Hammett $\sigma p$ value" and the numerical value of each substituent in the invention, the description on the $\sigma p$ value may be referred to in Hansch, C.et.al., Chem. Rev., 91, 165-195(1991).

The acceptor group that may be possessed by $A^1$ and $A^2$ is more preferably a group having a Hammett $\sigma p$ value greater than 0.2. Examples of the group having a Hammett $\sigma p$ value greater than 0.2 include a cyano group, an aryl group substituted with at least a cyano group, a fluorine atom-containing group, and a substituted or unsubstituted heteroaryl group containing a nitrogen atom as a ring skeleton forming atom. The aryl group substituted with at least a cyano group, which is mentioned herein, may be substituted with a substituent other than the cyano group (for example, an alkyl group or an aryl group), but may be an aryl group substituted with only a cyano group. The aryl group substituted with at least a cyano group is preferably a phenyl group substituted with at least a cyano group. The number of substitutions of the cyano group is preferably one or two, and, for example, may be one, or may be two. As the fluorine atom-containing group, a fluorine atom, an alkyl fluoride group, and an aryl group substituted with at least a fluorine atom or an alkyl fluoride group may be mentioned. The alkyl fluoride group is preferably a perfluoroalkyl group, and the number of carbon atoms is preferably 1 to 6, more preferably 1 to 3. Further, the heteroaryl group containing a nitrogen atom as a ring skeleton forming atom may be a monocycle, or may be a condensed ring in which two or more rings are condensed. In the case of a condensed ring, the number of rings after condensation is preferably two to six, and, for example, may be selected from two to four, or may be two. Specific examples of the ring forming the heteroaryl group include a pyridine ring, a pyrimidine ring, a pyrazine ring, a triazine ring, a quinoline ring, an isoquinoline ring, a quinazoline ring, a quinoxaline ring, and a naphthyridine ring other than the quinazoline ring or the quinoxaline ring. The ring forming the heteroaryl group may be substituted with a deuterium atom or a substituent, and as for the substituent, for example, one group selected from the group consisting of an alkyl group, an aryl group and a heteroaryl group or a group formed by combining two or more thereof may be mentioned. As the acceptor group that $A^1$ and $A^2$ may have, a cyano group is particularly preferable.

In one aspect of the invention, at least one of $A^1$ and $A^2$ is an acceptor group. In one aspect of the invention, only one of $A^1$ and $A^2$ is an acceptor group. In one aspect of the invention, both $A^1$ and $A^2$ are the same acceptor groups. In one aspect of the invention, $A^1$ and $A^2$ are different acceptor groups. In one aspect of the invention, $A^1$ and $A^2$ are

cyano groups. In one aspect of the invention, $A^1$ and $A^2$ are halogen atoms, e.g., bromine atoms.

Hereinafter, specific examples of the acceptor group that may be adopted in the invention will be illustrated. Meanwhile, the acceptor group that may be used in the invention is not construed as limiting to the following specific examples. In the present specification, the indication of a methyl group is omitted. Thus, for example, A15 indicates a group including two 4-methyl phenyl groups. Further, "D" represents a deuterium atom. * represents a bond position.

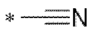 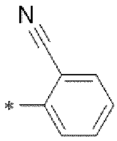 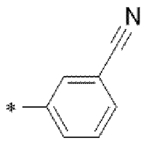 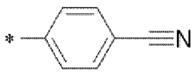 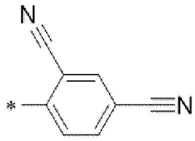 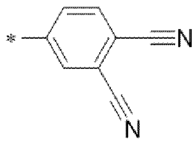

A1 A2 A3 A4 A5 A6

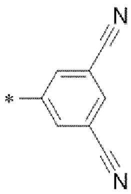 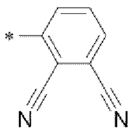 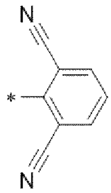 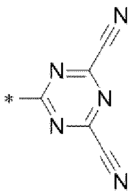 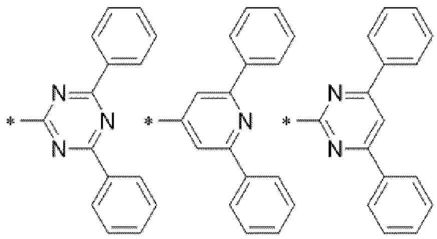

A7 A8 A9 A10 A11 A12 A13

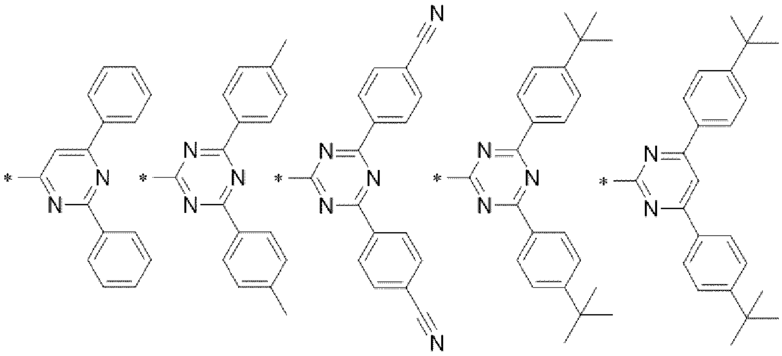

A14 A15 A16 A17 A18

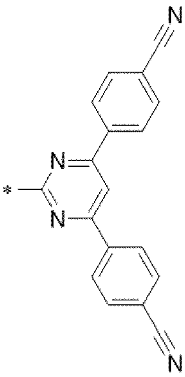 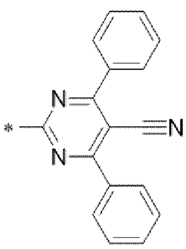 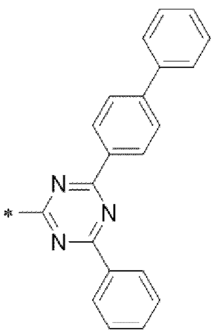 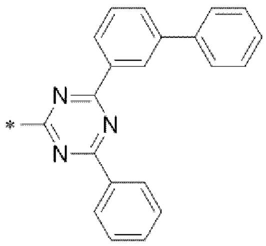

A19 A20 A21 A22

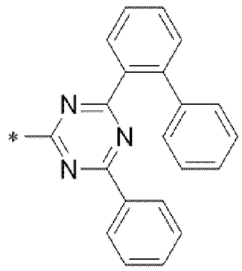 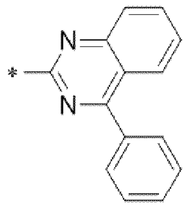 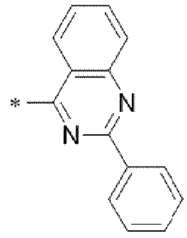

A23 A24 A25

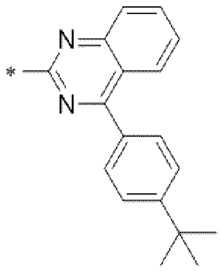 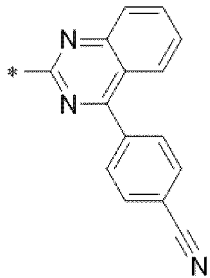 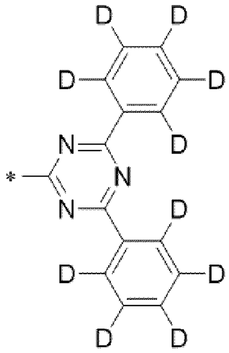 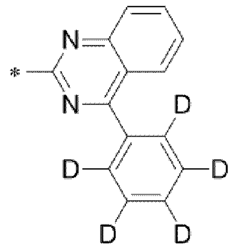

A26 A27 A28 A29

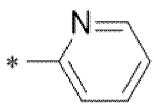 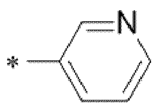 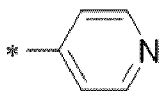 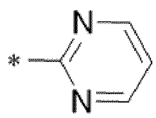 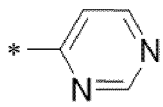

A30 A31 A32 A33 A34

$*-CF_3$ $*-C_2F_5$ 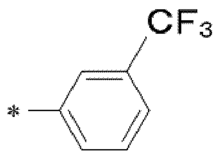 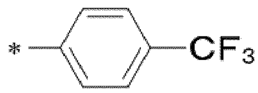

A35 A36 A37 A38

$*-F$ $*-Cl$ $*-Br$ $*-I$

A39 A40 A41 A42

When $X^1$ is a nitrogen atom, $R^7$ and $R^8$ are bonded via a nitrogen atom to form a 6-membered ring, $R^{21}$ and $R^{22}$ are bonded via a nitrogen atom to form a 6-membered ring, and $R^{17}$ and $R^{18}$ are bonded to each other to form a single bond, at least one of $R^1$ to $R^6$ is a substituted or unsubstituted aryl group, or in any of $R^1$ and $R^2$, $R^2$ and $R^3$, $R^3$ and $R^4$, $R^4$ and $R^5$, and $R^5$ and $R^6$, an aromatic ring (a substituted or unsubstituted benzene ring which may be condensed) or a heteroaromatic ring (preferably a substituted or unsubstituted furan ring of benzofuran which may be condensed, or a substituted or unsubstituted thiophene ring of benzothiophene which may be condensed) is formed through bonding.

Further, when $X^1$ is a boron atom, $X^2$ is a nitrogen atom, and $R^7$ and $R^8$, and $R^{17}$ and $R^{18}$ are bonded to each other to form boron atom-containing ring structures, the ring structure is a 5 to 7-membered ring. In the case of a 6-membered ring, $R^7$ and $R^8$, and $R^{17}$ and $R^{18}$ are bonded to each other to form -B($R^{32}$)-, -CO-, -CS- or -N($R^{27}$)-. $R^{27}$ preferably represents a hydrogen atom, a deuterium atom, or a substituent.

When $X^1$ of the formula (1) is a nitrogen atom, the compound of the invention has the following skeleton (1a). When $X^2$ of the formula (1) is a nitrogen atom, the compound of the invention has the following skeleton (1b).

Skeleton (1a)

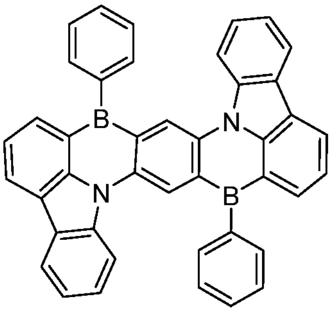

Skeleton (1b)

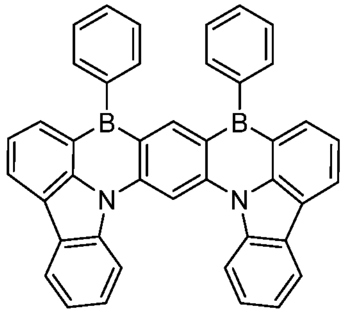

In the skeletons (1a) and (1b), each hydrogen atom may be substituted with a deuterium atom or a substituent. Further, it may be substituted with a linking group together with an adjacent hydrogen atom to form a ring structure. For details, corresponding descriptions on $R^1$ to $R^{26}$, $A^1$, and $A^2$ in the formula (1) may be referred to. Compounds, in which all phenyl groups bonded to boron atoms in the skeletons (1a) and (1b) are substituted with mesityl groups, 2,6-diisopropyl phenyl groups or 2,4,6-triisopropyl phenyl groups, may be exemplified. In one aspect of the invention, each hydrogen atom in the skeletons (1a) and (1b) is not substituted with a linking group together with an adjacent hydrogen atom to form a ring structure.

As one preferable group of compounds having the skeleton (1a), compounds represented by the following formula (1a) may be exemplified.

Formula (1a)

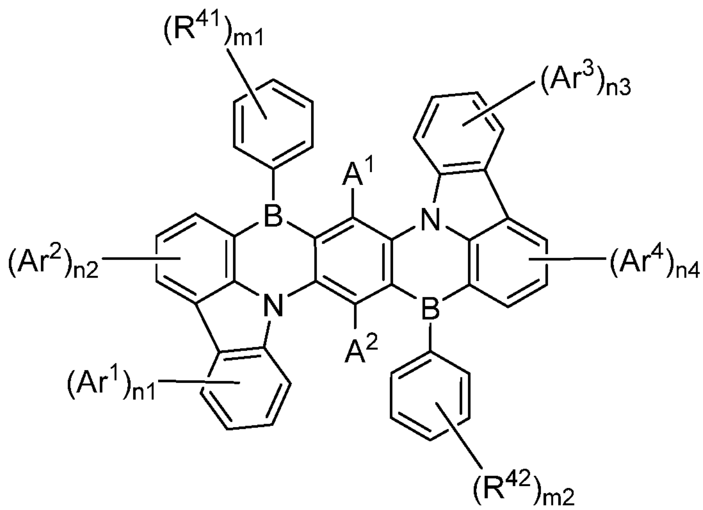

In the formula (1a), each of $Ar^1$ to $Ar^4$ independently represents a substituted or unsubstituted aryl group, a substituted or unsubstituted heteroaryl group, or a substituted or unsubstituted alkyl group, and, for example, a substituted or unsubstituted aryl group may be preferably selected. Each of $R^{41}$ and $R^{42}$ independently represents a substituted or unsubstituted alkyl group. Each of m1 and m2 independently represents an integer of 0 to 5, each of n1 and n3 independently represents an integer of 0 to 4, and each of n2 and n4 independently represents an integer of 0 to 3. Each of $A^1$ and $A^2$ independently represents a hydrogen atom, a deuterium atom, or a substituent. It is preferable that at least one of n1 to n4 is 1 or more, and each of m1 and m2 is independently any integer of 1 to 5.

In one aspect of the invention, each of n1 to n4 independently represents an integer of 0 to 2. In a preferred aspect of the invention, at least one of n1 to n4 is 1 or more. Preferably, at least one of n1 and n2 is 1 or more, and at least one of n3 and n4 is 1 or more. In one aspect of the invention, each of n1 and n3 is independently 1 or 2, and n2 and n4 are 0. In one aspect of the invention, each of n2 and n4 is independently 1 or 2, and n1 and n3 are 0. In one aspect of the invention, each of n1 to n4 is independently 1 or 2. In one aspect of the invention, n1 and n3 are the same, and n2 and n4 are the same. In one aspect of the invention, n1 and n3 are 1, and n2 and n4 are 0. In one aspect of the invention, n1 and n3 are 0, and n2 and n4 are 1. In one aspect of the invention, n1 to n4 are all 1. The bond positions of $Ar^1$ to $Ar^4$ may be at least one of 3 and 6 positions in a carbazole ring, may be at least one of 2 and 7 positions, may

be at least one of 1 and 8 positions, or may be at least one of 4 and 5 positions. The bond positions of $Ar^1$ to $Ar^4$ may be both of 3 and 6 positions in the carbazole ring, may be both of 2 and 7 positions, may be both of 1 and 8 positions, or may be both of 4 and 5 positions. For example, at least one of 3 and 6 positions may be preferably selected, or both of 3 and 6 positions may be further preferably selected. In a preferred aspect of the invention, $Ar^1$ to $Ar^4$ are all the same group. In a preferred aspect of the invention, each of $Ar^1$ to $Ar^4$ is independently a substituted or unsubstituted aryl group, more preferably a substituted or unsubstituted phenyl group or naphthyl group, further preferably a substituted or unsubstituted phenyl group. As the substituent, a group selected from any of substituent groups Ato E to be described below may be mentioned, but an unsubstituted phenyl group is also preferable. Specific preferable examples of $Ar^1$ to $Ar^4$ include a phenyl group, an o-biphenyl group, a m-biphenyl group, a p-biphenyl group, and a terphenyl group.

In one aspect of the invention, each of m1 and m2 is independently 0. In one aspect of the invention, each of m1 and m2 is independently any integer of 1 to 5. In one aspect of the invention, m1 and m2 are the same. In one aspect of the invention, $R^{41}$ and $R^{42}$ are alkyl groups having 1 to 6 carbon atoms and may be selected from, for example, alkyl groups having 1 to 3 carbon atoms, or a methyl group may be selected. When a carbon atom bonded to a boron atom is the 1-position, as the substitution position of the alkyl group, only the 2-position, only the 3-position, only the 4-position, the 3 and 5 positions, the 2 and 4 positions, the 2 and 6 positions, the 2, 4, and 6 positions, and the like may be exemplified. At least the 2-position is preferable, and at least 2 and 6 positions are more preferable.

For descriptions and preferable ranges of $A^1$ and $A^2$, corresponding descriptions on the formula (1) may be referred to.

Hereinafter, specific examples of the compound represented by the formula (1a) will be given. Compounds of the formula (1a) that may be used in the invention are not construed as limiting to specific examples in the following one group. For example, as one preferable group, a group including all the following compounds, except for the compound at the center in the fourth row and the compound at the center in the eighth row, may be mentioned.

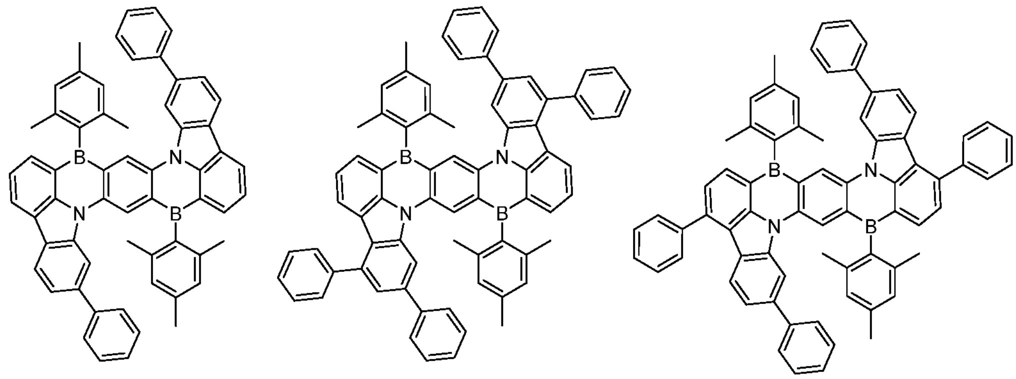

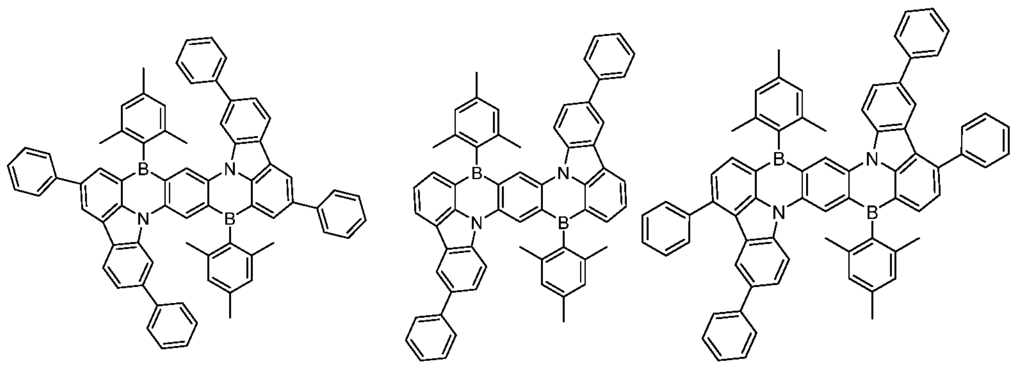

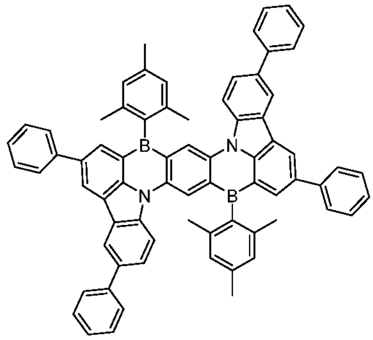
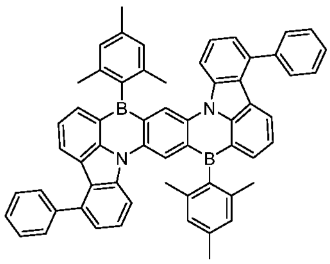
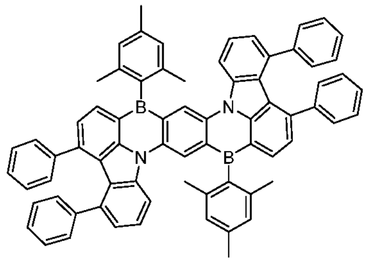

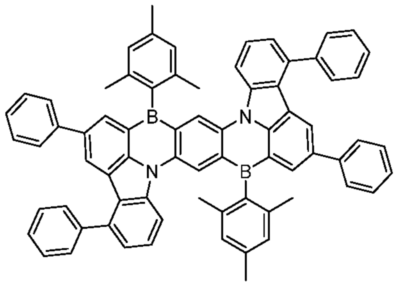
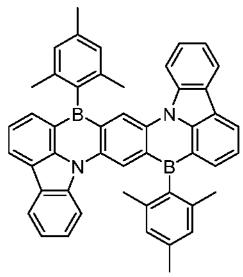
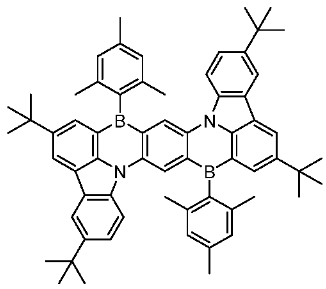

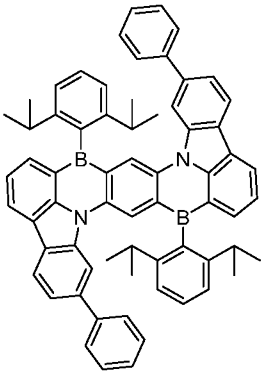
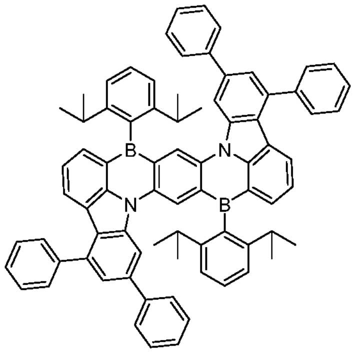
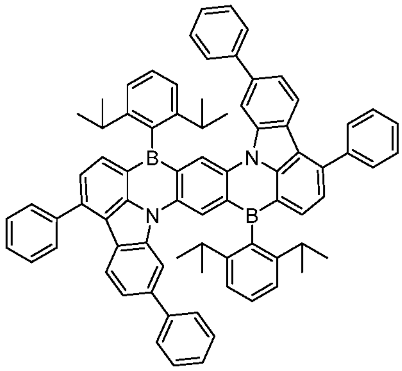

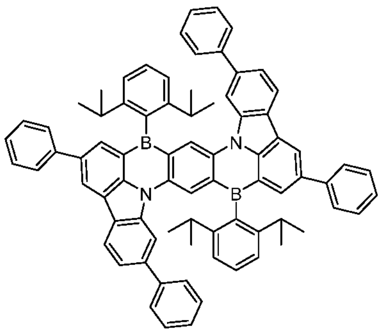
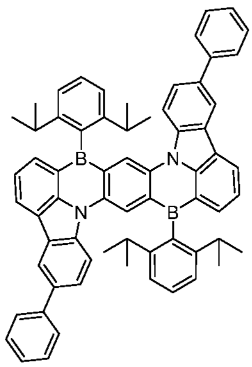
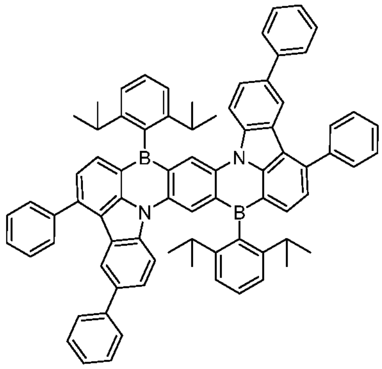

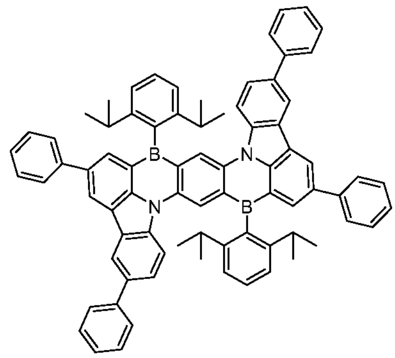
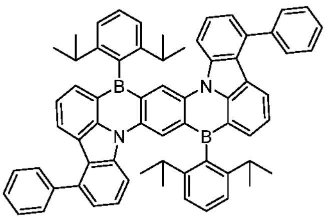
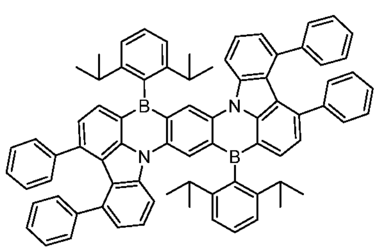

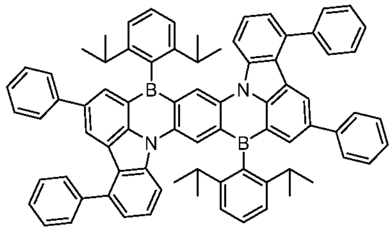
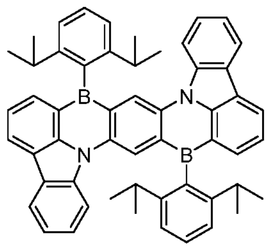
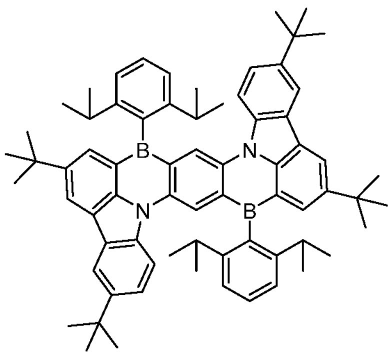

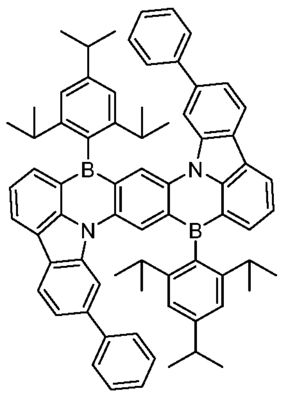
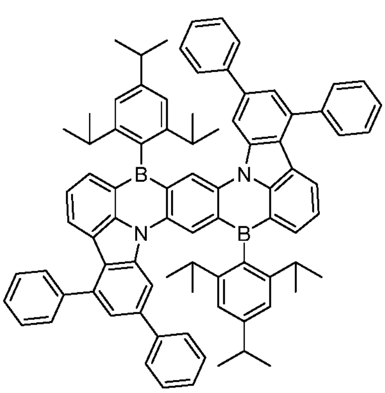
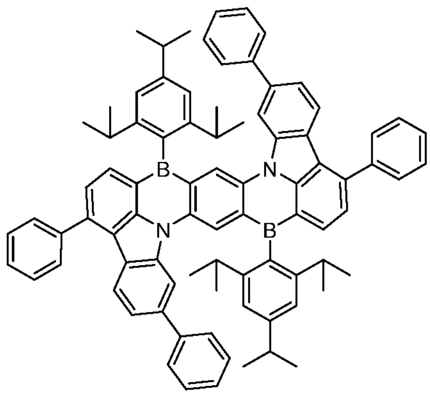

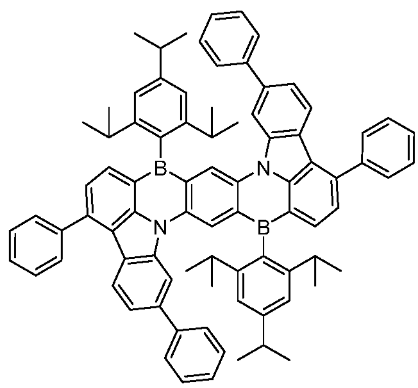
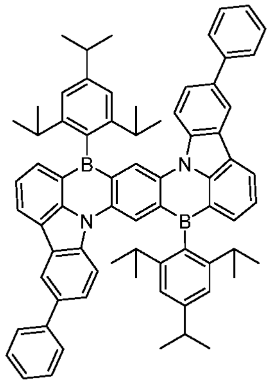
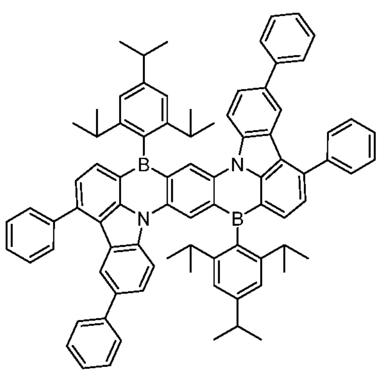

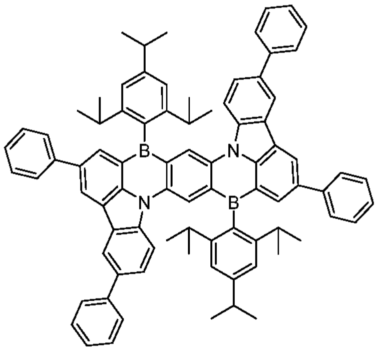

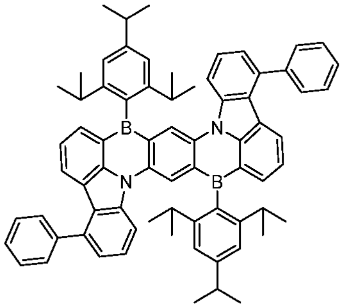

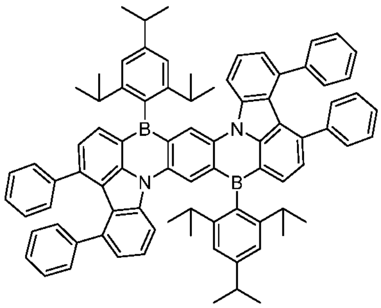

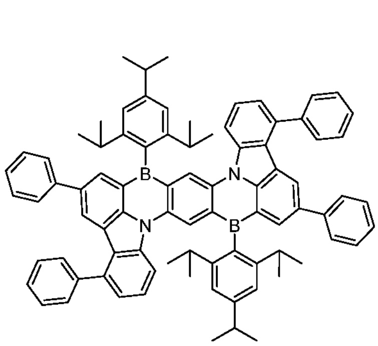

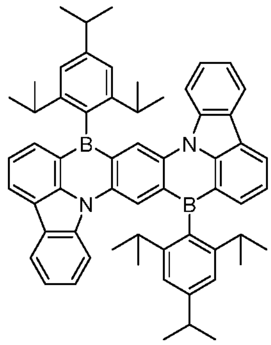

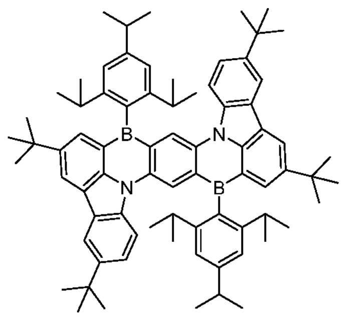

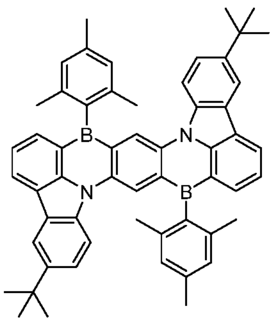

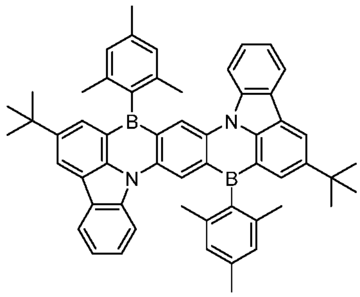

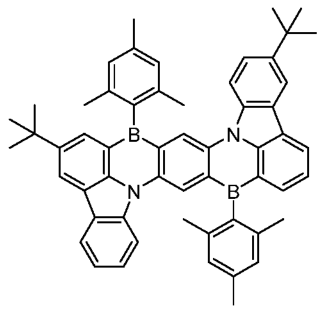

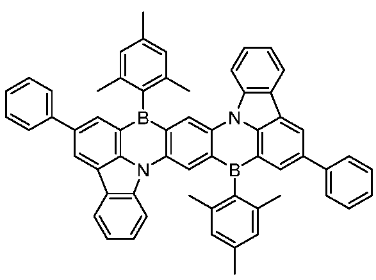

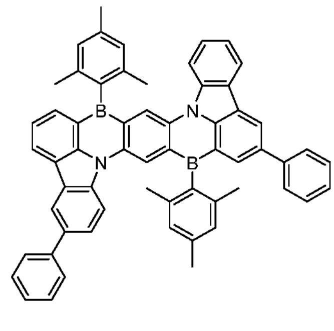

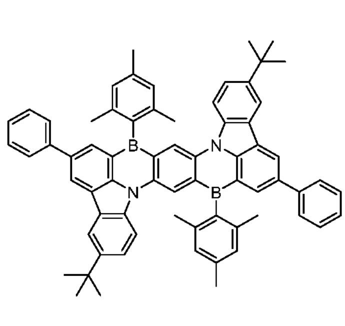

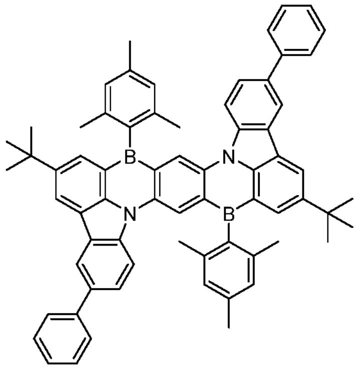

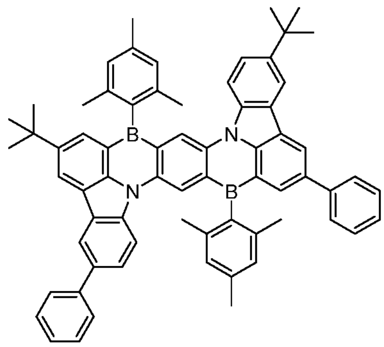

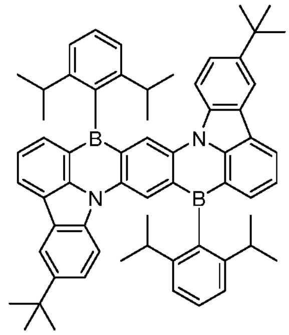

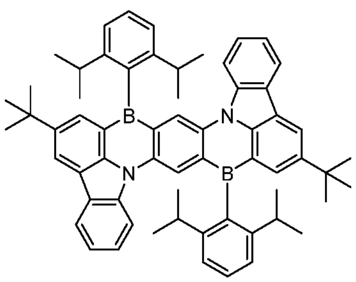

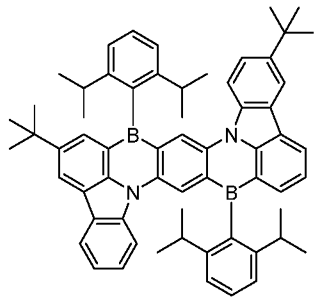

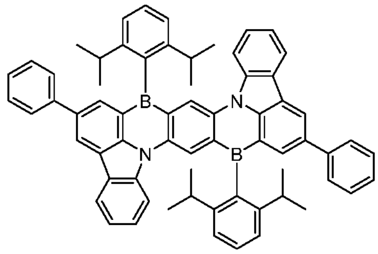

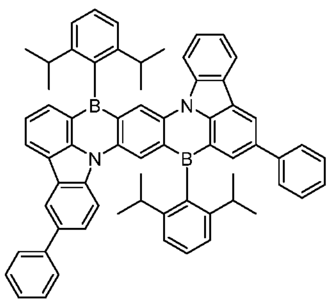

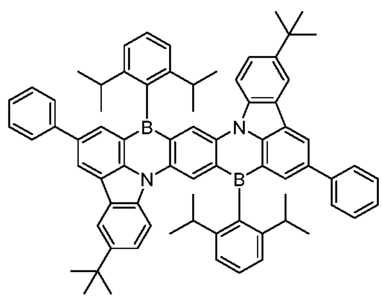

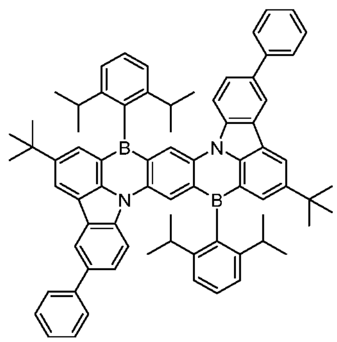

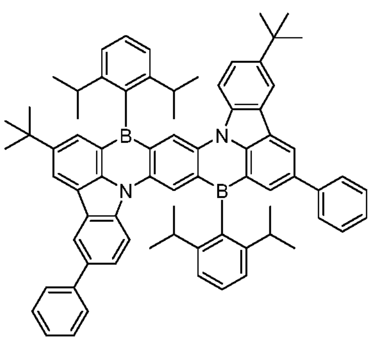

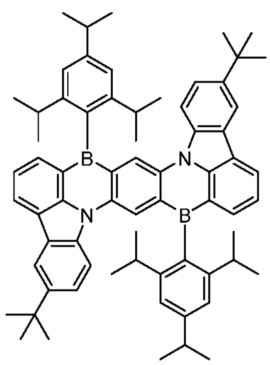

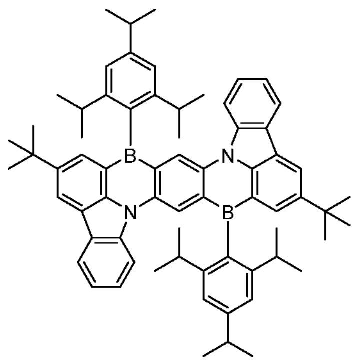

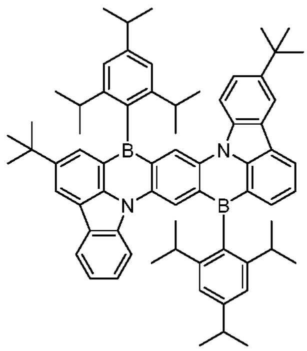
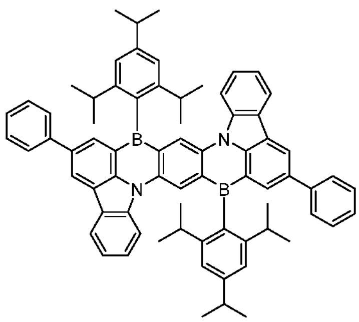
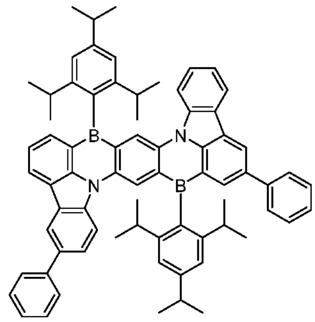

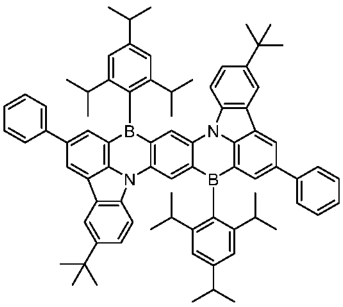
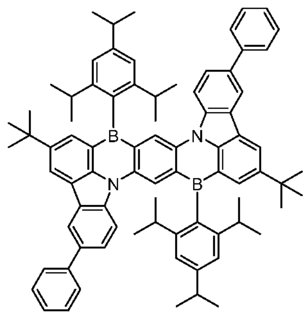
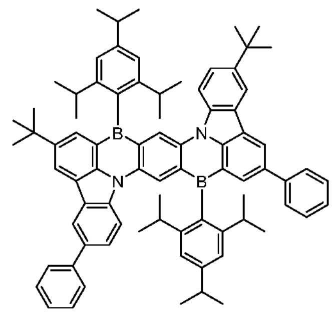

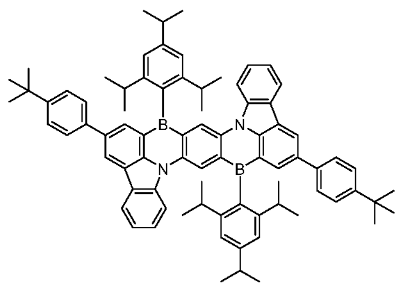
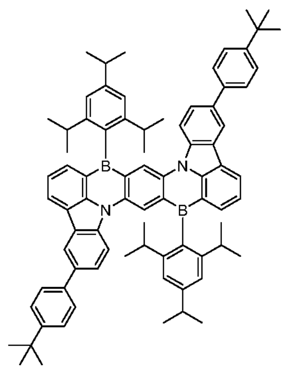
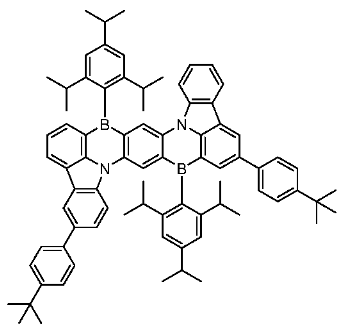

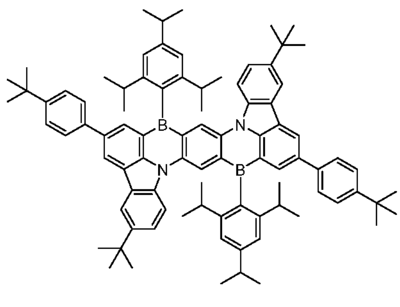
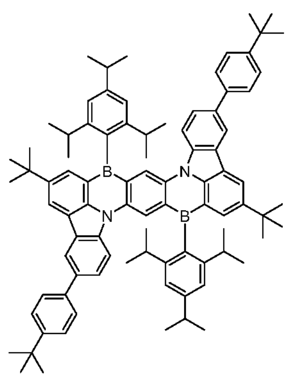
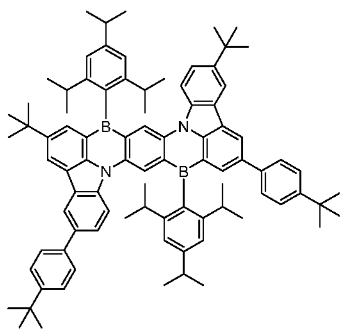

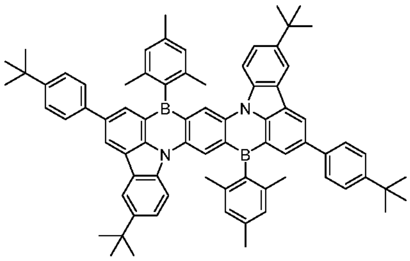
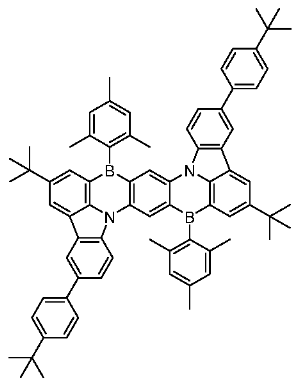
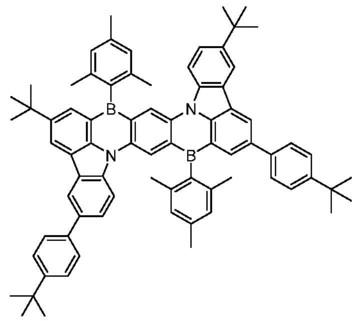

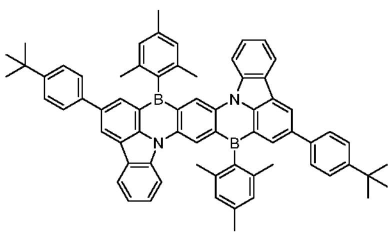
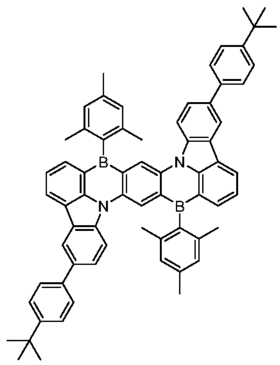
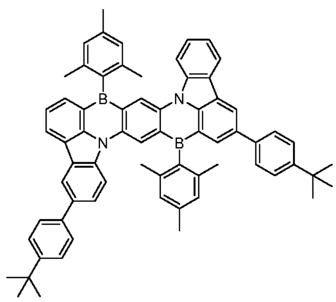

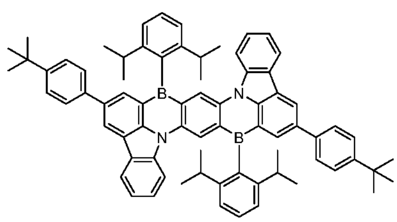
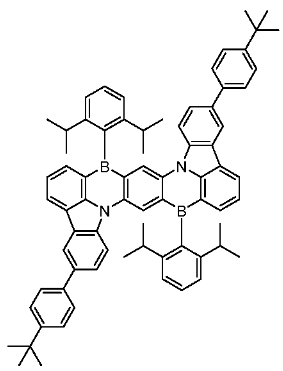
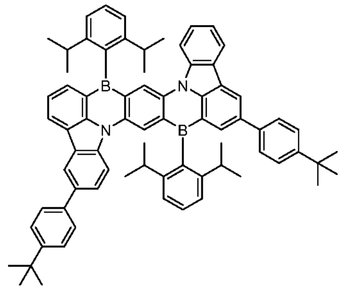

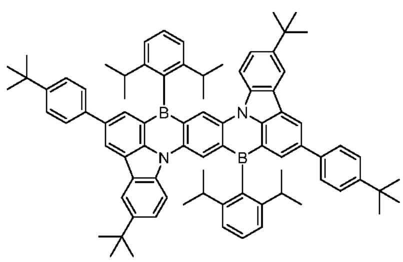
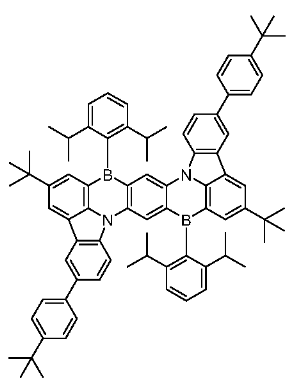
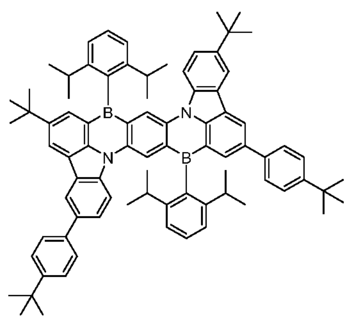

Hereinafter, another group of specific examples of the compound represented by the formula (1a) will be given. Compounds of the formula (1a) that may be used in the invention are not construed as limiting to specific examples in the following one group.

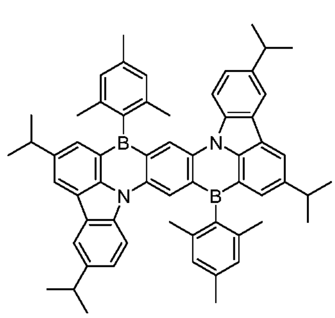

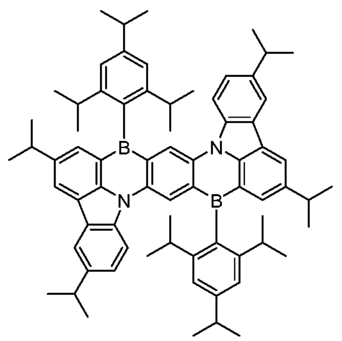

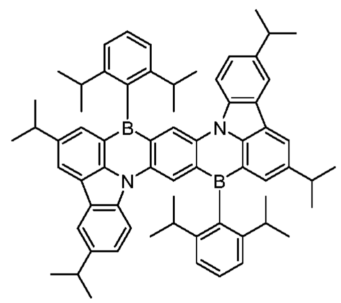

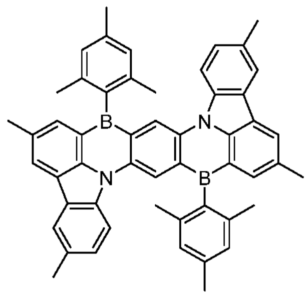

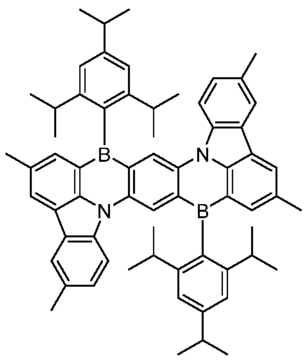

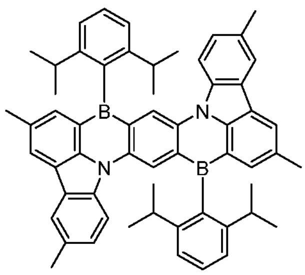

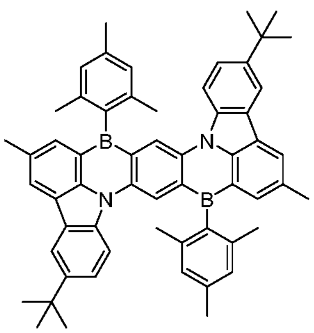

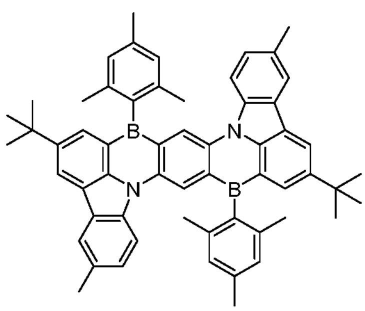

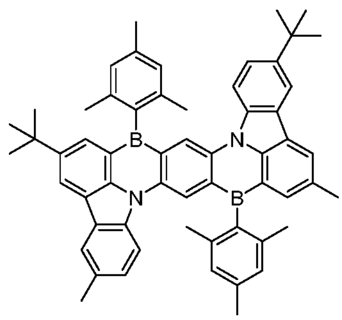

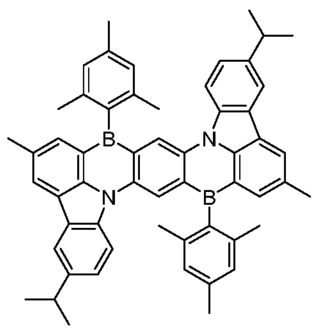

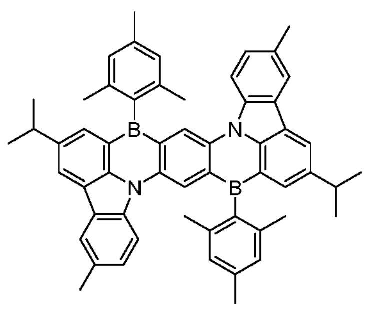

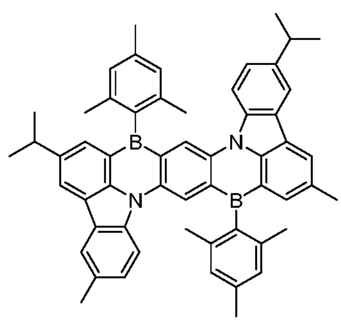

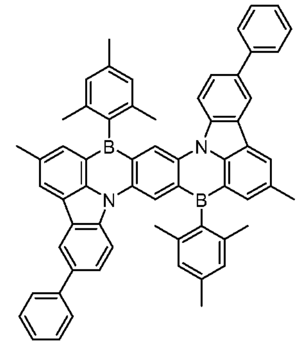
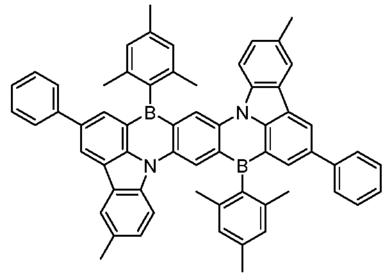
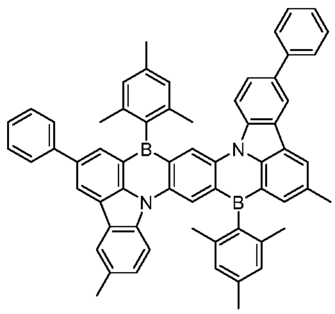

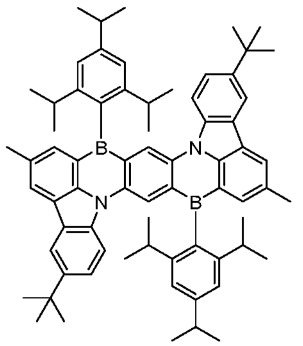
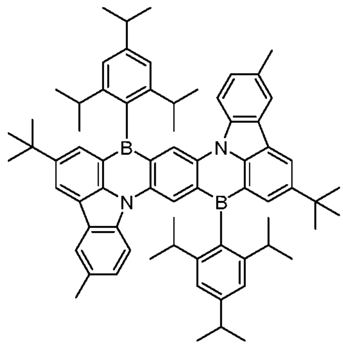
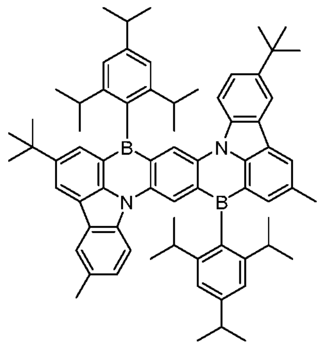

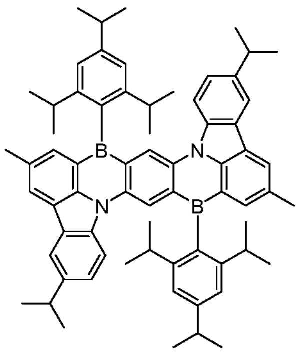
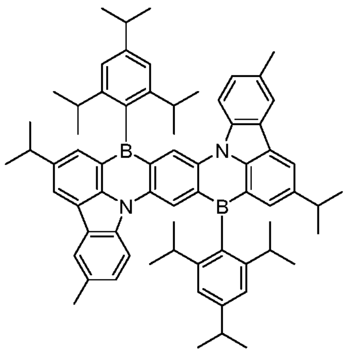
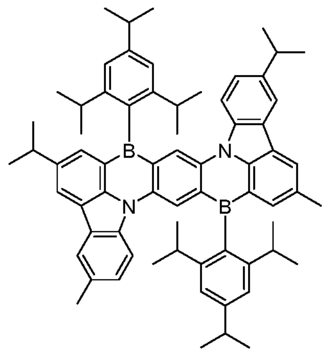

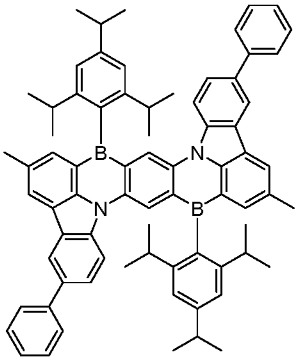
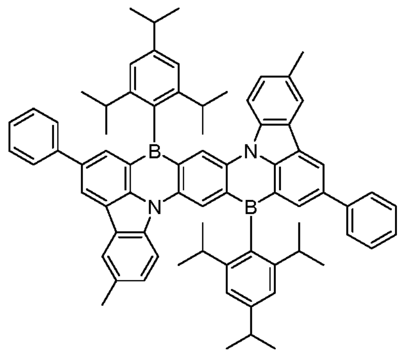
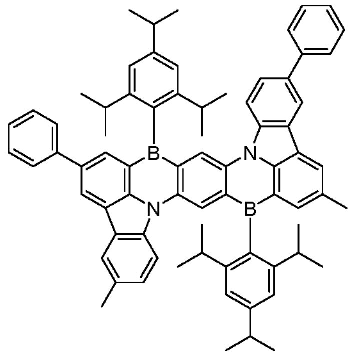

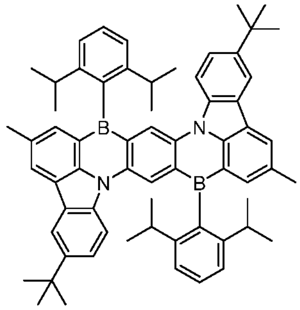
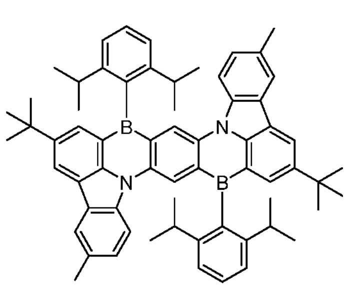
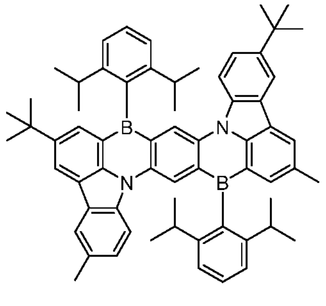

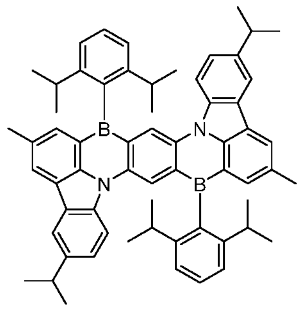
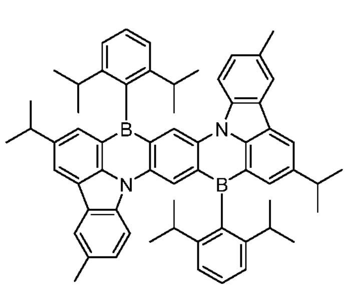
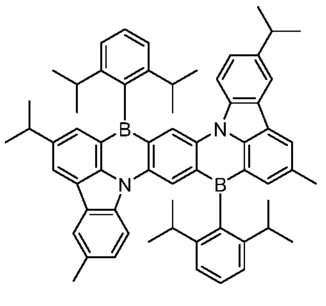

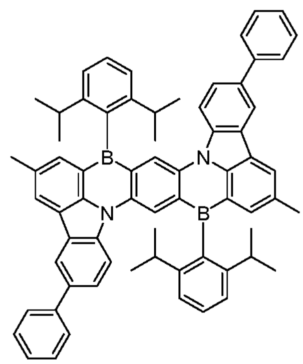
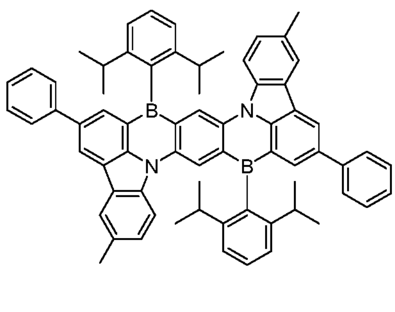
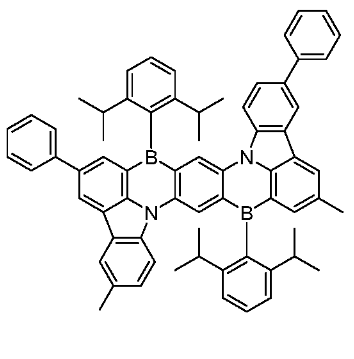

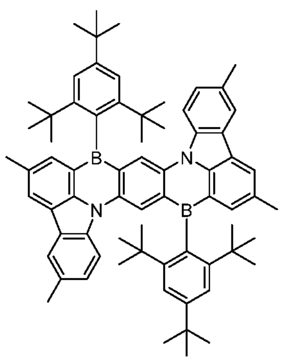
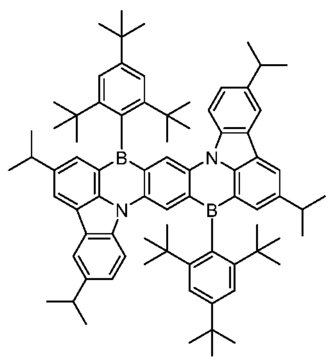
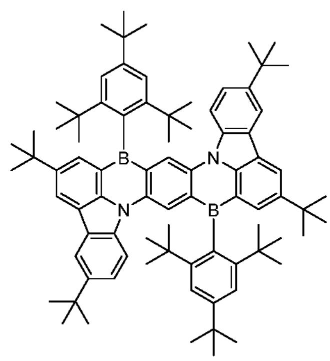

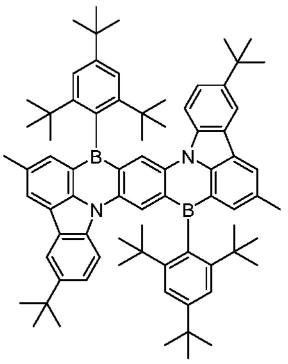
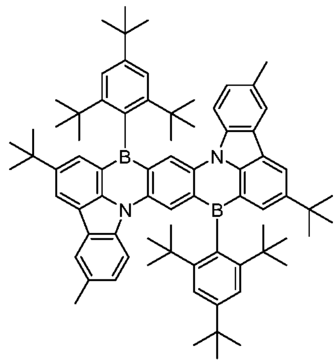
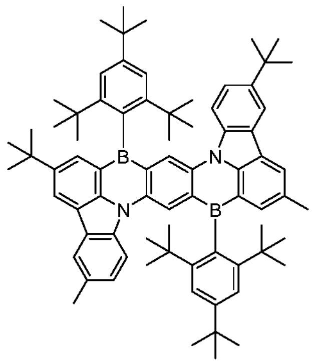

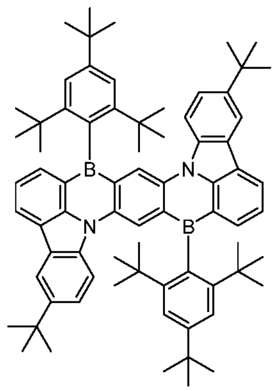
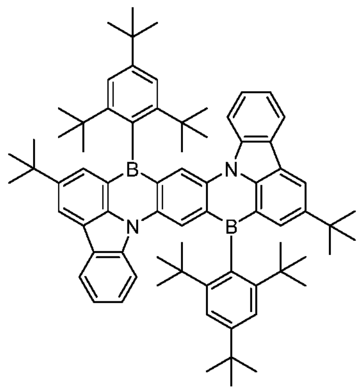
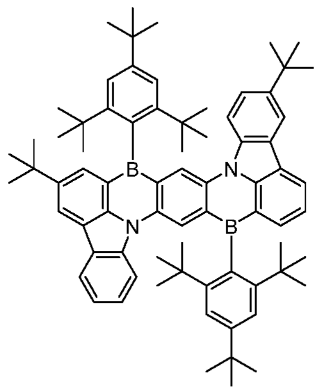

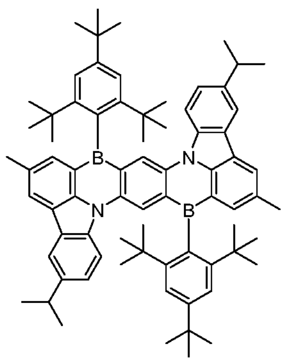
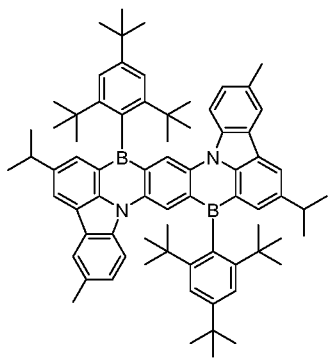
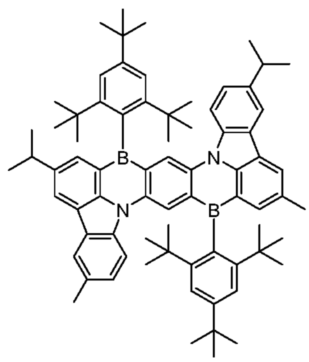

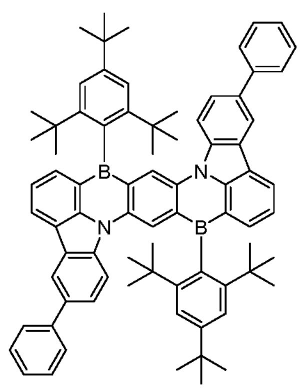
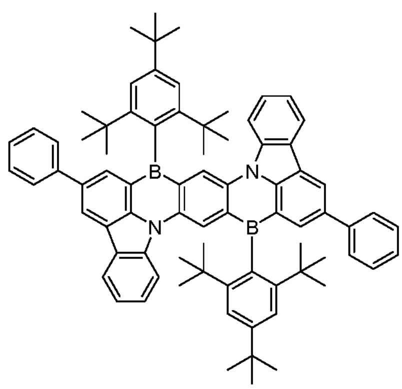
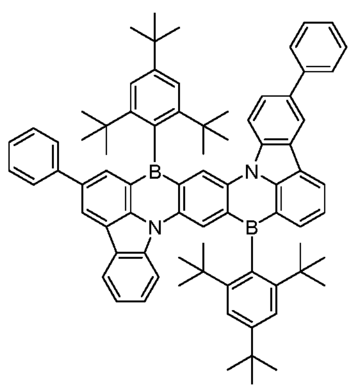

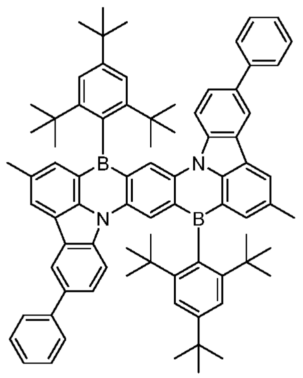
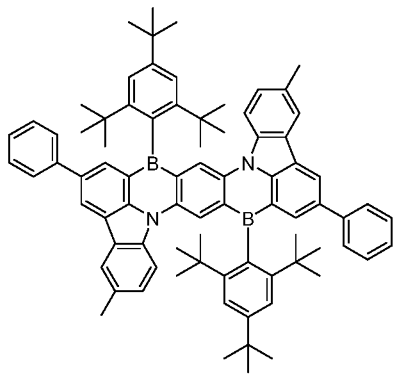
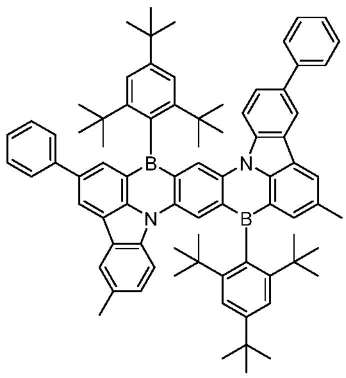

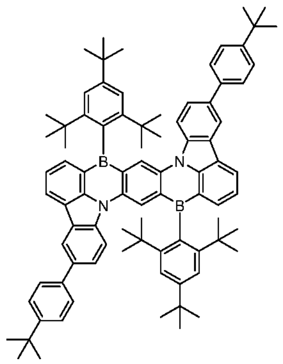
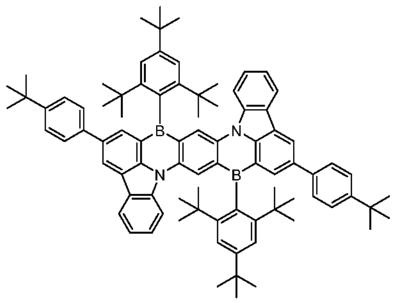
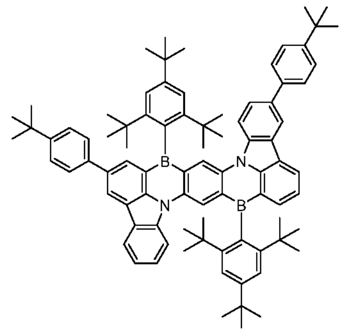

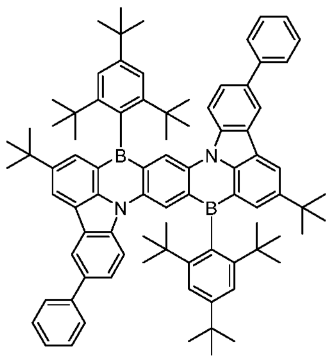
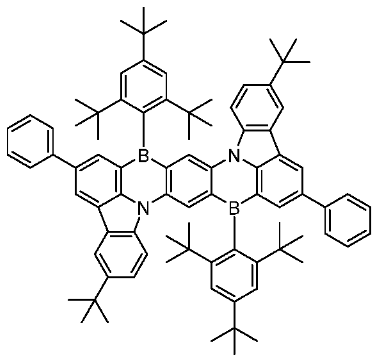
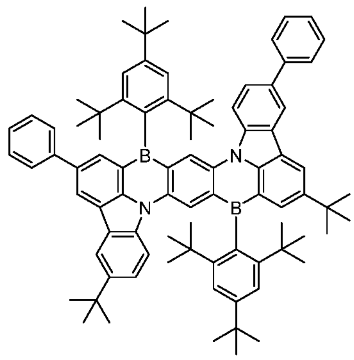

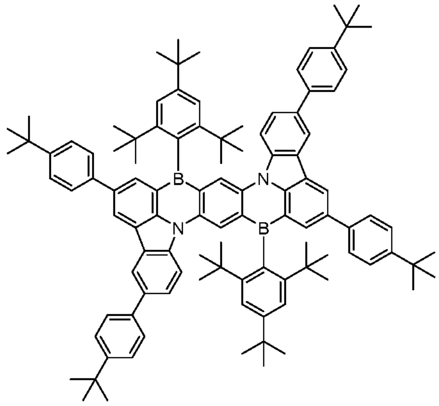
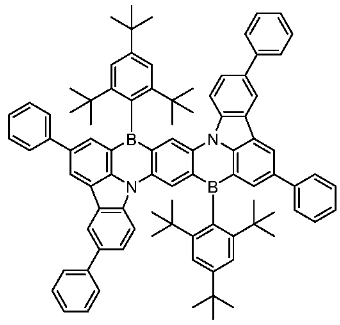
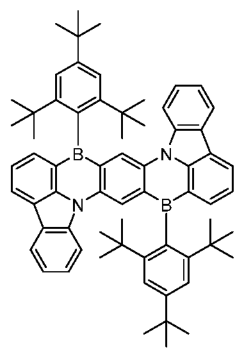

As one preferable group of compounds having the skeleton (1b), compounds represented by the following formula (1b) may be exemplified.

Formula (1b)

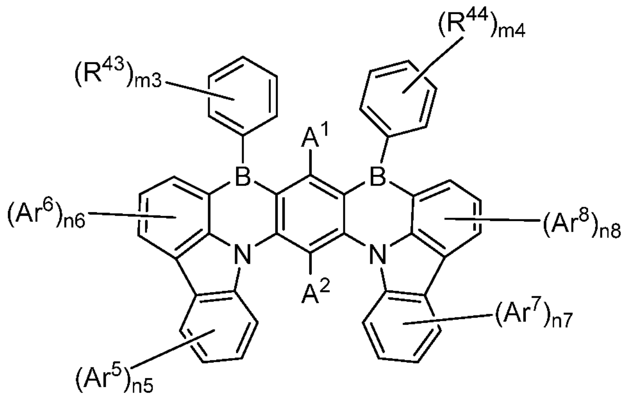

In the formula (1b), each of $Ar^5$ to $Ar^8$ independently represents a substituted or unsubstituted aryl group, a substituted or unsubstituted heteroaryl group, or a substituted or unsubstituted alkyl group, and, for example, a substituted or unsubstituted aryl group may be preferably selected. Each of $R^{43}$ and $R^{44}$ independently represents a substituted or unsubstituted alkyl group. Each of m3 and m4 independently represents an integer of 0 to 5, each of n6 and n8 independently represents an integer of 0 to 3, and each of n5 and n7 independently represents an integer of 0 to 4. Each of $A^1$ and $A^2$ independently represents a hydrogen atom, a deuterium atom, or a substituent. In relation to details of $Ar^5$ to $Ar^8$, $R^{43}$ and $R^{44}$, m3 and m4, n5 to n8, $A^1$, and $A^2$, the descriptions on $Ar^1$ to $Ar^4$, $R^{41}$ and $R^{42}$, m1 and m2, n1 to n4, $A^1$, and $A^2$ in the formula (1a) may be referred to. It is preferable that at least one of n5 to n8 is 1 or more, and each of m3 and m4 is independently any integer of 1 to 5.

Hereinafter, specific examples of the compound represented by the formula (1b) will be given. Compounds of the formula (1b) that may be used in the invention are not construed as limiting to the following specific examples.

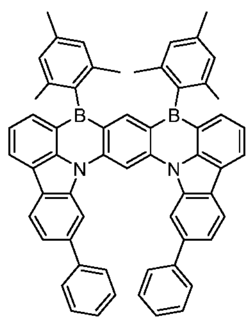

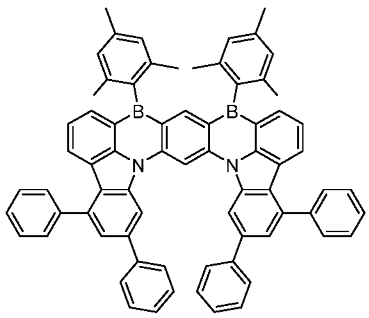

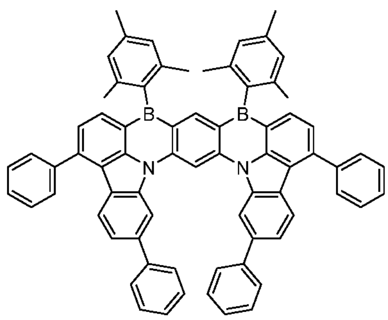

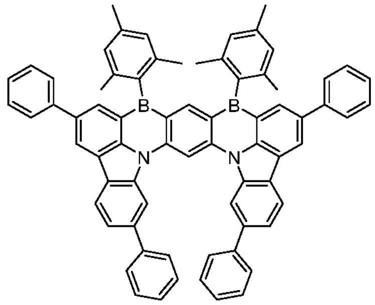

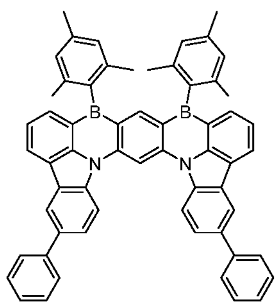

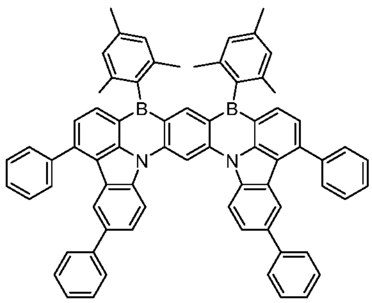

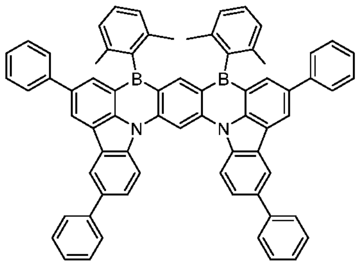
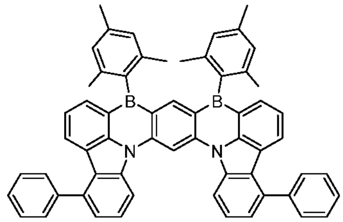
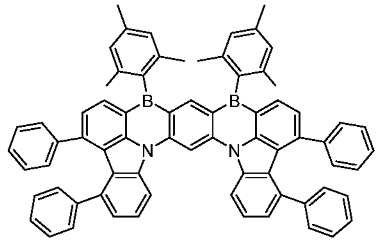

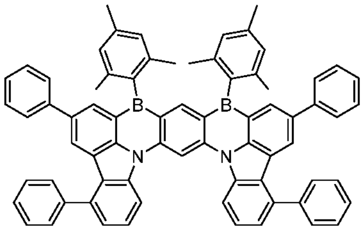
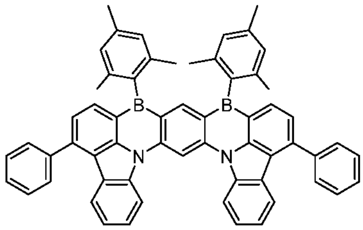
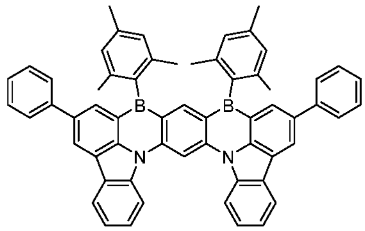

When $R^7$ and $R^8$ in the formula (1) are bonded to each other to form N-Ph, the compound of the invention has, for example, the following skeleton (2a) if $X^1$ is a nitrogen atom, and, has for example, the following skeleton (2b) if $X^2$ is a nitrogen atom. Ph is a phenyl group.

Skeleton (2a)

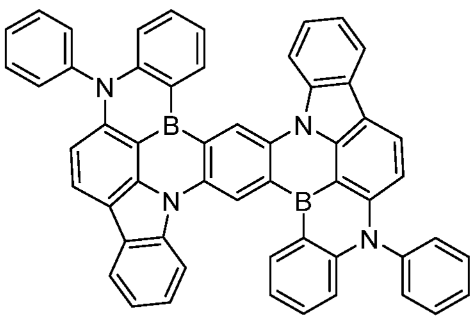

Skeleton (2b)

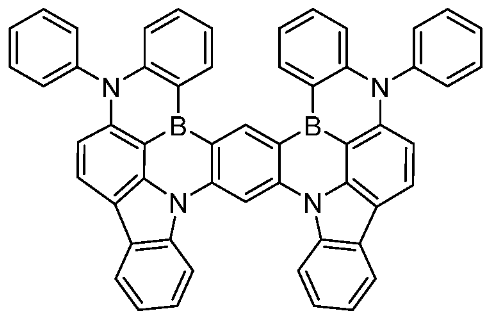

In the skeletons (2a) and (2b), each hydrogen atom may be substituted with a deuterium atom or a substituent. Further, it may be substituted with a linking group together with an adjacent hydrogen atom to form a ring structure. For details, corresponding descriptions on $R^1$ to $R^{26}$, $A^1$, and $A^2$ in the formula (1) may be referred to. At least one hydrogen atom of a benzene ring forming a carbazole partial structure included in the skeleton (2a) is substituted with a substituted or unsubstituted aryl group. In one aspect of the invention, each hydrogen atom in the skeletons (2a) and (2b) is not substituted with a linking group together with an adjacent hydrogen atom to form a ring structure.

As one preferable group of compounds having the skeleton (2a), compounds represented by the following formula (2a) may be exemplified.

Formula (2a)

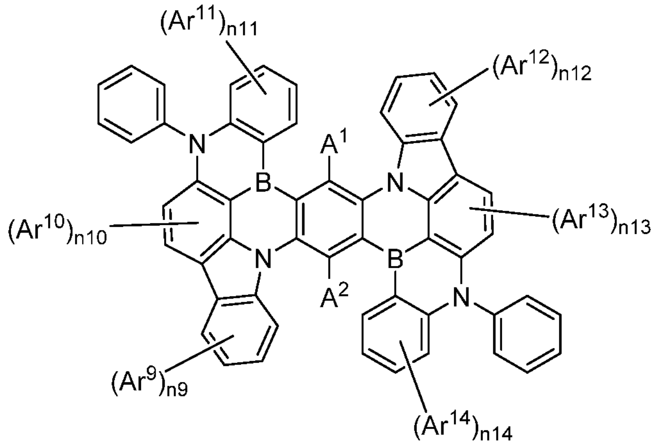

In the formula (2a), each of $Ar^9$ to $Ar^{14}$ independently represents a substituted or unsubstituted aryl group, a substituted or unsubstituted heteroaryl group, or a substituted or unsubstituted alkyl group, and for example, a substituted or unsubstituted aryl group may be preferably selected. Each of n9, n11, n12, and n14 independently represents an integer of 0 to 4, and each of n10 and n13 independently represents an integer of 0 to 2. Meanwhile, at least one of n9, n10, n12, and n13 is 1 or more. Each of $A^1$, and $A^2$ independently represents a hydrogen atom, a deuterium atom, or a substituent.

In one aspect of the invention, each of n9 to n14 independently represents an integer of 0 to 2. In one aspect of the invention, at least one of n9 to n14 is 1 or more. For example, n9 and n12 may be 1 or more or n10 and n13 may be 1 or more. In a preferred aspect of the invention, at least one of n9, n10, n12, and n13 is 1 or more. In one aspect of the invention, each of n9 and n12 is independently 1 or 2, and n10, n11, n13, and n14 are 0. In one aspect of the invention, each of n10 and n13 is independently 1 or 2, and n9, n11, n12, and n14 are 0. In one aspect of the invention, each of n9 and n12 is independently 1 or 2, each of n10 and n13 is independently 1 or 2, and n11 and n14 are 0. In one aspect of the invention, n9 to n14 are all 1. The bond positions of $Ar^9$ to $Ar^{14}$ may be 3 and 6 positions of a carbazole ring, or may be other positions. In a preferred aspect of the invention, $Ar^9$ to $Ar^{14}$ are all the same group. For preferable groups for $Ar^9$ to $Ar^{14}$, corresponding descriptions on $Ar^1$ to $Ar^4$ may be referred to. For descriptions and preferable ranges of $A^1$ and $A^2$, corresponding descriptions on the formula (1) may be referred to.

Hereinafter, specific examples of the compound represented by the formula (2a) will be given. Compounds of the formula (2a) that may be used in the invention are not construed as limiting to the following specific examples.

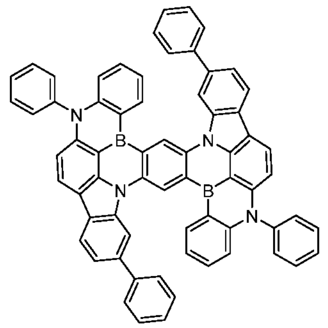
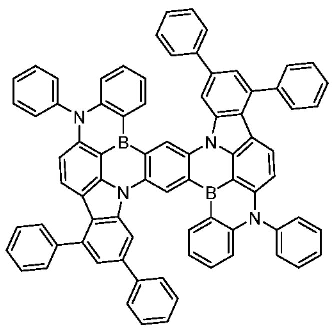
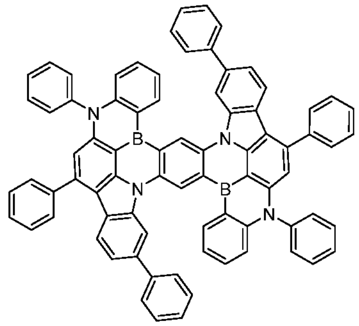

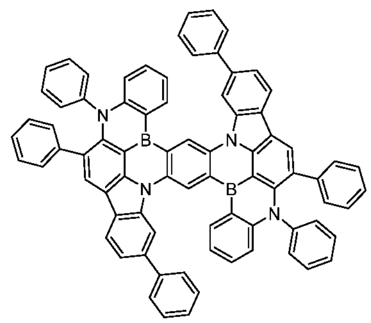
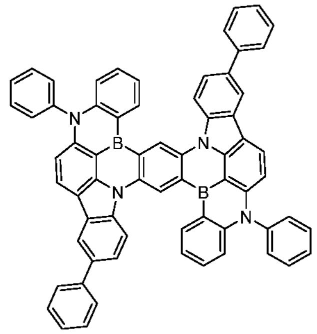
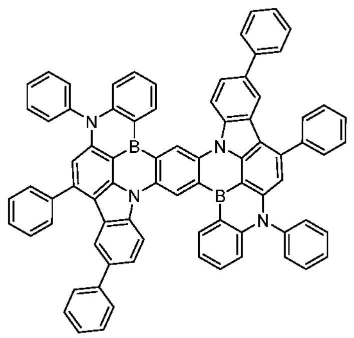

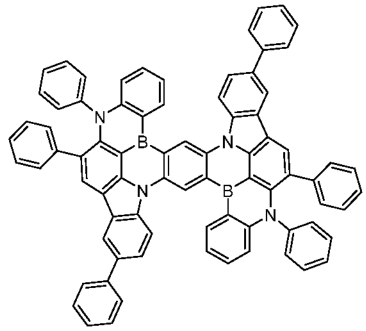
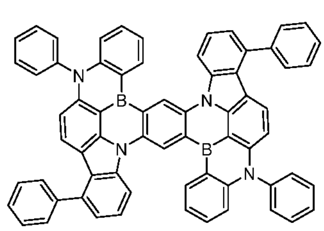
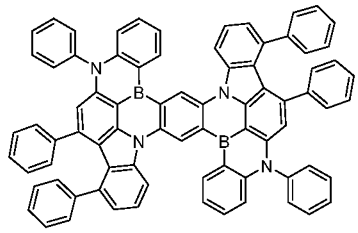

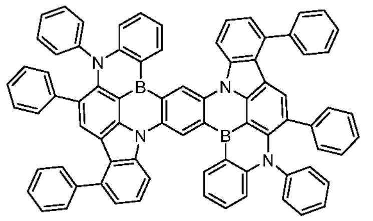
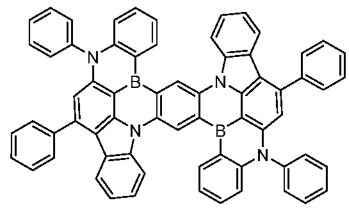
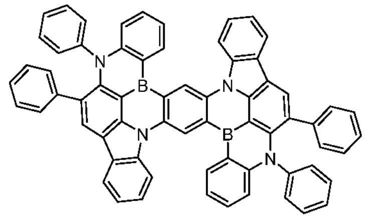

As one preferable group of compounds having the skeleton (2b), compounds represented by the following formula (2b) may be exemplified.

Formula (2b)

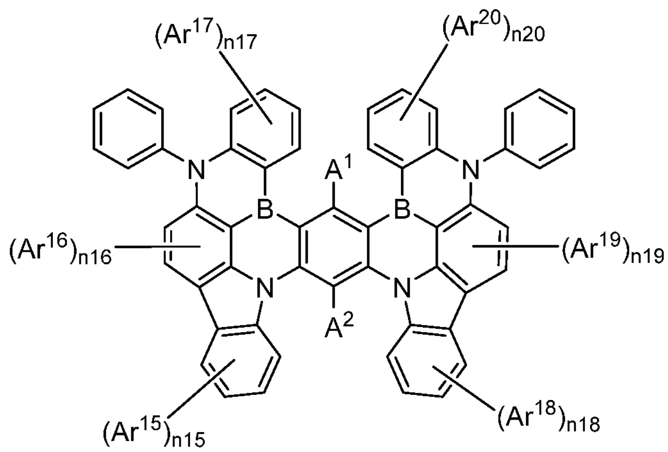

In the formula (2b), each of $Ar^{15}$ to $Ar^{20}$ independently represents a substituted or unsubstituted aryl group, a substituted or unsubstituted heteroaryl group, or a substituted or unsubstituted alkyl group, and, for example, a substituted or unsubstituted aryl group may be preferably selected. Each of n15, n17, n18, and n20 independently represents an integer of 0 to 4, and each of n16 and n19 independently represents an integer of 0 to 2. Each of $A^1$, and $A^2$ independently represents a hydrogen atom, a deuterium atom, or a substituent. For details of $Ar^{15}$ to $Ar^{20}$, n15 to n20, $A^1$, and $A^2$,

descriptions on $Ar^9$ to $Ar^{14}$, n9 to n14, $A^1$, and $A^2$ in the formula (2a) may be referred to in this order.

Hereinafter, specific examples of the compound represented by the formula (2b) will be given. Compounds of the formula (2b) that may be used in the invention are not construed as limiting to the following specific examples.

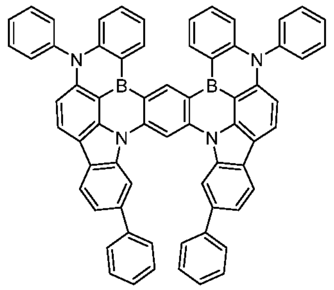

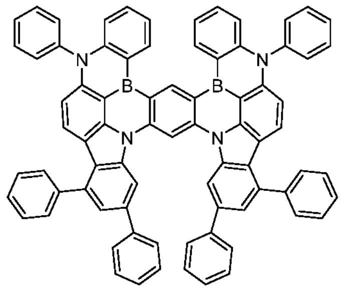

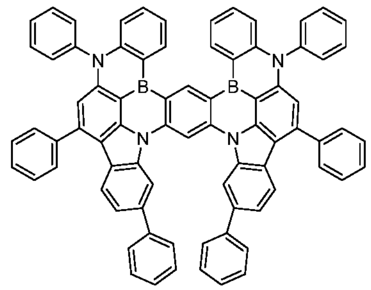

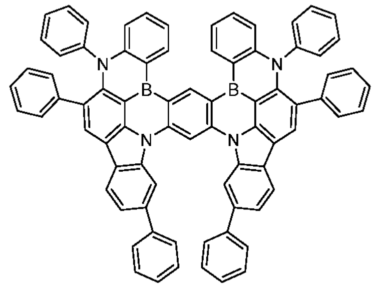

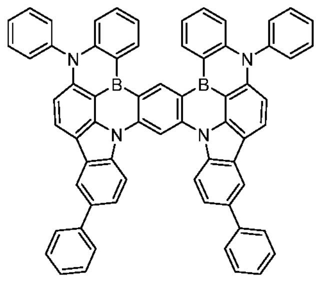

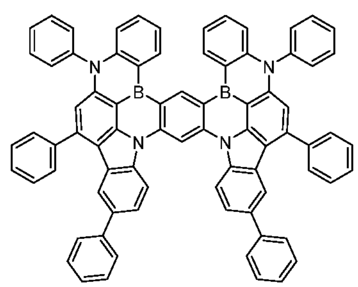

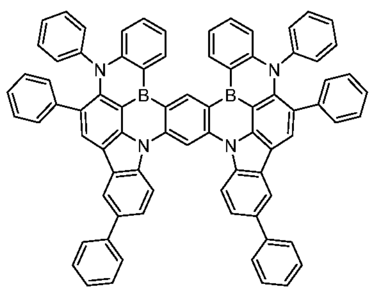

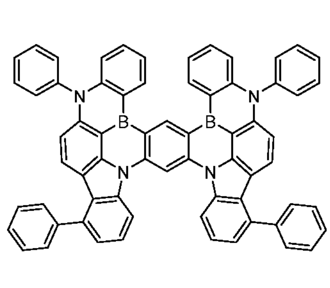

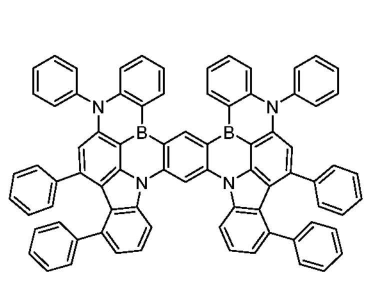

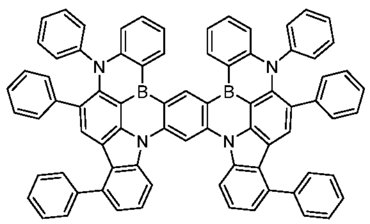

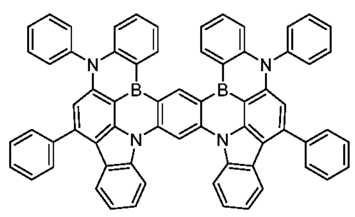

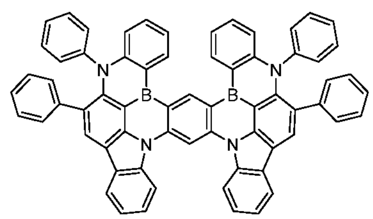

When R' and $R^8$ in the formula (1) are bonded to each other to form a single bond, the compound of the invention has, for example, the following skeleton (3a) if $X^1$ is a nitrogen atom, and has, for example, the following skeleton (3b) if $X^2$ is a nitrogen atom.

Skeleton (3a)

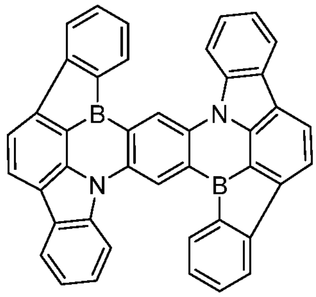

Skeleton (3b)

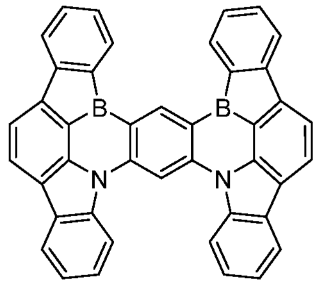

In the skeletons (3a) and (3b), each hydrogen atom may be substituted with a deuterium atom or a substituent. Further, it may be substituted with a linking group together with an adjacent hydrogen atom to form a ring structure. For details, corresponding descriptions on $R^1$ to $R^{26}$, $A^1$, and $A^2$ in the formula (1) may be referred to. In one aspect of the invention, each hydrogen atom in the skeletons (3a) and (3b) is not substituted with a linking group together with an adjacent hydrogen atom to form a ring structure.

As one preferable group of compounds having the skeleton (3a), compounds represented by the following formula (3a) may be exemplified.

Formula (3a)

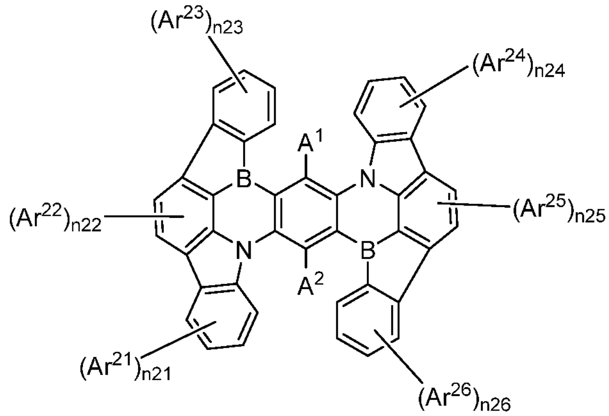

In the formula (3a), each of $Ar^{21}$ to $Ar^{26}$ independently represents a substituted or unsubstituted aryl group, a substituted or unsubstituted heteroaryl group, or a substituted or unsubstituted alkyl group, and for example, a substituted or unsubstituted aryl group may be preferably selected. Each of n21, n23, n24, and n26 independently represents an integer of 0 to 4, and each of n22 and n25 independently represents an integer of 0 to 2. Each of $A^1$ and $A^2$ independently represents a hydrogen atom, a deuterium atom, or a substituent. For details of $Ar^{21}$ to $Ar^{25}$, and n21 to n25, descriptions on $Ar^9$ to $Ar^{14}$, n9 to n14, $A^1$, and $A^2$ in the formula (2a) may be referred to.

Hereinafter, specific examples of the compound represented by the formula (3a) will be given. Compounds of the formula (3a) that may be used in the invention are not construed as limiting to the following specific examples.

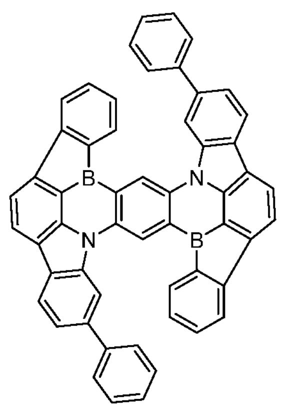
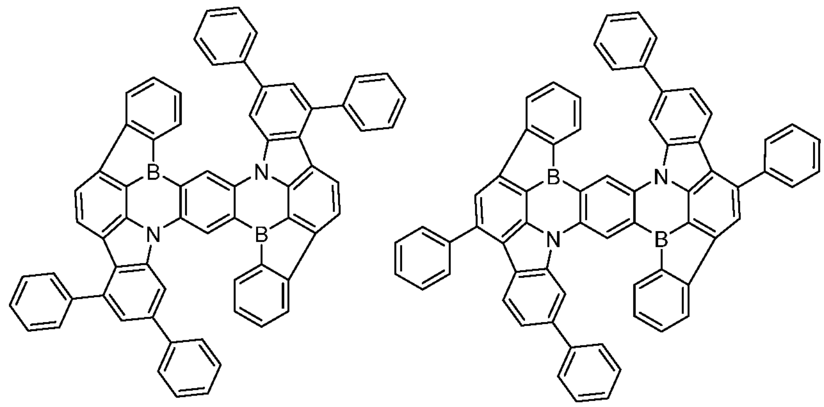

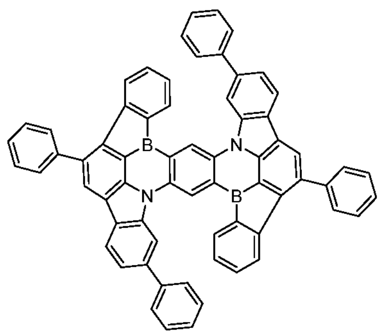
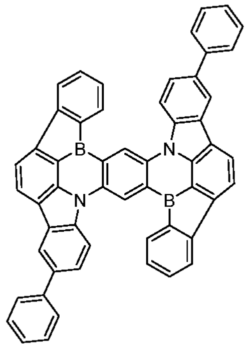
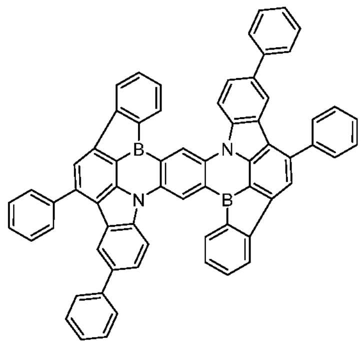

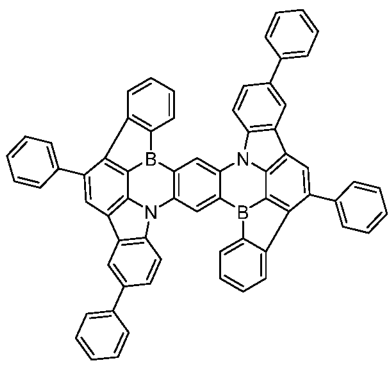
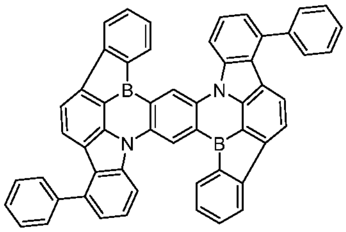
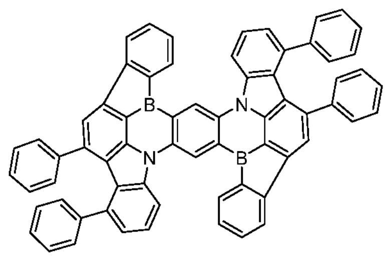

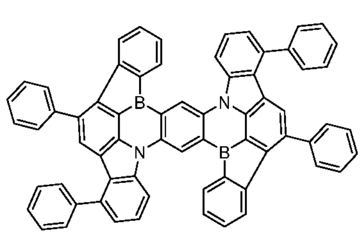
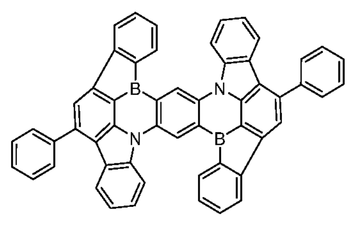
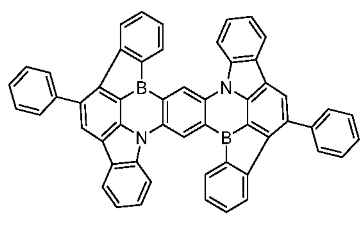

As one preferable group of compounds having the skeleton (3b), compounds represented by the following formula (3b) may be exemplified.

Formula (3b)

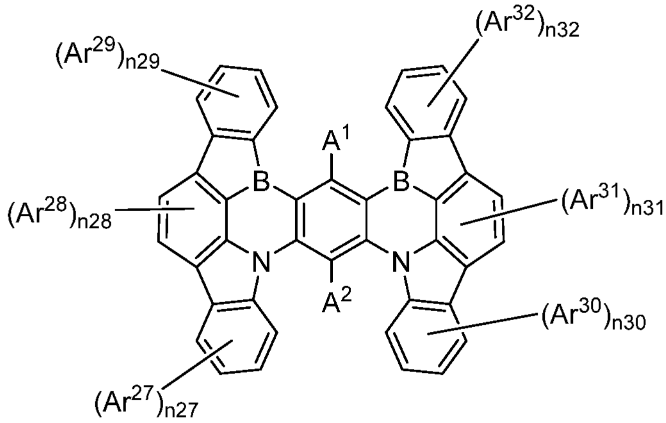

In the formula (3b), each of $Ar^{27}$ to $Ar^{32}$ independently represents a substituted or unsubstituted aryl group, a substituted or unsubstituted heteroaryl group, or a substituted or unsubstituted alkyl group, and for example, a substituted or unsubstituted aryl group may be preferably selected. Each of n27, n29, n30, and n32 independently represents an integer of 0 to 4, and each of n28 and n31 independently represents an integer of 0 to 2. Each of $A^1$ and $A^2$ independently represents a hydrogen atom, a deuterium atom, or a substituent. For details of $Ar^{27}$ to $Ar^{32}$, n27 to n32, $A^1$, and $A^2$, descriptions on $Ar^{15}$ to $Ar^{20}$, n15 to n20, $A^1$, and $A^2$ in the formula (2b) may be referred to in this order.

Hereinafter, specific examples of the compound represented by the formula (3b) will be given. Compounds of the formula (3b) that may be used in the invention are not construed as limiting to the following specific examples.

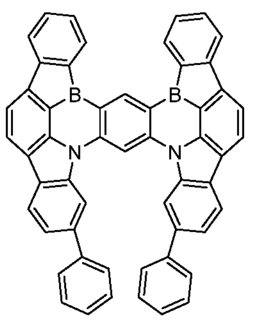
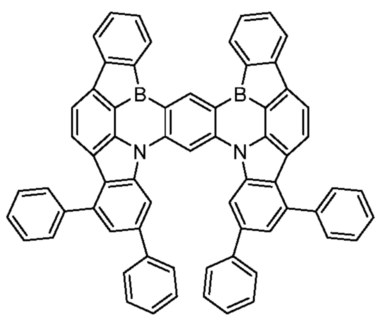
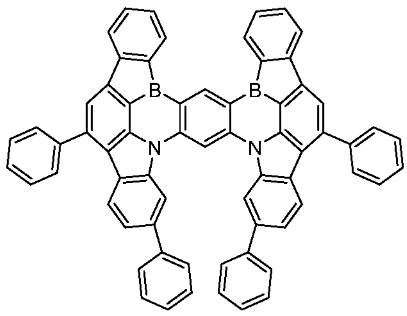

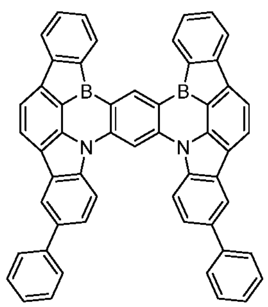
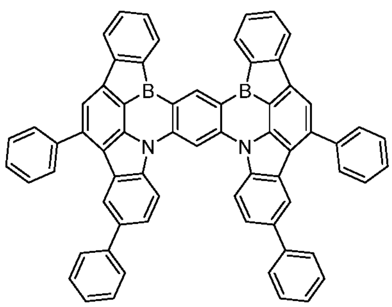
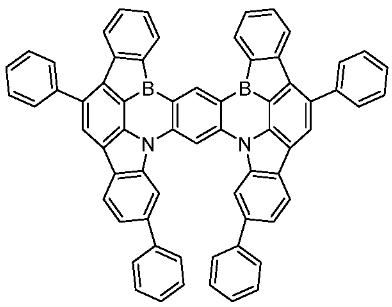

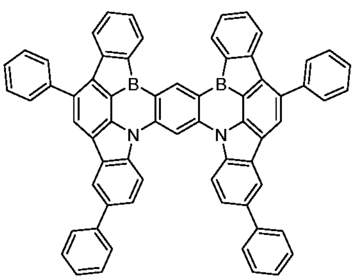
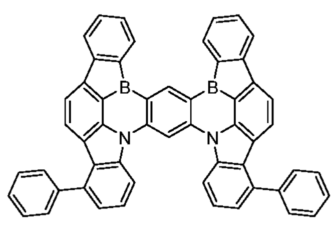
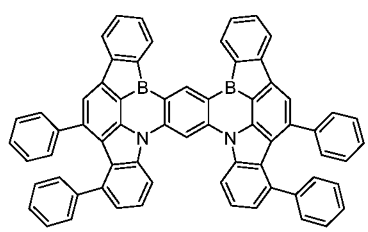

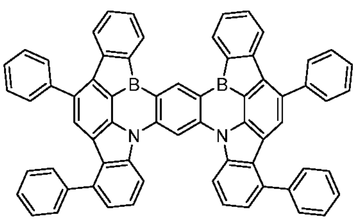
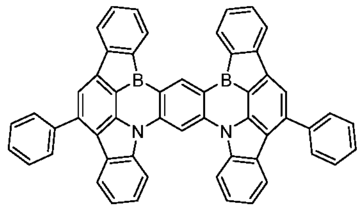
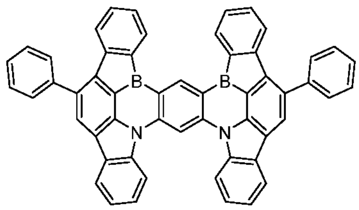

In a preferred aspect of the invention, compounds in which another ring is condensed with two benzene rings forming a carbazole partial structure existing in the formula (1) are selected. Among them, a compound in which a benzofuran ring is condensed, a compound in which a benzothiophene ring is condensed, and a compound in which a benzene ring is condensed may be particularly preferably selected. Hereinafter, compounds in which these rings are condensed will be described with reference to specific examples.

A compound in which a benzofuran ring or a benzothiophene ring is condensed with a benzene ring to which a boron atom is not directly bonded, between two benzene rings forming a carbazole partial structure existing in the formula (1), may be preferably mentioned. Examples of such a compound include a compound having the following skeleton (4a), and a compound having the following skeleton (4b).

Skeleton (4a)

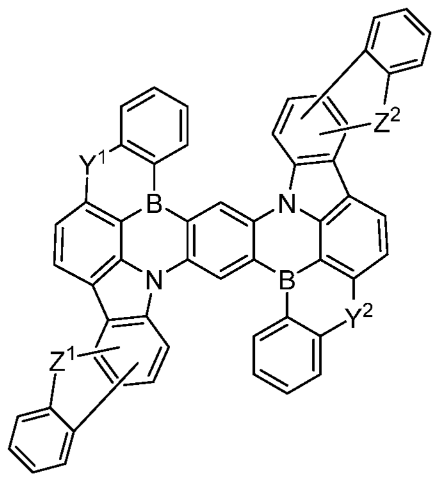

Skeleton (4b)

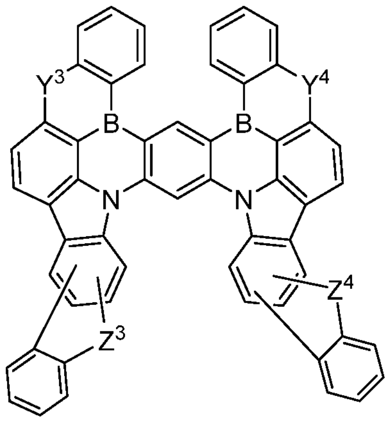

In the skeletons (4a) and (4b), each of $Y^1$ to $Y^4$ independently represents two hydrogen atoms, a single bond or $N(R^{27})$. Two hydrogen atoms mentioned herein indicate a state where two benzene rings bonded to a boron atom are not linked to each other. It is preferable that $Y^1$ and $Y^2$ are the same, and $Y^3$ and $Y^4$ are the same, but they may be different from each other. In one aspect of the invention, $Y^1$ to $Y^4$ are single bonds. In one aspect of the invention, $Y^1$ to $Y^4$ are $N(R^{27})$. $R^{27}$ represents a hydrogen atom, a deuterium atom, or a substituent.

Each of $Z^1$ to $Z^4$ independently represents an oxygen atom or a sulfur atom. It is preferable that $Z^1$ and $Z^2$ are the same, and $Z^3$ and $Z^4$ are the same, but they may be different from each other. In one aspect of the invention, $Z^1$ to $Z^4$ are oxygen atoms. Here, a furan ring of benzofuran is condensed with the benzene ring forming the carbazole partial structure in (4a) and (4b). The orientation of the condensed furan ring is not limited. In one aspect of the invention, $Z^1$ to $Z^4$ are sulfur atoms. Here, a thiophene ring of benzothiophene is condensed with the benzene ring forming the carbazole partial structure in (4a) and (4b). The orientation of the condensed thiophene ring is not limited.

Each hydrogen atom in the skeletons (4a) and (4b) may be substituted with a deuterium atom or a substituent. Further, it may be substituted with a linking group together with an adjacent hydrogen atom to form a ring structure. For details, corresponding descriptions on $R^1$ to $R^{26}$, $A^1$, and $A^2$ in the formula (1) may be referred to. In one aspect of the invention, each hydrogen atom in the skeletons (4a) and (4b) is not substituted with a linking group together with an adjacent hydrogen atom to form a ring structure.

As one preferable group of compounds having the skeleton (4a), compounds represented by the following formula (4a) may be exemplified. It is assumed that X in specific examples is an oxygen atom or a sulfur atom, and a compound in which X is an oxygen atom and a compound in which X is a sulfur atom are disclosed, respectively. Further, in specific examples of compounds represented by other subsequent formulas, X has the same meaning.

Formula (4a)

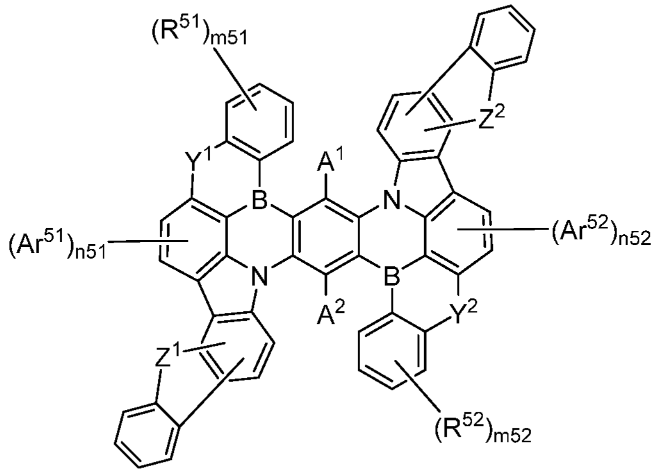

In the formula (4a), each of $Ar^{51}$ and $Ar^{52}$ independently represents a substituted or unsubstituted aryl group, a substituted or unsubstituted heteroaryl group, or a substituted or unsubstituted alkyl group, and for example, a substituted or unsubstituted aryl group may be preferably selected. Each of $R^{51}$ and $R^{52}$ independently represents a substituted or unsubstituted alkyl group. Each of m51 and m52 independently represents an integer of 0 to 4. Each of n51 and n52 independently represents an integer of 0 to 2. Each of $Y^1$ to $Y^4$ independently represents two hydrogen atoms, a single bond or $N(R^{27})$. $R^{27}$ represents a hydrogen atom, a deuterium atom, or a substituent. Each of $Z^1$ to $Z^4$ independently represents an oxygen atom or a sulfur atom. Each of $A^1$ and $A^2$ independently represents a hydrogen atom, a deuterium atom, or a substituent.

In one aspect of the invention, n51 and n52 are the same number. For example, n51 and n52 may be 0, and n51 and n52 may be 1. In one aspect of the invention, m51 and m52 are the same number. In one aspect of the invention, m51 and m52 are integers of 0 to 3. For example, m51 and m52 may be 0, m51 and m52 may be 1, m51 and m52 may be 2, and m51 and m52 may be 3. In relation to preferable groups for $Ar^{51}$, $Ar^{52}$, $R^{51}$, $R^{52}$, $A^1$, and $A^2$, corresponding descriptions on $Ar^1$ to $Ar^4$, $R^{41}$ to $R^{42}$, $A^1$, and $A^2$ in the formula (1a) may be referred to.

Hereinafter, specific examples of the compound represented by the formula (4a) will be given. Compounds of the formula (4a) that may be used in the invention are not construed as limiting to specific examples in the following one group. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

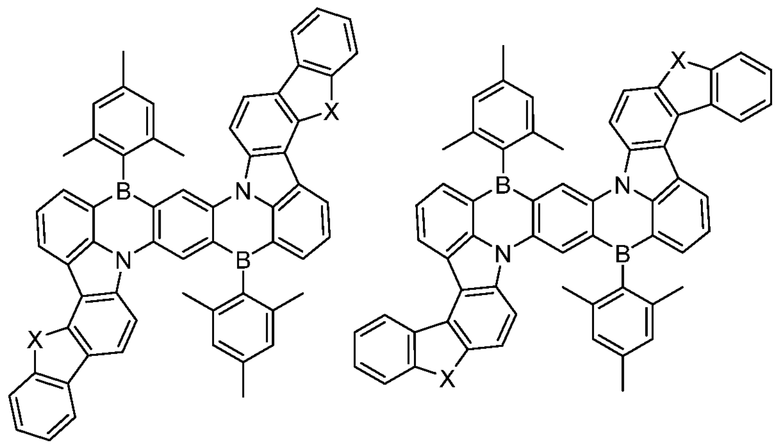

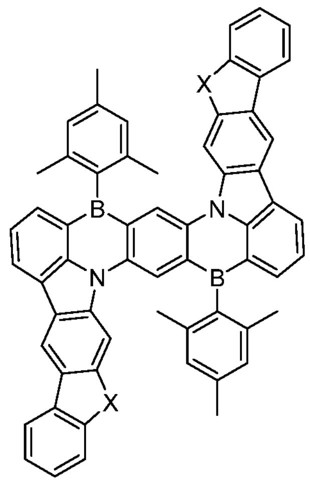

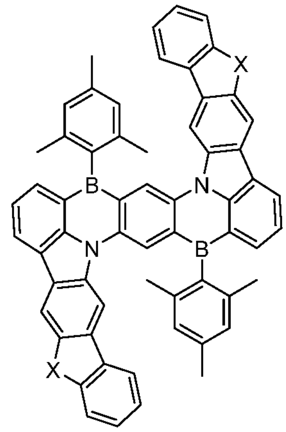
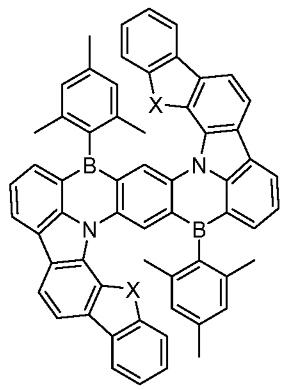
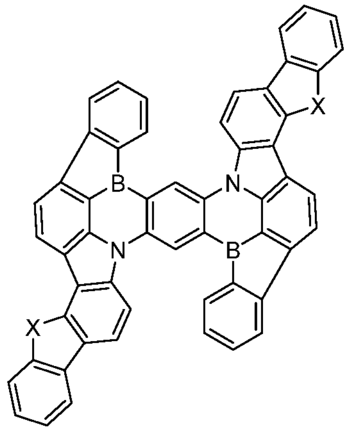

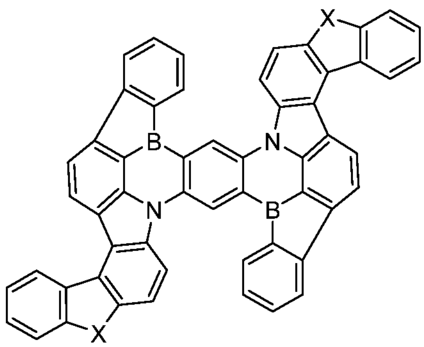
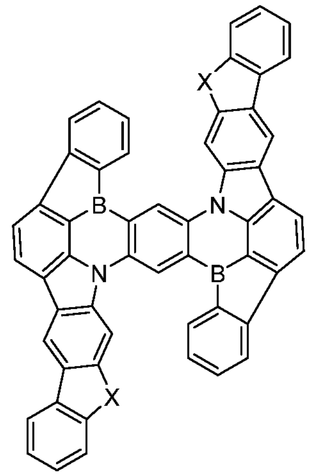
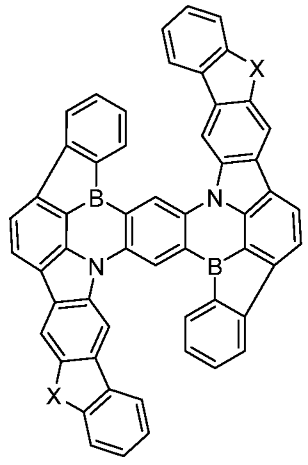

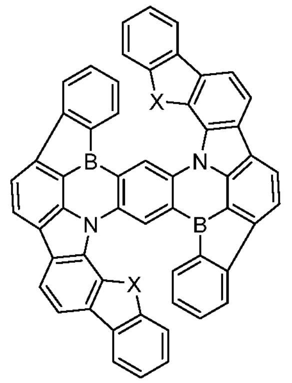
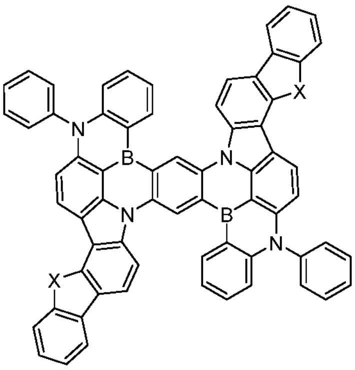
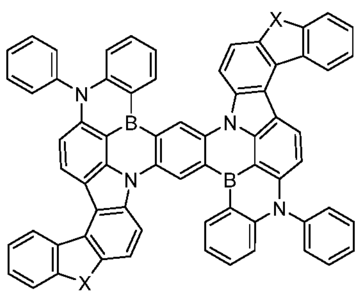

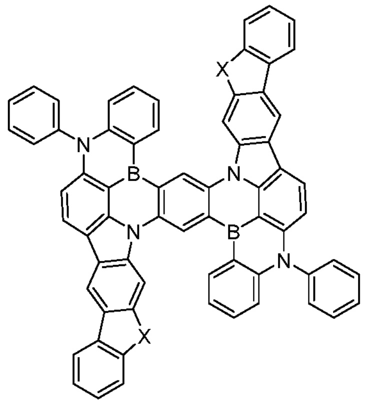

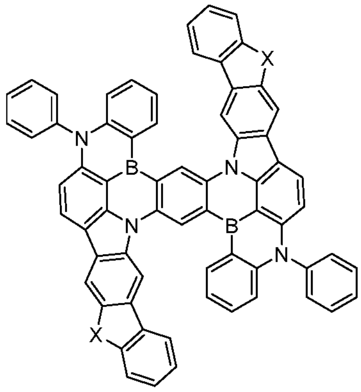

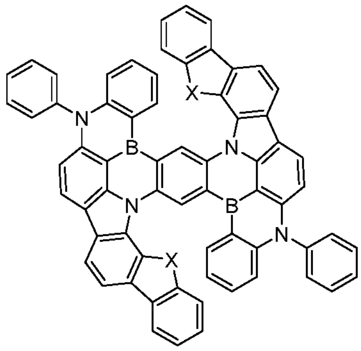

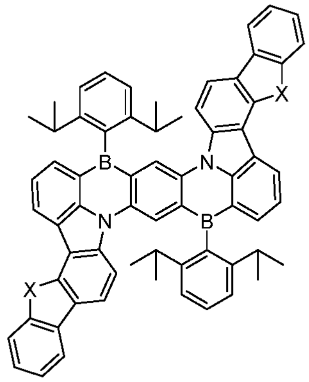

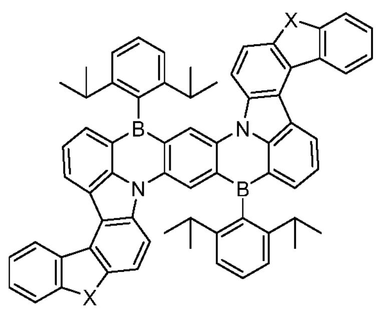

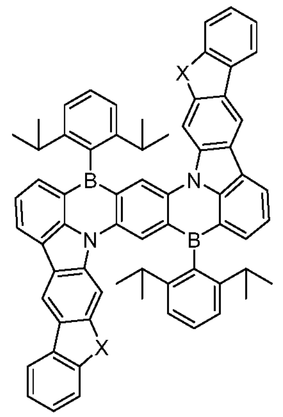

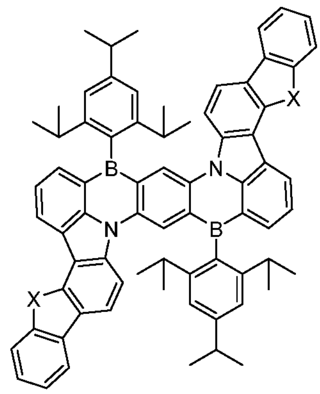

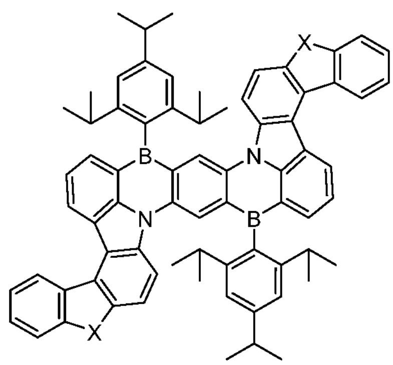

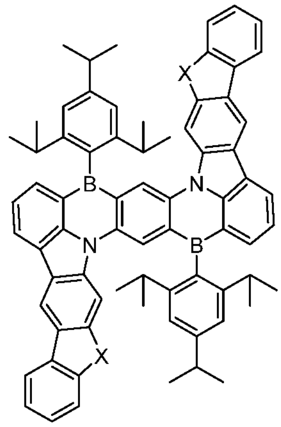

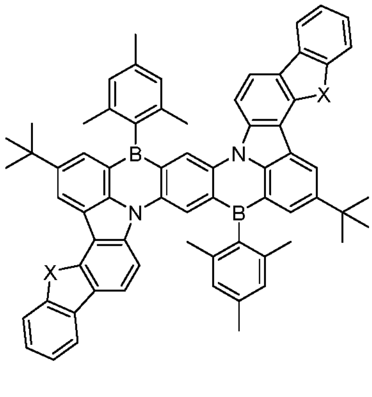

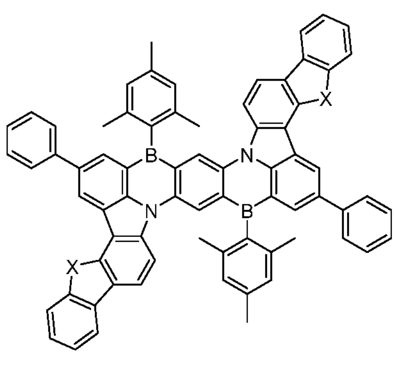

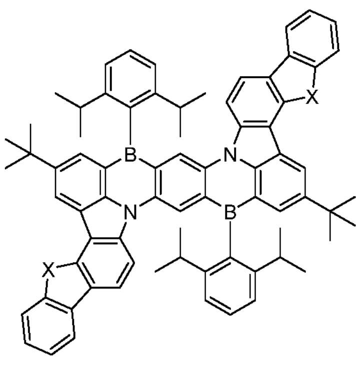

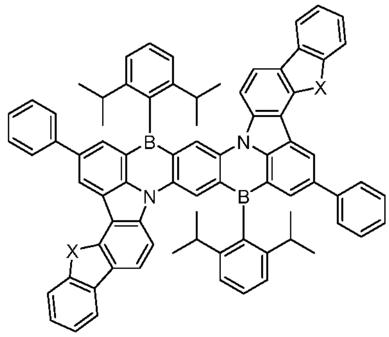

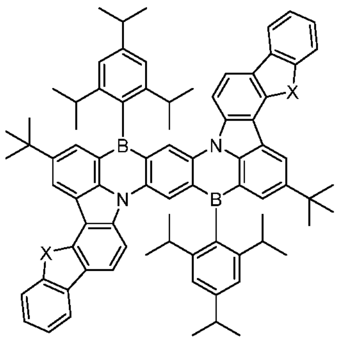

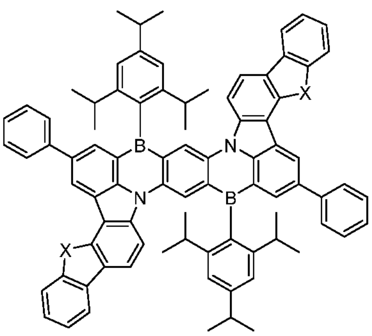

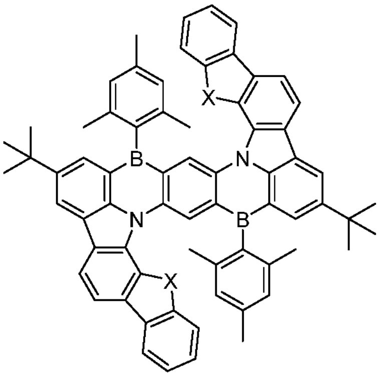

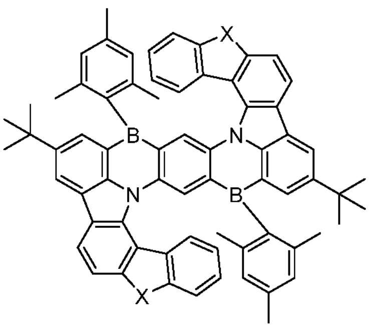

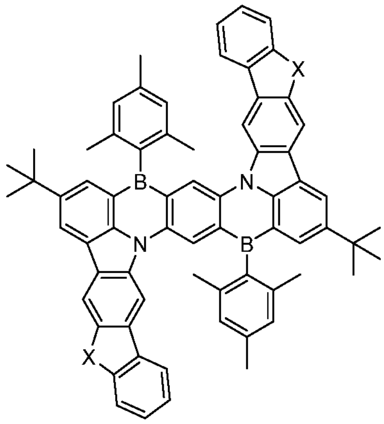

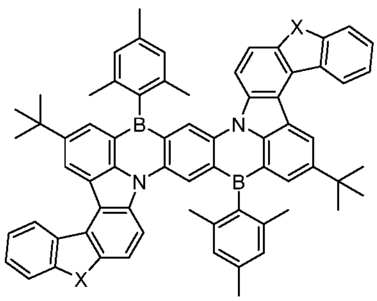

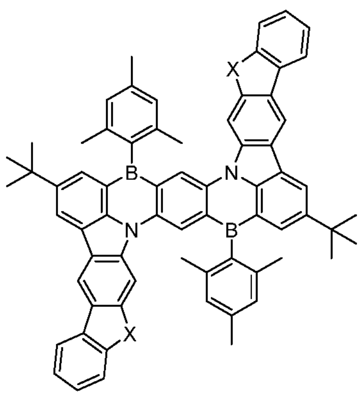

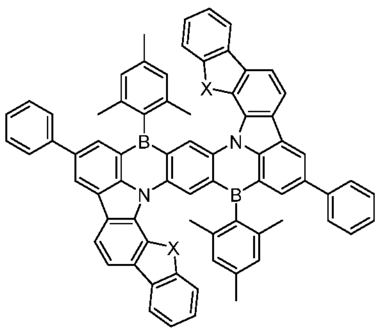

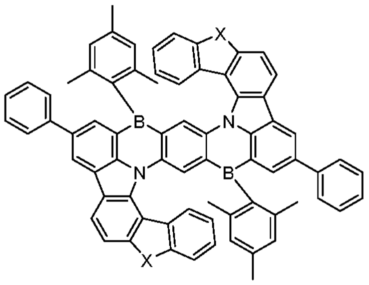

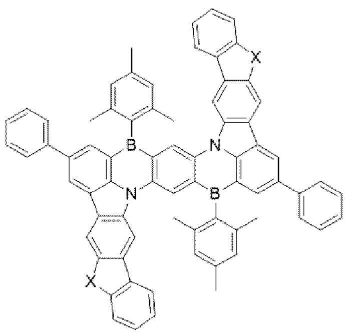

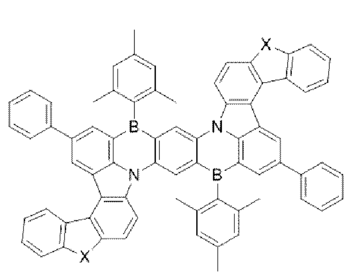

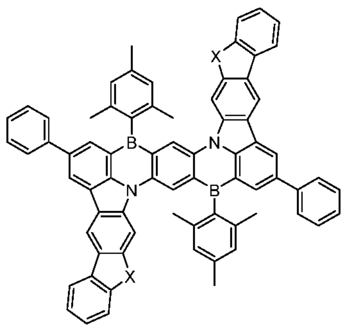
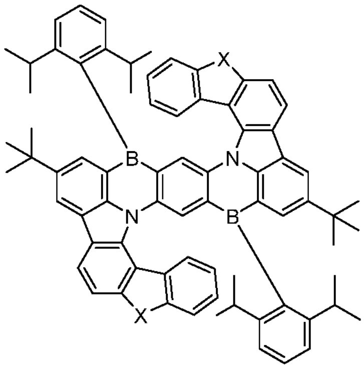
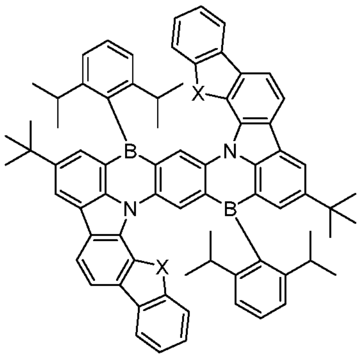

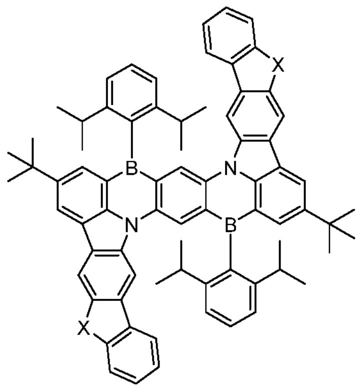
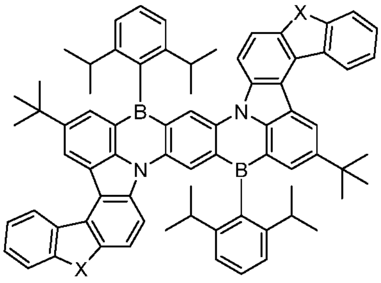
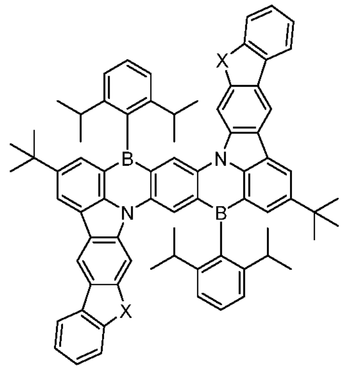

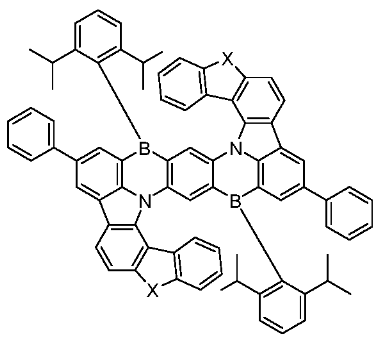
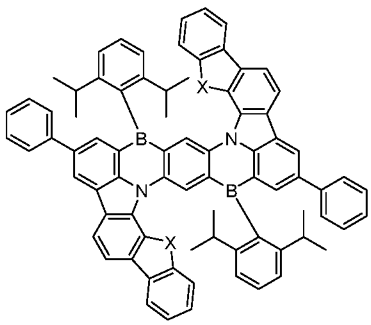
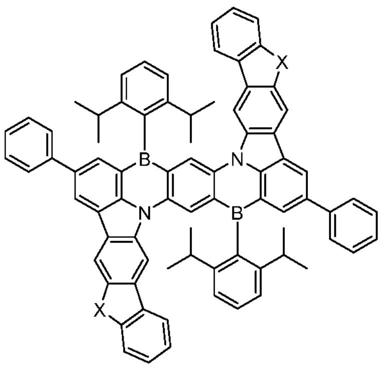

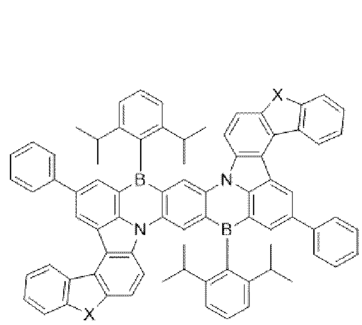
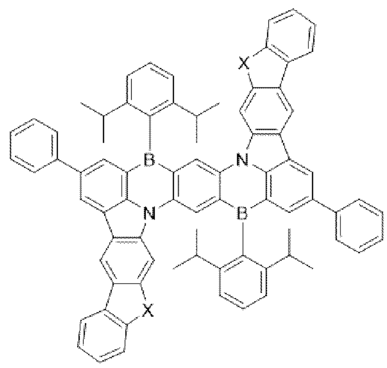
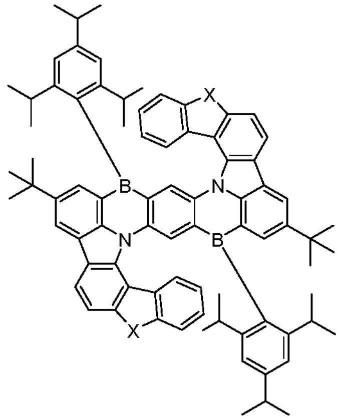

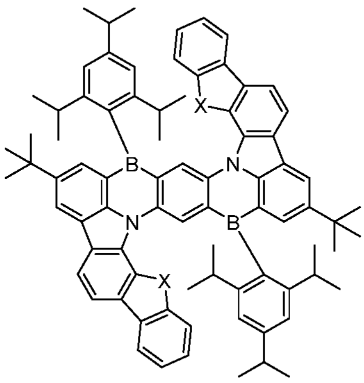

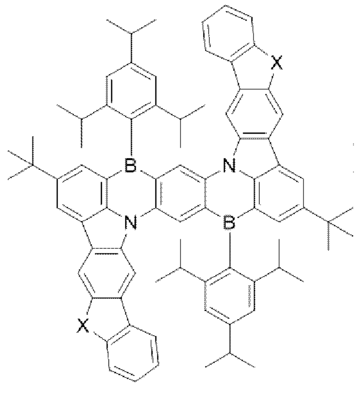

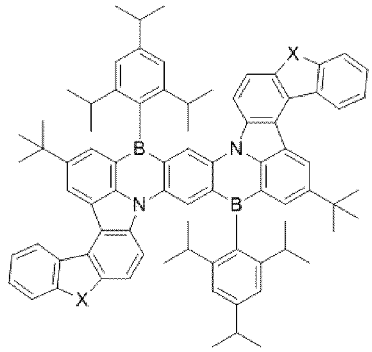

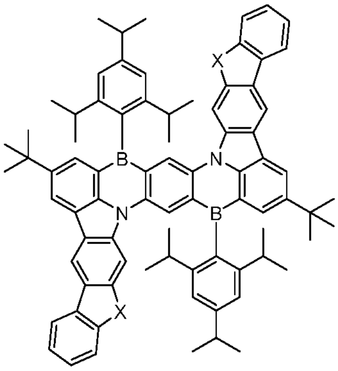

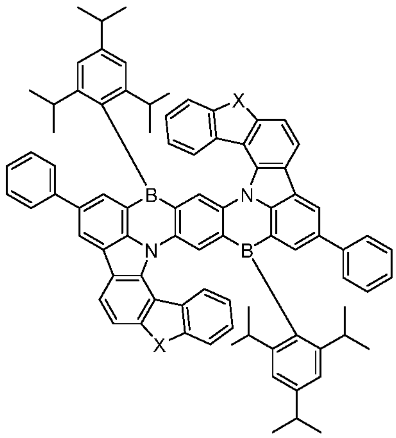

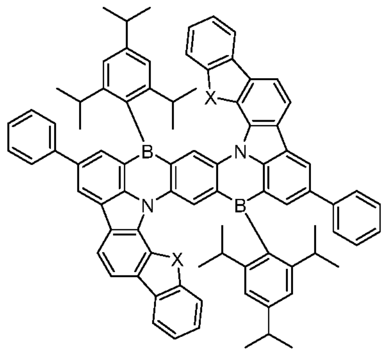

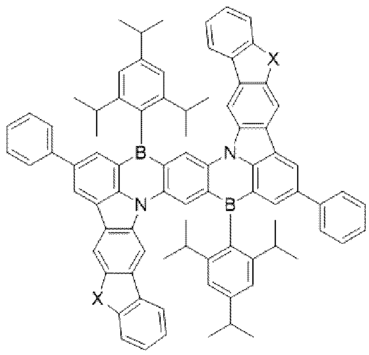

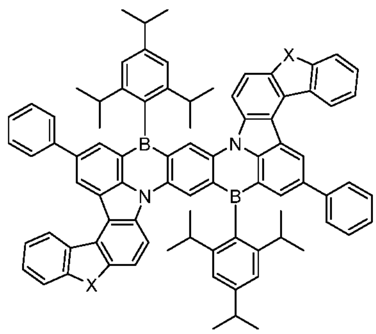

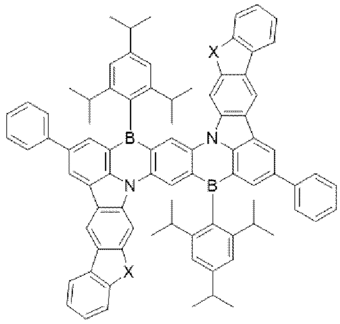

Hereinafter, another group of specific examples of the compound represented by the formula (4a) will be given. Compounds of the formula (4a) that may be used in the invention are not construed as limiting to specific examples in the following one group.

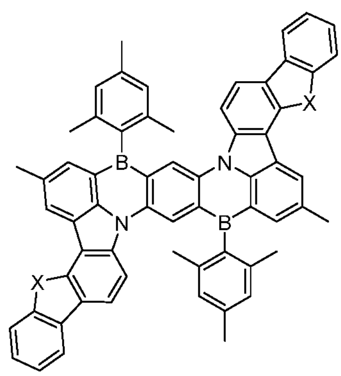

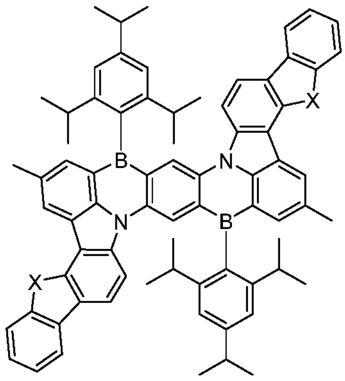

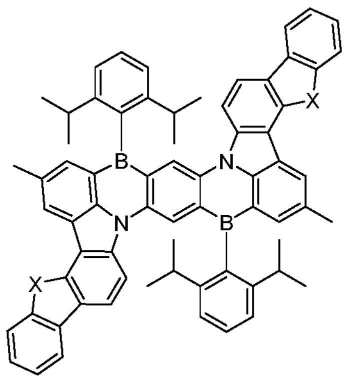

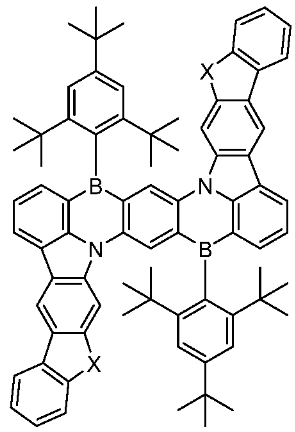

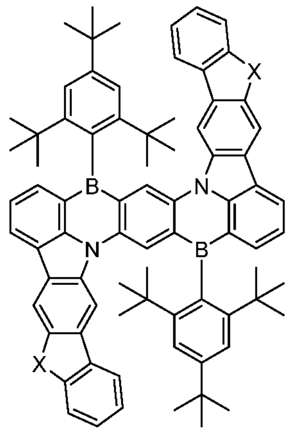

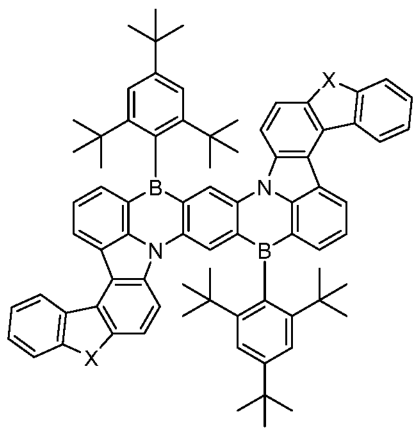

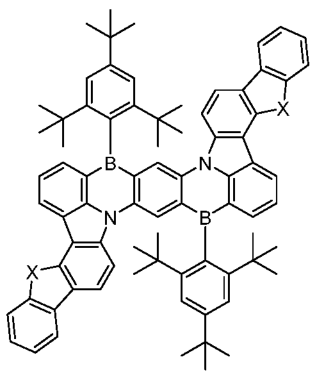

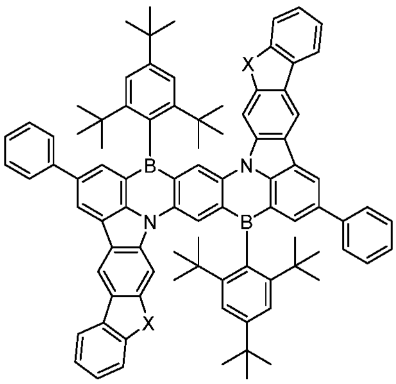

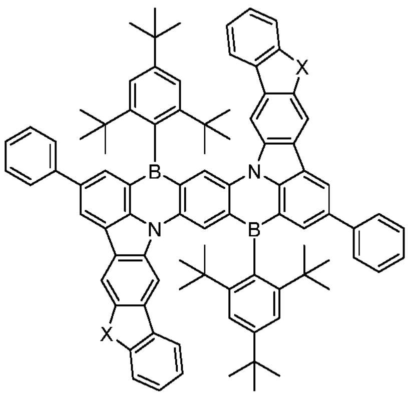

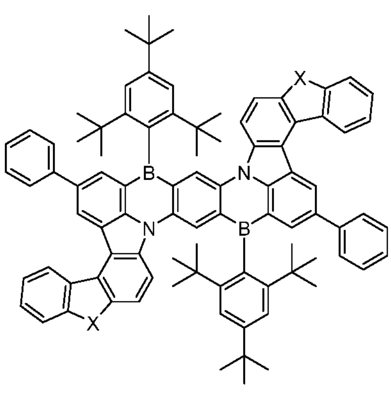

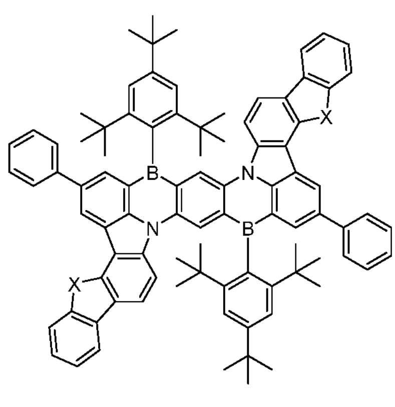

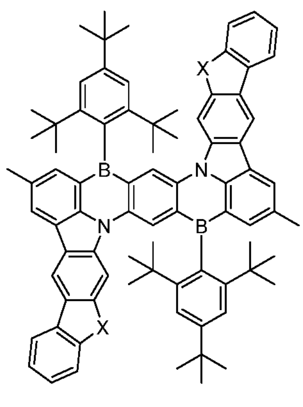

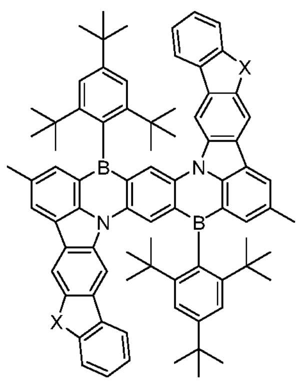
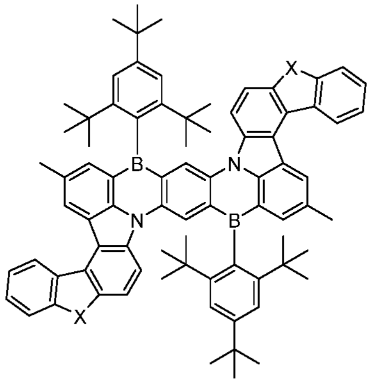
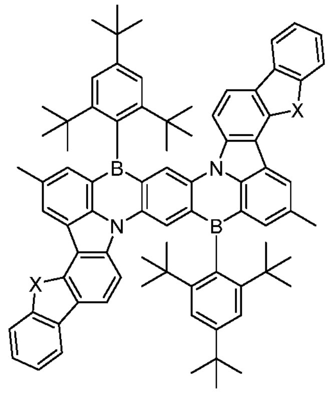

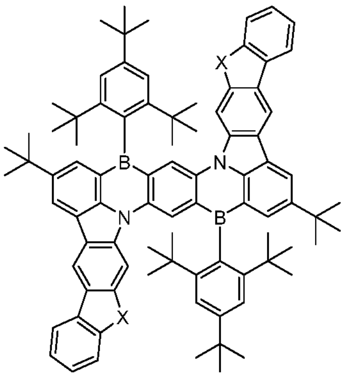
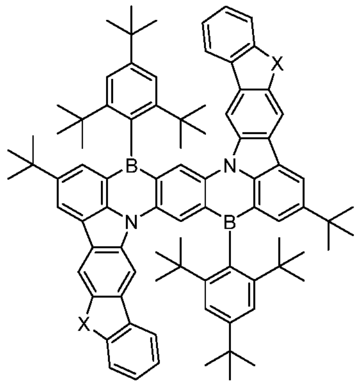
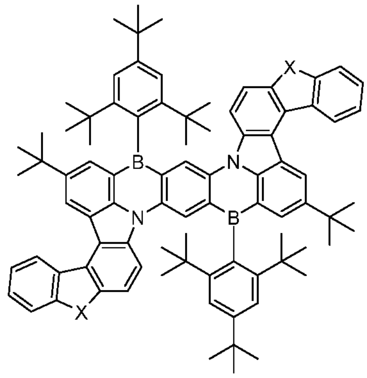

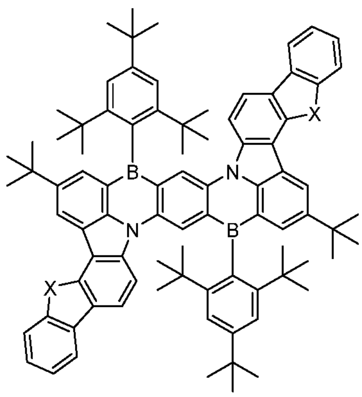

As one preferable group of compounds having the skeleton (4b), compounds represented by the following formula (4b) may be exemplified.

Formula (4b)

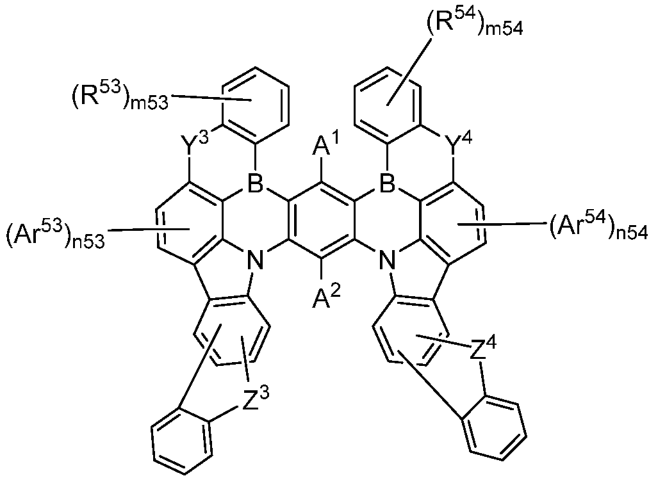

In the formula (4b), each of $Ar^{53}$ and $Ar^{54}$ independently represents a substituted or unsubstituted aryl group, a substituted or unsubstituted heteroaryl group, or a substituted or unsubstituted alkyl group, and for example, a substituted or unsubstituted aryl group may be preferably selected. Each of $R^{53}$ and $R^{54}$ independently represents a substituted or unsubstituted alkyl group. Each of m53 and m54 independently represents an integer of 0 to 4. Each of n53 and n54 independently represents an integer of 0 to 2. Each of $Y^3$ and $Y^4$ independently represents two hydrogen atoms, a single bond or $N(R^{27})$. $R^{27}$ represents a hydrogen atom, a deuterium atom, or a substituent. Each of $Z^3$ and $Z^4$ independently represents an oxygen atom or a sulfur atom. Each of $A^1$ and $A^2$ independently represents a hydrogen atom, a deuterium atom, or a substituent. In relation to details of $Ar^{53}$, $Ar^{54}$, $R^{53}$, $R^{54}$, m53, m54, n53, n54, $A^1$, and $A^2$, the descriptions on $Ar^{51}$, $Ar^{52}$, $R^{51}$, $R^{52}$, m51, m52, n51, n52, $A^1$, and $A^2$ in the formula (4a) may be referred to.

Hereinafter, specific examples of the compound represented by the formula (4b) will be given. Compounds of the formula (4b) that may be used in the invention are not construed as limiting to the following specific examples. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

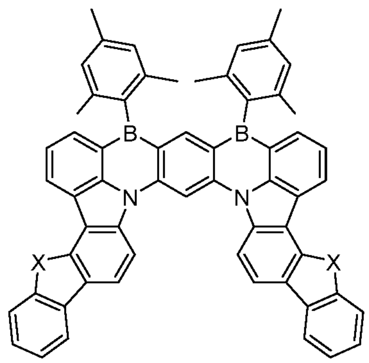

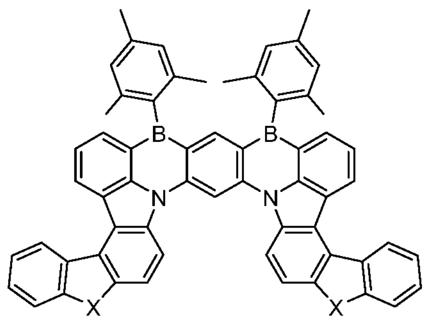

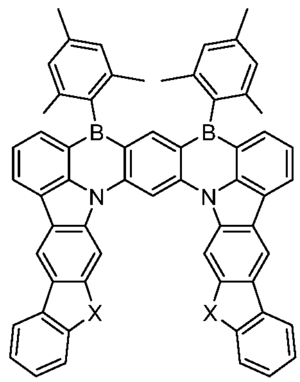

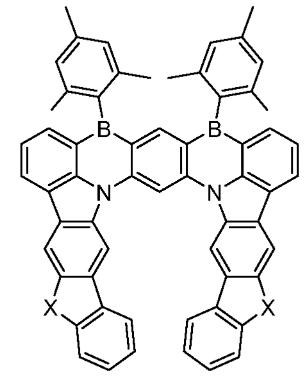
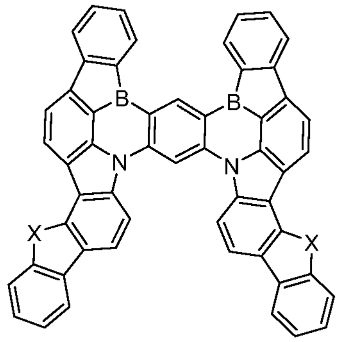
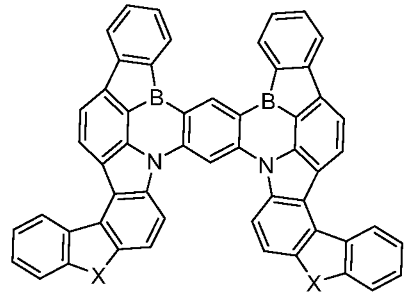

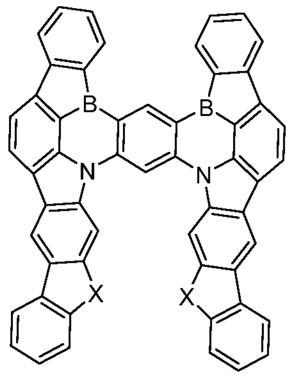
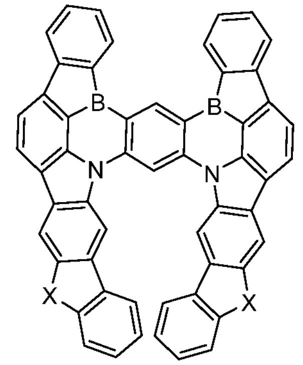
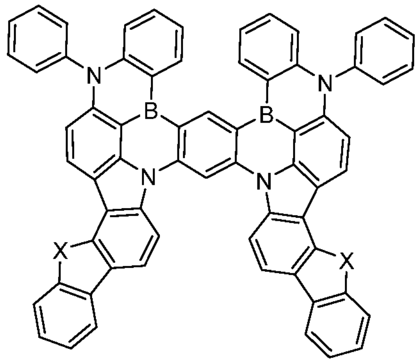

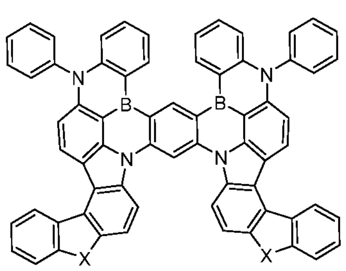
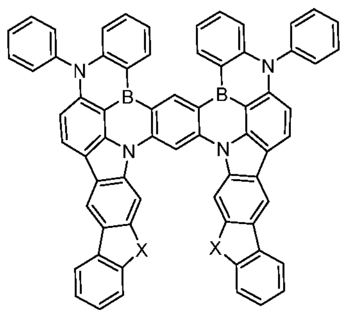
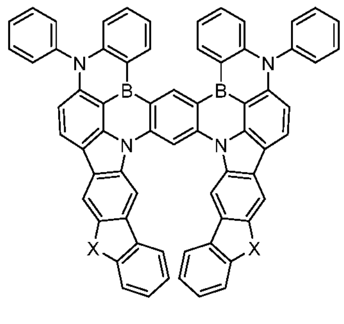

A compound in which a benzofuran ring or a benzothiophene ring is condensed with a benzene ring to which a boron atom is directly bonded, between two benzene rings forming a carbazole partial structure existing in the formula (1), may be preferably mentioned. Examples of such a compound include a compound having the following skeleton (5a) and a compound having the following skeleton (5b).

Skeleton (5a)

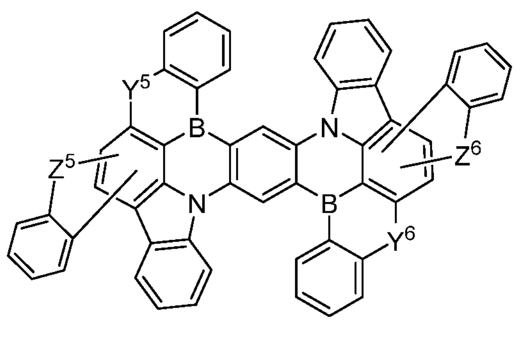

Skeleton (5b)

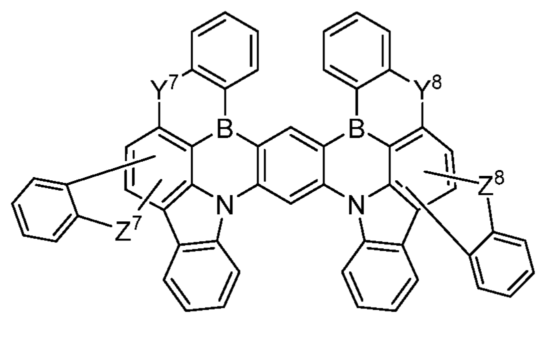

In the skeletons (5a) and (5b), each of $Y^5$ to $Y^8$ independently represents two hydrogen atoms, a single bond

or $N(R^{27})$. Each of $Z^5$ to $Z^8$ independently represents an oxygen atom or a sulfur atom. In relation to details of $Y^5$ to $Y^8$, and $Z^5$ to $Z^8$, corresponding descriptions for the skeletons (4a) and (4b) may be referred to. In one aspect of the invention, each hydrogen atom in the skeletons (5a) and (5b) is not substituted with a linking group together with an adjacent hydrogen atom to form a ring structure.

As one preferable group of compounds having the skeleton (5a), compounds represented by the following formula (5a) may be exemplified.

Formula (5a)

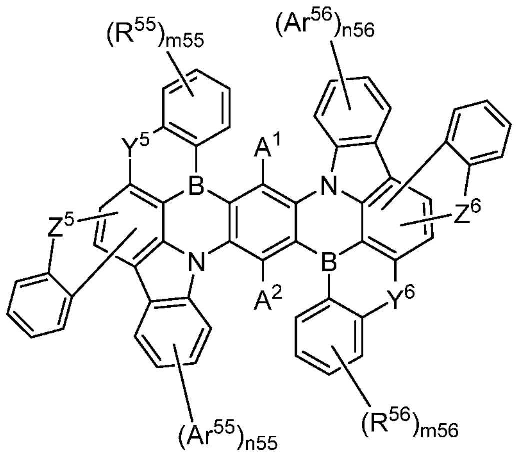

In the formula (5a), each of $Ar^{55}$ and $Ar^{56}$ independently represents a substituted or unsubstituted aryl group, a substituted or unsubstituted heteroaryl group, or a substituted or unsubstituted alkyl group, and for example, a substituted or unsubstituted aryl group may be preferably selected. Each of $R^{55}$ and $R^{56}$ independently represents a substituted or unsubstituted alkyl group. Each of m55 and m56 independently represents an integer of 0 to 4. Each of n55 and n56 independently represents an integer of 0 to 4. Each of $Y^5$ and $Y^6$ independently represents two hydrogen atoms, a single bond or $N(R^{27})$. $R^{27}$ represents a hydrogen atom, a deuterium atom, or a substituent. Each of $Z^5$ and $Z^6$ independently represents an oxygen atom or a sulfur atom. Each of $A^1$ and $A^2$ independently represents a hydrogen atom, a deuterium atom, or a substituent.

In one aspect of the invention, n55 and n56 are integers of 0 to 2. For example, n55 and n56 may be 0, and n55 and n56 may be 1. In one aspect of the invention, m51 and m52 are the same number. In relation to details of m55 and m56, descriptions on m51 and m52 in the formula (4a) may be referred to. In relation to preferable groups for $Ar^{55}$, $Ar^{56}$, $R^{55}$, $R^{56}$, $A^1$, and $A^2$, corresponding descriptions on $Ar^1$, $Ar^3$, $R^{41}$, $R^{42}$, $A^1$, and $A^2$ in the formula (1a) may be referred to.

Hereinafter, specific examples of the compound represented by the formula (5a) will be given. Compounds of the formula (5a) that may be used in the invention are not construed as limiting to specific examples in the following one group. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

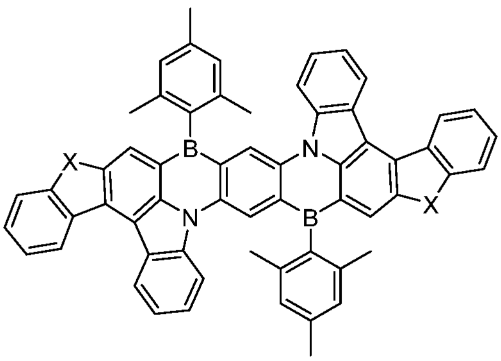

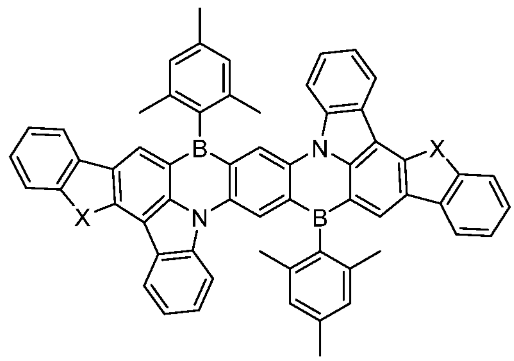

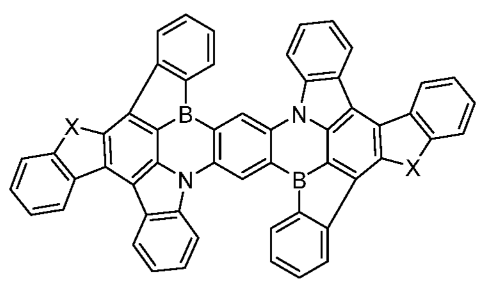
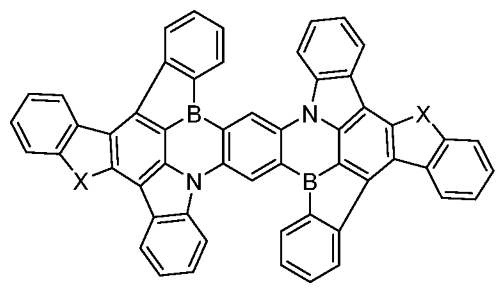

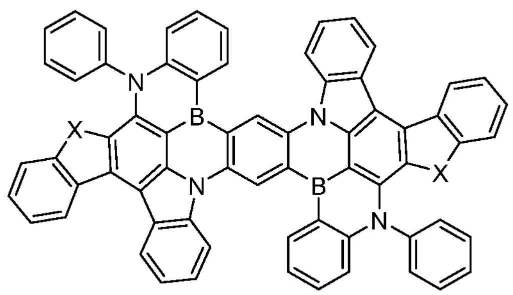
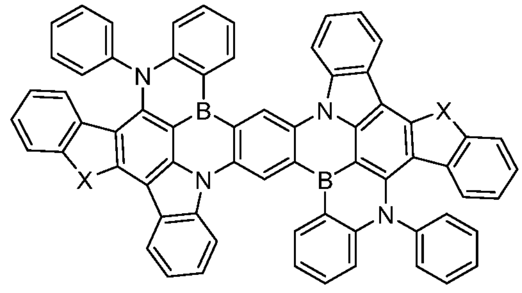

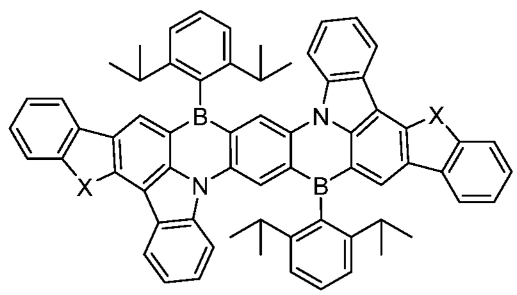
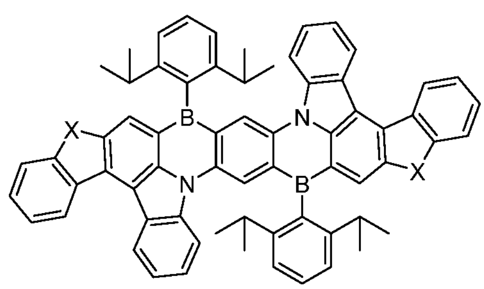

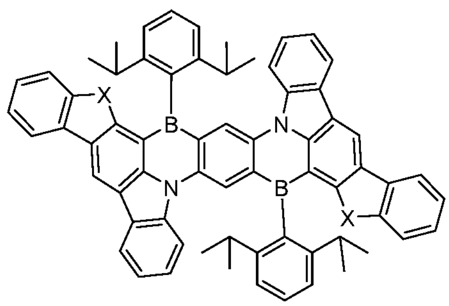
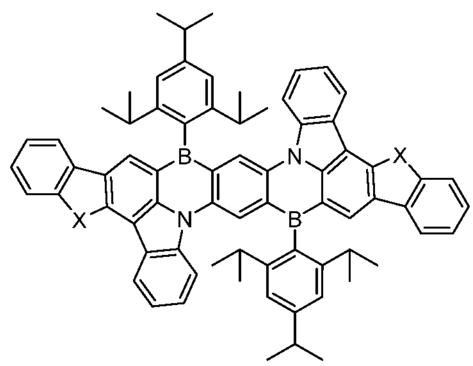

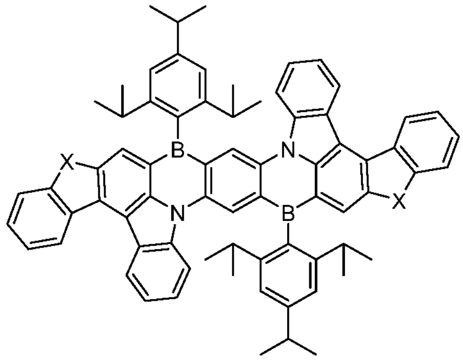

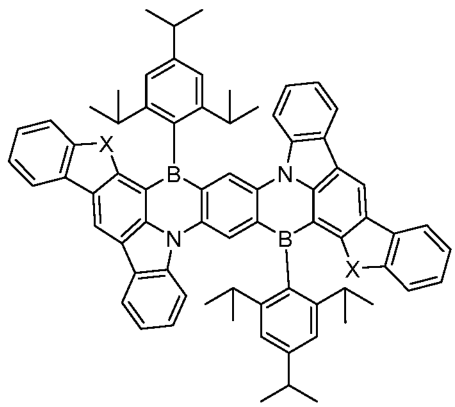

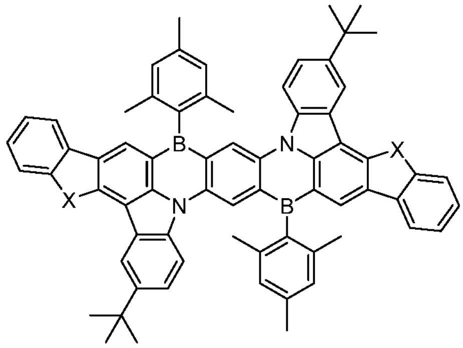

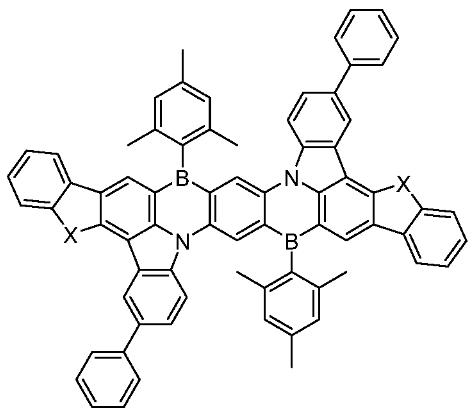

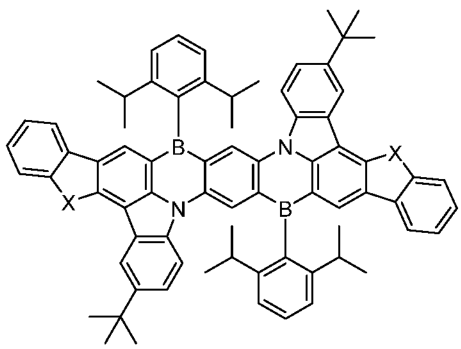

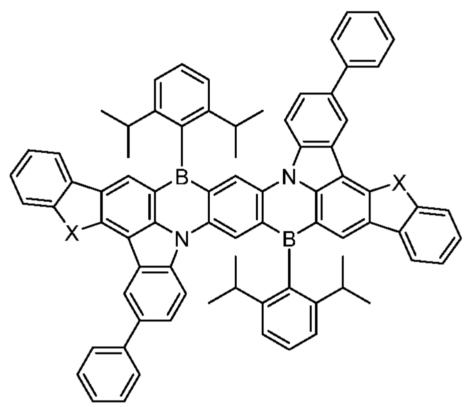

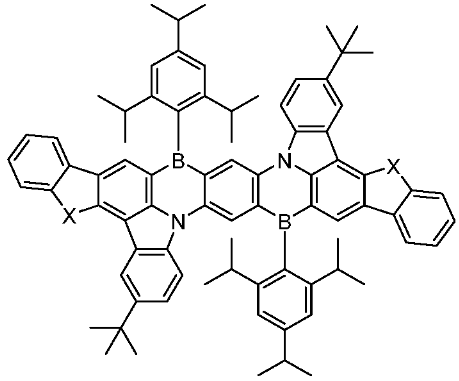

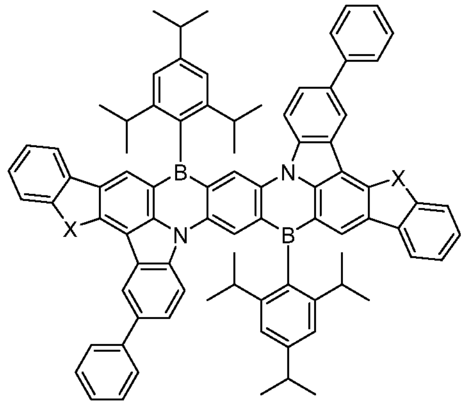

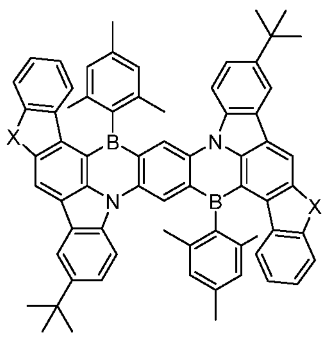
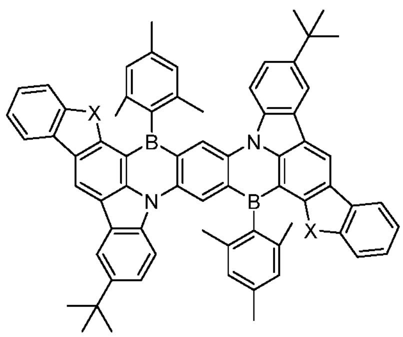
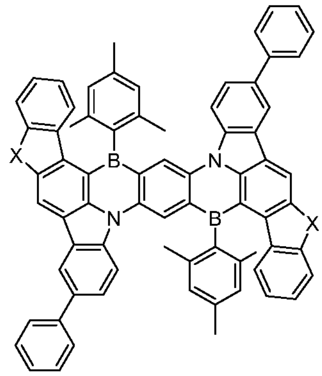

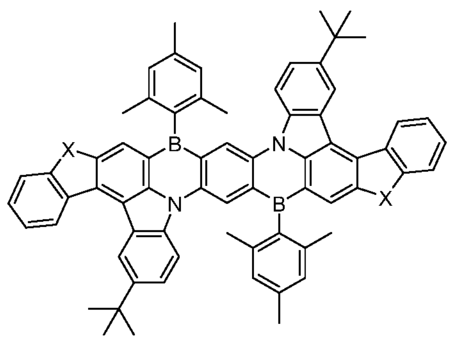
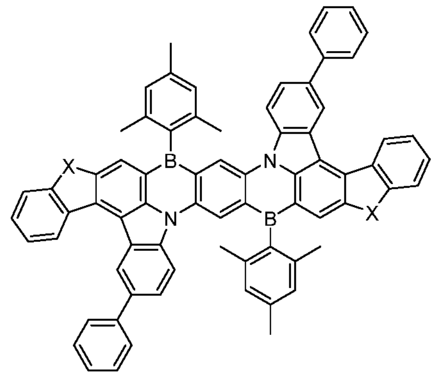

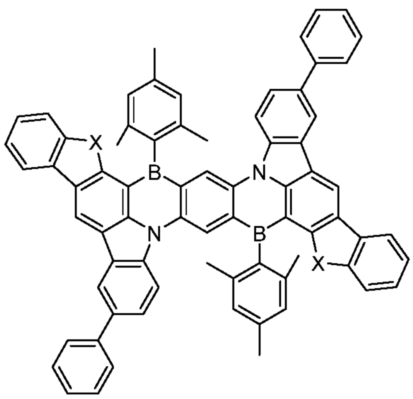
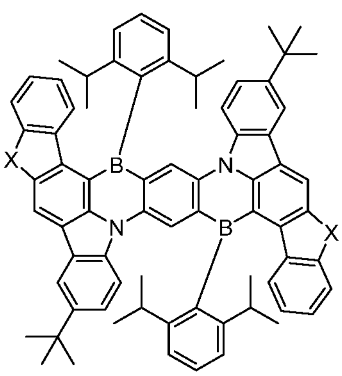
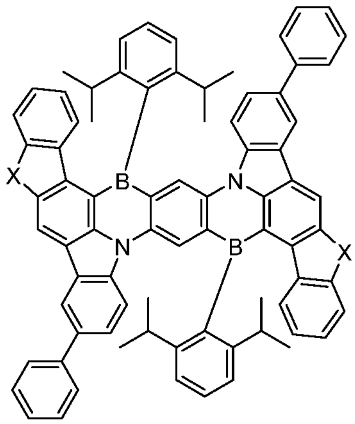

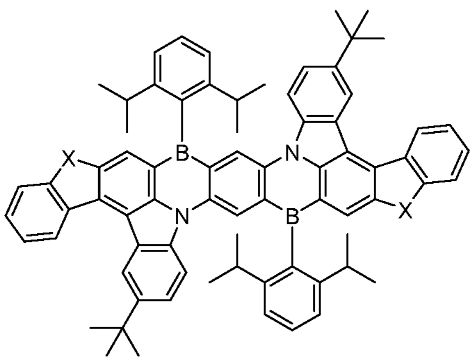
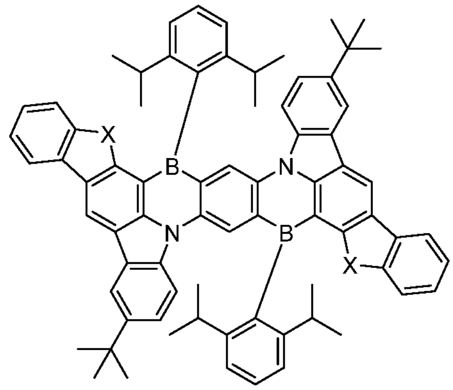

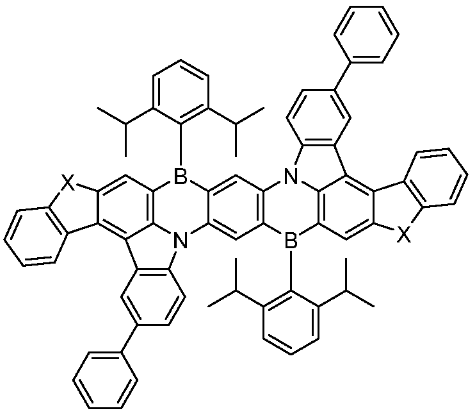

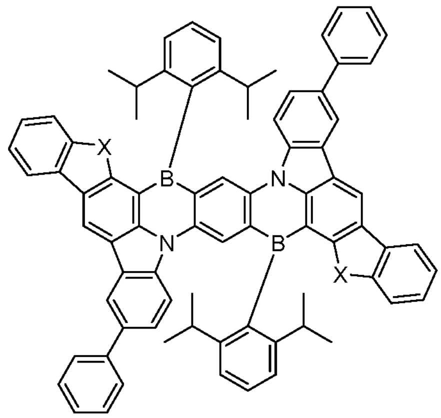

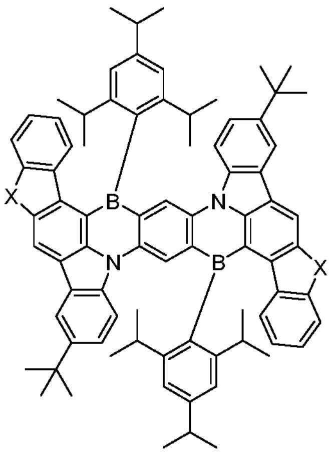

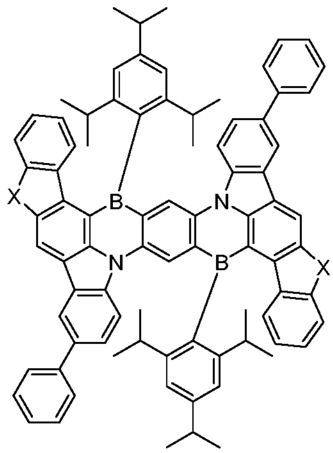

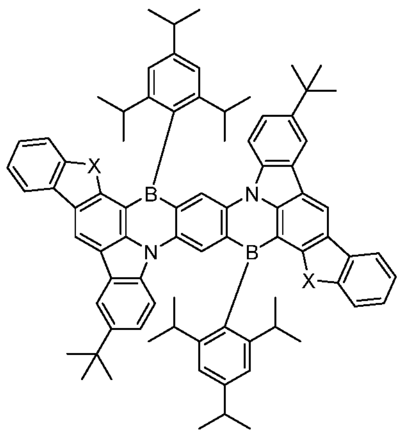

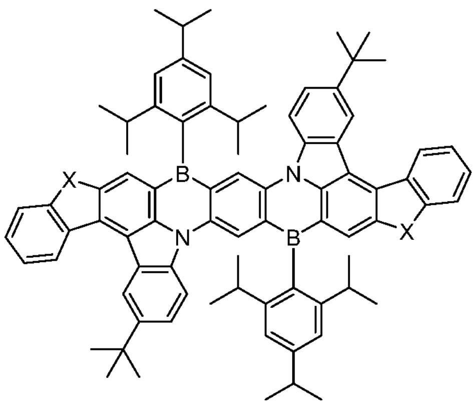

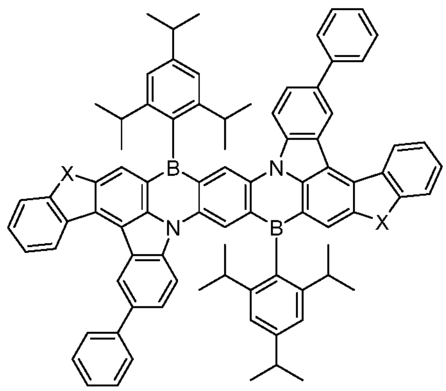

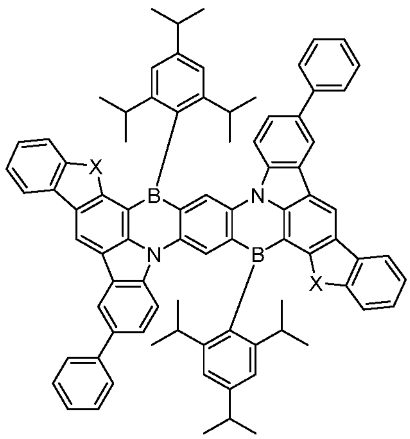

Hereinafter, another group of specific examples of the compound represented by the formula (5a) will be given. Compounds of the formula (5a) that may be used in the invention are not construed as limiting to specific examples in the following one group.

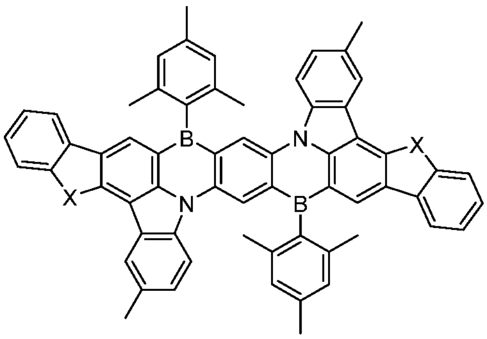

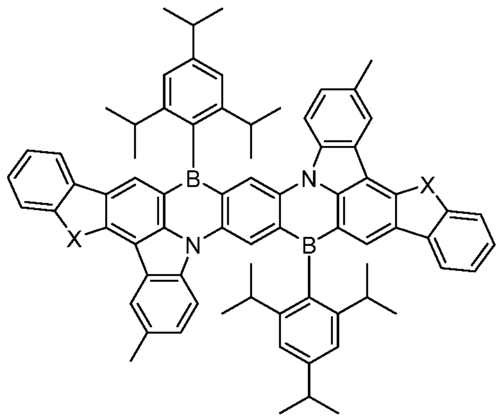

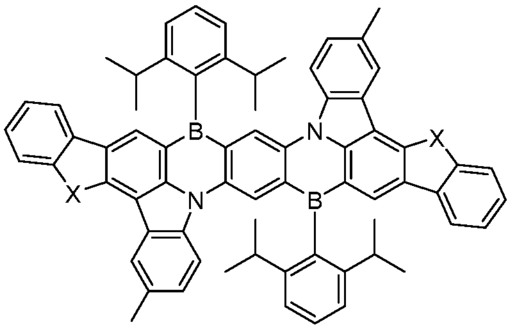

As one preferable group of compounds having the skeleton (5b), compounds represented by the following formula (5b) may be exemplified.

Formula (5b)

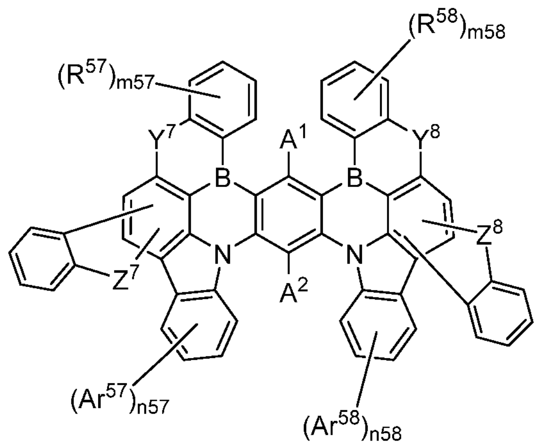

In the formula (5b), each of $Ar^{57}$ and $Ar^{58}$ independently represents a substituted or unsubstituted aryl group, a substituted or unsubstituted heteroaryl group, or a substituted or unsubstituted alkyl group, and for example, a substituted or unsubstituted aryl group may be preferably selected. Each of $R^{57}$ and $R^{58}$ independently represents a substituted or unsubstituted alkyl group. Each of m57 and m58 independently represents an integer of 0 to 4. Each of n57 and n58 independently represents an integer of 0 to 4. Each of $Y^7$ and $Y^8$ independently represents two hydrogen atoms, a single bond or $N(R^{27})$. $R^{27}$ represents a hydrogen atom, a deuterium atom, or a substituent. Each of $Z^7$ and $Z^8$ independently represents an oxygen atom or a sulfur atom. Each of $A^1$ and $A^2$ independently represents a hydrogen atom, a deuterium atom, or a substituent. In relation to details of $Ar^{57}$, $Ar^{58}$, $R^{57}$, $R^{58}$, m57, m58, n57, n58, $A^1$, and $A^2$, descriptions on $Ar^{55}$, $Ar^{56}$, $R^{55}$, $R^{56}$, m55, m56, n55, n56, $A^1$, and $A^2$ in the formula (5a) may be referred to.

Hereinafter, specific examples of the compound represented by the formula (5b) will be given. Compounds of the formula (5b) that may be used in the invention are not construed as limiting to specific examples in the following one group. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms and the rest are sulfur atoms may also be adopted.

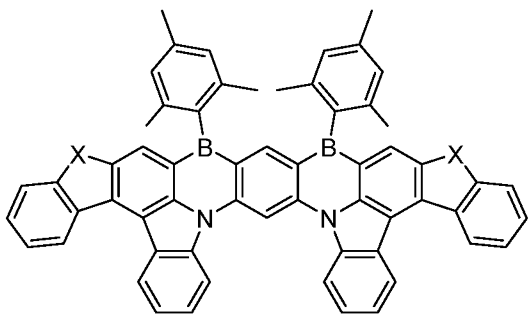

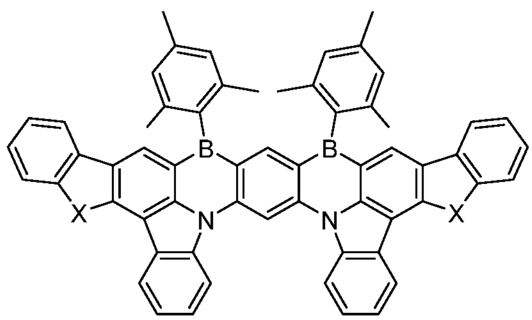

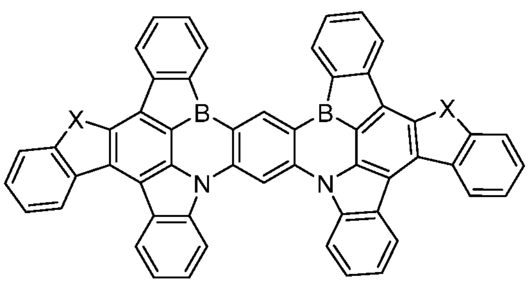

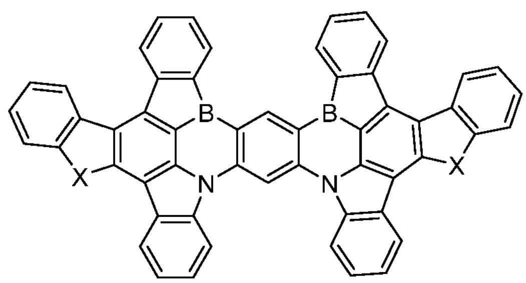

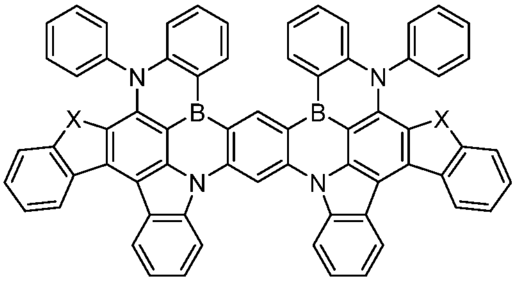

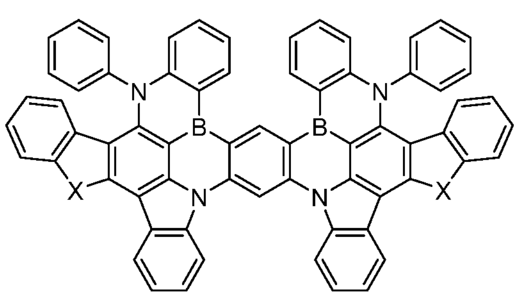

Hereinafter, another group of specific examples of the compound represented by the formula (5b) will be given. Compounds of the formula (5b) that may be used in the invention are not construed as limiting to specific examples in the following one group.

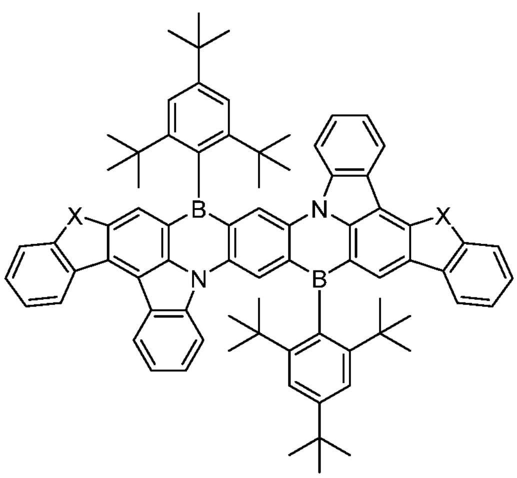

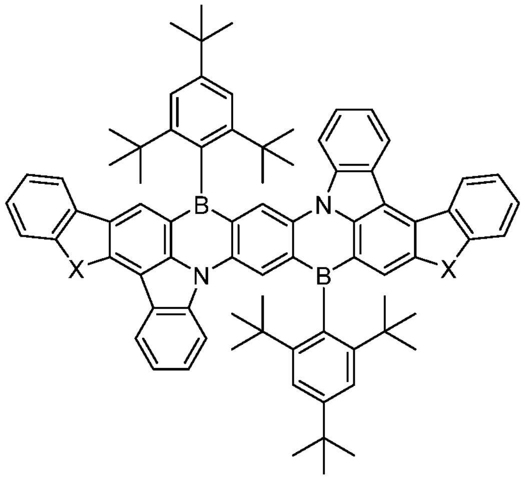

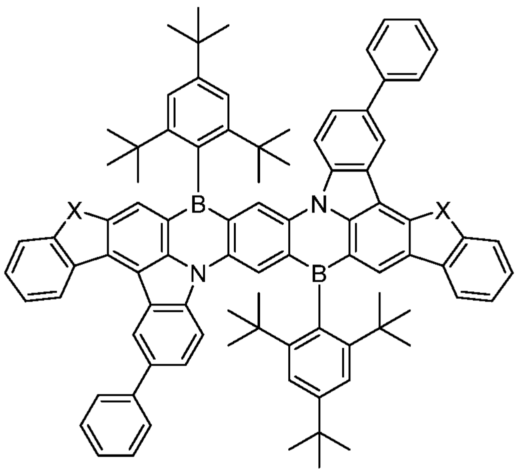

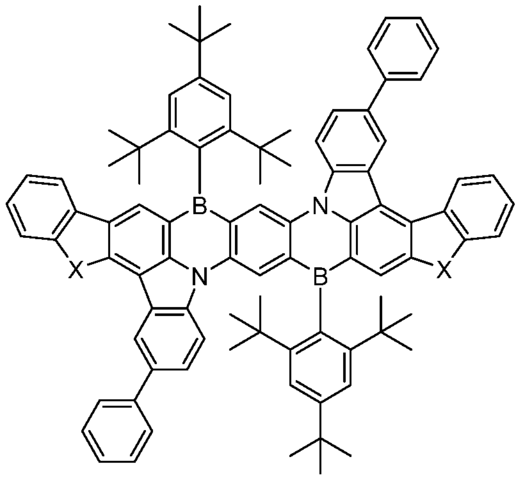

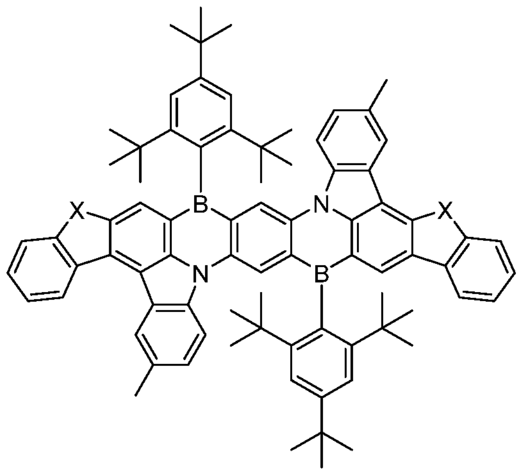

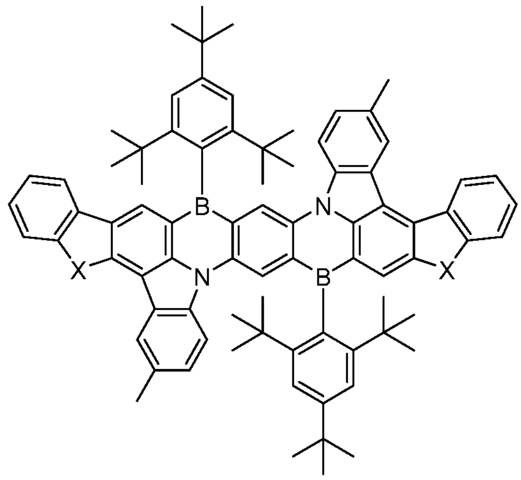

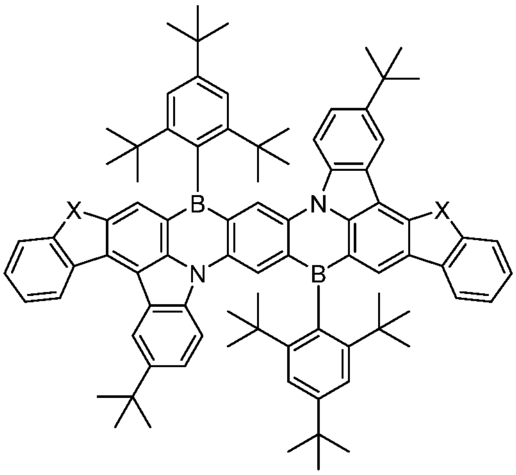

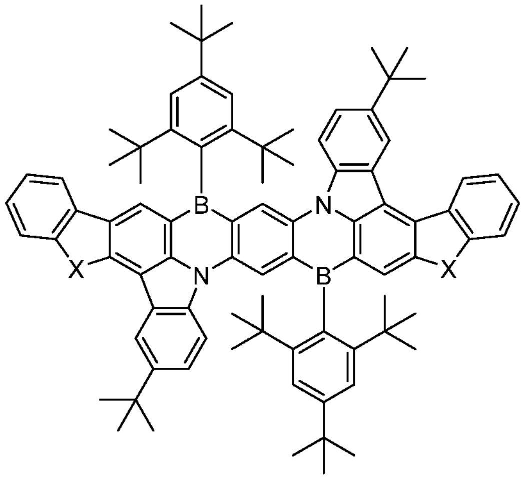

A compound in which benzofuran rings or benzothiophene rings are condensed with both of two benzene rings forming a carbazole partial structure existing in the formula (1) may be preferably mentioned. Examples of such a compound include a compound having the following skeleton (6a), and a compound having the following skeleton (6b).

Skeleton (6a)

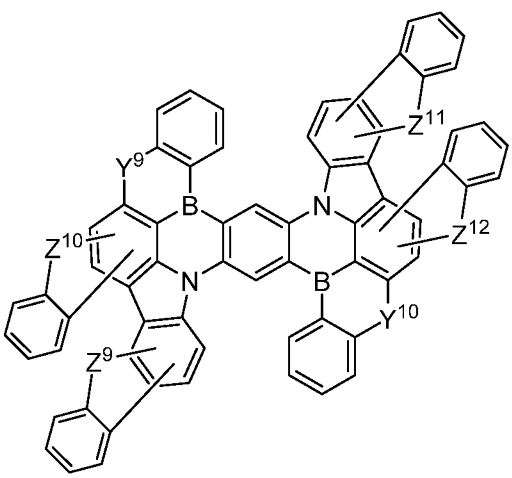

Skeleton (6b)

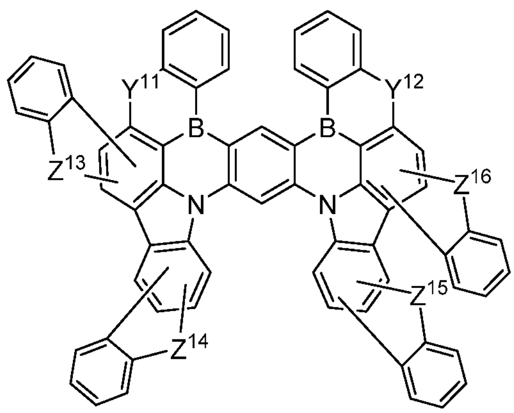

In the skeletons (6a) and (6b), each of $Y^9$ to $Y^{12}$ independently represents two hydrogen atoms, a single bond

or N($R^{27}$). Each of $Z^9$ to $Z^{16}$ independently represents an oxygen atom or a sulfur atom. It is preferable that $Z^9$ to $Z^{16}$ are the same, but they may be different. In one aspect of the invention, $Z^9$ to $Z^{16}$ are oxygen atoms. In one aspect of the invention, $Z^9$ to $Z^{16}$ are sulfur atoms. In relation to details of $Y^9$ to $Y^{12}$, corresponding descriptions for the skeletons (4a) and (4b) may be referred to. In one aspect of the invention, each hydrogen atom in the skeletons (6a) and (6b) is not substituted with a linking group together with an adjacent hydrogen atom to form a ring structure.

As one preferable group of compounds having the skeleton (6a), compounds represented by the following formula (6a) may be exemplified.

Formula (6a)

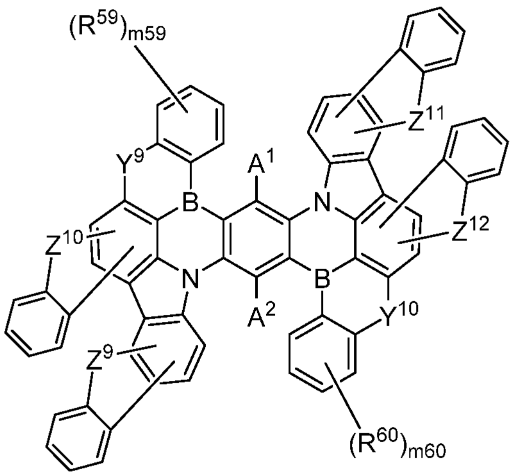

In the formula (6a), each of $R^{59}$ and $R^{60}$ independently represents a substituted or unsubstituted alkyl group. Each of m59 and m60 independently represents an integer of 0 to 4. Each of $Y^9$ and $Y^{10}$ independently represents two hydrogen atoms, a single bond or N($R^{27}$). $R^{27}$ represents a hydrogen atom, a deuterium atom, or a substituent. Each of $Z^9$ to $Z^{12}$ independently represents an oxygen atom or a sulfur atom. Each of $A^1$ and $A^2$ independently represents a hydrogen atom, a deuterium atom, or a substituent. In relation to details of $R^{59}$, $R^{60}$, m59, m60, $Z^9$ to $Z^{12}$, $A^1$, and $A^2$, descriptions on $R^{55}$, $R^{56}$, m55, m56, $A^1$, and $A^2$ in the formula (5a) and $Z^9$ to $Z^{12}$ in the skeleton (6a) may be referred to.

Hereinafter, specific examples of the compound represented by the formula (6a) will be given. Compounds of the formula (6a) that may be used in the invention are not construed as limiting to the following specific examples. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

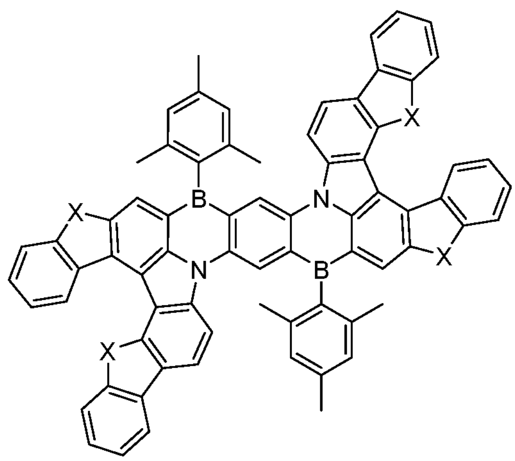

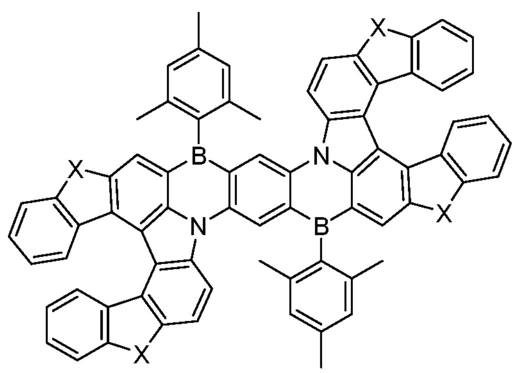

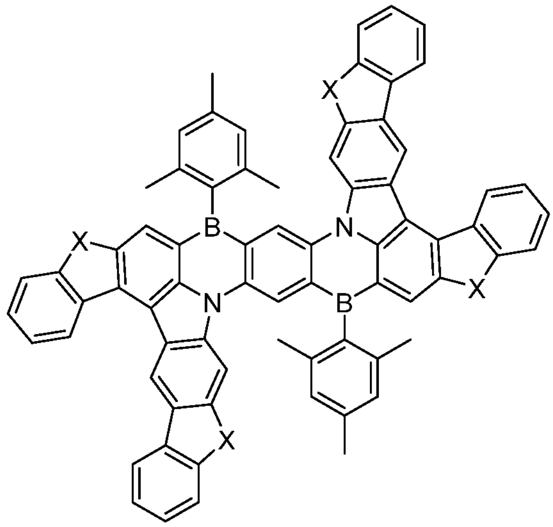

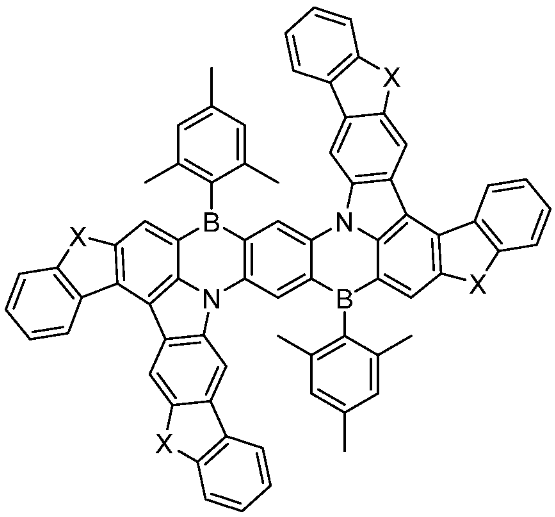

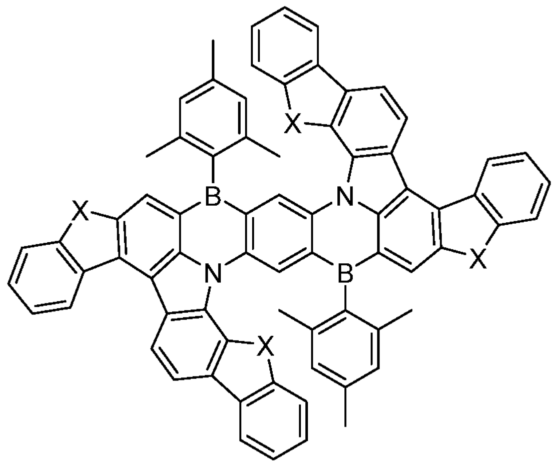

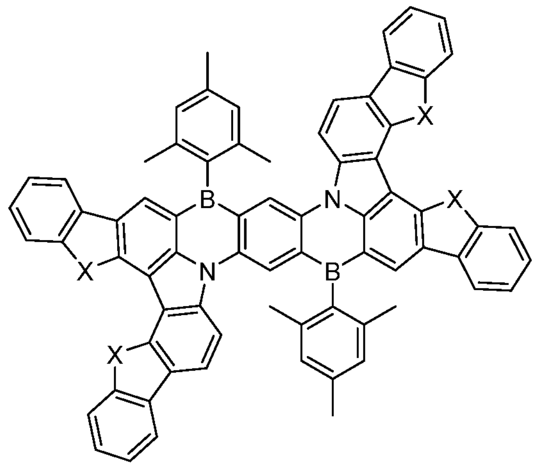

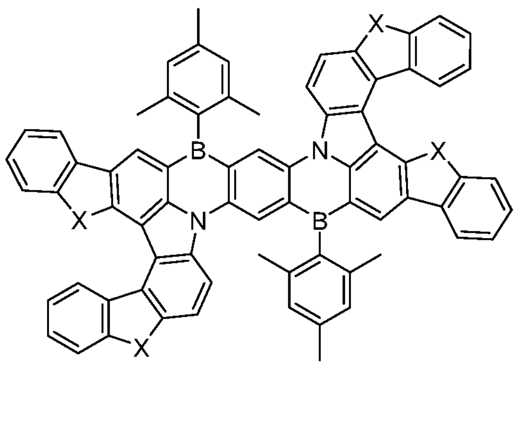

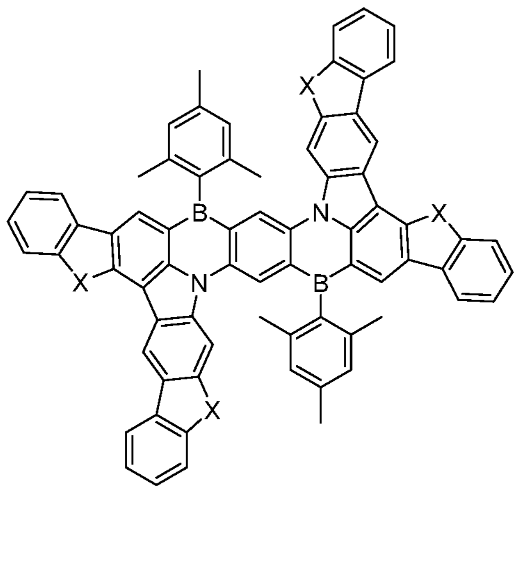

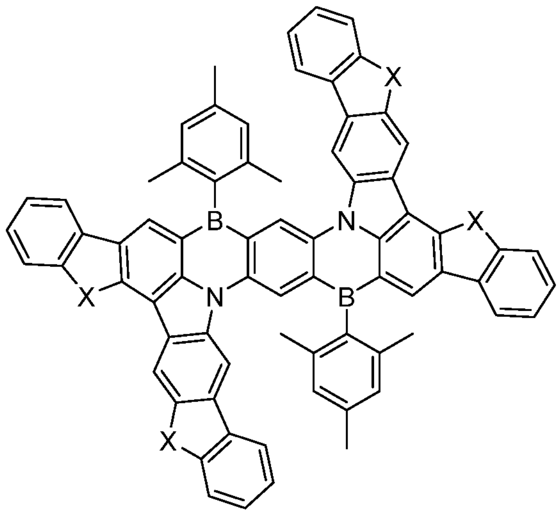

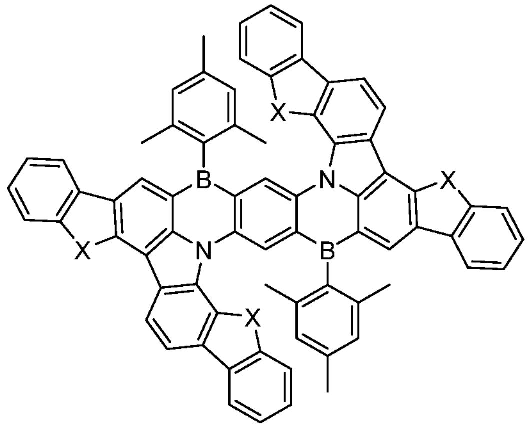

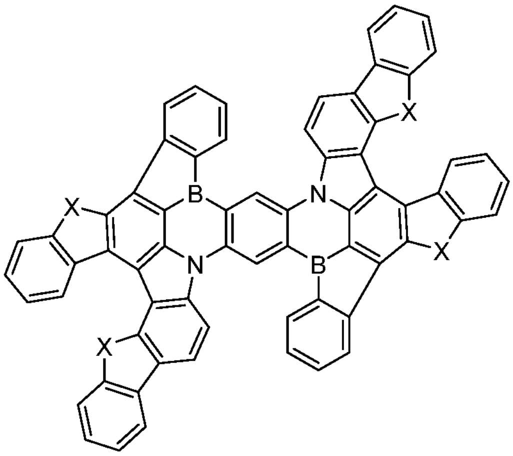

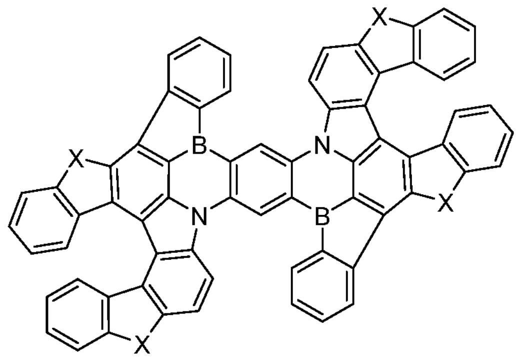

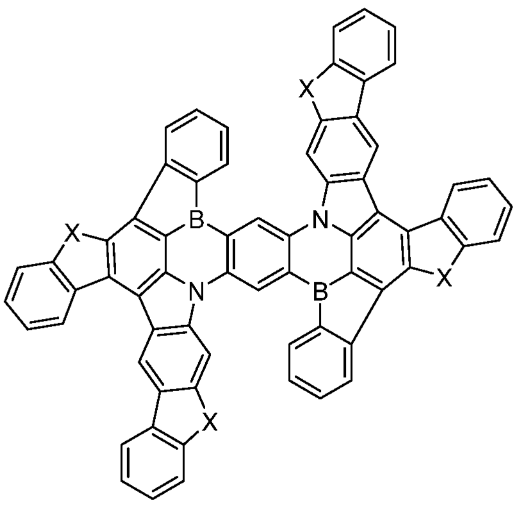

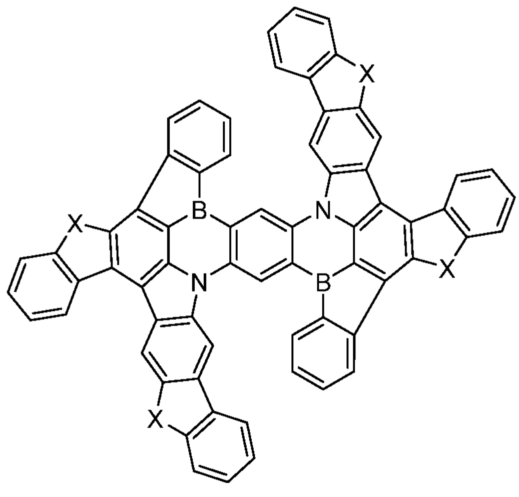

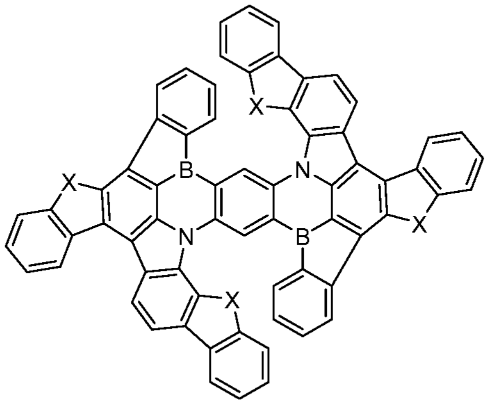

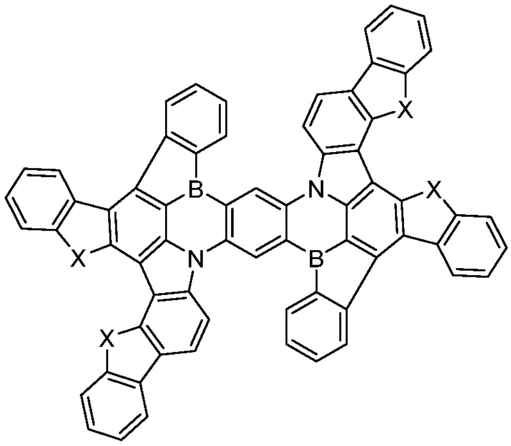

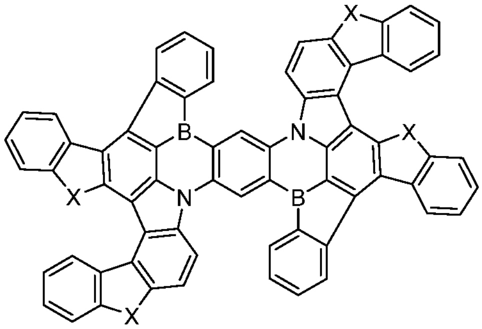

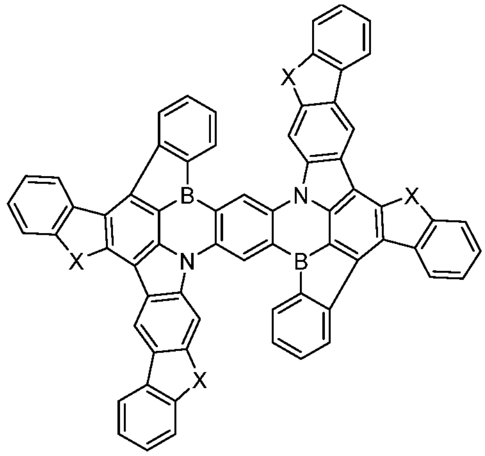

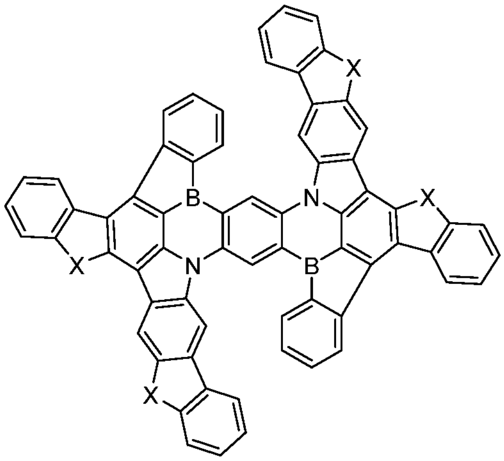

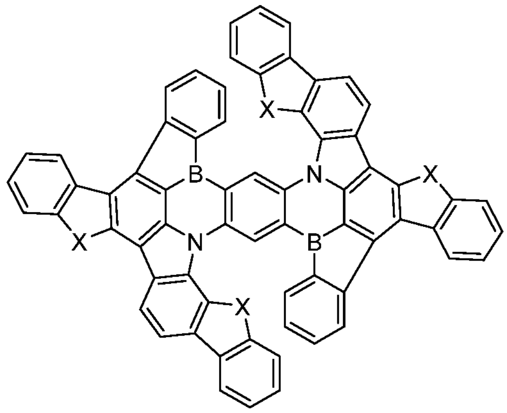

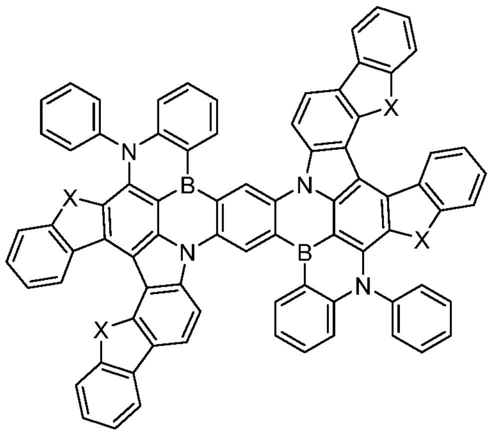

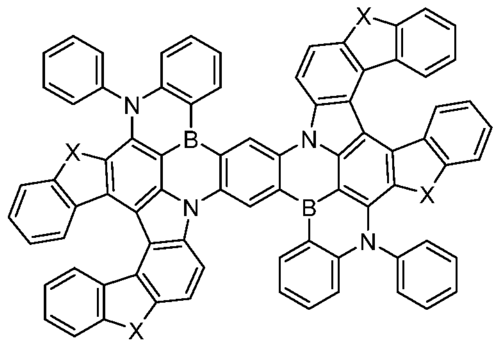

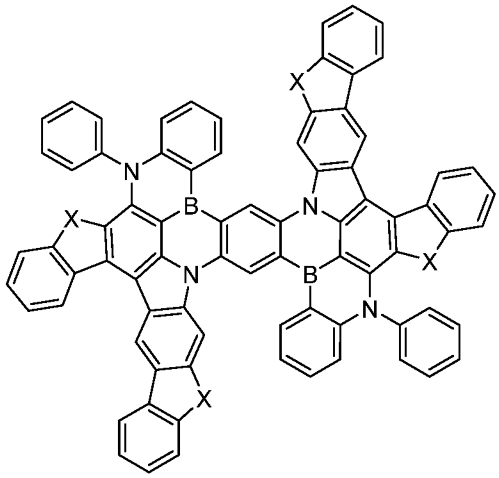

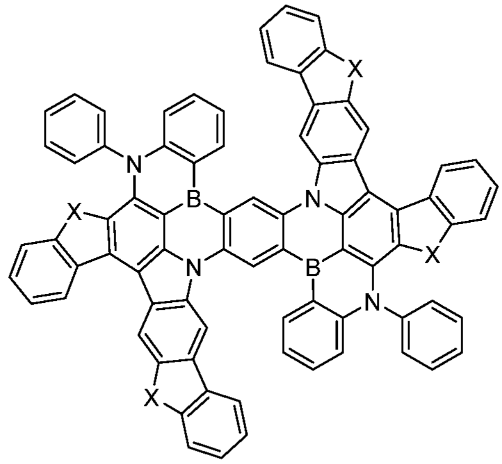

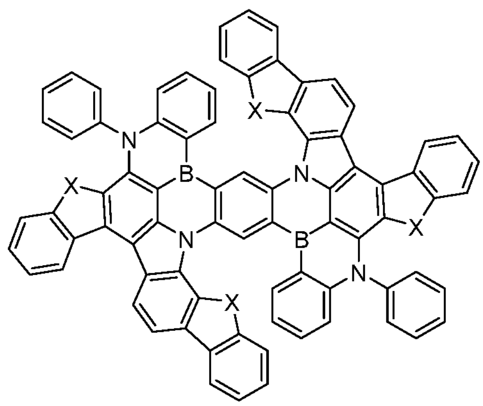

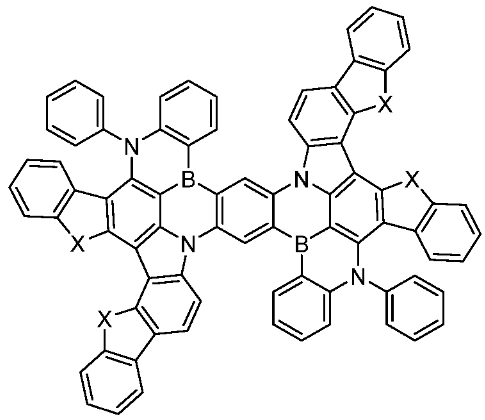

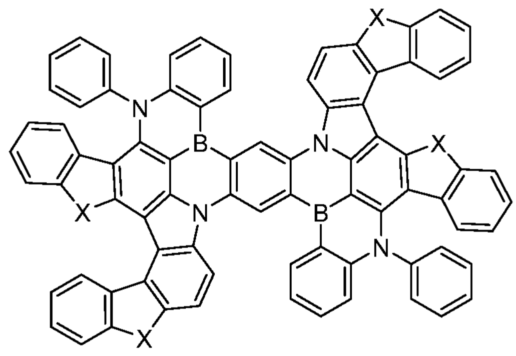

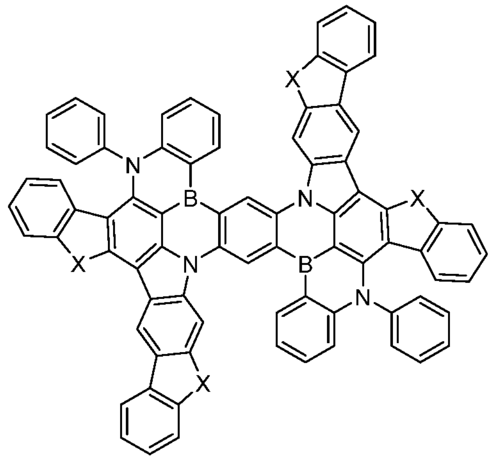

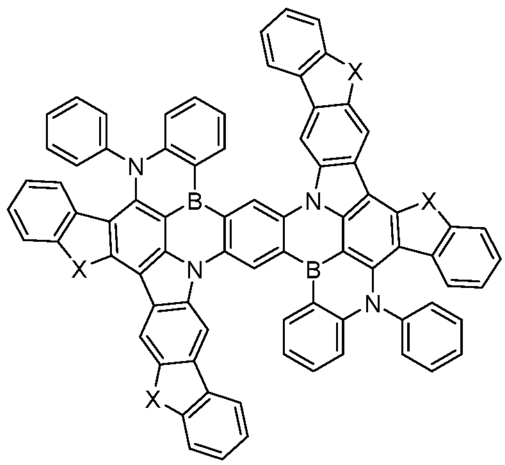

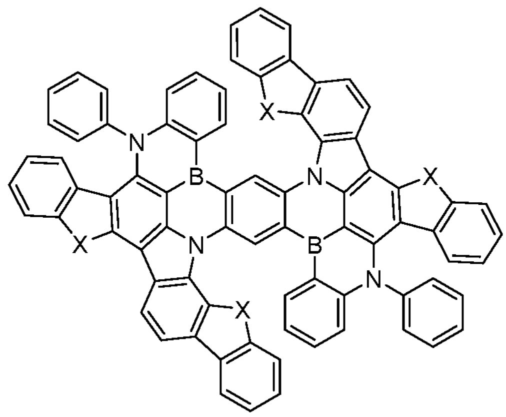

As one preferable group of compounds having the skeleton (6b), compounds represented by the following formula (6b) may be exemplified.

Formula (6b)

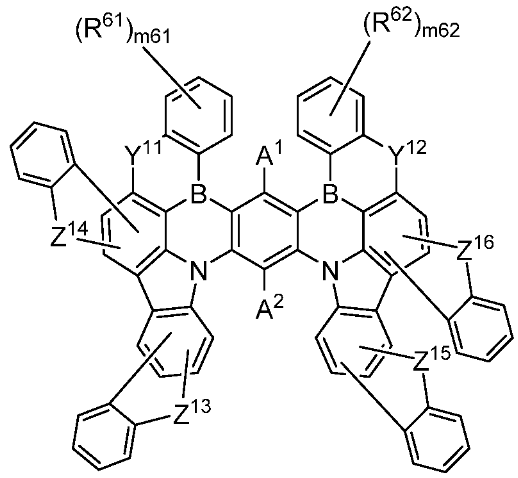

In the formula (6b), each of $R^{61}$ and $R^{62}$ independently represents a substituted or unsubstituted alkyl group. Each of m61 and m60 independently represents an integer of 0 to 4. Each of $Y^{11}$ and $Y^{12}$ independently represents two hydrogen atoms, a single bond or $N(R^{27})$. $R^{27}$ represents a hydrogen atom, a deuterium atom, or a substituent. Each of $Z^{13}$ to $Z^{16}$ independently represents an oxygen atom or a sulfur atom. Each of $A^1$ and $A^2$ independently represents

a hydrogen atom, a deuterium atom, or a substituent. In relation to details of $R^{61}$, $R^{62}$, m61, m62, $Z^{13}$ to $Z^{16}$, $A^1$, and $A^2$, descriptions on $R^{59}$, $R^{60}$, m59, m60, $A^1$, and $A^2$ in the formula (6a), and $Z^{13}$ to $Z^{16}$ in the skeleton (6b) may be referred to.

Hereinafter, specific examples of the compound represented by the formula (6b) will be given. Compounds of the formula (6b) that may be used in the invention are not construed as limiting to the following specific examples. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

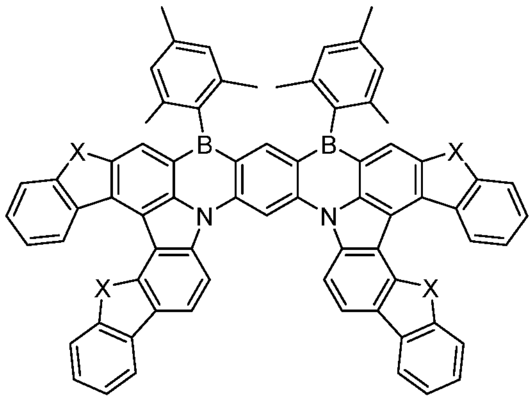

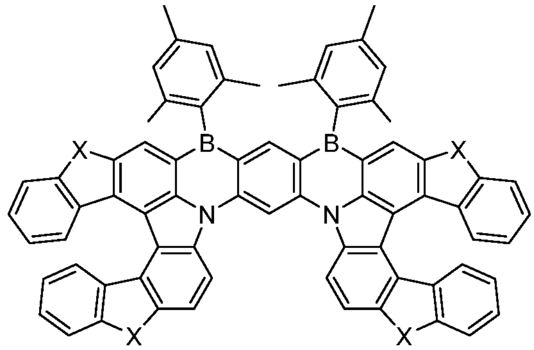

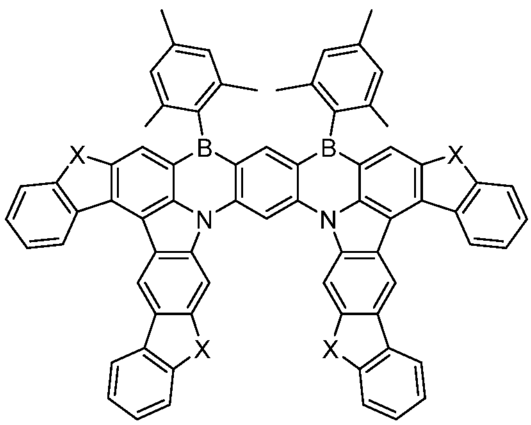

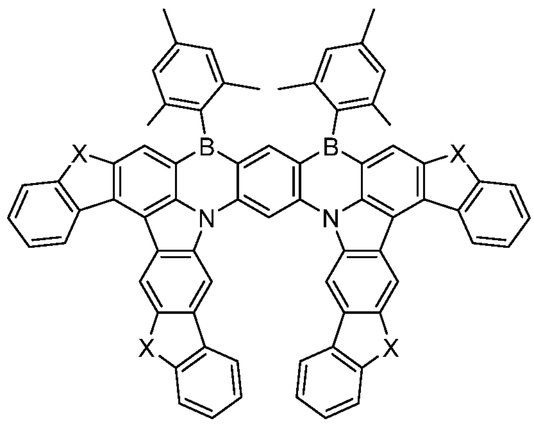

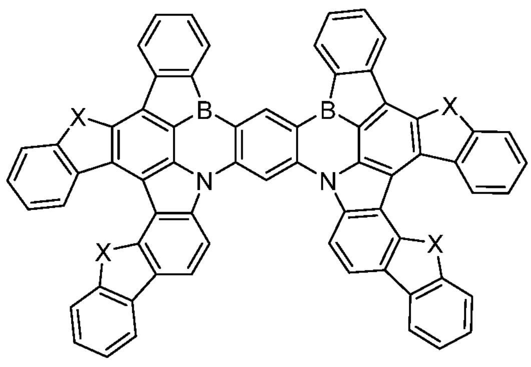

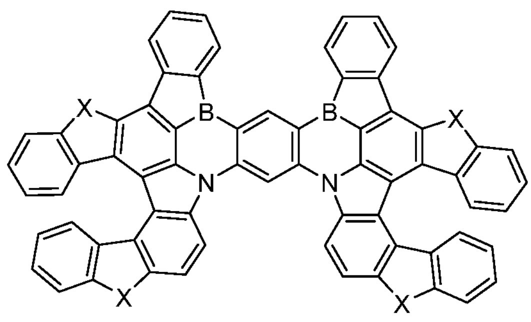

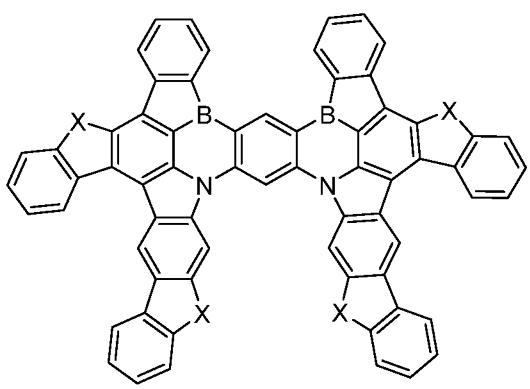

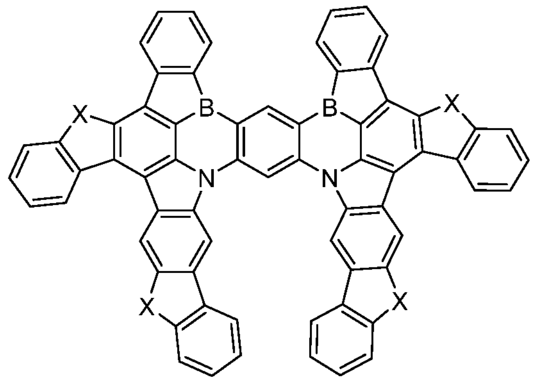

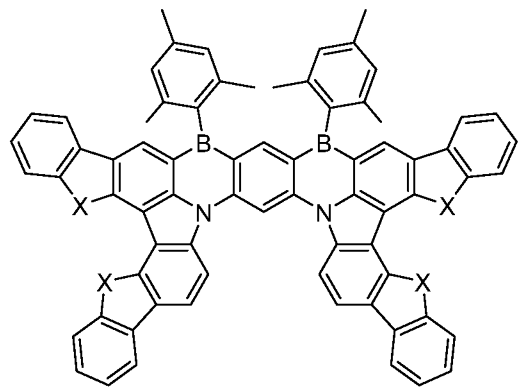

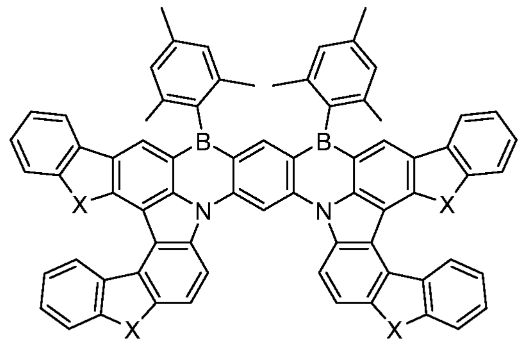

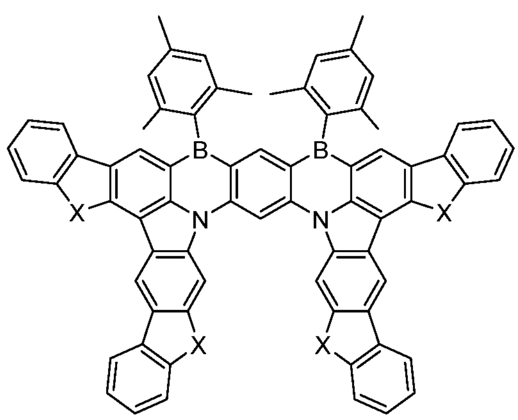

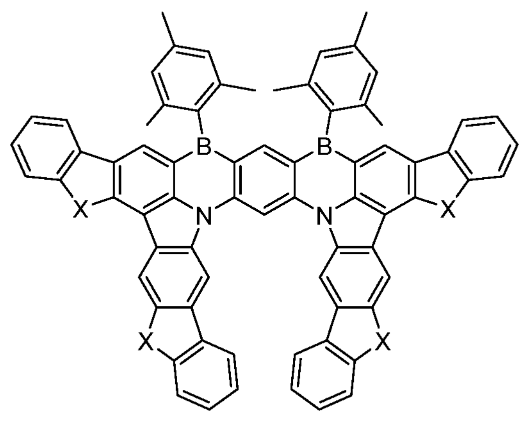

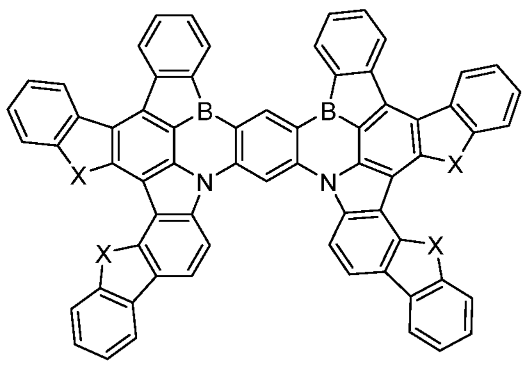

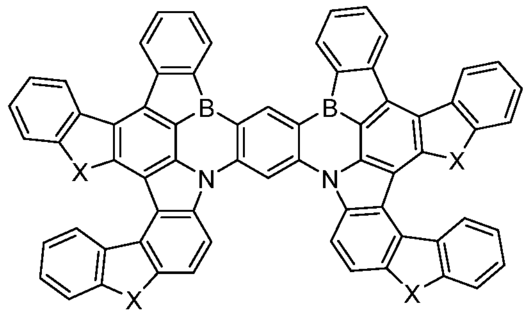

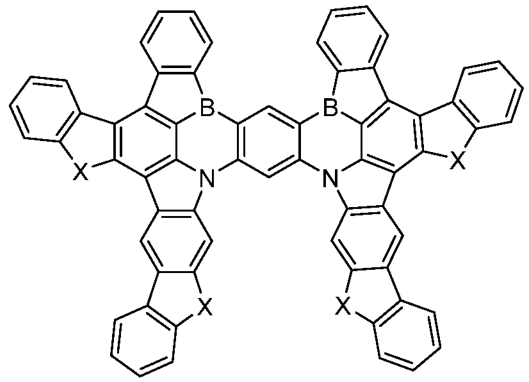

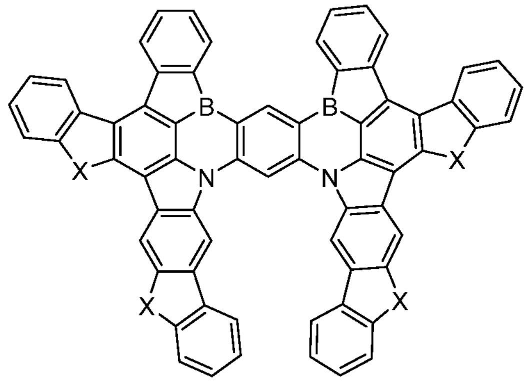

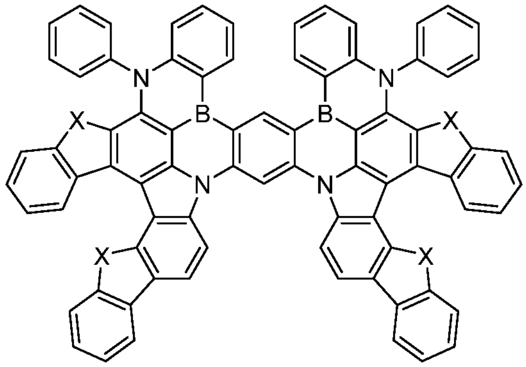

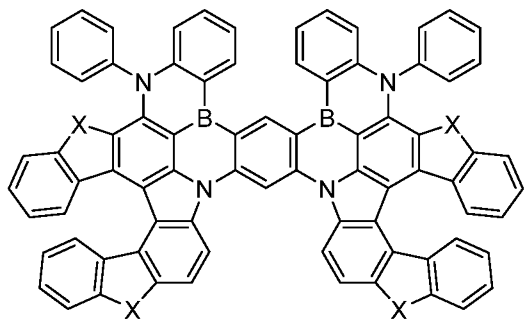

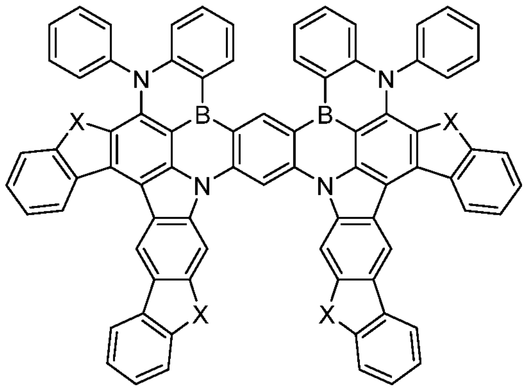

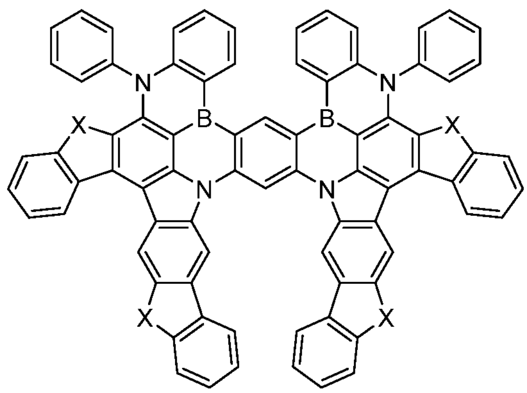

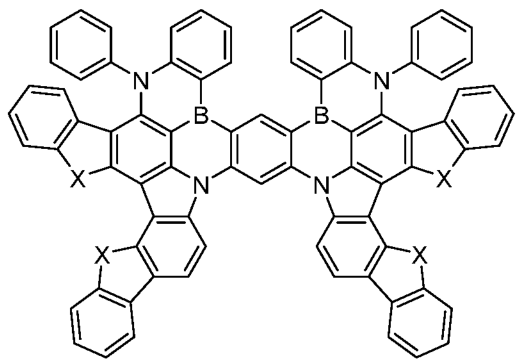

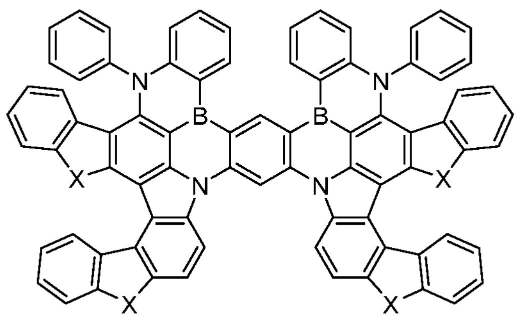

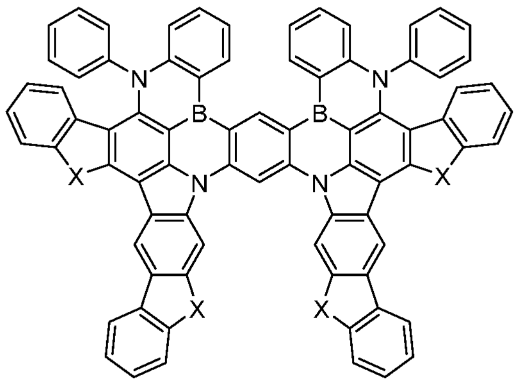

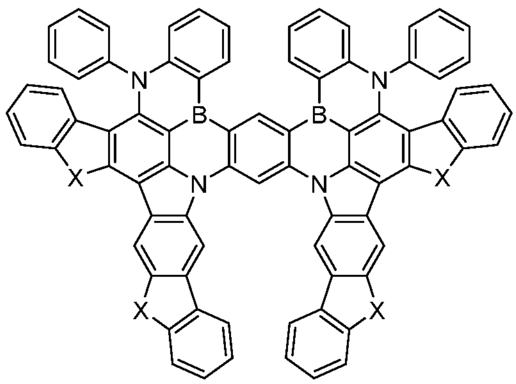

A compound in which a benzene ring is condensed with a benzene ring to which a boron atom is not directly bonded, between two benzene rings forming a carbazole partial structure existing in the formula (1), may be preferably mentioned. Examples of such a compound include a compound having the following skeleton (7a), and a compound having the following skeleton (7b).

Skeleton (7a)

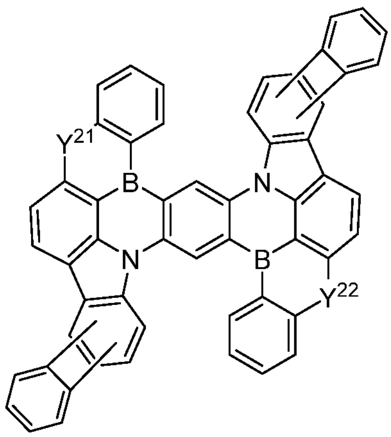

Skeleton (7b)

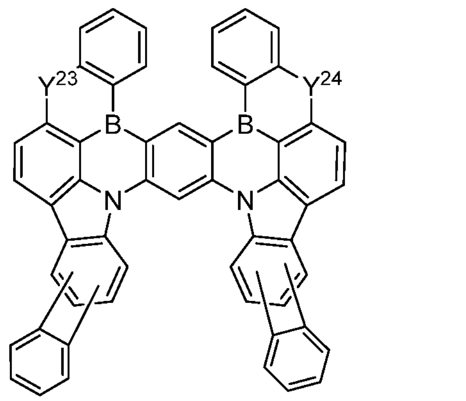

In the skeletons (7a) and (7b), each of $Y^{21}$ to $Y^{24}$ independently represents two hydrogen atoms, a single bond or $N(R^{27})$. In relation to details of $Y^{21}$ to $Y^{24}$, descriptions on $Y^1$ to $Y^4$ in the skeletons (4a) and (4b) may be referred to. In one aspect of the invention, each hydrogen atom in the skeletons (7a) and (7b) is not substituted with a linking group together with an adjacent hydrogen atom to form a ring structure.

As one preferable group of compounds having the skeleton (7a), compounds represented by the following formula (7a) may be exemplified.

Formula (7a)

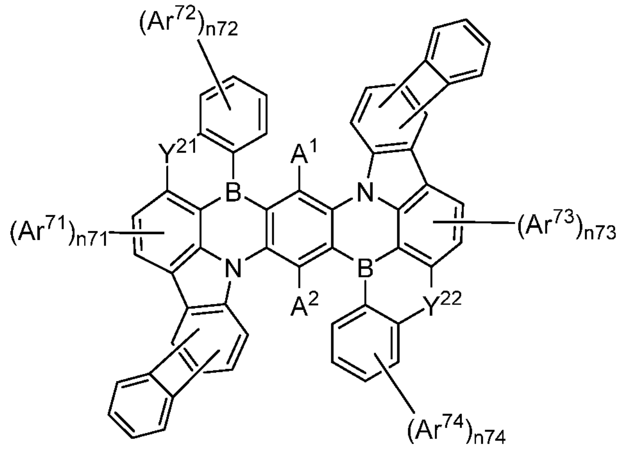

In the formula (7a), each of $Ar^{71}$ to $Ar^{74}$ independently represents a substituted or unsubstituted aryl group, a substituted or unsubstituted heteroaryl group, or a substituted or unsubstituted alkyl group, and, for example, a substituted or unsubstituted aryl group may be preferably selected. Each of n71 and n73 independently represents an integer of 0 to 2. Each of n72 and n74 independently represents an integer of 0 to 4. Each of $Y^{21}$ and $Y^{22}$ independently represents two hydrogen atoms, a single bond or $N(R^{27})$. $R^{27}$ represents a hydrogen atom, a deuterium atom, or a substituent. Each of $A^1$ and $A^2$ independently represents a hydrogen atom, a deuterium atom, or a substituent.

In one aspect of the invention, n71 to n74 are integers of 0 to 2. In one aspect of the invention, n71 and n73 are the same number, and n72 and n74 are the same number. n71 to n74 may be the same number. For example, n71 to n74 may be 0. n71 to n74 may be all 1. Further, for example, n71 and n73 may be 0, and n72 and n74 may be 1. In relation to preferable groups for $Ar^{71}$ to $Ar^{74}$, $A^1$, and $A^2$, corresponding descriptions on $Ar^1$ to $Ar^4$, $A^1$, and $A^2$ in the formula (1a) may be referred to.

Hereinafter, specific examples of the compound represented by the formula (7a) will be given. Compounds of the formula (7a) that may be used in the invention are not construed as limiting to the following specific examples.

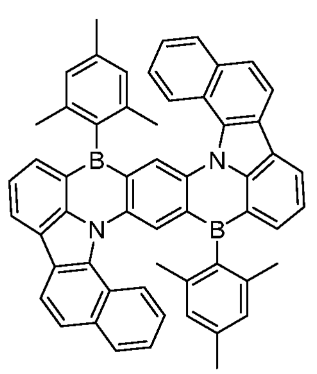
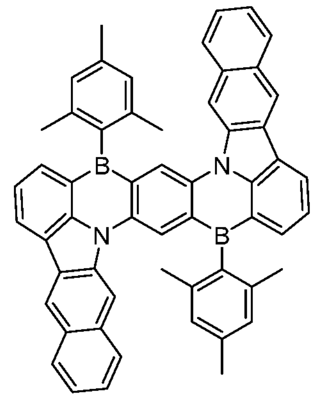
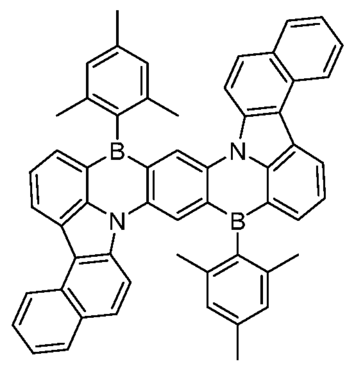

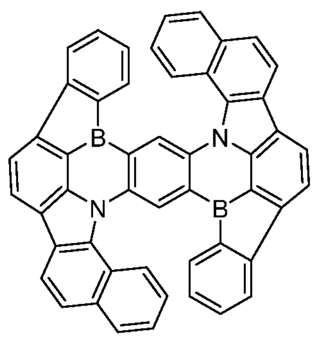
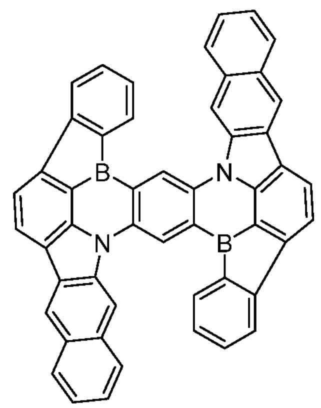
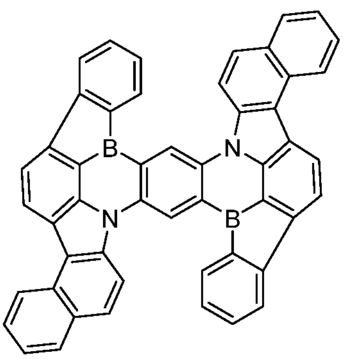

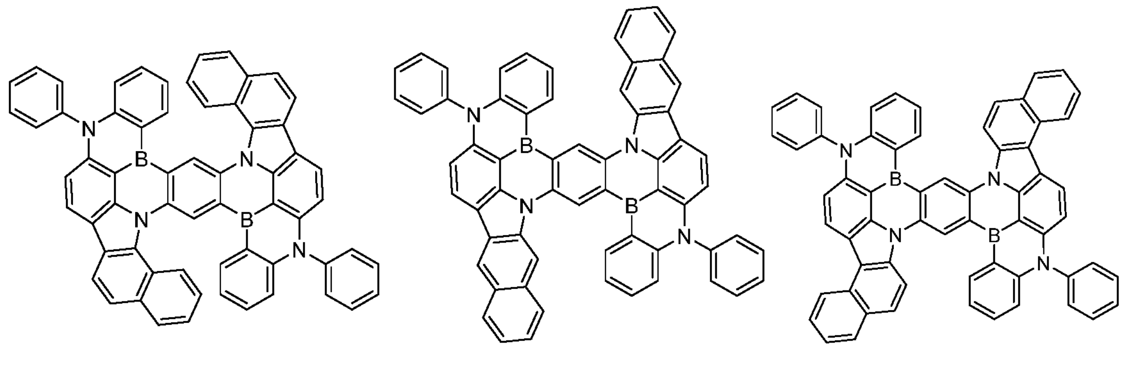

As one preferable group of compounds having the skeleton (7b), compounds represented by the following formula (7b) may be exemplified.

Formula (7b)

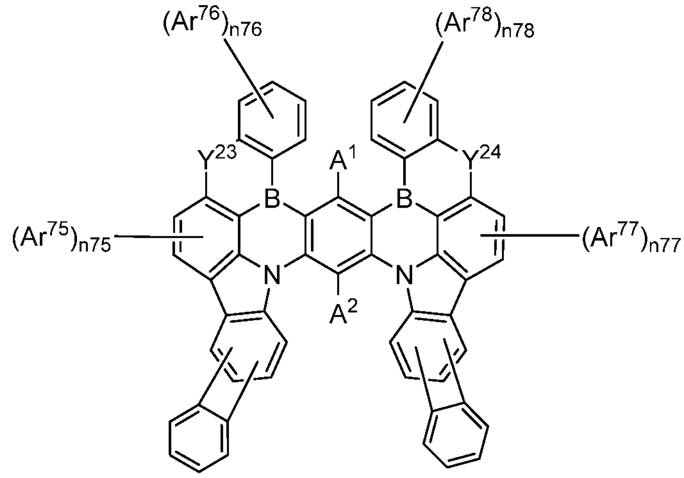

In the formula (7b), each of $Ar^{75}$ to $Ar^{78}$ independently represents a substituted or unsubstituted aryl group, a substituted or unsubstituted heteroaryl group, or a substituted or unsubstituted alkyl group, and, for example, a substituted or unsubstituted aryl group may be preferably selected. Each of n75 and n77 independently represents an integer of 0 to 2. Each of n76 and n78 independently represents an integer of 0 to 4. Each of $Y^{23}$ and $Y^{24}$ independently represents two hydrogen atoms, a single bond or $N(R^{27})$. $R^{27}$ represents a hydrogen atom, a deuterium atom, or a substituent. For detailed descriptions of n75 to n78, descriptions on n71 to n74 in the formula (7a) may be referred to in this order. In relation to preferable groups for $Ar^{75}$ to $Ar^{78}$, corresponding descriptions on $Ar^1$ to $Ar^4$ in the formula (1a) may be referred to.

Hereinafter, specific examples of the compound represented by the formula (7b) will be given. Compounds of the formula (7b) that may be used in the invention are not construed as limiting to the following specific examples.

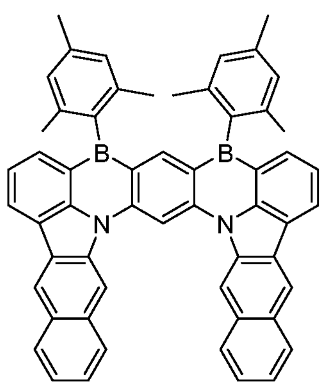

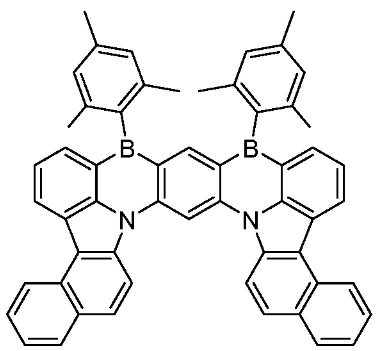

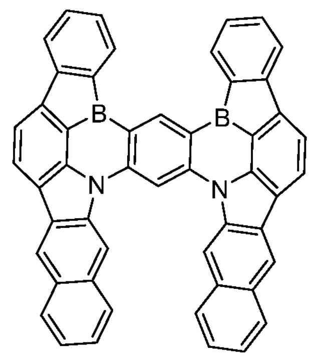

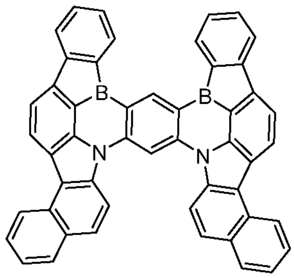
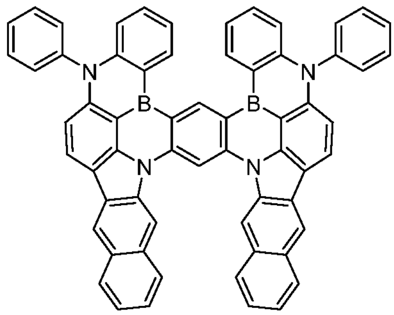
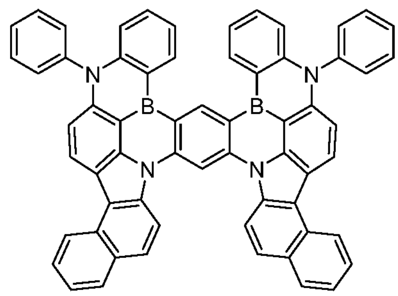

A compound in which a benzene ring is condensed with a benzene ring to which a boron atom is directly bonded, between two benzene rings forming a carbazole partial structure existing in the formula (1), may be preferably mentioned. Examples of such a compound include a compound having the following skeleton (8a), and a compound having the following skeleton (8b).

Skeleton (8a)

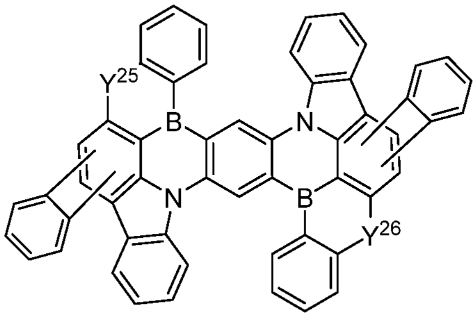

Skeleton (8b)

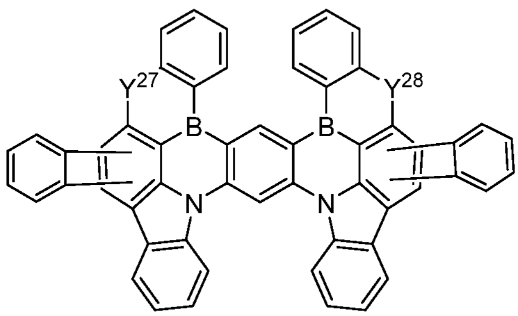

In the skeletons (8a) and (8b), each of $Y^{25}$ to $Y^{28}$ independently represents two hydrogen atoms, a single bond or $N(R^{27})$. In relation to details of $Y^{25}$ to $Y^{28}$, corresponding descriptions for the skeletons (4a) and (4b) may be referred to. In one aspect of the invention, each hydrogen atom in the skeletons (8a) and (8b) is not substituted with a linking group together with an adjacent hydrogen atom to form a ring structure.

As one preferable group of compounds having the skeleton (8a), compounds represented by the following formula (8a) may be exemplified.

Formula (8a)

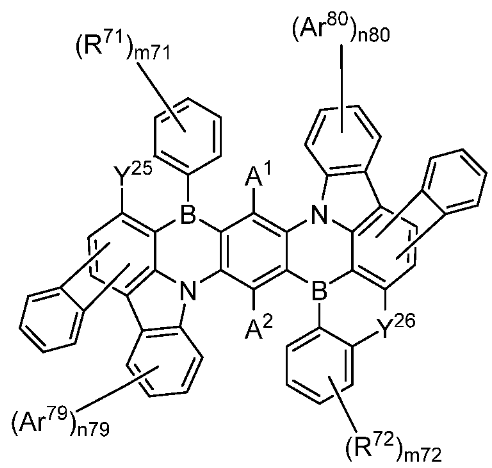

In the formula (8a), each of $Ar^{79}$ and $Ar^{80}$ independently represents a substituted or unsubstituted aryl group, a substituted or unsubstituted heteroaryl group, or a substituted or unsubstituted alkyl group, and, for example, a substituted or unsubstituted aryl group may be preferably selected. Each of $R^{71}$ and $R^{72}$ independently represents a sub-

stituted or unsubstituted alkyl group. Each of m71 and m72 independently represents an integer of 0 to 4. Each of n79 and n80 independently represents an integer of 0 to 4. Each of $Y^{25}$ and $Y^{26}$ independently represents two hydrogen atoms, a single bond or $N(R^{27})$. $R^{27}$ represents a hydrogen atom, a deuterium atom, or a substituent. Each of $A^1$ and $A^2$ independently represents a hydrogen atom, a deuterium atom, or a substituent.

In one aspect of the invention, n79 and n80 are integers of 0 to 2. In one aspect of the invention, n79 and n80 are the same number, and for example, may be all 0, or may be all 1. In one aspect of the invention, m71 and m72 are integers of 0 to 2. In one aspect of the invention, m71 and m72 are the same number, and for example, may be all 0, or may be all 1. In relation to preferable groups for $Ar^{79}$, $Ar^{80}$, $R^{71}$, $R^{72}$, $A^1$, and $A^2$, corresponding descriptions on $Ar^1$, $Ar^3$, $R^{41}$, $R^{42}$, $A^1$, and $A^2$ in the formula (1a) may be referred to.

Hereinafter, specific examples of the compound represented by the formula (8a) will be given. Compounds of the formula (8a) that may be used in the invention are not construed as limiting to the following specific examples.

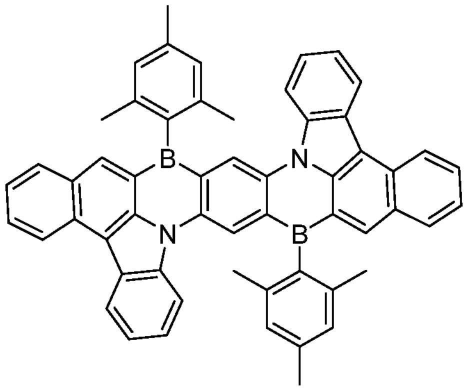

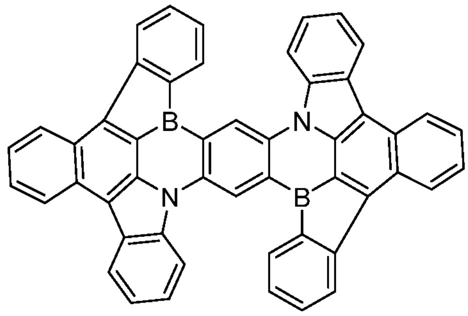

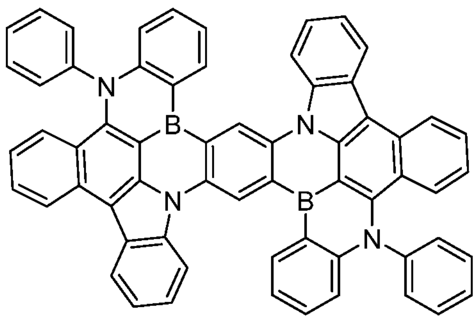

As one preferable group of compounds having the skeleton (8b), compounds represented by the following formula (8b) may be exemplified.

Formula (8b)

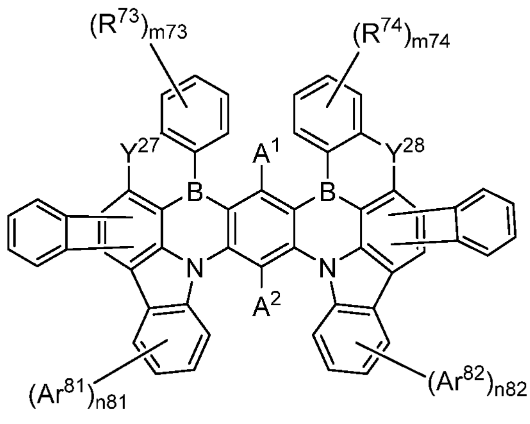

In the formula (8b), each of $Ar^{81}$ and $Ar^{82}$ independently represents a substituted or unsubstituted aryl group, a substituted or unsubstituted heteroaryl group, or a substituted or unsubstituted alkyl group, and, for example, a substituted or unsubstituted aryl group may be preferably selected. Each of $R^{73}$ and $R^{74}$ independently represents a substituted or unsubstituted alkyl group. Each of m73 and m74 independently represents an integer of 0 to 4. Each of n81 and n82 independently represents an integer of 0 to 4. Each of $Y^{27}$ and $Y^{28}$ independently represents two hydrogen atoms, a single bond or $N(R^{27})$. $R^{27}$ represents a hydrogen atom, a deuterium atom, or a substituent. Each of $A^1$ and $A^2$ independently represents a hydrogen atom, a deuterium atom, or a substituent.

In relation to detailed descriptions of m73, m74, n81, and n82, descriptions on m71, m72, n79, and n80 in the formula (8a) may be referred to. In relation to preferable groups for $Ar^{81}$, $Ar^{82}$, $R^{73}$, $R^{74}$, $A^1$, and $A^2$, corresponding descriptions on $Ar^1$, $Ar^3$, $R^{41}$, $R^{42}$, $A^1$, and $A^2$ in the formula (1a) may be referred to.

Hereinafter, specific examples of the compound represented by the formula (8b) will be given. Compounds of the formula (8b) that may be used in the invention are not construed as limiting to the following specific examples.

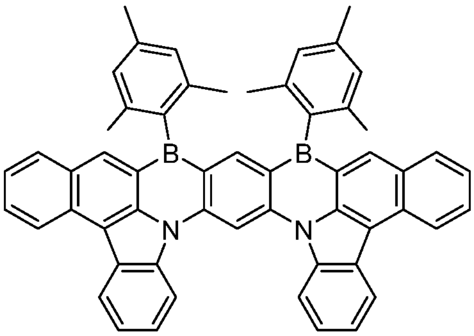

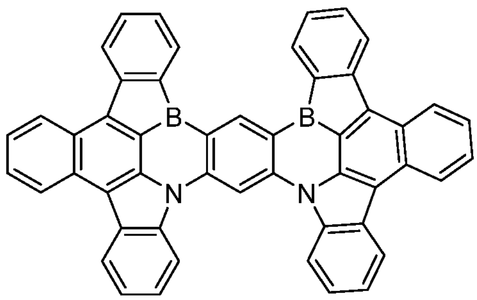

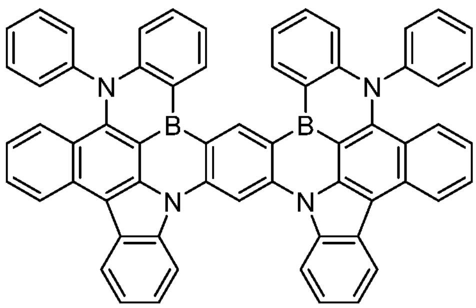

A compound in which benzene rings are condensed with both of two benzene rings forming a carbazole partial structure existing in the formula (1) may be preferably mentioned. Examples of such a compound include a compound having the following skeleton (9a), and a compound having the following skeleton (9b).

Skeleton (9a)

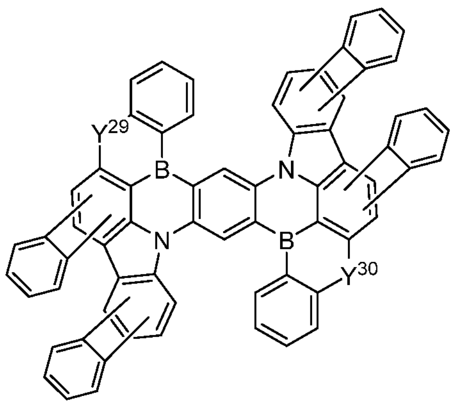

Skeleton (9b)

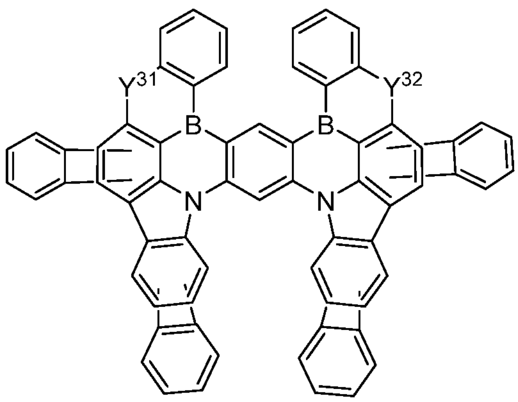

In the skeletons (9a) and (9b), each of $Y^{29}$ to $Y^{32}$ independently represents two hydrogen atoms, a single bond or $N(R^{27})$. In relation to details of $Y^{29}$ to $^{y32}$, corresponding descriptions for the skeletons (4a) and (4b) may be referred

to. In one aspect of the invention, each hydrogen atom in the skeletons (9a) and (9b) is not substituted with a linking group together with an adjacent hydrogen atom to form a ring structure.

As one preferable group of compounds having the skeleton (9a), compounds represented by the following formula (9a) may be exemplified.

Formula (9a)

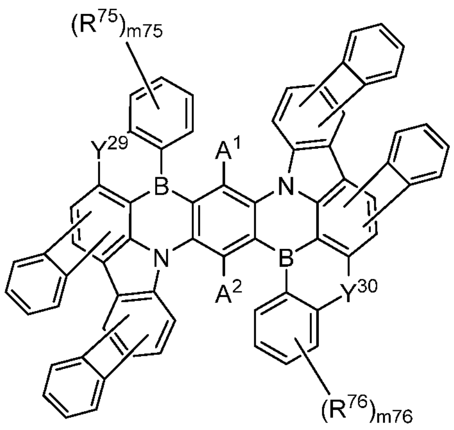

In the formula (9a), each of $R^{75}$ and $R^{76}$ independently represents a substituted or unsubstituted alkyl group. Each of m75 and m76 independently represents an integer of 0 to 4. Each of $Y^{29}$ and $Y^{30}$ independently represents two hydrogen atoms, a single bond or $N(R^{27})$. $R^{27}$ represents a hydrogen atom, a deuterium atom, or a substituent. Each of $A^1$ and $A^2$ independently represents a hydrogen atom, a deuterium atom, or a substituent. In relation to details of $R^{75}$, $R^{76}$, m75, m76, $A^1$, and $A^2$, descriptions on $R^{71}$, $R^{72}$, m71, m72, $A^1$, and $A^2$ in the formula (8a) may be referred to.

Hereinafter, specific examples of the compound represented by the formula (9a) will be given. Compounds of the formula (9a) that may be used in the invention are not construed as limiting to the following specific examples.

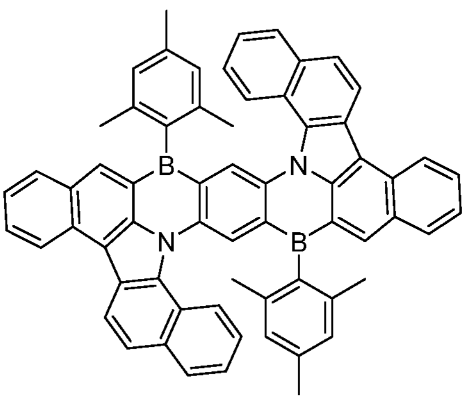

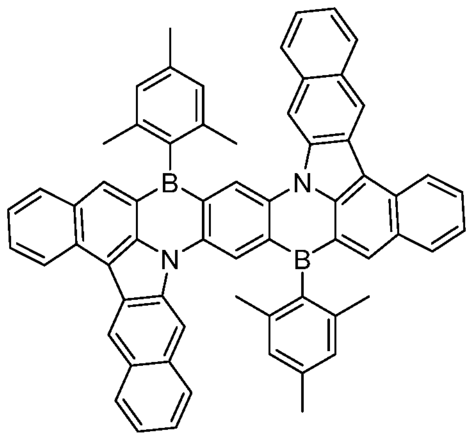

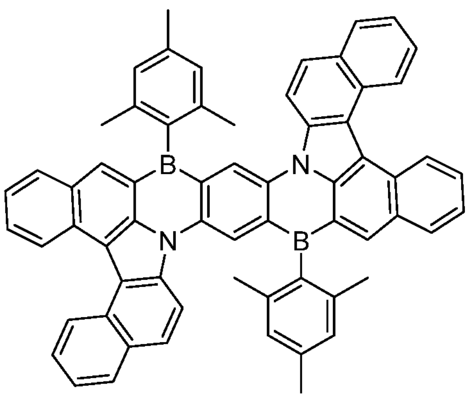

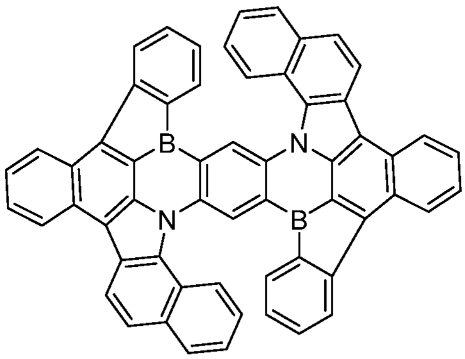

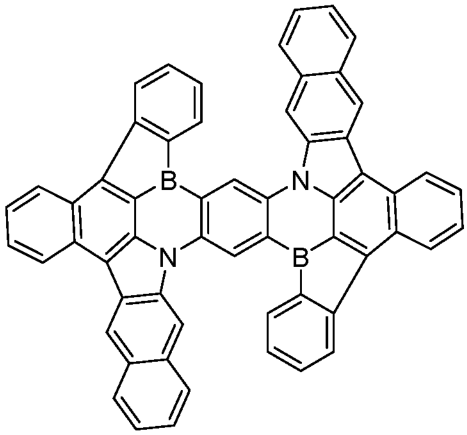

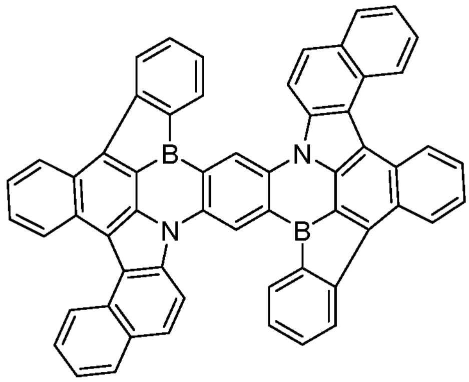

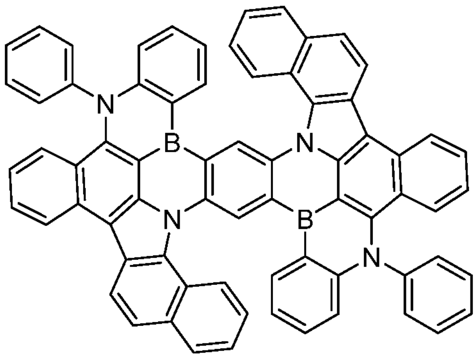

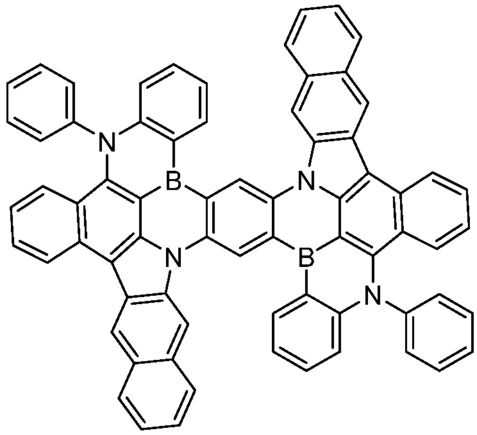

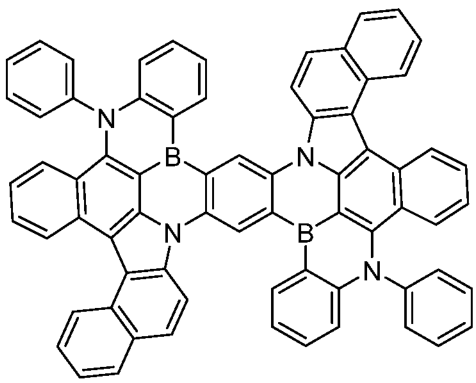

As one preferable group of compounds having the skeleton (9b), compounds represented by the following formula (9b) may be exemplified.

Formula (9b)

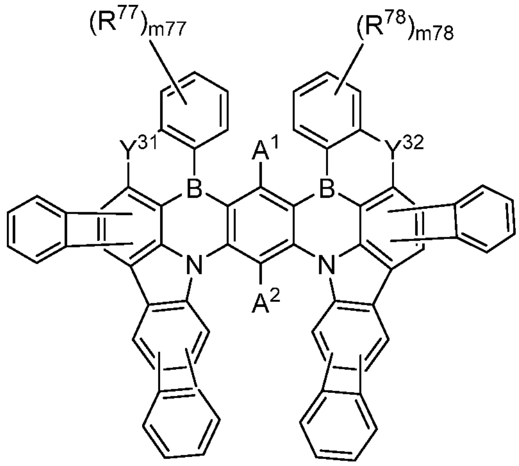

In the formula (9b), each of $R^{77}$ and $R^{78}$ independently represents a substituted or unsubstituted alkyl group. Each of m77 and m78 independently represents an integer of 0 to 4. Each of $Y^{31}$ and $Y^{32}$ independently represents two hydrogen atoms, a single bond or $N(R^{27})$. $R^{27}$ represents a hydrogen atom, a deuterium atom, or a substituent. Each of $A^1$ and $A^2$ independently represents a hydrogen atom, a deuterium atom, or a substituent. In relation to details of $R^{77}$, $R^{78}$, m77, m78, $A^1$, and $A^2$, descriptions on $R^{71}$, $R^{72}$, m71, m72, $A^1$, and $A^2$ in the formula (8a) may be referred to.

Hereinafter, specific examples of the compound represented by the formula (9b) will be given. Compounds of the formula (9b) that may be used in the invention are not construed as limiting to the following specific examples.

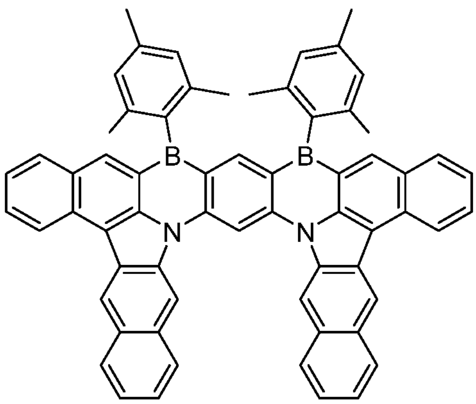

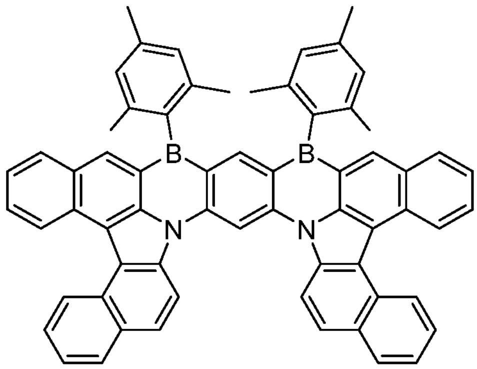

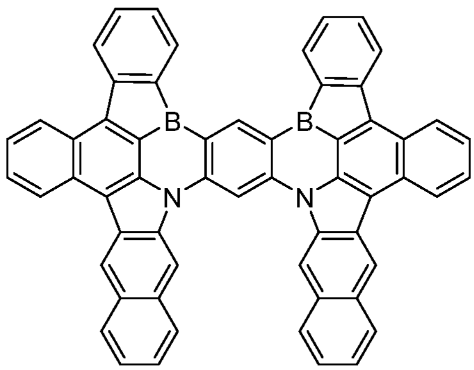

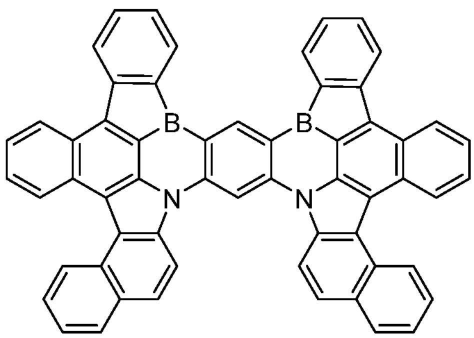

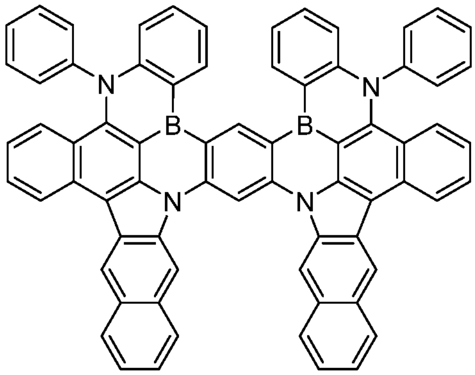

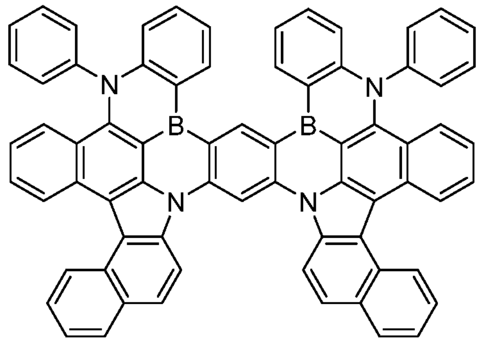

As the compound represented by the formula (1), a compound in which four or more carbazole partial structures are included in the molecule is also preferable. As an example of such a compound, a compound having the following skeleton (10) may be exemplified.

Skeleton (10)

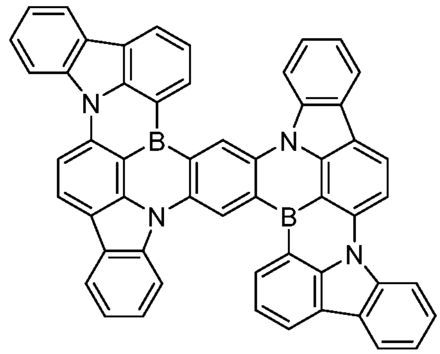

Each hydrogen atom in the skeleton (10) may be substituted with a deuterium atom or a substituent. Further, it may be substituted with a linking group together with an adjacent hydrogen atom to form a ring structure. For details, corresponding descriptions on $R^1$ to $R^{26}$, $A^1$, and $A^2$ in the formula (1) may be referred to. At least one hydrogen atom of a benzene ring forming a carbazole partial structure included in the skeleton (10) is substituted with a substituted or unsubstituted aryl group. In one aspect of the invention, each hydrogen atom in the skeleton (10) is not substituted with a linking group together with an adjacent hydrogen atom to form a ring structure.

As one preferable group of compounds having the skeleton (10), compounds represented by the following formula (10) may be exemplified.

Formula (10)

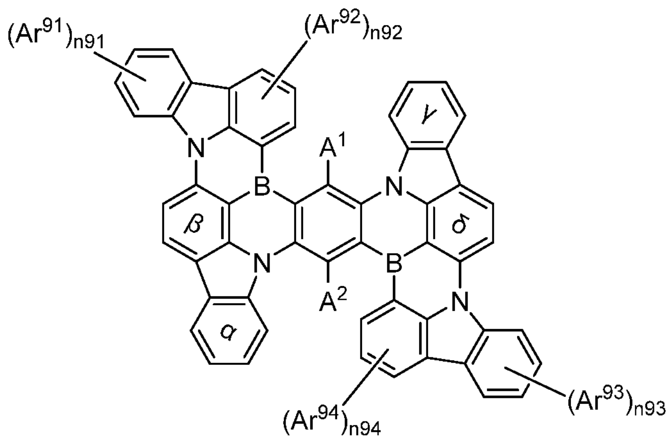

In the formula (10), each of $Ar^{91}$ to $Ar^{94}$ independently represents a substituted or unsubstituted aryl group, a

substituted or unsubstituted heteroaryl group, or a substituted or unsubstituted alkyl group, and, for example, a substituted or unsubstituted aryl group may be preferably selected. Each of n91 and n93 independently represents an integer of 0 to 4, and each of n92 and n94 independently represents an integer of 0 to 3. An $\alpha$ ring, a $\beta$ ring, a $\gamma$ ring, and a $\delta$ ring may be substituted. At least one ring is substituted with a substituted or unsubstituted aryl group, is condensed with a benzene ring that may be substituted, or is condensed with a substituted or unsubstituted furan ring of benzofuran or a substituted or unsubstituted thiophene ring of thiophene. Each of $A^1$ and $A^2$ independently represents a hydrogen atom, a deuterium atom, or a substituent.

In one aspect of the invention, n91 to n94 are integers of 0 to 2. In one aspect of the invention, n91 and n93 are the same number, and n92 and n94 are the same number. n91 to n94 may be all the same number, and for example may be all 0, or may be all 1. In relation to preferable groups for $Ar^{91}$ to $Ar^{94}$, corresponding descriptions on $Ar^1$ to $Ar^4$ in the formula (1a) may be referred to. In one aspect of the invention, the $\alpha$ ring and the $\gamma$ ring have the same substituents or have the same condensed structures, and the $\beta$ ring and the $\delta$ ring have the same substituents or have the same condensed structures. In one aspect of the invention, both the $\beta$ ring and the $\delta$ ring are substituted with substituted or unsubstituted aryl groups, are condensed with benzene rings that may be substituted, or are condensed with substituted or unsubstituted furan rings of benzofuran or substituted or unsubstituted thiophene rings of thiophene. In one aspect of the invention, both the $\alpha$ ring and the $\gamma$ ring are substituted with substituted or unsubstituted aryl groups, are condensed with benzene rings that may be substituted, or are condensed with substituted or unsubstituted furan rings of benzofuran or substituted or unsubstituted thiophene rings of thiophene. In one aspect of the invention, all of the $\alpha$ ring, the $\beta$ ring, the $\gamma$ ring, and the $\delta$ ring are substituted with substituted or unsubstituted aryl groups, are condensed with benzene rings that may be substituted, or are condensed with substituted or unsubstituted furan rings of benzofuran or substituted or unsubstituted thiophene rings of thiophene. In relation to descriptions and preferable ranges of $A^1$ and $A^2$, corresponding descriptions for the formula (1) may be referred to.

Hereinafter, specific examples of the compound represented by the formula (10) will be given. Compounds of the formula (10) that may be used in the invention are not construed as limiting to the following specific examples.

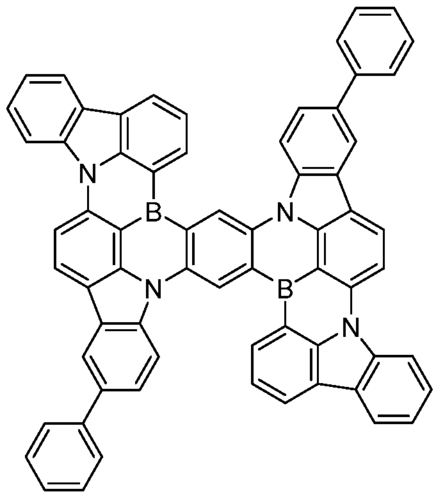

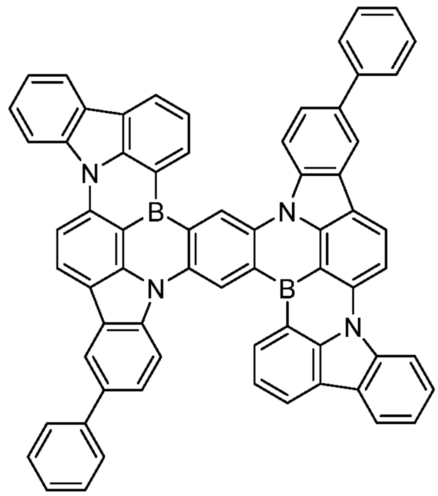

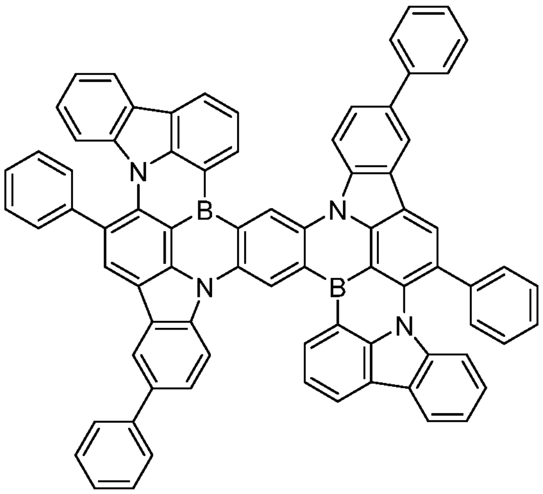

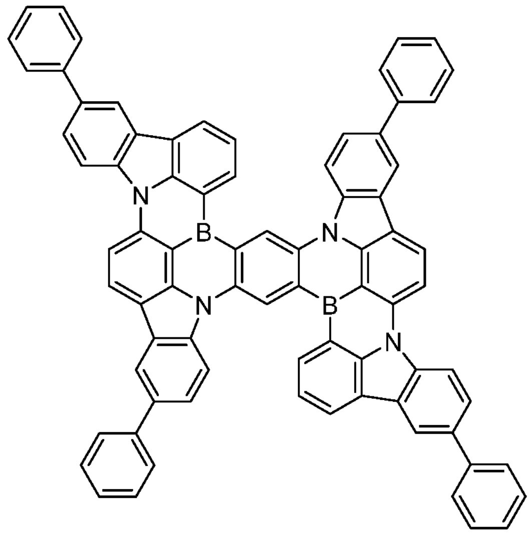

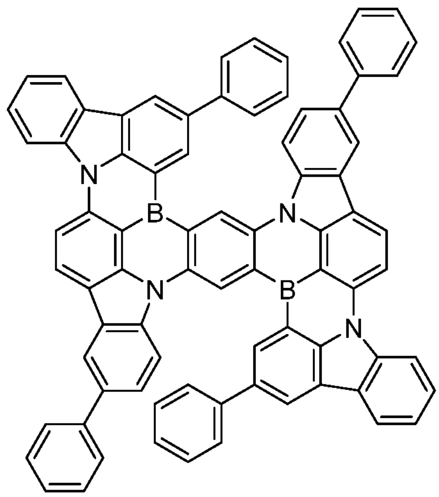

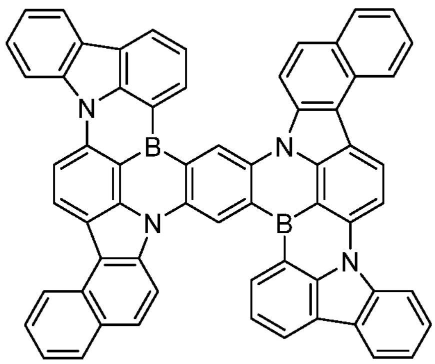

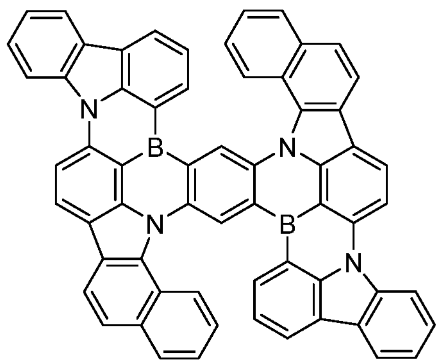

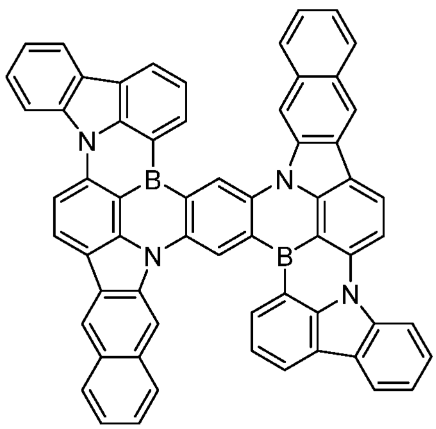

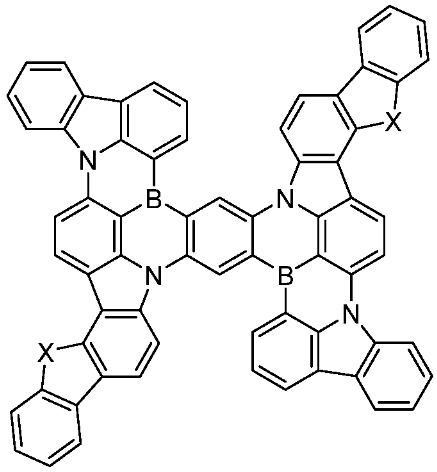

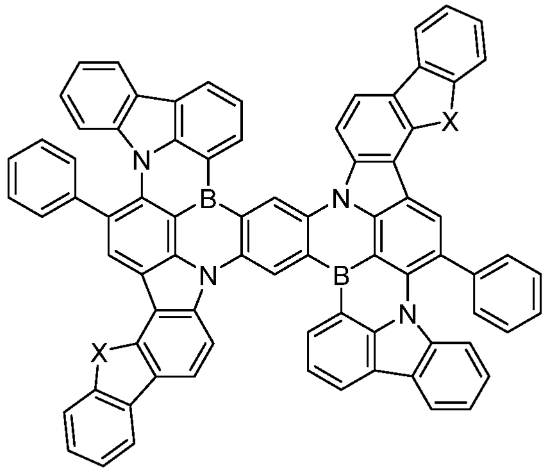

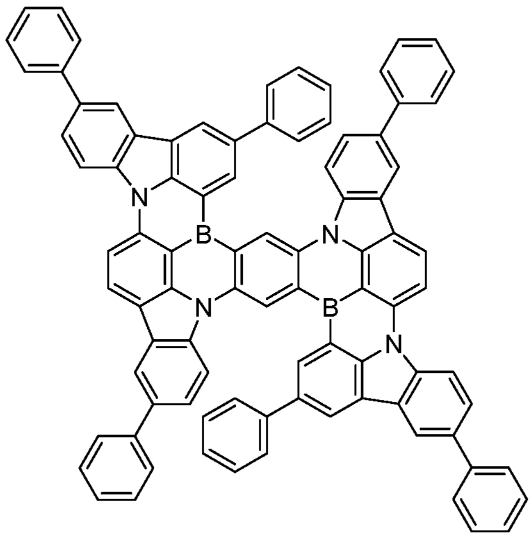

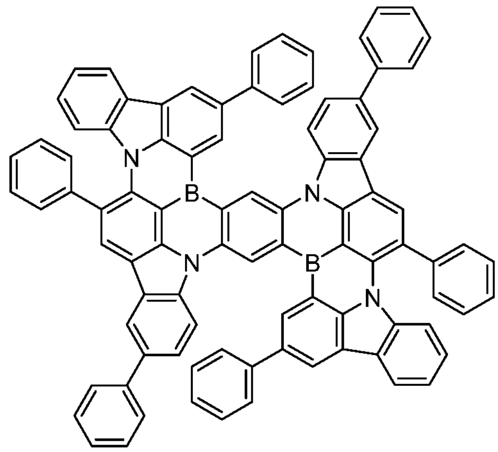

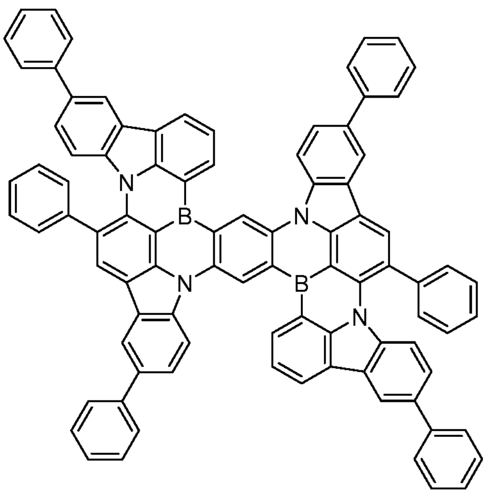

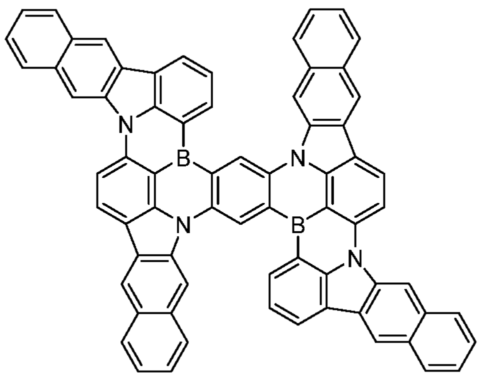

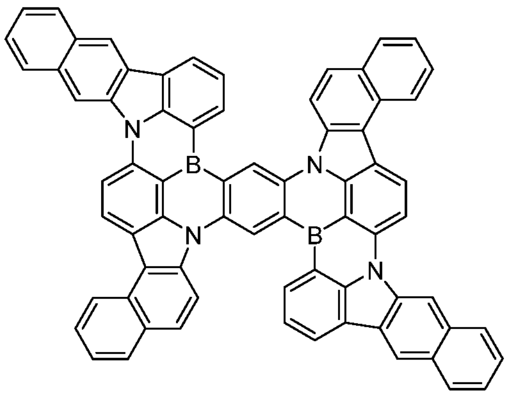

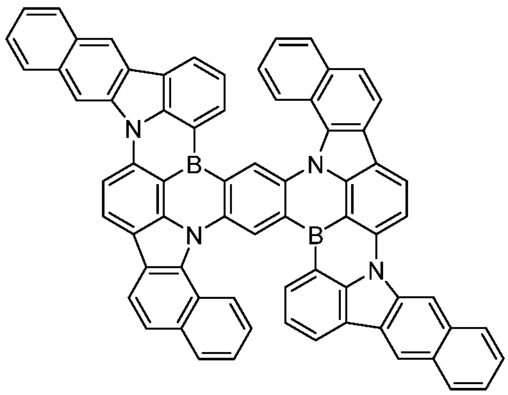

The compound represented by the formula (1) may have a skeleton having no symmetry. For example, it may be a compound having an asymmetric skeleton such as the following skeleton (11a) or the following skeleton (11b).

Skeleton (11a)

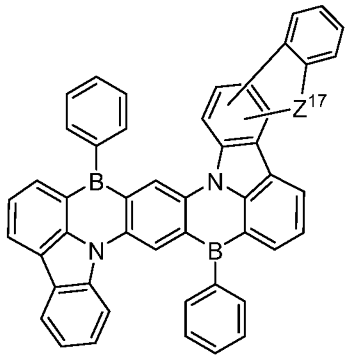

Skeleton (11b)

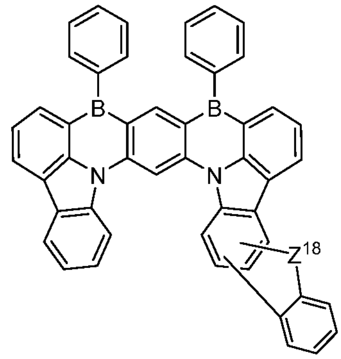

In the skeletons (11a) and (11b), each of $Z^{17}$ and $Z^{18}$ independently represents an oxygen atom or a sulfur atom. In one aspect of the invention, each hydrogen atom in the skeletons (11a) and (11b) is not substituted with a linking group together with an adjacent hydrogen atom to form a ring structure.

As one preferable group of compounds having the skeleton (11a), compounds represented by the following formula (11a) may be exemplified.

Formula (11a)

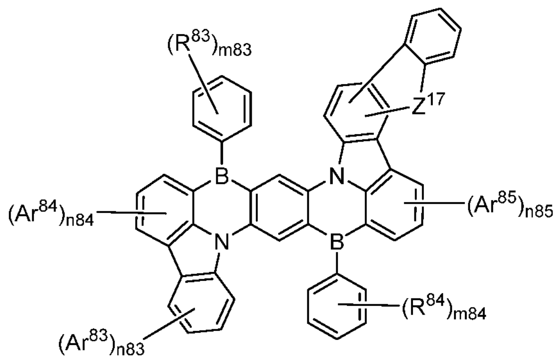

In the formula (11a), each of $Ar^{83}$ to $Ar^{85}$ independently represents a substituted or unsubstituted aryl group, a substituted or unsubstituted heteroaryl group, or a substituted or unsubstituted alkyl group, and, for example, a substituted or unsubstituted aryl group may be preferably selected. Each of $R^{83}$ and $R^{84}$ independently represents a substituted or unsubstituted alkyl group. $Z^{17}$ represents an oxygen atom or a sulfur atom. Each of m83 and m84 independently represents an integer of 0 to 5. n83 represents an integer of 0 to 4, and each of n84 and n85 independently represents an integer of 0 to 3.

For detailed descriptions and preferable ranges of $Ar^{83}$ to $Ar^{85}$, $R^{83}$, $R^{84}$, m83, m84, and n83 to n85, descriptions

on $Ar^1$, $Ar^2$, $Ar^4$, $R^{41}$, $R^{42}$, m1, m2, n1, n2, and n4 in the formula (1a) may be referred to.

Hereinafter, specific examples of the compound represented by the formula (11a) will be given. Compounds of the formula (11a) that may be used in the invention are not construed as limiting to the following specific examples. In relation to the following specific examples, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

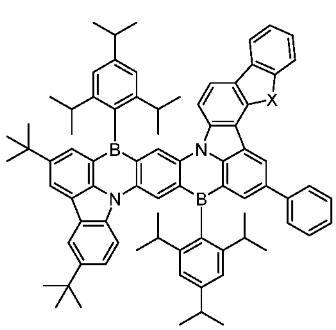

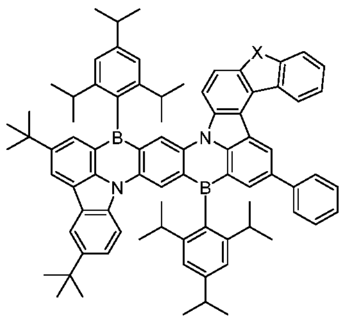

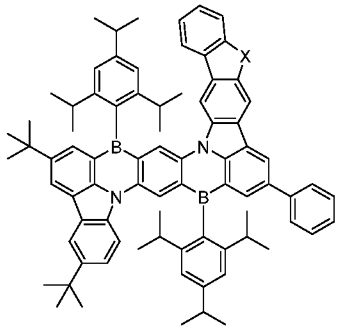

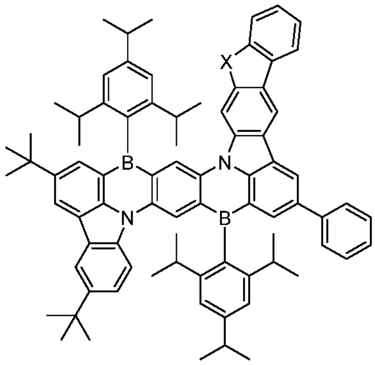

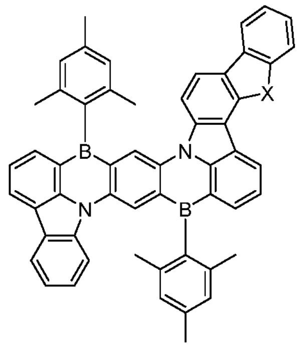

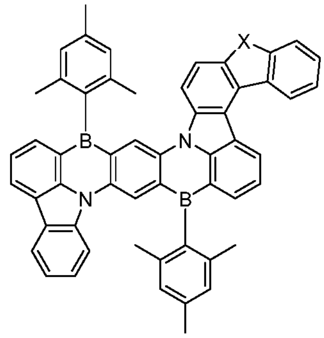

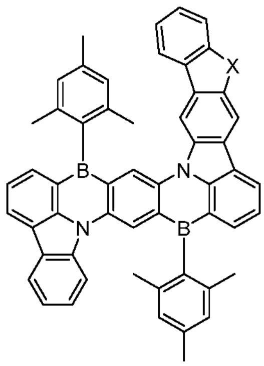

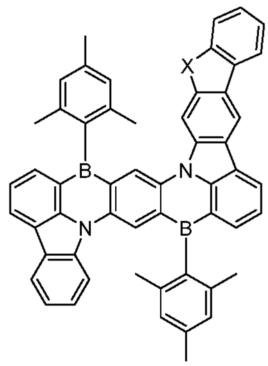

As one preferable group of compounds having the skeleton (11b), compounds represented by the following formula (11b) may be exemplified.

Formula (11b)

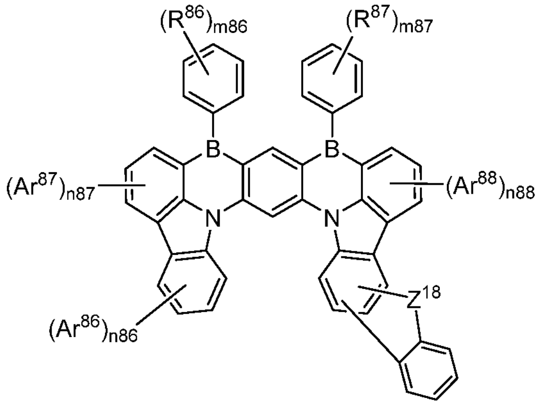

In the formula (11b), each of $Ar^{86}$ to $Ar^{88}$ independently represents a substituted or unsubstituted aryl group, a substituted or unsubstituted heteroaryl group, or a substituted or unsubstituted alkyl group, and, for example, a substituted or unsubstituted aryl group may be preferably selected. Each of $R^{86}$ and $R^{87}$ independently represents a substituted or unsubstituted alkyl group. $Z^{18}$ represents an oxygen atom or a sulfur atom. Each of m86 and m87 independently represents an integer of 0 to 5. n86 represents an integer of 0 to 4, and each of n87 and n88 independently represents an integer of 0 to 3.

For detailed descriptions and preferable ranges of $Ar^{86}$ to $Ar^{88}$, $R^{86}$, $R^{87}$, m86, m87, and n86 to n88, descriptions on $Ar^1$, $Ar^2$, $Ar^4$, $R^{41}$, $R^{42}$, m1, m2, n1, n2, and n4 in the formula (1a) may be referred to.

Hereinafter, specific examples of the compound represented by the formula (11b) will be given. Compounds of the formula (11b) that may be used in the invention are not construed as limiting to the following specific examples. In relation to the following specific examples, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms can also be adopted.

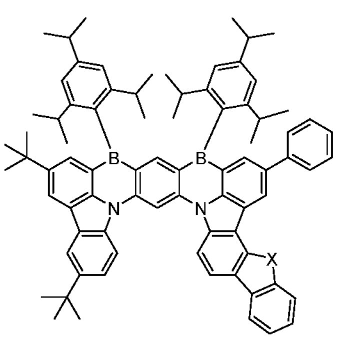
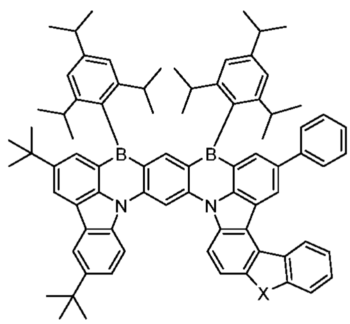
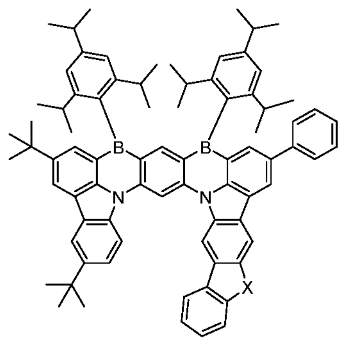

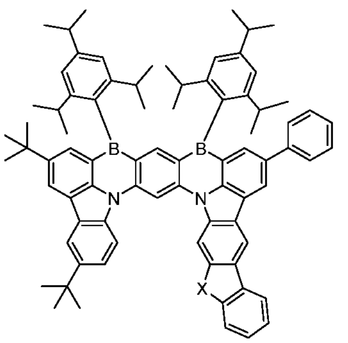
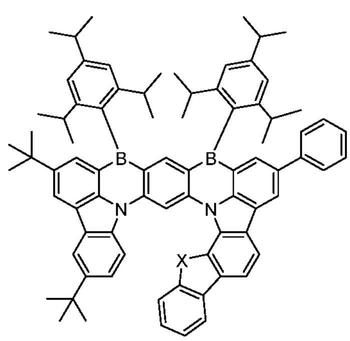
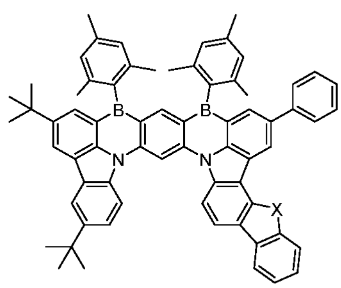

As the compound represented by the formula (1), a compound in which $R^5$ is a donor group may be preferably adopted. The compound in which $R^5$ is a donor group has a high molar coefficient extinction, and thus tends to have a high luminous efficiency. For example, it exhibits excellent luminescence characteristics as compared to a compound in which $R^3$ is a donor group. In a preferred aspect of the invention, $R^3$ is not a donor group. In a preferred aspect of the

invention, among $R^1$ to $R^7$, only $R^5$ is a donor group, or none of them is a donor group (in particular, a donor group having a σp value of -0.2 or less). The donor group is a group having a negative Hammett σp value. The σp value of the donor group for $R^5$ is preferably -0.2 or less, and may be, for example, - 0.4 or less, or may be, for example, -0.6 or less. As a preferable donor group, a substituted amino group may be mentioned, and a substituted or unsubstituted diaryl amino group is preferable. The aryl group may be a monocycle, or may be a condensed ring in which two or more rings are condensed. In the case of the condensed ring, the number of rings after the condensation is preferably two to six, and, for example, may be selected from two to four, or may be two. Two aryl groups constituting the diaryl amino group may be the same or different. Further, the two aryl groups may be linked by a single bond or a linking group. As the substituted or unsubstituted diaryl amino group, a substituted or unsubstituted diphenyl amino group is preferable. A substituted or unsubstituted carbazole-9-yl group in which two phenyl groups are bonded by a single bond may be adopted, or a substituted or unsubstituted diphenyl amino group in which two phenyl groups are not bonded by a single bond may be adopted. When any of $R^1$ to $R^7$ in the formula (1) is a substituted amino group, preferably at least $R^5$ is a substituted amino group, more preferably only $R^5$ is a substituted amino group. In one aspect of the invention, $R^3$ is not a substituted amino group.

When $R^5$ is a donor group, and $X^1$ is a nitrogen atom, it is preferable that $R^{16}$ or $R^{19}$ is a donor group, and it is more preferable that $R^{19}$ is a donor group. Here, all of the rest of $R^1$ to $R^{26}$ may be, for example, hydrogen atoms or deuterium atoms. For example, at least one of $R^3$, $R^6$, $R^{15}$, and $R^{20}$ may be a substituent (preferably, a substituted or unsubstituted alkyl group, or a substituted or unsubstituted aryl group) and the others may be hydrogen atoms or deuterium atoms.

When $R^5$ is a donor group, and $X^1$ is a boron atom, it is preferable that $R^{20}$ or $R^{23}$ is a donor group, and it is more preferable that $R^{20}$ is a donor group. Here, all of the rest of $R^1$ to $R^{26}$ may be, for example, hydrogen atoms or deuterium atoms. For example, at least one of $R^3$, $R^6$, $R^{19}$, and $R^{24}$ may be a substituent (preferably, a substituted or unsubstituted alkyl group, or a substituted or unsubstituted aryl group) and the others may be hydrogen atoms or deuterium atom.

As one preferable group of compounds in which $R^5$ is a donor group, a compound represented by the following formula (12a) and a compound represented by the following formula (12b) may be exemplified.

Formula (12a)

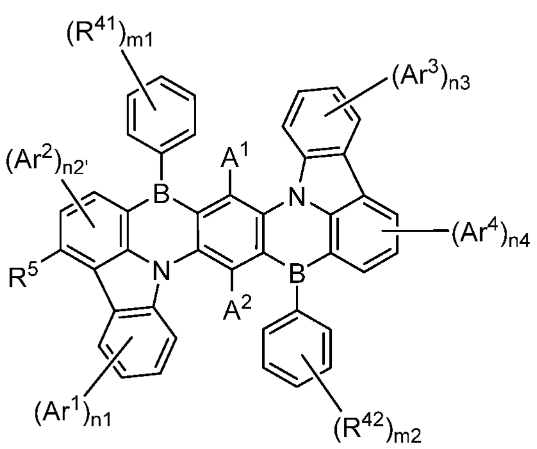

Formula (12b)

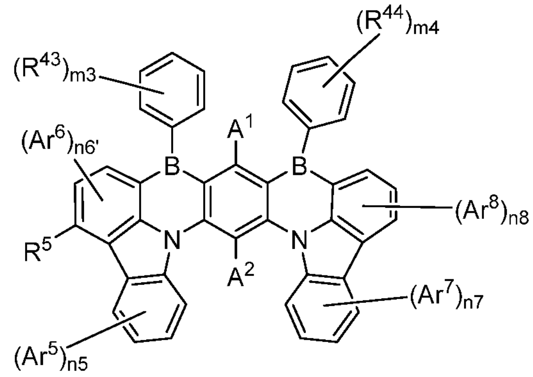

In the formula (12a) and the formula (12b), each of $Ar^1$ to $Ar^8$ independently represents a substituted or unsubstituted aryl group, a substituted or unsubstituted heteroaryl group, or a substituted or unsubstituted alkyl group. For example, a substituted or unsubstituted alkyl group may be preferably selected, or a substituted or unsubstituted aryl group may be preferably selected. $R^5$ represents a donor group. Each of $R^{41}$ to $R^{44}$ independently represents a substituted or unsubstituted alkyl group. Each of m1 to m4 independently represents an integer of 0 to 5. Each of n1, n3, n5, and n7 independently represents an integer of 0 to 4, n4 and n8 represent integers of 0 to 3, and n2' and n6' represent integers of 0 to 2. Each of $A^1$ and $A^2$ independently represents a hydrogen atom, a deuterium atom, or a substituent. In relation to details of $Ar^1$ to $Ar^8$, $R^{41}$ to $R^{44}$, m1 to m4, n1, n3 to n5, n7, n8, $A^1$, and $A^2$, the corresponding descriptions for the formula (1a) and the formula (1b) may be referred to. Meanwhile, $Ar^1$'s bonded to adjacent carbon atoms, $Ar^3$'s bonded to adjacent carbon atoms, $Ar^5$'s bonded to adjacent carbon atoms, and $Ar^7$'s bonded to adjacent carbon atoms may be bonded to each other to form ring structures. Preferably, benzofuran (condensed as a furan ring) or benzothiophene (condensed as a thiophene ring) may be formed.

Hereinafter, specific examples of the compounds represented by the formula (12a) and the formula (12b) will be given. Meanwhile, the compounds of the formula (12a) and the formula (12b), which may be used in the invention,

are not construed as limiting to the following specific examples. In the following specific examples, R, Ar, and X in the formulas F1 to F56 are specified in the table so that the structure of each compound is defined. R is selected from A to D described below, Ar is selected from a to d described below, and X is selected from $\alpha$ to $\gamma$. For example, the No.1 compound in the table is a compound of the formula F1, which has a structure in which R is A, and Ar is a.

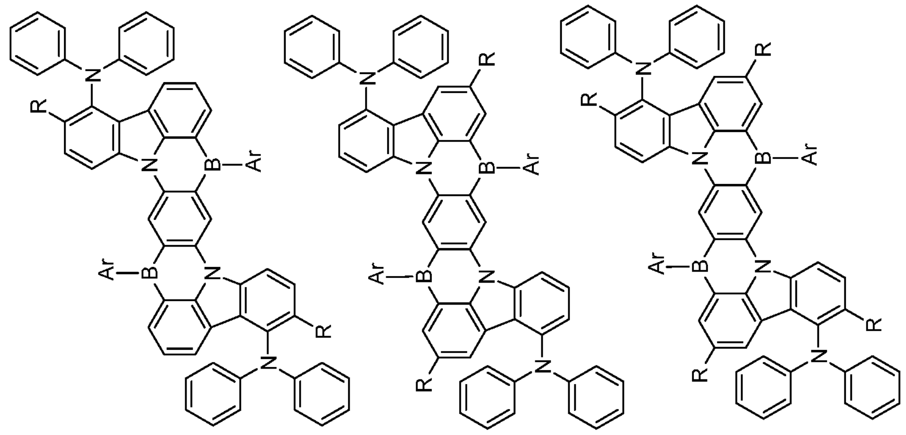

F7

F8

F9

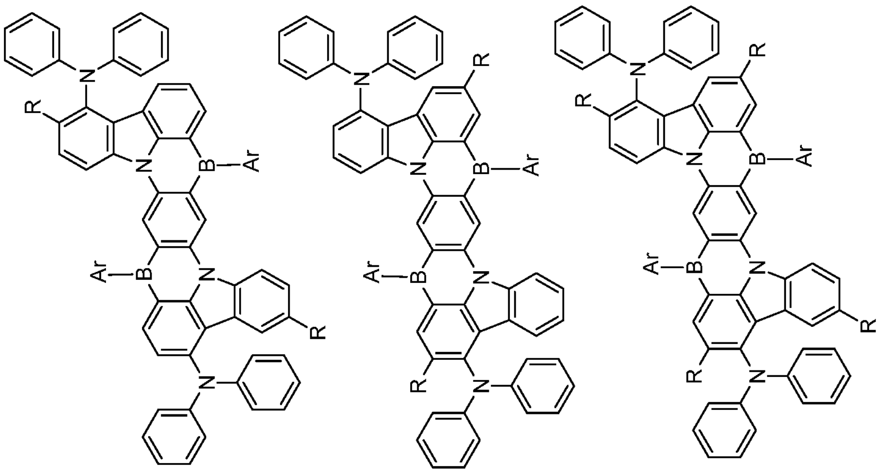

F4

F5

F6

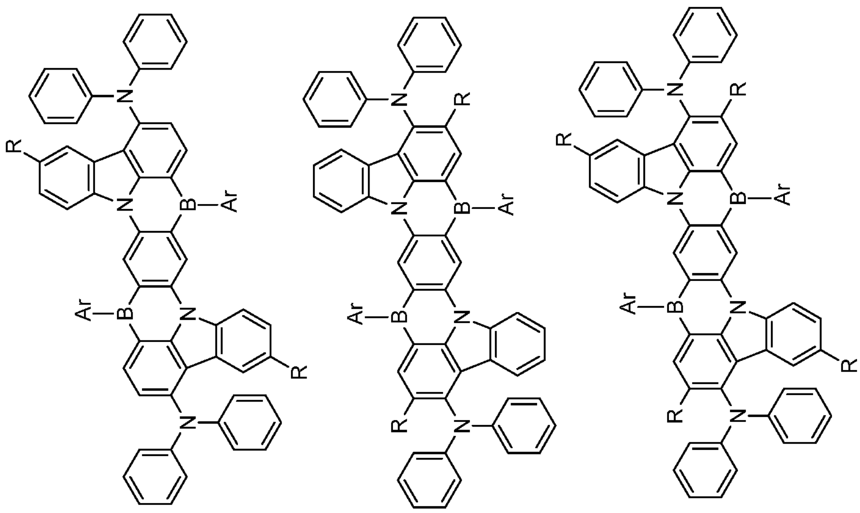

F1

F2

F3

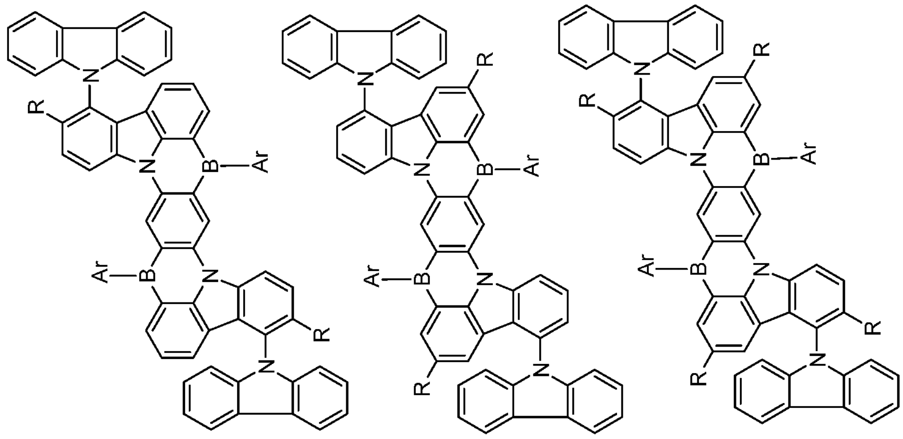

F16 F17 F18

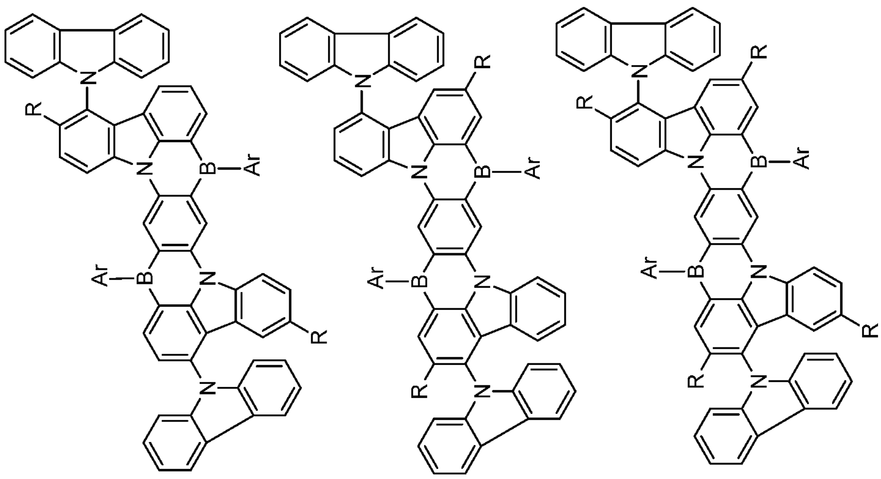

F13 F14 F15

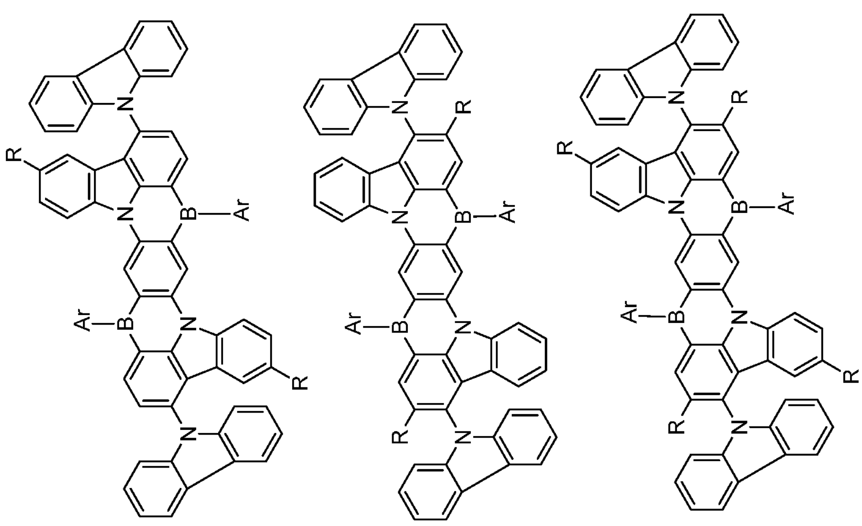

F10 F11 F12

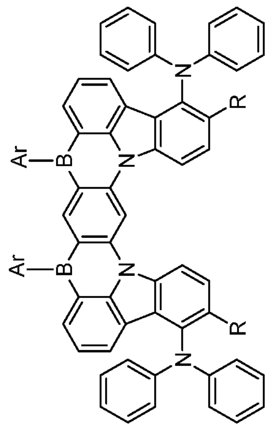

F25

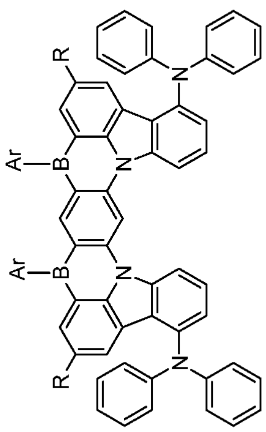

F26

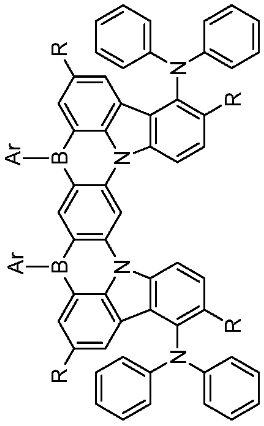

F27

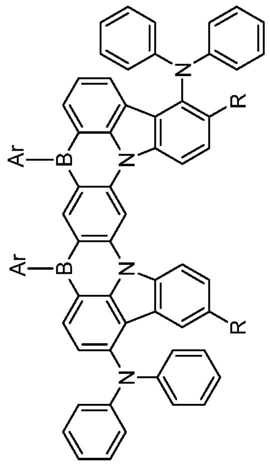

F22

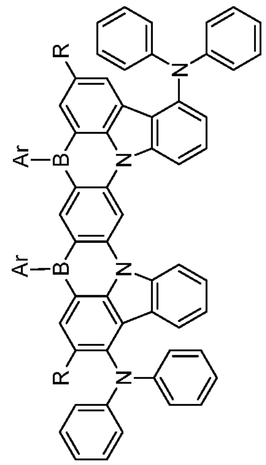

F23

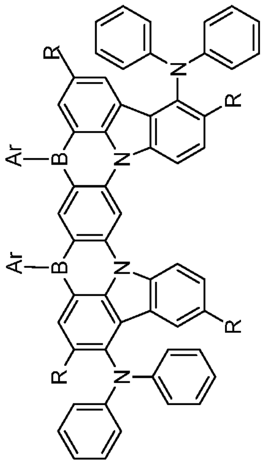

F24

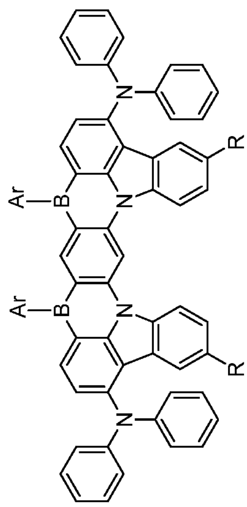

F19

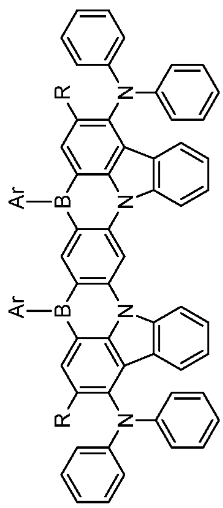

F20

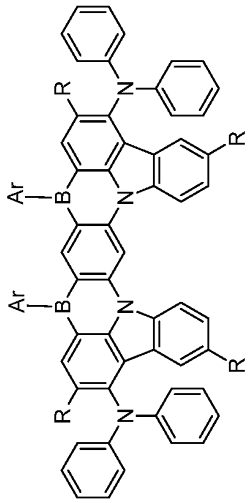

F21

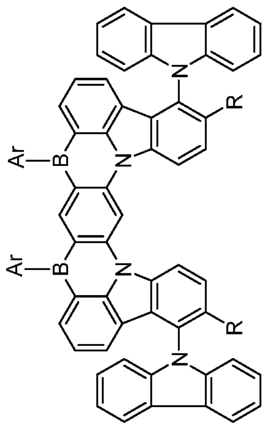

F34

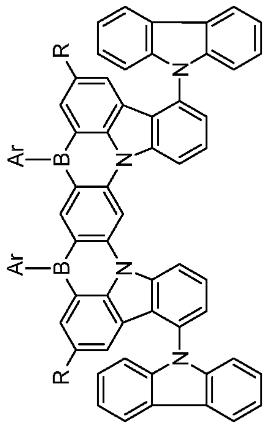

F35

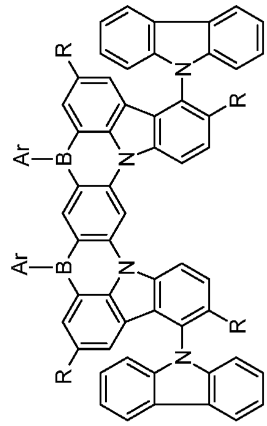

F36

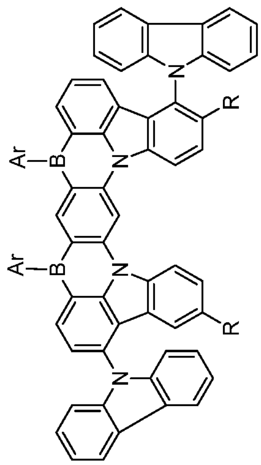

F31

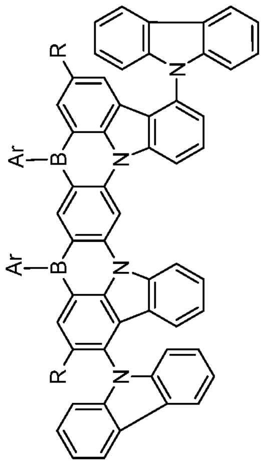

F32

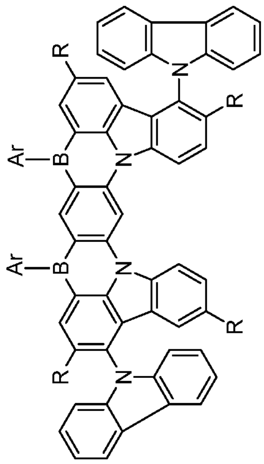

F33

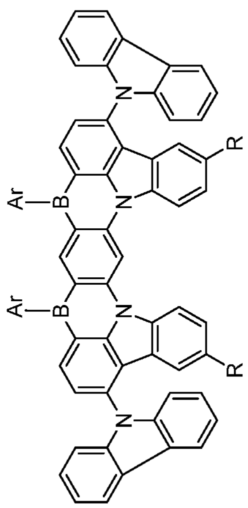

F28

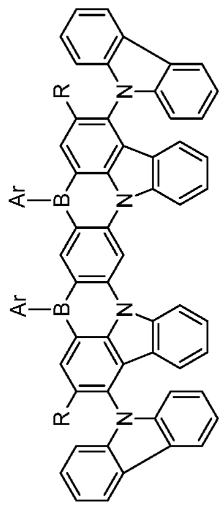

F29

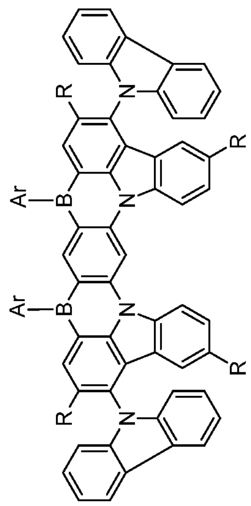

F30

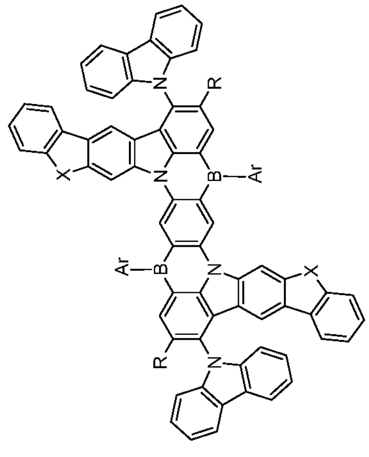

F43

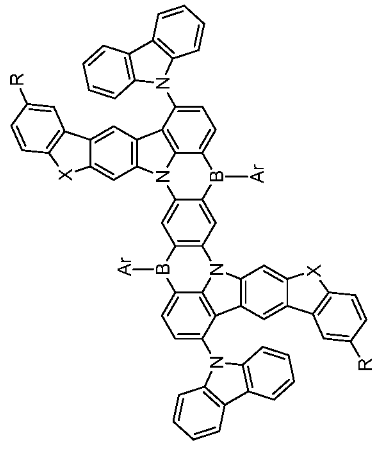

F44

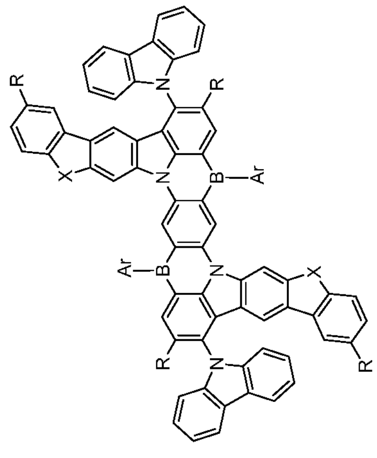

F45

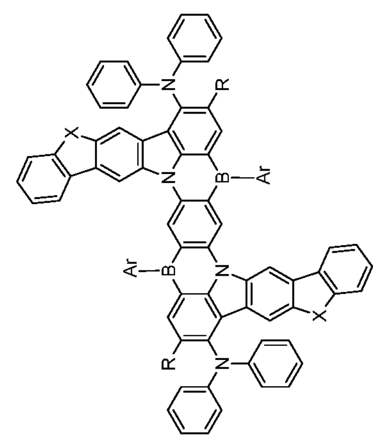

F40

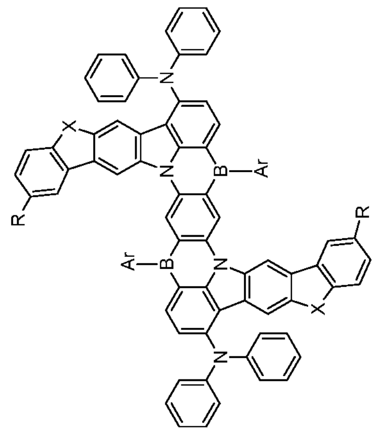

F41

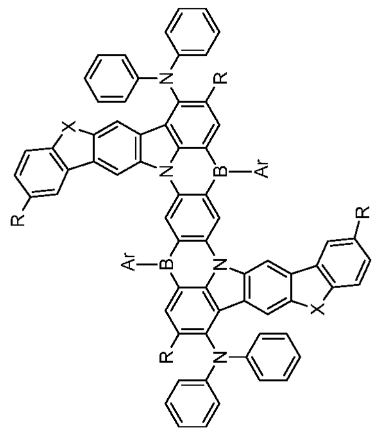

F42

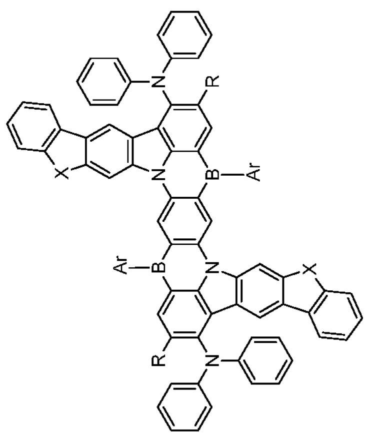

F37

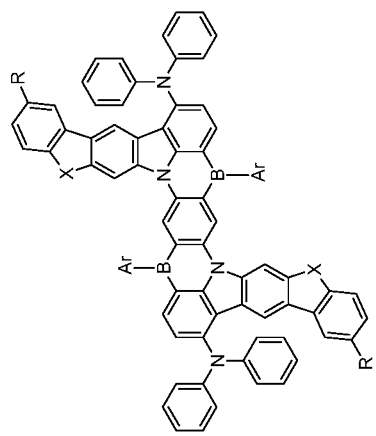

F38

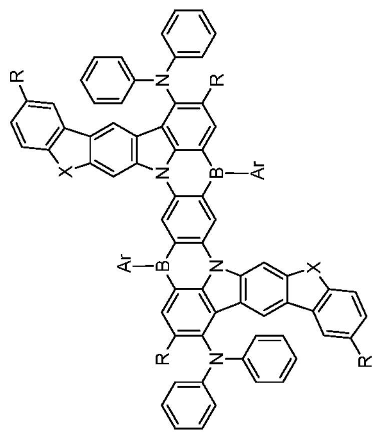

F39

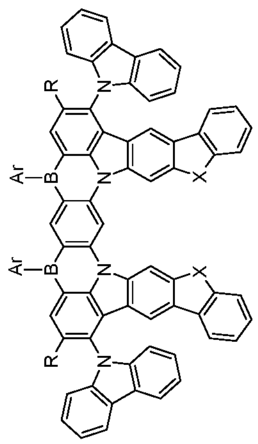

F52

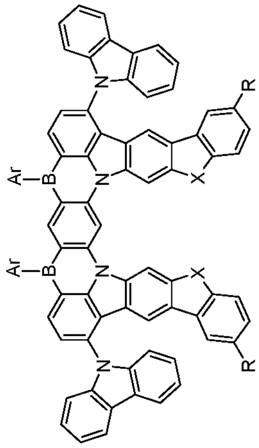

F53

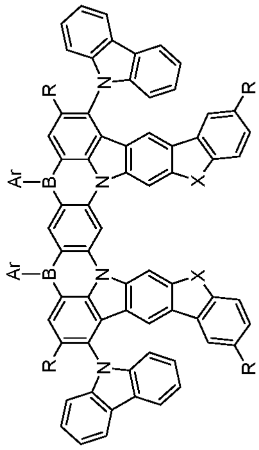

F54

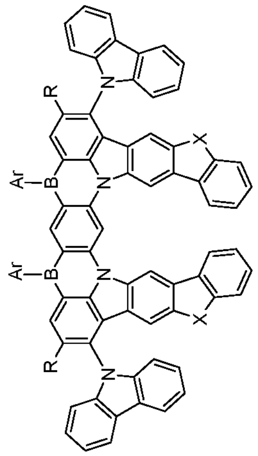

F56

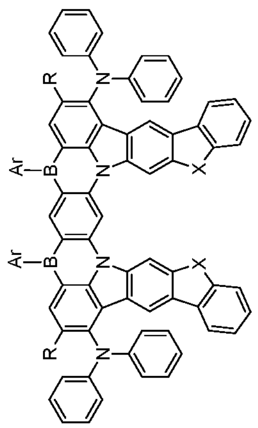

F49

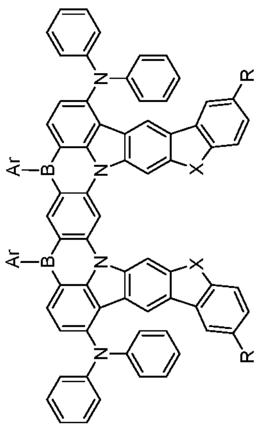

F50

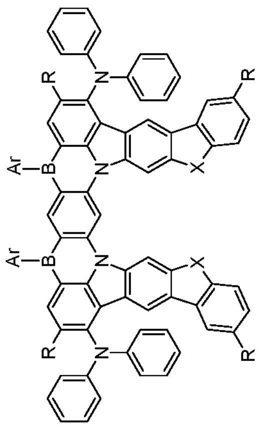

F51

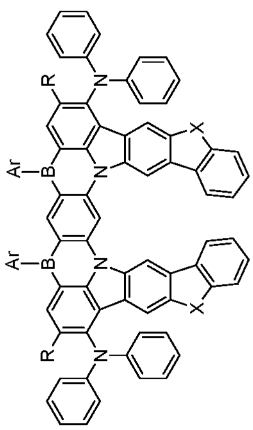

F55

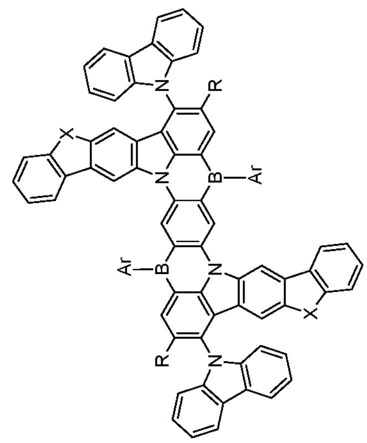

F46

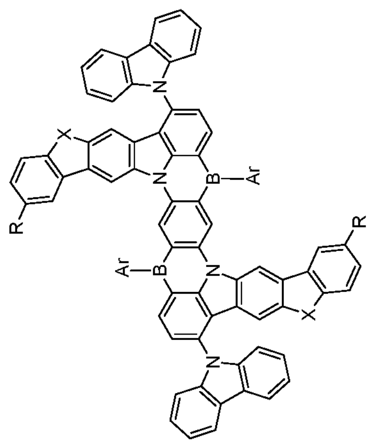

F47

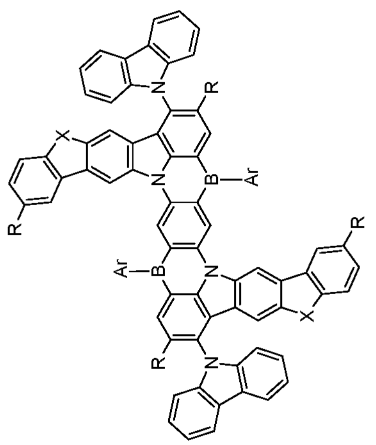

F48

Table 1-1

| No. | F | R | Ar |
|---|---|---|---|
| 1 | F1 | A | a |
| 2 | F1 | A | b |
| 3 | F1 | A | c |
| 4 | F1 | A | d |
| 5 | F1 | B | a |
| 6 | F1 | B | b |
| 7 | F1 | B | c |
| 8 | F1 | B | d |
| 9 | F1 | C | a |
| 10 | F1 | C | b |
| 11 | F1 | C | c |
| 12 | F1 | C | d |
| 13 | F1 | D | a |
| 14 | F1 | D | b |
| 15 | F1 | D | c |
| 16 | F1 | D | d |
| 17 | F2 | A | a |
| 18 | F2 | A | b |
| 19 | F2 | A | c |
| 20 | F2 | A | d |
| 21 | F2 | B | a |
| 22 | F2 | B | b |
| 23 | F2 | B | c |
| 24 | F2 | B | d |
| 25 | F2 | C | a |
| 26 | F2 | C | b |
| 27 | F2 | C | c |
| 28 | F2 | C | d |
| 29 | F3 | A | a |
| 30 | F3 | A | b |
| 31 | F3 | A | c |
| 32 | F3 | A | d |
| 33 | F3 | B | a |
| 34 | F3 | B | b |
| 35 | F3 | B | c |
| 36 | F3 | B | d |
| 37 | F3 | C | a |
| 38 | F3 | C | b |

(continued)

| No. | F | R | Ar |
|---|---|---|---|
| 39 | F3 | C | c |
| 40 | F3 | C | d |
| 41 | F4 | A | a |
| 42 | F4 | A | b |
| 43 | F4 | A | c |
| 44 | F4 | A | d |
| 45 | F4 | B | a |
| 46 | F4 | B | b |
| 47 | F4 | B | c |
| 48 | F4 | B | d |
| 49 | F4 | C | a |
| 50 | F4 | C | b |
| 51 | F4 | C | c |
| 52 | F4 | C | d |
| 53 | F4 | D | a |
| 54 | F4 | D | b |
| 55 | F4 | D | c |
| 56 | F4 | D | d |
| 57 | F5 | A | a |
| 58 | F5 | A | b |
| 59 | F5 | A | c |
| 60 | F5 | A | d |
| 61 | F5 | B | a |
| 62 | F5 | B | b |
| 63 | F5 | B | c |
| 64 | F5 | B | d |
| 65 | F5 | C | a |
| 66 | F5 | C | b |
| 67 | F5 | C | c |
| 68 | F5 | C | d |
| 69 | F6 | A | a |
| 70 | F6 | A | b |
| 71 | F6 | A | c |
| 72 | F6 | A | d |
| 73 | F6 | B | a |
| 74 | F6 | B | b |
| 75 | F6 | B | c |
| 76 | F6 | B | d |

(continued)

| No. | F | R | Ar |
|---|---|---|---|
| 77 | F6 | C | a |
| 78 | F6 | C | b |
| 79 | F6 | C | c |
| 80 | F6 | C | d |
| 81 | F7 | A | a |
| 82 | F7 | A | b |
| 83 | F7 | A | c |
| 84 | F7 | A | d |
| 85 | F7 | B | a |
| 86 | F7 | B | b |
| 87 | F7 | B | c |
| 88 | F7 | B | d |
| 89 | F7 | C | a |
| 90 | F7 | C | b |
| 91 | F7 | C | c |
| 92 | F7 | C | d |
| 93 | F7 | D | a |
| 94 | F7 | D | b |
| 95 | F7 | D | c |
| 96 | F7 | D | d |
| 97 | F8 | A | a |
| 98 | F8 | A | b |
| 99 | F8 | A | c |
| 100 | F8 | A | d |
| 101 | F8 | B | a |
| 102 | F8 | B | b |
| 103 | F8 | B | c |
| 104 | F8 | B | d |
| 105 | F8 | C | a |
| 106 | F8 | C | b |
| 107 | F8 | C | c |
| 108 | F8 | C | d |
| 109 | F9 | A | a |
| 110 | F9 | A | b |
| 111 | F9 | A | c |
| 112 | F9 | A | d |
| 113 | F9 | B | a |
| 114 | F9 | B | b |

(continued)

| No. | F | R | Ar |
|---|---|---|---|
| 115 | F9 | B | c |
| 116 | F9 | B | d |
| 117 | F9 | C | a |
| 118 | F9 | C | b |
| 119 | F9 | C | c |
| 120 | F9 | C | d |
| 121 | F10 | A | a |
| 122 | F10 | A | b |
| 123 | F10 | A | c |
| 124 | F10 | A | d |
| 125 | F10 | B | a |
| 126 | F10 | B | b |
| 127 | F10 | B | c |
| 128 | F10 | B | d |
| 129 | F10 | C | a |
| 130 | F10 | C | b |
| 131 | F10 | C | c |
| 132 | F10 | C | d |
| 133 | F10 | D | a |
| 134 | F10 | D | b |
| 135 | F10 | D | c |
| 136 | F10 | D | d |
| 137 | F11 | A | a |
| 138 | F11 | A | b |
| 139 | F11 | A | c |
| 140 | F11 | A | d |
| 141 | F11 | B | a |
| 142 | F11 | B | b |
| 143 | F11 | B | c |
| 144 | F11 | B | d |
| 145 | F11 | C | a |
| 146 | F11 | C | b |
| 147 | F11 | C | c |
| 148 | F11 | C | d |
| 149 | F12 | A | a |
| 150 | F12 | A | b |
| 151 | F12 | A | c |
| 152 | F12 | A | d |

(continued)

| No. | F | R | Ar |
|---|---|---|---|
| 153 | F12 | B | a |
| 154 | F12 | B | b |
| 155 | F12 | B | c |
| 156 | F12 | B | d |
| 157 | F12 | C | a |
| 158 | F12 | C | b |
| 159 | F12 | C | c |
| 160 | F12 | C | d |

Table 1-2

| No. | F | R | Ar |
|---|---|---|---|
| 161 | F13 | A | a |
| 162 | F13 | A | b |
| 163 | F13 | A | c |
| 164 | F13 | A | d |
| 165 | F13 | B | a |
| 166 | F13 | B | b |
| 167 | F13 | B | c |
| 168 | F13 | B | d |
| 169 | F13 | C | a |
| 170 | F13 | C | b |
| 171 | F13 | C | c |
| 172 | F13 | C | d |
| 173 | F13 | D | a |
| 174 | F13 | D | b |
| 175 | F13 | D | c |
| 176 | F13 | D | d |
| 177 | F14 | A | a |
| 178 | F14 | A | b |
| 179 | F14 | A | c |
| 180 | F14 | A | d |
| 181 | F14 | B | a |
| 182 | F14 | B | b |
| 183 | F14 | B | c |
| 184 | F14 | B | d |
| 185 | F14 | C | a |
| 186 | F14 | C | b |

(continued)

| No. | F | R | Ar |
|---|---|---|---|
| 187 | F14 | C | c |
| 188 | F14 | C | d |
| 189 | F15 | A | a |
| 190 | F15 | A | b |
| 191 | F15 | A | c |
| 192 | F15 | A | d |
| 193 | F15 | B | a |
| 194 | F15 | B | b |
| 195 | F15 | B | c |
| 196 | F15 | B | d |
| 197 | F15 | C | a |
| 198 | F15 | C | b |
| 199 | F15 | C | c |
| 200 | F15 | C | d |
| 201 | F16 | A | a |
| 202 | F16 | A | b |
| 203 | F16 | A | c |
| 204 | F16 | A | d |
| 205 | F16 | B | a |
| 206 | F16 | B | b |
| 207 | F16 | B | c |
| 208 | F16 | B | d |
| 209 | F16 | C | a |
| 210 | F16 | C | b |
| 211 | F16 | C | c |
| 212 | F16 | C | d |
| 213 | F16 | D | a |
| 214 | F16 | D | b |
| 215 | F16 | D | c |
| 216 | F16 | D | d |
| 217 | F17 | A | a |
| 218 | F17 | A | b |
| 219 | F17 | A | c |
| 220 | F17 | A | d |
| 221 | F17 | B | a |
| 222 | F17 | B | b |
| 223 | F17 | B | c |
| 224 | F17 | B | d |

(continued)

| No. | F | R | Ar |
|---|---|---|---|
| 225 | F17 | C | a |
| 226 | F17 | C | b |
| 227 | F17 | C | c |
| 228 | F17 | C | d |
| 229 | F18 | A | a |
| 230 | F18 | A | b |
| 231 | F18 | A | c |
| 232 | F18 | A | d |
| 233 | F18 | B | a |
| 234 | F18 | B | b |
| 235 | F18 | B | c |
| 236 | F18 | B | d |
| 237 | F18 | C | a |
| 238 | F18 | C | b |
| 239 | F18 | C | c |
| 240 | F18 | C | d |
| 241 | F19 | A | a |
| 242 | F19 | A | b |
| 243 | F19 | A | c |
| 244 | F19 | A | d |
| 245 | F19 | B | a |
| 246 | F19 | B | b |
| 247 | F19 | B | c |
| 248 | F19 | B | d |
| 249 | F19 | C | a |
| 250 | F19 | C | b |
| 251 | F19 | C | c |
| 252 | F19 | C | d |
| 253 | F19 | D | a |
| 254 | F19 | D | b |
| 255 | F19 | D | c |
| 256 | F19 | D | d |
| 257 | F20 | A | a |
| 258 | F20 | A | b |
| 259 | F20 | A | c |
| 260 | F20 | A | d |
| 261 | F20 | B | a |
| 262 | F20 | B | b |

(continued)

| No. | F | R | Ar |
|---|---|---|---|
| 263 | F20 | B | c |
| 264 | F20 | B | d |
| 265 | F20 | C | a |
| 266 | F20 | C | b |
| 267 | F20 | C | c |
| 268 | F20 | C | d |
| 269 | F21 | A | a |
| 270 | F21 | A | b |
| 271 | F21 | A | c |
| 272 | F21 | A | d |
| 273 | F21 | B | a |
| 274 | F21 | B | b |
| 275 | F21 | B | c |
| 276 | F21 | B | d |
| 277 | F21 | C | a |
| 278 | F21 | C | b |
| 279 | F21 | C | c |
| 280 | F21 | C | d |
| 281 | F22 | A | a |
| 282 | F22 | A | b |
| 283 | F22 | A | c |
| 284 | F22 | A | d |
| 285 | F22 | B | a |
| 286 | F22 | B | b |
| 287 | F22 | B | c |
| 288 | F22 | B | d |
| 289 | F22 | C | a |
| 290 | F22 | C | b |
| 291 | F22 | C | c |
| 292 | F22 | C | d |
| 293 | F22 | D | a |
| 294 | F22 | D | b |
| 295 | F22 | D | c |
| 296 | F22 | D | d |
| 297 | F23 | A | a |
| 298 | F23 | A | b |
| 299 | F23 | A | c |
| 300 | F23 | A | d |

(continued)

| No. | F | R | Ar |
|---|---|---|---|
| 301 | F23 | B | a |
| 302 | F23 | B | b |
| 303 | F23 | B | c |
| 304 | F23 | B | d |
| 305 | F23 | C | a |
| 306 | F23 | C | b |
| 307 | F23 | C | c |
| 308 | F23 | C | d |
| 309 | F24 | A | a |
| 310 | F24 | A | b |
| 311 | F24 | A | c |
| 312 | F24 | A | d |
| 313 | F24 | B | a |
| 314 | F24 | B | b |
| 315 | F24 | B | c |
| 316 | F24 | B | d |
| 317 | F24 | C | a |
| 318 | F24 | C | b |
| 319 | F24 | C | c |
| 320 | F24 | C | d |

Table 1-3

| No. | F | R | Ar |
|---|---|---|---|
| 321 | F25 | A | a |
| 322 | F25 | A | b |
| 323 | F25 | A | c |
| 324 | F25 | A | d |
| 325 | F25 | B | a |
| 326 | F25 | B | b |
| 327 | F25 | B | c |
| 328 | F25 | B | d |
| 329 | F25 | C | a |
| 330 | F25 | C | b |
| 331 | F25 | C | c |
| 332 | F25 | C | d |
| 333 | F25 | D | a |
| 334 | F25 | D | b |

(continued)

| No. | F | R | Ar |
|---|---|---|---|
| 335 | F25 | D | c |
| 336 | F25 | D | d |
| 337 | F26 | A | a |
| 338 | F26 | A | b |
| 339 | F26 | A | c |
| 340 | F26 | A | d |
| 341 | F26 | B | a |
| 342 | F26 | B | b |
| 343 | F26 | B | c |
| 344 | F26 | B | d |
| 345 | F26 | C | a |
| 346 | F26 | C | b |
| 347 | F26 | C | c |
| 348 | F26 | C | d |
| 349 | F27 | A | a |
| 350 | F27 | A | b |
| 351 | F27 | A | c |
| 352 | F27 | A | d |
| 353 | F27 | B | a |
| 354 | F27 | B | b |
| 355 | F27 | B | c |
| 356 | F27 | B | d |
| 357 | F27 | C | a |
| 358 | F27 | C | b |
| 359 | F27 | C | c |
| 360 | F27 | C | d |
| 361 | F28 | A | a |
| 362 | F28 | A | b |
| 363 | F28 | A | c |
| 364 | F28 | A | d |
| 365 | F28 | B | a |
| 366 | F28 | B | b |
| 367 | F28 | B | c |
| 368 | F28 | B | d |
| 369 | F28 | C | a |
| 370 | F28 | C | b |
| 371 | F28 | C | c |
| 372 | F28 | C | d |

(continued)

| No. | F | R | Ar |
|---|---|---|---|
| 373 | F28 | D | a |
| 374 | F28 | D | b |
| 375 | F28 | D | c |
| 376 | F28 | D | d |
| 377 | F29 | A | a |
| 378 | F29 | A | b |
| 379 | F29 | A | c |
| 380 | F29 | A | d |
| 381 | F29 | B | a |
| 382 | F29 | B | b |
| 383 | F29 | B | c |
| 384 | F29 | B | d |
| 385 | F29 | C | a |
| 386 | F29 | C | b |
| 387 | F29 | C | c |
| 388 | F29 | C | d |
| 389 | F30 | A | a |
| 390 | F30 | A | b |
| 391 | F30 | A | c |
| 392 | F30 | A | d |
| 393 | F30 | B | a |
| 394 | F30 | B | b |
| 395 | F30 | B | c |
| 396 | F30 | B | d |
| 397 | F30 | C | a |
| 398 | F30 | C | b |
| 399 | F30 | C | c |
| 400 | F30 | C | d |
| 401 | F31 | A | a |
| 402 | F31 | A | b |
| 403 | F31 | A | c |
| 404 | F31 | A | d |
| 405 | F31 | B | a |
| 406 | F31 | B | b |
| 407 | F31 | B | c |
| 408 | F31 | B | d |
| 409 | F31 | C | a |
| 410 | F31 | C | b |

(continued)

| No. | F | R | Ar |
|---|---|---|---|
| 411 | F31 | C | c |
| 412 | F31 | C | d |
| 413 | F31 | D | a |
| 414 | F31 | D | b |
| 415 | F31 | D | c |
| 416 | F31 | D | d |
| 417 | F32 | A | a |
| 418 | F32 | A | b |
| 419 | F32 | A | c |
| 420 | F32 | A | d |
| 421 | F32 | B | a |
| 422 | F32 | B | b |
| 423 | F32 | B | c |
| 424 | F32 | B | d |
| 425 | F32 | C | a |
| 426 | F32 | C | b |
| 427 | F32 | C | c |
| 428 | F32 | C | d |
| 429 | F33 | A | a |
| 430 | F33 | A | b |
| 431 | F33 | A | c |
| 432 | F33 | A | d |
| 433 | F33 | B | a |
| 434 | F33 | B | b |
| 435 | F33 | B | c |
| 436 | F33 | B | d |
| 437 | F33 | C | a |
| 438 | F33 | C | b |
| 439 | F33 | C | c |
| 440 | F33 | C | d |
| 441 | F34 | A | a |
| 442 | F34 | A | b |
| 443 | F34 | A | c |
| 444 | F34 | A | d |
| 445 | F34 | B | a |
| 446 | F34 | B | b |
| 447 | F34 | B | c |
| 448 | F34 | B | d |

(continued)

| No. | F | R | Ar |
|---|---|---|---|
| 449 | F34 | C | a |
| 450 | F34 | C | b |
| 451 | F34 | C | c |
| 452 | F34 | C | d |
| 453 | F34 | D | a |
| 454 | F34 | D | b |
| 455 | F34 | D | c |
| 456 | F34 | D | d |
| 457 | F35 | A | a |
| 458 | F35 | A | b |
| 459 | F35 | A | c |
| 460 | F35 | A | d |
| 461 | F35 | B | a |
| 462 | F35 | B | b |
| 463 | F35 | B | c |
| 464 | F35 | B | d |
| 465 | F35 | C | a |
| 466 | F35 | C | b |
| 467 | F35 | C | c |
| 468 | F35 | C | d |
| 469 | F36 | A | a |
| 470 | F36 | A | b |
| 471 | F36 | A | c |
| 472 | F36 | A | d |
| 473 | F36 | B | a |
| 474 | F36 | B | b |
| 475 | F36 | B | c |
| 476 | F36 | B | d |
| 477 | F36 | C | a |
| 478 | F36 | C | b |
| 479 | F36 | C | c |
| 480 | F36 | C | d |

Table 1-4

| No. | F | R | Ar | X |
|---|---|---|---|---|
| 481 | F37 | A | a | $\alpha$ |
| 482 | F37 | A | a | $\beta$ |

(continued)

| No. | F | R | Ar | X |
|---|---|---|---|---|
| 483 | F37 | A | a | γ |
| 484 | F37 | A | b | α |
| 485 | F37 | A | b | β |
| 486 | F37 | A | b | γ |
| 487 | F37 | A | c | α |
| 488 | F37 | A | c | β |
| 489 | F37 | A | c | γ |
| 490 | F37 | A | d | α |
| 491 | F37 | A | d | β |
| 492 | F37 | A | d | γ |
| 493 | F37 | B | a | α |
| 494 | F37 | B | a | β |
| 495 | F37 | B | a | γ |
| 496 | F37 | B | b | α |
| 497 | F37 | B | b | β |
| 498 | F37 | B | b | γ |
| 499 | F37 | B | c | α |
| 500 | F37 | B | c | β |
| 501 | F37 | B | c | r |
| 502 | F37 | B | d | α |
| 503 | F37 | B | d | β |
| 504 | F37 | B | d | r |
| 505 | F37 | C | a | α |
| 506 | F37 | C | a | β |
| 507 | F37 | C | a | γ |
| 508 | F37 | C | b | α |
| 509 | F37 | C | b | β |
| 510 | F37 | C | b | γ |
| 511 | F37 | C | c | α |
| 512 | F37 | C | c | β |
| 513 | F37 | C | c | γ |
| 514 | F37 | C | d | α |
| 515 | F37 | C | d | β |
| 516 | F37 | C | d | γ |
| 517 | F37 | D | a | α |
| 518 | F37 | D | a | β |
| 519 | F37 | D | a | γ |
| 520 | F37 | D | b | α |

(continued)

| No. | F | R | Ar | X |
|---|---|---|---|---|
| 521 | F37 | D | b | β |
| 522 | F37 | D | b | γ |
| 523 | F37 | D | c | α |
| 524 | F37 | D | c | β |
| 525 | F37 | D | c | γ |
| 526 | F37 | D | d | α |
| 527 | F37 | D | d | β |
| 528 | F37 | D | d | γ |
| 529 | F38 | A | a | α |
| 530 | F38 | A | a | β |
| 531 | F38 | A | a | γ |
| 532 | F38 | A | b | α |
| 533 | F38 | A | b | β |
| 534 | F38 | A | b | γ |
| 535 | F38 | A | c | α |
| 536 | F38 | A | c | β |
| 537 | F38 | A | c | γ |
| 538 | F38 | A | d | α |
| 539 | F38 | A | d | β |
| 540 | F38 | A | d | γ |
| 541 | F38 | B | a | α |
| 542 | F38 | B | a | β |
| 543 | F38 | B | a | γ |
| 544 | F38 | B | b | α |
| 545 | F38 | B | b | β |
| 546 | F38 | B | b | γ |
| 547 | F38 | B | c | α |
| 548 | F38 | B | c | β |
| 549 | F38 | B | c | γ |
| 550 | F38 | B | d | α |
| 551 | F38 | B | d | β |
| 552 | F38 | B | d | γ |
| 553 | F38 | C | a | α |
| 554 | F38 | C | a | β |
| 555 | F38 | C | a | γ |
| 556 | F38 | C | b | α |
| 557 | F38 | C | b | β |
| 558 | F38 | C | b | γ |

(continued)

| No. | F | R | Ar | X |
|---|---|---|---|---|
| 559 | F38 | C | c | $\alpha$ |
| 560 | F38 | C | c | $\beta$ |
| 561 | F38 | C | c | $\gamma$ |
| 562 | F38 | C | d | $\alpha$ |
| 563 | F38 | C | d | $\beta$ |
| 564 | F38 | C | d | $\gamma$ |
| 565 | F39 | A | a | $\alpha$ |
| 566 | F39 | A | a | $\beta$ |
| 567 | F39 | A | a | $\gamma$ |
| 568 | F39 | A | b | $\alpha$ |
| 569 | F39 | A | b | $\beta$ |
| 570 | F39 | A | b | $\gamma$ |
| 571 | F39 | A | c | $\alpha$ |
| 572 | F39 | A | c | $\beta$ |
| 573 | F39 | A | c | $\gamma$ |
| 574 | F39 | A | d | $\alpha$ |
| 575 | F39 | A | d | $\beta$ |
| 576 | F39 | A | d | $\gamma$ |
| 577 | F39 | B | a | $\alpha$ |
| 578 | F39 | B | a | $\beta$ |
| 579 | F39 | B | a | $\gamma$ |
| 580 | F39 | B | b | $\alpha$ |
| 581 | F39 | B | b | $\beta$ |
| 582 | F39 | B | b | $\gamma$ |
| 583 | F39 | B | c | $\alpha$ |
| 584 | F39 | B | c | $\beta$ |
| 585 | F39 | B | c | $\gamma$ |
| 586 | F39 | B | d | $\alpha$ |
| 587 | F39 | B | d | $\beta$ |
| 588 | F39 | B | d | $\gamma$ |
| 589 | F39 | C | a | $\alpha$ |
| 590 | F39 | C | a | $\beta$ |
| 591 | F39 | C | a | $\gamma$ |
| 592 | F39 | C | b | $\alpha$ |
| 593 | F39 | C | b | $\beta$ |
| 594 | F39 | C | b | $\gamma$ |
| 595 | F39 | C | c | $\alpha$ |
| 596 | F39 | C | c | $\beta$ |

(continued)

| No. | F | R | Ar | X |
|---|---|---|---|---|
| 597 | F39 | C | c | $\gamma$ |
| 598 | F39 | C | d | $\alpha$ |
| 599 | F39 | C | d | $\beta$ |
| 600 | F39 | C | d | $\gamma$ |
| 601 | F40 | A | a | $\alpha$ |
| 602 | F40 | A | a | $\beta$ |
| 603 | F40 | A | a | $\gamma$ |
| 604 | F40 | A | b | $\alpha$ |
| 605 | F40 | A | b | $\beta$ |
| 606 | F40 | A | b | $\gamma$ |
| 607 | F40 | A | c | $\alpha$ |
| 608 | F40 | A | c | $\beta$ |
| 609 | F40 | A | c | $\gamma$ |
| 610 | F40 | A | d | $\alpha$ |
| 611 | F40 | A | d | $\beta$ |
| 612 | F40 | A | d | $\gamma$ |
| 613 | F40 | B | a | $\alpha$ |
| 614 | F40 | B | a | $\beta$ |
| 615 | F40 | B | a | $\gamma$ |
| 616 | F40 | B | b | $\alpha$ |
| 617 | F40 | B | b | $\beta$ |
| 618 | F40 | B | b | $\gamma$ |
| 619 | F40 | B | c | $\alpha$ |
| 620 | F40 | B | c | $\beta$ |
| 621 | F40 | B | c | $\gamma$ |
| 622 | F40 | B | d | $\alpha$ |
| 623 | F40 | B | d | $\beta$ |
| 624 | F40 | B | d | $\gamma$ |
| 625 | F40 | C | a | $\alpha$ |
| 626 | F40 | C | a | $\beta$ |
| 627 | F40 | C | a | $\gamma$ |
| 628 | F40 | C | b | $\alpha$ |
| 629 | F40 | C | b | $\beta$ |
| 630 | F40 | C | b | $\gamma$ |
| 631 | F40 | C | c | $\alpha$ |
| 632 | F40 | C | c | $\beta$ |
| 633 | F40 | C | c | $\gamma$ |
| 634 | F40 | C | d | $\alpha$ |

(continued)

| No. | F | R | Ar | X |
|---|---|---|---|---|
| 635 | F40 | C | d | $\beta$ |
| 636 | F40 | C | d | $\gamma$ |
| 637 | F40 | D | a | $\alpha$ |
| 638 | F40 | D | a | $\beta$ |
| 639 | F40 | D | a | $\gamma$ |
| 640 | F40 | D | b | $\alpha$ |

Table 1-5

| No. | F | R | Ar | X |
|---|---|---|---|---|
| 641 | F40 | D | b | $\beta$ |
| 642 | F40 | D | b | $\gamma$ |
| 643 | F40 | D | c | $\alpha$ |
| 644 | F40 | D | c | $\beta$ |
| 645 | F40 | D | c | $\gamma$ |
| 646 | F40 | D | d | $\alpha$ |
| 647 | F40 | D | d | $\beta$ |
| 648 | F40 | D | d | $\gamma$ |
| 649 | F41 | A | a | $\alpha$ |
| 650 | F41 | A | a | $\beta$ |
| 651 | F41 | A | a | $\gamma$ |
| 652 | F41 | A | b | $\alpha$ |
| 653 | F41 | A | b | $\beta$ |
| 654 | F41 | A | b | $\gamma$ |
| 655 | F41 | A | c | $\alpha$ |
| 656 | F41 | A | c | $\beta$ |
| 657 | F41 | A | c | $\gamma$ |
| 658 | F41 | A | d | $\alpha$ |
| 659 | F41 | A | d | $\beta$ |
| 660 | F41 | A | d | $\gamma$ |
| 661 | F41 | B | a | $\alpha$ |
| 662 | F41 | B | a | $\beta$ |
| 663 | F41 | B | a | $\gamma$ |
| 664 | F41 | B | b | $\alpha$ |
| 665 | F41 | B | b | $\beta$ |
| 666 | F41 | B | b | $\gamma$ |
| 667 | F41 | B | c | $\alpha$ |
| 668 | F41 | B | c | $\beta$ |

(continued)

| No. | F | R | Ar | X |
|---|---|---|---|---|
| 669 | F41 | B | c | $\gamma$ |
| 670 | F41 | B | d | $\alpha$ |
| 671 | F41 | B | d | $\beta$ |
| 672 | F41 | B | d | $\gamma$ |
| 673 | F41 | C | a | $\alpha$ |
| 674 | F41 | C | a | $\beta$ |
| 675 | F41 | C | a | $\gamma$ |
| 676 | F41 | C | b | $\alpha$ |
| 677 | F41 | C | b | $\beta$ |
| 678 | F41 | C | b | $\gamma$ |
| 679 | F41 | C | c | $\alpha$ |
| 680 | F41 | C | c | $\beta$ |
| 681 | F41 | C | c | $\gamma$ |
| 682 | F41 | C | d | $\alpha$ |
| 683 | F41 | C | d | $\beta$ |
| 684 | F41 | C | d | $\gamma$ |
| 685 | F42 | A | a | $\alpha$ |
| 686 | F42 | A | a | $\beta$ |
| 687 | F42 | A | a | $\gamma$ |
| 688 | F42 | A | b | $\alpha$ |
| 689 | F42 | A | b | $\beta$ |
| 690 | F42 | A | b | $\gamma$ |
| 691 | F42 | A | c | $\alpha$ |
| 692 | F42 | A | c | $\beta$ |
| 693 | F42 | A | c | $\gamma$ |
| 694 | F42 | A | d | $\alpha$ |
| 695 | F42 | A | d | $\beta$ |
| 696 | F42 | A | d | $\gamma$ |
| 697 | F42 | B | a | $\alpha$ |
| 698 | F42 | B | a | $\beta$ |
| 699 | F42 | B | a | $\gamma$ |
| 700 | F42 | B | b | $\alpha$ |
| 701 | F42 | B | b | $\beta$ |
| 702 | F42 | B | b | $\gamma$ |
| 703 | F42 | B | c | $\alpha$ |
| 704 | F42 | B | c | $\beta$ |
| 705 | F42 | B | c | $\gamma$ |
| 706 | F42 | B | d | $\alpha$ |

(continued)

| No. | F | R | Ar | X |
|---|---|---|---|---|
| 707 | F42 | B | d | $\beta$ |
| 708 | F42 | B | d | $\gamma$ |
| 709 | F42 | C | a | $\alpha$ |
| 710 | F42 | C | a | $\beta$ |
| 711 | F42 | C | a | $\gamma$ |
| 712 | F42 | C | b | $\alpha$ |
| 713 | F42 | C | b | $\beta$ |
| 714 | F42 | C | b | $\gamma$ |
| 715 | F42 | C | c | $\alpha$ |
| 716 | F42 | C | c | $\beta$ |
| 717 | F42 | C | c | $\gamma$ |
| 718 | F42 | C | d | $\alpha$ |
| 719 | F42 | C | d | $\beta$ |
| 720 | F42 | C | d | $\gamma$ |
| 721 | F43 | A | a | $\alpha$ |
| 722 | F43 | A | a | $\beta$ |
| 723 | F43 | A | a | $\gamma$ |
| 724 | F43 | A | b | $\alpha$ |
| 725 | F43 | A | b | $\beta$ |
| 726 | F43 | A | b | $\gamma$ |
| 727 | F43 | A | c | $\alpha$ |
| 728 | F43 | A | c | $\beta$ |
| 729 | F43 | A | c | $\gamma$ |
| 730 | F43 | A | d | $\alpha$ |
| 731 | F43 | A | d | $\beta$ |
| 732 | F43 | A | d | $\gamma$ |
| 733 | F43 | B | a | $\alpha$ |
| 734 | F43 | B | a | $\beta$ |
| 735 | F43 | B | a | $\gamma$ |
| 736 | F43 | B | b | $\alpha$ |
| 737 | F43 | B | b | $\beta$ |
| 738 | F43 | B | b | $\gamma$ |
| 739 | F43 | B | c | $\alpha$ |
| 740 | F43 | B | c | $\beta$ |
| 741 | F43 | B | c | $\gamma$ |
| 742 | F43 | B | d | $\alpha$ |
| 743 | F43 | B | d | $\beta$ |
| 744 | F43 | B | d | $\gamma$ |

(continued)

| No. | F | R | Ar | X |
|---|---|---|---|---|
| 745 | F43 | C | a | $\alpha$ |
| 746 | F43 | C | a | $\beta$ |
| 747 | F43 | C | a | $\gamma$ |
| 748 | F43 | C | b | $\alpha$ |
| 749 | F43 | C | b | $\beta$ |
| 750 | F43 | C | b | $\gamma$ |
| 751 | F43 | C | c | $\alpha$ |
| 752 | F43 | C | c | $\beta$ |
| 753 | F43 | C | c | $\gamma$ |
| 754 | F43 | C | d | $\alpha$ |
| 755 | F43 | C | d | $\beta$ |
| 756 | F43 | C | d | $\gamma$ |
| 757 | F43 | D | a | $\alpha$ |
| 758 | F43 | D | a | $\beta$ |
| 759 | F43 | D | a | $\gamma$ |
| 760 | F43 | D | b | $\alpha$ |
| 761 | F43 | D | b | $\beta$ |
| 762 | F43 | D | b | $\gamma$ |
| 763 | F43 | D | c | $\alpha$ |
| 764 | F43 | D | c | $\beta$ |
| 765 | F43 | D | c | $\gamma$ |
| 766 | F43 | D | d | $\alpha$ |
| 767 | F43 | D | d | $\beta$ |
| 768 | F43 | D | d | $\gamma$ |
| 769 | F44 | A | a | $\alpha$ |
| 770 | F44 | A | a | $\beta$ |
| 771 | F44 | A | a | $\gamma$ |
| 772 | F44 | A | b | $\alpha$ |
| 773 | F44 | A | b | $\beta$ |
| 774 | F44 | A | b | $\gamma$ |
| 775 | F44 | A | c | $\alpha$ |
| 776 | F44 | A | c | $\beta$ |
| 777 | F44 | A | c | $\gamma$ |
| 778 | F44 | A | d | $\alpha$ |
| 779 | F44 | A | d | $\beta$ |
| 780 | F44 | A | d | $\gamma$ |
| 781 | F44 | B | a | $\alpha$ |
| 782 | F44 | B | a | $\beta$ |

(continued)

| No. | F | R | Ar | X |
|---|---|---|---|---|
| 783 | F44 | B | a | $\gamma$ |
| 784 | F44 | B | b | $\alpha$ |
| 785 | F44 | B | b | $\beta$ |
| 786 | F44 | B | b | $\gamma$ |
| 787 | F44 | B | c | $\alpha$ |
| 788 | F44 | B | c | $\beta$ |
| 789 | F44 | B | c | $\gamma$ |
| 790 | F44 | B | d | $\alpha$ |
| 791 | F44 | B | d | $\beta$ |
| 792 | F44 | B | d | $\gamma$ |
| 793 | F44 | C | a | $\alpha$ |
| 794 | F44 | C | a | $\beta$ |
| 795 | F44 | C | a | $\gamma$ |
| 796 | F44 | C | b | $\alpha$ |
| 797 | F44 | C | b | $\beta$ |
| 798 | F44 | C | b | $\gamma$ |
| 799 | F44 | C | c | $\alpha$ |
| 800 | F44 | C | c | $\beta$ |

Table 1-6

| No. | F | R | Ar | X |
|---|---|---|---|---|
| 801 | F44 | C | c | $\gamma$ |
| 802 | F44 | C | d | $\alpha$ |
| 803 | F44 | C | d | $\beta$ |
| 804 | F44 | C | d | $\gamma$ |
| 805 | F45 | A | a | $\alpha$ |
| 806 | F45 | A | a | $\beta$ |
| 807 | F45 | A | a | $\gamma$ |
| 808 | F45 | A | b | $\alpha$ |
| 809 | F45 | A | b | $\beta$ |
| 810 | F45 | A | b | $\gamma$ |
| 811 | F45 | A | c | $\alpha$ |
| 812 | F45 | A | c | $\beta$ |
| 813 | F45 | A | c | $\gamma$ |
| 814 | F45 | A | d | $\alpha$ |
| 815 | F45 | A | d | $\beta$ |
| 816 | F45 | A | d | $\gamma$ |

(continued)

| No. | F | R | Ar | X |
|---|---|---|---|---|
| 817 | F45 | B | a | $\alpha$ |
| 818 | F45 | B | a | $\beta$ |
| 819 | F45 | B | a | $\gamma$ |
| 820 | F45 | B | b | $\alpha$ |
| 821 | F45 | B | b | $\beta$ |
| 822 | F45 | B | b | $\gamma$ |
| 823 | F45 | B | c | $\alpha$ |
| 824 | F45 | B | c | $\beta$ |
| 825 | F45 | B | c | $\gamma$ |
| 826 | F45 | B | d | $\alpha$ |
| 827 | F45 | B | d | $\beta$ |
| 828 | F45 | B | d | $\gamma$ |
| 829 | F45 | C | a | $\alpha$ |
| 830 | F45 | C | a | $\beta$ |
| 831 | F45 | C | a | $\gamma$ |
| 832 | F45 | C | b | $\alpha$ |
| 833 | F45 | C | b | $\beta$ |
| 834 | F45 | C | b | $\gamma$ |
| 835 | F45 | C | c | $\alpha$ |
| 836 | F45 | C | c | $\beta$ |
| 837 | F45 | C | c | $\gamma$ |
| 838 | F45 | C | d | $\alpha$ |
| 839 | F45 | C | d | $\beta$ |
| 840 | F45 | C | d | $\gamma$ |
| 841 | F46 | A | a | $\alpha$ |
| 842 | F46 | A | a | $\beta$ |
| 843 | F46 | A | a | $\gamma$ |
| 844 | F46 | A | b | $\alpha$ |
| 845 | F46 | A | b | $\beta$ |
| 846 | F46 | A | b | $\gamma$ |
| 847 | F46 | A | c | $\alpha$ |
| 848 | F46 | A | c | $\beta$ |
| 849 | F46 | A | c | $\gamma$ |
| 850 | F46 | A | d | $\alpha$ |
| 851 | F46 | A | d | $\beta$ |
| 852 | F46 | A | d | $\gamma$ |
| 853 | F46 | B | a | $\alpha$ |
| 854 | F46 | B | a | $\beta$ |

(continued)

| No. | F | R | Ar | X |
|---|---|---|---|---|
| 855 | F46 | B | a | $\gamma$ |
| 856 | F46 | B | b | $\alpha$ |
| 857 | F46 | B | b | $\beta$ |
| 858 | F46 | B | b | $\gamma$ |
| 859 | F46 | B | c | $\alpha$ |
| 860 | F46 | B | c | $\beta$ |
| 861 | F46 | B | c | $\gamma$ |
| 862 | F46 | B | d | $\alpha$ |
| 863 | F46 | B | d | $\beta$ |
| 864 | F46 | B | d | $\gamma$ |
| 865 | F46 | C | a | $\alpha$ |
| 866 | F46 | C | a | $\beta$ |
| 867 | F46 | C | a | $\gamma$ |
| 868 | F46 | C | b | $\alpha$ |
| 869 | F46 | C | b | $\beta$ |
| 870 | F46 | C | b | $\gamma$ |
| 871 | F46 | C | c | $\alpha$ |
| 872 | F46 | C | c | $\beta$ |
| 873 | F46 | C | c | $\gamma$ |
| 874 | F46 | C | d | $\alpha$ |
| 875 | F46 | C | d | $\beta$ |
| 876 | F46 | C | d | $\gamma$ |
| 877 | F46 | D | a | $\alpha$ |
| 878 | F46 | D | a | $\beta$ |
| 879 | F46 | D | a | $\gamma$ |
| 880 | F46 | D | b | $\alpha$ |
| 881 | F46 | D | b | $\beta$ |
| 882 | F46 | D | b | $\gamma$ |
| 883 | F46 | D | c | $\alpha$ |
| 884 | F46 | D | c | $\beta$ |
| 885 | F46 | D | c | $\gamma$ |
| 886 | F46 | D | d | $\alpha$ |
| 887 | F46 | D | d | $\beta$ |
| 888 | F46 | D | d | $\gamma$ |
| 889 | F47 | A | a | $\alpha$ |
| 890 | F47 | A | a | $\beta$ |
| 891 | F47 | A | a | $\gamma$ |
| 892 | F47 | A | b | $\alpha$ |

(continued)

| No. | F | R | Ar | X |
|---|---|---|---|---|
| 893 | F47 | A | b | $\beta$ |
| 894 | F47 | A | b | $\gamma$ |
| 895 | F47 | A | c | $\alpha$ |
| 896 | F47 | A | c | $\beta$ |
| 897 | F47 | A | c | $\gamma$ |
| 898 | F47 | A | d | $\alpha$ |
| 899 | F47 | A | d | $\beta$ |
| 900 | F47 | A | d | $\gamma$ |
| 901 | F47 | B | a | $\alpha$ |
| 902 | F47 | B | a | $\beta$ |
| 903 | F47 | B | a | $\gamma$ |
| 904 | F47 | B | b | $\alpha$ |
| 905 | F47 | B | b | $\beta$ |
| 906 | F47 | B | b | $\gamma$ |
| 907 | F47 | B | c | $\alpha$ |
| 908 | F47 | B | c | $\beta$ |
| 909 | F47 | B | c | $\gamma$ |
| 910 | F47 | B | d | $\alpha$ |
| 911 | F47 | B | d | $\beta$ |
| 912 | F47 | B | d | $\gamma$ |
| 913 | F47 | C | a | $\alpha$ |
| 914 | F47 | C | a | $\beta$ |
| 915 | F47 | C | a | $\gamma$ |
| 916 | F47 | C | b | $\alpha$ |
| 917 | F47 | C | b | $\beta$ |
| 918 | F47 | C | b | $\gamma$ |
| 919 | F47 | C | c | $\alpha$ |
| 920 | F47 | C | c | $\beta$ |
| 921 | F47 | C | c | $\gamma$ |
| 922 | F47 | C | d | $\alpha$ |
| 923 | F47 | C | d | $\beta$ |
| 924 | F47 | C | d | $\gamma$ |
| 925 | F48 | A | a | $\alpha$ |
| 926 | F48 | A | a | $\beta$ |
| 927 | F48 | A | a | Y |
| 928 | F48 | A | b | $\alpha$ |
| 929 | F48 | A | b | $\beta$ |
| 930 | F48 | A | b | $\gamma$ |

(continued)

| No. | F | R | Ar | X |
|---|---|---|---|---|
| 931 | F48 | A | c | $\alpha$ |
| 932 | F48 | A | c | $\beta$ |
| 933 | F48 | A | c | $\gamma$ |
| 934 | F48 | A | d | $\alpha$ |
| 935 | F48 | A | d | $\beta$ |
| 936 | F48 | A | d | $\gamma$ |
| 937 | F48 | B | a | $\alpha$ |
| 938 | F48 | B | a | $\beta$ |
| 939 | F48 | B | a | $\gamma$ |
| 940 | F48 | B | b | $\alpha$ |
| 941 | F48 | B | b | $\beta$ |
| 942 | F48 | B | b | $\gamma$ |
| 943 | F48 | B | c | $\alpha$ |
| 944 | F48 | B | c | $\beta$ |
| 945 | F48 | B | c | $\gamma$ |
| 946 | F48 | B | d | $\alpha$ |
| 947 | F48 | B | d | $\beta$ |
| 948 | F48 | B | d | $\gamma$ |
| 949 | F48 | C | a | $\alpha$ |
| 950 | F48 | C | a | $\beta$ |
| 951 | F48 | C | a | $\gamma$ |
| 952 | F48 | C | b | $\alpha$ |
| 953 | F48 | C | b | $\beta$ |
| 954 | F48 | C | b | $\gamma$ |
| 955 | F48 | C | c | $\alpha$ |
| 956 | F48 | C | c | $\beta$ |
| 957 | F48 | C | c | $\gamma$ |
| 958 | F48 | C | d | $\alpha$ |
| 959 | F48 | C | d | $\beta$ |
| 960 | F48 | C | d | $\gamma$ |

Table 1-7

| No. | F | R | Ar | X |
|---|---|---|---|---|
| 961 | F49 | A | a | $\alpha$ |
| 962 | F49 | A | a | $\beta$ |
| 963 | F49 | A | a | $\gamma$ |
| 964 | F49 | A | b | $\alpha$ |

(continued)

| No. | F | R | Ar | X |
|---|---|---|---|---|
| 965 | F49 | A | b | $\beta$ |
| 966 | F49 | A | b | $\gamma$ |
| 967 | F49 | A | c | $\alpha$ |
| 968 | F49 | A | c | $\beta$ |
| 969 | F49 | A | c | $\gamma$ |
| 970 | F49 | A | d | $\alpha$ |
| 971 | F49 | A | d | $\beta$ |
| 972 | F49 | A | d | $\gamma$ |
| 973 | F49 | B | a | $\alpha$ |
| 974 | F49 | B | a | $\beta$ |
| 975 | F49 | B | a | $\gamma$ |
| 976 | F49 | B | b | $\alpha$ |
| 977 | F49 | B | b | $\beta$ |
| 978 | F49 | B | b | $\gamma$ |
| 979 | F49 | B | c | $\alpha$ |
| 980 | F49 | B | c | $\beta$ |
| 981 | F49 | B | c | $\gamma$ |
| 982 | F49 | B | d | $\alpha$ |
| 983 | F49 | B | d | $\beta$ |
| 984 | F49 | B | d | $\gamma$ |
| 985 | F49 | C | a | $\alpha$ |
| 986 | F49 | C | a | $\beta$ |
| 987 | F49 | C | a | $\gamma$ |
| 988 | F49 | C | b | $\alpha$ |
| 989 | F49 | C | b | $\beta$ |
| 990 | F49 | C | b | $\gamma$ |
| 991 | F49 | C | c | $\alpha$ |
| 992 | F49 | C | c | $\beta$ |
| 993 | F49 | C | c | $\gamma$ |
| 994 | F49 | C | d | $\alpha$ |
| 995 | F49 | C | d | $\beta$ |
| 996 | F49 | C | d | $\gamma$ |
| 997 | F49 | D | a | $\alpha$ |
| 998 | F49 | D | a | $\beta$ |
| 999 | F49 | D | a | $\gamma$ |
| 1000 | F49 | D | b | $\alpha$ |
| 1001 | F49 | D | b | $\beta$ |
| 1002 | F49 | D | b | $\gamma$ |

(continued)

| No. | F | R | Ar | X |
|---|---|---|---|---|
| 1003 | F49 | D | c | $\alpha$ |
| 1004 | F49 | D | c | $\beta$ |
| 1005 | F49 | D | c | $\gamma$ |
| 1006 | F49 | D | d | $\alpha$ |
| 1007 | F49 | D | d | $\beta$ |
| 1008 | F49 | D | d | $\gamma$ |
| 1009 | F50 | A | a | $\alpha$ |
| 1010 | F50 | A | a | $\beta$ |
| 1011 | F50 | A | a | $\gamma$ |
| 1012 | F50 | A | b | $\alpha$ |
| 1013 | F50 | A | b | $\beta$ |
| 1014 | F50 | A | b | $\gamma$ |
| 1015 | F50 | A | c | $\alpha$ |
| 1016 | F50 | A | c | $\beta$ |
| 1017 | F50 | A | c | $\gamma$ |
| 1018 | F50 | A | d | $\alpha$ |
| 1019 | F50 | A | d | $\beta$ |
| 1020 | F50 | A | d | $\gamma$ |
| 1021 | F50 | B | a | $\alpha$ |
| 1022 | F50 | B | a | $\beta$ |
| 1023 | F50 | B | a | $\gamma$ |
| 1024 | F50 | B | b | $\alpha$ |
| 1025 | F50 | B | b | $\beta$ |
| 1026 | F50 | B | b | $\gamma$ |
| 1027 | F50 | B | c | $\alpha$ |
| 1028 | F50 | B | c | $\beta$ |
| 1029 | F50 | B | c | $\gamma$ |
| 1030 | F50 | B | d | $\alpha$ |
| 1031 | F50 | B | d | $\beta$ |
| 1032 | F50 | B | d | $\gamma$ |
| 1033 | F50 | C | a | $\alpha$ |
| 1034 | F50 | C | a | $\beta$ |
| 1035 | F50 | C | a | $\gamma$ |
| 1036 | F50 | C | b | $\alpha$ |
| 1037 | F50 | C | b | $\beta$ |
| 1038 | F50 | C | b | $\gamma$ |
| 1039 | F50 | C | c | $\alpha$ |
| 1040 | F50 | C | c | $\beta$ |

(continued)

| No. | F | R | Ar | X |
|---|---|---|---|---|
| 1041 | F50 | C | c | $\gamma$ |
| 1042 | F50 | C | d | $\alpha$ |
| 1043 | F50 | C | d | $\beta$ |
| 1044 | F50 | C | d | $\gamma$ |
| 1045 | F51 | A | a | $\alpha$ |
| 1046 | F51 | A | a | $\beta$ |
| 1047 | F51 | A | a | $\gamma$ |
| 1048 | F51 | A | b | $\alpha$ |
| 1049 | F51 | A | b | $\beta$ |
| 1050 | F51 | A | b | $\gamma$ |
| 1051 | F51 | A | c | $\alpha$ |
| 1052 | F51 | A | c | $\beta$ |
| 1053 | F51 | A | c | $\gamma$ |
| 1054 | F51 | A | d | $\alpha$ |
| 1055 | F51 | A | d | $\beta$ |
| 1056 | F51 | A | d | $\gamma$ |
| 1057 | F51 | B | a | $\alpha$ |
| 1058 | F51 | B | a | $\beta$ |
| 1059 | F51 | B | a | $\gamma$ |
| 1060 | F51 | B | b | $\alpha$ |
| 1061 | F51 | B | b | $\beta$ |
| 1062 | F51 | B | b | $\gamma$ |
| 1063 | F51 | B | c | $\alpha$ |
| 1064 | F51 | B | c | $\beta$ |
| 1065 | F51 | B | c | $\gamma$ |
| 1066 | F51 | B | d | $\alpha$ |
| 1067 | F51 | B | d | $\beta$ |
| 1068 | F51 | B | d | $\gamma$ |
| 1069 | F51 | C | a | $\alpha$ |
| 1070 | F51 | C | a | $\beta$ |
| 1071 | F51 | C | a | $\gamma$ |
| 1072 | F51 | C | b | $\alpha$ |
| 1073 | F51 | C | b | $\beta$ |
| 1074 | F51 | C | b | $\gamma$ |
| 1075 | F51 | C | c | $\alpha$ |
| 1076 | F51 | C | c | $\beta$ |
| 1077 | F51 | C | c | $\gamma$ |
| 1078 | F51 | C | d | $\alpha$ |

(continued)

| No. | F | R | Ar | X |
|---|---|---|---|---|
| 1079 | F51 | C | d | β |
| 1080 | F51 | C | d | γ |
| 1081 | F52 | A | a | α |
| 1082 | F52 | A | a | β |
| 1083 | F52 | A | a | γ |
| 1084 | F52 | A | b | α |
| 1085 | F52 | A | b | β |
| 1086 | F52 | A | b | γ |
| 1087 | F52 | A | c | α |
| 1088 | F52 | A | c | β |
| 1089 | F52 | A | c | γ |
| 1090 | F52 | A | d | α |
| 1091 | F52 | A | d | β |
| 1092 | F52 | A | d | γ |
| 1093 | F52 | B | a | α |
| 1094 | F52 | B | a | β |
| 1095 | F52 | B | a | γ |
| 1096 | F52 | B | b | α |
| 1097 | F52 | B | b | β |
| 1098 | F52 | B | b | γ |
| 1099 | F52 | B | c | α |
| 1100 | F52 | B | c | β |
| 1101 | F52 | B | c | γ |
| 1102 | F52 | B | d | α |
| 1103 | F52 | B | d | β |
| 1104 | F52 | B | d | γ |
| 1105 | F52 | C | a | α |
| 1106 | F52 | C | a | β |
| 1107 | F52 | C | a | γ |
| 1108 | F52 | C | b | α |
| 1109 | F52 | C | b | β |
| 1110 | F52 | C | b | γ |
| 1111 | F52 | C | c | α |
| 1112 | F52 | C | c | β |
| 1113 | F52 | C | c | γ |
| 1114 | F52 | C | d | α |
| 1115 | F52 | C | d | β |
| 1116 | F52 | C | d | γ |

(continued)

| No. | F | R | Ar | X |
|---|---|---|---|---|
| 1117 | F52 | D | a | $\alpha$ |
| 1118 | F52 | D | a | $\beta$ |
| 1119 | F52 | D | a | $\gamma$ |
| 1120 | F52 | D | b | $\alpha$ |

Table 1-8

| No. | F | R | Ar | X |
|---|---|---|---|---|
| 1121 | F52 | D | b | $\beta$ |
| 1122 | F52 | D | b | $\gamma$ |
| 1123 | F52 | D | c | $\alpha$ |
| 1124 | F52 | D | c | $\beta$ |
| 1125 | F52 | D | c | $\gamma$ |
| 1126 | F52 | D | d | $\alpha$ |
| 1127 | F52 | D | d | $\beta$ |
| 1128 | F52 | D | d | $\gamma$ |
| 1129 | F53 | A | a | $\alpha$ |
| 1130 | F53 | A | a | $\beta$ |
| 1131 | F53 | A | a | $\gamma$ |
| 1132 | F53 | A | b | $\alpha$ |
| 1133 | F53 | A | b | $\beta$ |
| 1134 | F53 | A | b | $\gamma$ |
| 1135 | F53 | A | c | $\alpha$ |
| 1136 | F53 | A | c | $\beta$ |
| 1137 | F53 | A | c | $\gamma$ |
| 1138 | F53 | A | d | $\alpha$ |
| 1139 | F53 | A | d | $\beta$ |
| 1140 | F53 | A | d | $\gamma$ |
| 1141 | F53 | B | a | $\alpha$ |
| 1142 | F53 | B | a | $\beta$ |
| 1143 | F53 | B | a | $\gamma$ |
| 1144 | F53 | B | b | $\alpha$ |
| 1145 | F53 | B | b | $\beta$ |
| 1146 | F53 | B | b | $\gamma$ |
| 1147 | F53 | B | c | $\alpha$ |
| 1148 | F53 | B | c | $\beta$ |
| 1149 | F53 | B | c | $\gamma$ |
| 1150 | F53 | B | d | $\alpha$ |

(continued)

| No. | F | R | Ar | X |
|---|---|---|---|---|
| 1151 | F53 | B | d | $\beta$ |
| 1152 | F53 | B | d | $\gamma$ |
| 1153 | F53 | C | a | $\alpha$ |
| 1154 | F53 | C | a | $\beta$ |
| 1155 | F53 | C | a | $\gamma$ |
| 1156 | F53 | C | b | $\alpha$ |
| 1157 | F53 | C | b | $\beta$ |
| 1158 | F53 | C | b | $\gamma$ |
| 1159 | F53 | C | c | $\alpha$ |
| 1160 | F53 | C | c | $\beta$ |
| 1161 | F53 | C | c | $\gamma$ |
| 1162 | F53 | C | d | $\alpha$ |
| 1163 | F53 | C | d | $\beta$ |
| 1164 | F53 | C | d | $\gamma$ |
| 1165 | F54 | A | a | $\alpha$ |
| 1166 | F54 | A | a | $\beta$ |
| 1167 | F54 | A | a | $\gamma$ |
| 1168 | F54 | A | b | $\alpha$ |
| 1169 | F54 | A | b | $\beta$ |
| 1170 | F54 | A | b | $\gamma$ |
| 1171 | F54 | A | c | $\alpha$ |
| 1172 | F54 | A | c | $\beta$ |
| 1173 | F54 | A | c | $\gamma$ |
| 1174 | F54 | A | d | $\alpha$ |
| 1175 | F54 | A | d | $\beta$ |
| 1176 | F54 | A | d | $\gamma$ |
| 1177 | F54 | B | a | $\alpha$ |
| 1178 | F54 | B | a | $\beta$ |
| 1179 | F54 | B | a | $\gamma$ |
| 1180 | F54 | B | b | $\alpha$ |
| 1181 | F54 | B | b | $\beta$ |
| 1182 | F54 | B | b | $\gamma$ |
| 1183 | F54 | B | c | $\alpha$ |
| 1184 | F54 | B | c | $\beta$ |
| 1185 | F54 | B | c | $\gamma$ |
| 1186 | F54 | B | d | $\alpha$ |
| 1187 | F54 | B | d | $\beta$ |
| 1188 | F54 | B | d | $\gamma$ |

(continued)

| No. | F | R | Ar | X |
|---|---|---|---|---|
| 1189 | F54 | C | a | $\alpha$ |
| 1190 | F54 | C | a | $\beta$ |
| 1191 | F54 | C | a | $\gamma$ |
| 1192 | F54 | C | b | $\alpha$ |
| 1193 | F54 | C | b | $\beta$ |
| 1194 | F54 | C | b | $\gamma$ |
| 1195 | F54 | C | c | $\alpha$ |
| 1196 | F54 | C | c | $\beta$ |
| 1197 | F54 | C | c | $\gamma$ |
| 1198 | F54 | C | d | $\alpha$ |
| 1199 | F54 | C | d | $\beta$ |
| 1200 | F54 | C | d | $\gamma$ |
| 1201 | F55 | A | a | $\alpha$ |
| 1202 | F55 | A | a | $\beta$ |
| 1203 | F55 | A | a | $\gamma$ |
| 1204 | F55 | A | b | $\alpha$ |
| 1205 | F55 | A | b | $\beta$ |
| 1206 | F55 | A | b | $\gamma$ |
| 1207 | F55 | A | c | $\alpha$ |
| 1208 | F55 | A | c | $\beta$ |
| 1209 | F55 | A | c | $\gamma$ |
| 1210 | F55 | A | d | $\alpha$ |
| 1211 | F55 | A | d | $\beta$ |
| 1212 | F55 | A | d | $\gamma$ |
| 1213 | F55 | B | a | $\alpha$ |
| 1214 | F55 | B | a | $\beta$ |
| 1215 | F55 | B | a | $\gamma$ |
| 1218 | F55 | B | b | $\alpha$ |
| 1217 | F55 | B | b | $\beta$ |
| 1218 | F55 | B | b | $\gamma$ |
| 1219 | F55 | B | c | $\alpha$ |
| 1220 | F55 | B | c | $\beta$ |
| 1221 | F55 | B | c | $\gamma$ |
| 1222 | F55 | B | d | $\alpha$ |
| 1223 | F55 | B | d | $\beta$ |
| 1224 | F55 | B | d | $\gamma$ |
| 1225 | F55 | C | a | $\alpha$ |
| 1226 | F55 | C | a | $\beta$ |

(continued)

| No. | F | R | Ar | X |
|---|---|---|---|---|
| 1227 | F55 | C | a | $\gamma$ |
| 1228 | F55 | C | b | $\alpha$ |
| 1229 | F55 | C | b | $\beta$ |
| 1230 | F55 | C | b | $\gamma$ |
| 1231 | F55 | C | c | $\alpha$ |
| 1232 | F55 | C | c | $\beta$ |
| 1233 | F55 | C | c | $\gamma$ |
| 1234 | F55 | C | d | $\alpha$ |
| 1235 | F55 | C | d | $\beta$ |
| 1236 | F55 | C | d | $\gamma$ |
| 1237 | F55 | D | a | $\alpha$ |
| 1238 | F55 | D | a | $\beta$ |
| 1239 | F55 | D | a | $\gamma$ |
| 1240 | F55 | D | b | $\alpha$ |
| 1241 | F55 | D | b | $\beta$ |
| 1242 | F55 | D | b | $\gamma$ |
| 1243 | F55 | D | c | $\alpha$ |
| 1244 | F55 | D | c | $\beta$ |
| 1245 | F55 | D | c | $\gamma$ |
| 1246 | F55 | D | d | $\alpha$ |
| 1247 | F55 | D | d | $\beta$ |
| 1248 | F55 | D | d | $\gamma$ |
| 1249 | F56 | A | a | $\alpha$ |
| 1250 | F56 | A | a | $\beta$ |
| 1251 | F56 | A | a | $\gamma$ |
| 1252 | F56 | A | b | $\alpha$ |
| 1253 | F56 | A | b | $\beta$ |
| 1254 | F56 | A | b | $\gamma$ |
| 1255 | F56 | A | c | $\alpha$ |
| 1256 | F56 | A | c | $\beta$ |
| 1257 | F56 | A | c | $\gamma$ |
| 1258 | F56 | A | d | $\alpha$ |
| 1259 | F56 | A | d | $\beta$ |
| 1260 | F56 | A | d | $\gamma$ |
| 1261 | F56 | B | a | $\alpha$ |
| 1262 | F56 | B | a | $\beta$ |
| 1263 | F56 | B | a | $\gamma$ |
| 1264 | F56 | B | b | $\alpha$ |

(continued)

| No. | F | R | Ar | X |
|---|---|---|---|---|
| 1265 | F56 | B | b | $\beta$ |
| 1266 | F56 | B | b | $\gamma$ |
| 1267 | F56 | B | c | $\alpha$ |
| 1268 | F56 | B | c | $\beta$ |
| 1269 | F56 | B | c | $\gamma$ |
| 1270 | F56 | B | d | $\alpha$ |
| 1271 | F56 | B | d | $\beta$ |
| 1272 | F56 | B | d | $\gamma$ |
| 1273 | F56 | C | a | $\alpha$ |
| 1274 | F56 | C | a | $\beta$ |
| 1275 | F56 | C | a | $\gamma$ |
| 1276 | F56 | C | b | $\alpha$ |
| 1277 | F56 | C | b | $\beta$ |
| 1278 | F56 | C | b | $\gamma$ |
| 1279 | F56 | C | c | $\alpha$ |
| 1280 | F56 | C | c | $\beta$ |

Table 1-9

| No. | F | R | Ar | X |
|---|---|---|---|---|
| 1281 | F56 | C | c | $\gamma$ |
| 1282 | F56 | C | d | $\alpha$ |
| 1283 | F56 | C | d | $\beta$ |
| 1284 | F56 | C | d | $\gamma$ |
| 1285 | F56 | D | a | $\alpha$ |
| 1286 | F56 | D | a | $\beta$ |
| 1287 | F56 | D | a | $\gamma$ |
| 1288 | F56 | D | b | $\alpha$ |
| 1289 | F56 | D | b | $\beta$ |
| 1290 | F56 | D | b | $\gamma$ |
| 1291 | F56 | D | c | $\alpha$ |
| 1292 | F56 | D | c | $\beta$ |

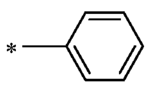
A

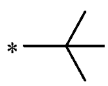
B

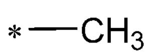
C

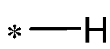
D

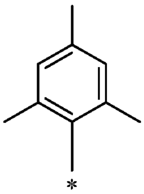

a

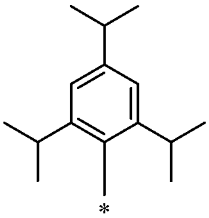

b

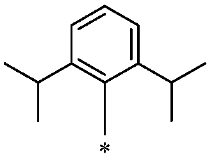

c

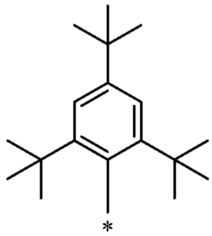

d

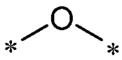

α

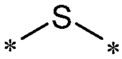

β

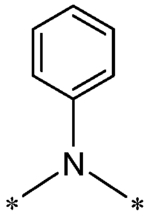

γ

In one aspect of the invention, the skeletons (1a) to (12b) are skeletons in which other rings are not further condensed. In one aspect of the invention, the skeletons (1a) to (12b) are skeletons in which other rings may be further condensed. Regarding other rings mentioned herein, the above descriptions on the ring structures formed by bonding $R^1$ and $R^2$, $R^2$ and $R^3$, $R^3$ and $R^4$, $R^4$ and $R^5$, $R^5$ and $R^6$, $R^6$ and $R^7$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{18}$ and $R^{19}$, $R^{19}$ and $R^{20}$, $R^{20}$ and $R^{21}$, $R^{22}$ and $R^{23}$, $R^{23}$ and $R^{24}$, $R^{24}$ and $R^{25}$, and $R^{25}$ and $R^{26}$ to each other may be referred to.

In one aspect of the invention, $A^1$ and $A^2$ in the formula (1) are acceptor groups. For example, a compound having acceptor groups at positions of $A^1$ and $A^2$ and having any of the skeletons (1a) to (12b) may be mentioned. In relation to descriptions and specific examples of the acceptor group, descriptions, and specific examples of the acceptor group for $A^1$ and $A^2$ in the formula (1) may be referred to.

Hereinafter, specific examples of a compound in which $A^1$ and $A^2$ are acceptor groups will be given. The compounds in which $A^1$ and $A^2$ are acceptor groups, which may be used in the invention, are not construed as limiting to the following specific examples. The following specific examples have structures in which both $A^1$ and $A^2$ are "A", and the structure of each compound is specified by individually specifying the "A".

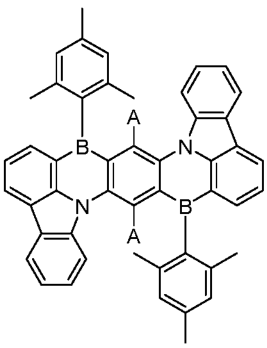

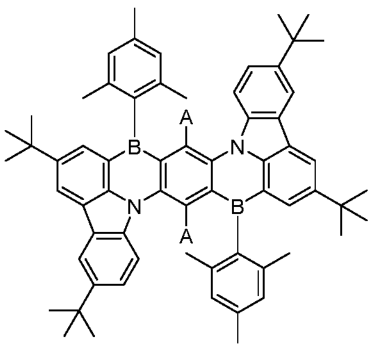

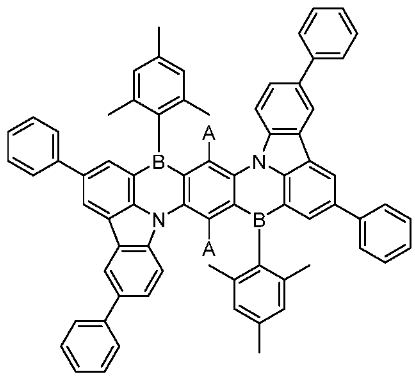

| | | |
|---|---|---|
| 1a:A=A1 | 2a:A=A1 | 3a:A=A1 |
| 1b:A=A2 | 2b:A=A2 | 3b:A=A2 |
| 1c:A=A3 | 2c:A=A3 | 3c:A=A3 |
| 1d:A=A4 | 2d:A=A4 | 3d:A=A4 |
| 1e:A=A7 | 2e:A=A7 | 3e:A=A7 |
| 1f:A=A10 | 2f:A=A10 | 3f:A=A10 |

(continued)

1g:A=A11 2g:A=A11 3g:A=A11
1h:A=A16 2h:A=A16 3h:A=A16
1i:A=A35 2i:A=A35 3i:A=A35
1j:A=A39 2j:A=A39 3j:A=A39
1k:A=A40 2k:A=A40 3k:A=A40
1l:A=A41 2l:A=A41 3l:A=A41
1m:A=A42 2m:A=A42 3m:A=A42

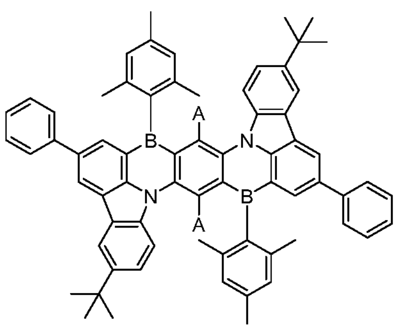
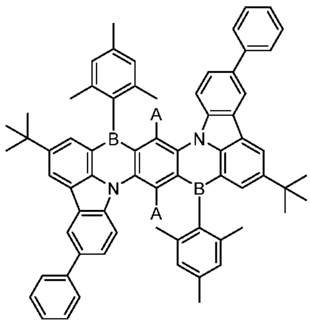
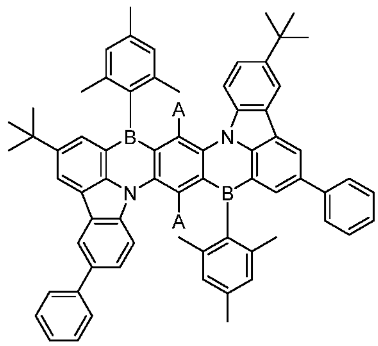

4a:A=A1 5a:A=A1 6a:A=A1
4b:A=A2 5b:A=A2 6b:A=A2
4c:A=A3 5c:A=A3 6c:A=A3
4d:A=A4 5d:A=A4 6d:A=A4
4e:A=A7 5e:A=A7 6e:A=A7
4f:A=A10 5f:A=A10 6f:A=A10
4g:A=A11 5g:A=A11 6g:A=A11
4h:A=A16 5h:A=A16 6h:A=A16
4i:A=A35 5i:A=A35 6i:A=A35
4j:A=A39 5j:A=A39 6j:A=A39
4k:A=A40 5k:A=A40 6k:A=A40
4l:A=A41 5l:A=A41 6l:A=A41
4m:A=A42 5m:A=A42 6m:A=A42

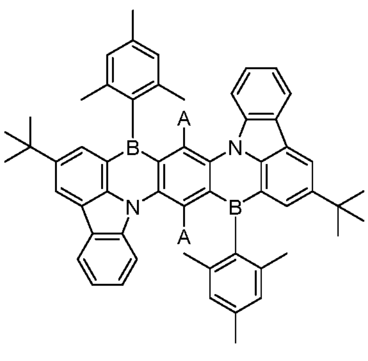
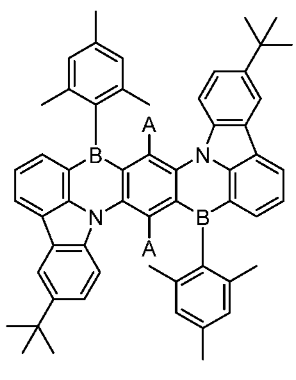
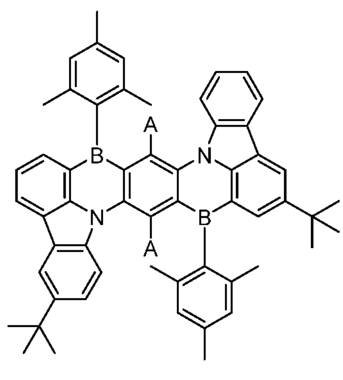

7a:A=A1 8a:A=A1 9a:A=A1
7b:A=A2 8b:A=A2 9b:A=A2
7c:A=A3 8c:A=A3 9c:A=A3

(continued)

| | | |
|---|---|---|
| 7d:A=A4 | 8d:A=A4 | 9d:A=A4 |
| 7e:A=A7 | 8e:A=A7 | 9e:A=A7 |
| 7f:A=A10 | 8f:A=A10 | 9f:A=A10 |
| 7g:A=A11 | 8g:A=A11 | 9g:A=A11 |
| 7h:A=A16 | 8h:A=A16 | 9h:A=A16 |
| 7i:A=A35 | 8i:A=A35 | 9i:A=A35 |
| 7j:A=A39 | 8j:A=A39 | 9j:A=A39 |
| 7k:A=A40 | 8k:A=A40 | 9k:A=A40 |
| 7l:A=A41 | 8l:A=A41 | 9l:A=A41 |
| 7m:A=A42 | 8m:A=A42 | 9m:A=A42 |

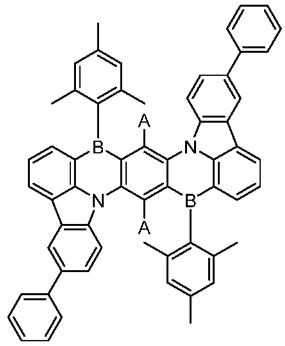

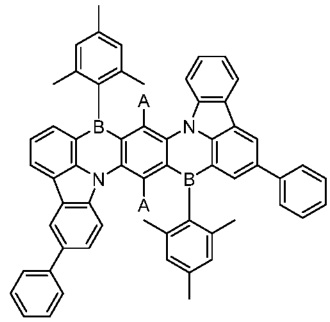

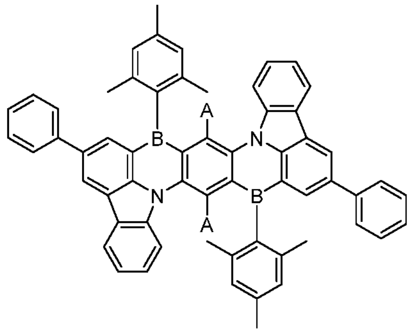

| | | |
|---|---|---|
| 10a:A=A1 | 11a:A=A1 | 12a:A=A1 |
| 10b:A=A2 | 11b:A=A2 | 12b:A=A2 |
| 10c:A=A3 | 11c:A=A3 | 12c:A=A3 |
| 10d:A=A4 | 11d:A=A4 | 12d:A=A4 |
| 10e:A=A7 | 11e:A=A7 | 12e:A=A7 |
| 10f:A=A10 | 11f:A=A10 | 12f:A=A10 |
| 10g:A=A11 | 11g:A=A11 | 12g:A=A11 |
| 10h:A=A16 | 11h:A=A16 | 12h:A=A16 |
| 10i:A=A35 | 11i:A=A35 | 12i:A=A35 |
| 10j:A=A39 | 11i:A=A39 | 12j:A=A39 |
| 10k:A=A40 | 11k:A=A40 | 12k:A=A40 |
| 10l:A=A41 | 11l:A=A41 | 12l:A=A41 |
| 10m:A=A42 | 11m:A=A42 | 12m:A=A42 |

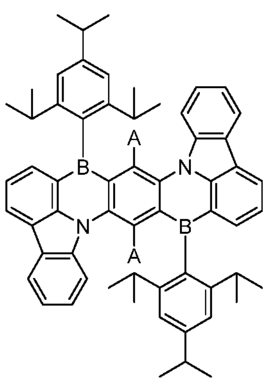

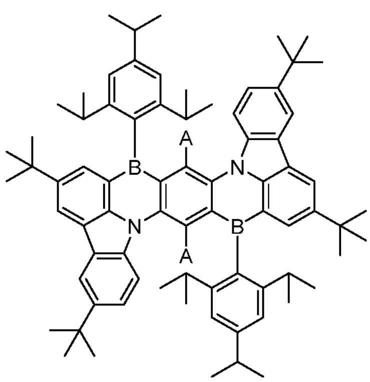

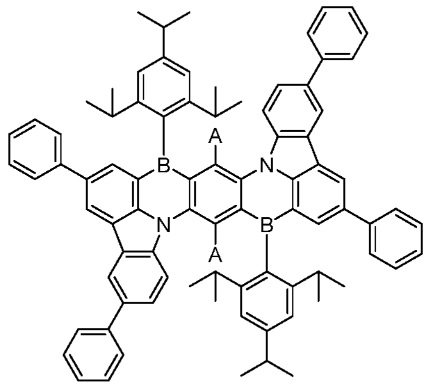

| | | |
|---|---|---|
| 13a:A=A1 | 14a:A=A1 | 15a:A=A1 |
| 13b:A=A2 | 14b:A=A2 | 15b:A=A2 |
| 13c:A=A3 | 14c:A=A3 | 15c:A=A3 |
| 13d:A=A4 | 14d:A=A4 | 15d:A=A4 |
| 13e:A=A7 | 14e:A=A7 | 15e:A=A7 |
| 13f:A=A10 | 14f:A=A10 | 15f:A=A10 |
| 13g:A=A11 | 14g:A=A11 | 15g:A=A11 |
| 13h:A=A16 | 14h:A=A16 | 15h:A=A16 |
| 13i:A=A35 | 14i:A=A35 | 15i:A=A35 |
| 13j:A=A39 | 14j:A=A39 | 15j:A=A39 |
| 13k:A=A40 | 14k:A=A40 | 15k:A=A40 |
| 13l:A=A41 | 14l:A=A41 | 15l:A=A41 |
| 13m:A=A42 | 14m:A=A42 | 15m:A=A42 |

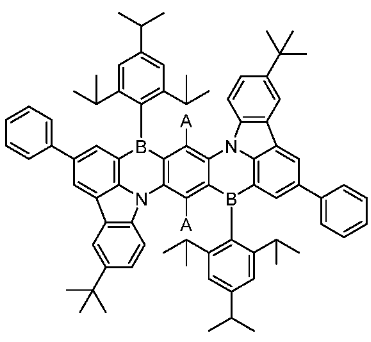
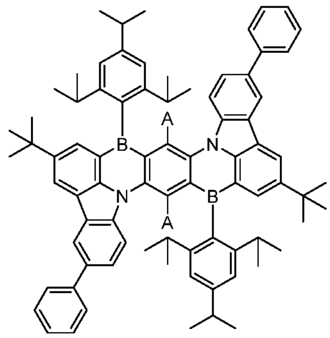
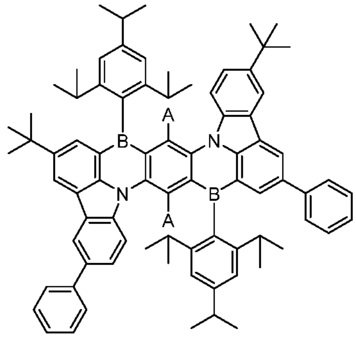

| | | |
|---|---|---|
| 16a:A=A1 | 17a:A=A1 | 18a:A=A1 |
| 16b:A=A2 | 17b:A=A2 | 18b:A=A2 |
| 16c:A=A3 | 17c:A=A3 | 18c:A=A3 |
| 16d:A=A4 | 17d:A=A4 | 18d:A=A4 |
| 16e:A=A7 | 17e:A=A7 | 18e:A=A7 |
| 16f:A=A10 | 17f:A=A10 | 18f:A=A10 |
| 16g:A=A11 | 17g:A=A11 | 18g:A=A11 |
| 16h:A=A16 | 17h:A=A16 | 18h:A=A16 |
| 16i:A=A35 | 17i:A=A35 | 18i:A=A35 |
| 16j:A=A39 | 17j:A=A39 | 18j:A=A39 |
| 16k:A=A40 | 17k:A=A40 | 18k:A=A40 |
| 16l:A=A41 | 17l:A=A41 | 18l:A=A41 |
| 16m:A=A42 | 17m:A=A42 | 18m:A=A42 |

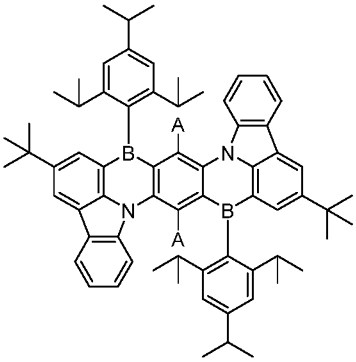

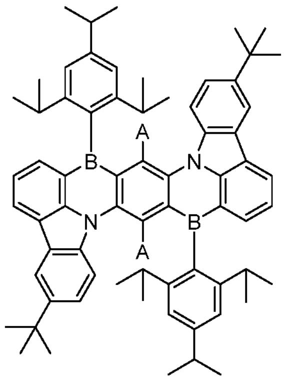

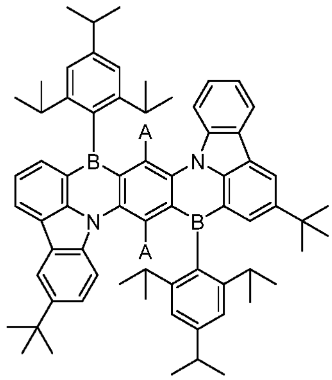

19a:A=A1
19b:A=A2
19c:A=A3
19d:A=A4
19e:A=A7
19f:A=A10
19g:A=A11
19h:A=A16
19i:A=A35
19j:A=A39
19k:A=A40
19l:A=A41
19m:A=A42

20a:A=A1
20b:A=A2
20c:A=A3
20d:A=A4
20e:A=A7
20f:A=A10
20g:A=A11
20h:A=A16
20i:A=A35
20j:A=A39
20k:A=A40
20l:A=A41
20m:A=A42

21a:A=A1
21b:A=A2
21c:A=A3
21d:A=A4
21e:A=A7
21f:A=A10
21g:A=A11
21h:A=A16
21i:A=A35
21j:A=A39
21k:A=A40
21l:A=A41
21m:A=A42

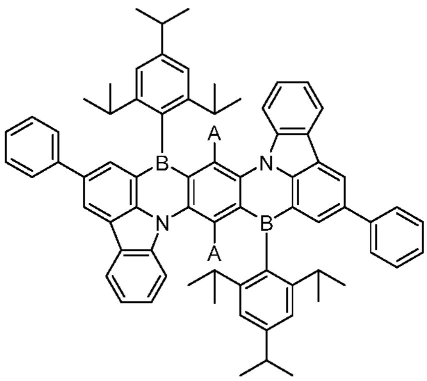

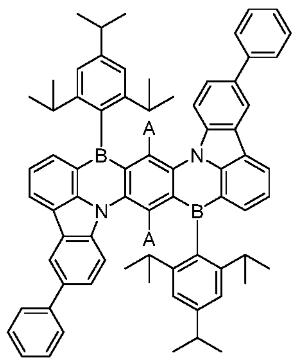

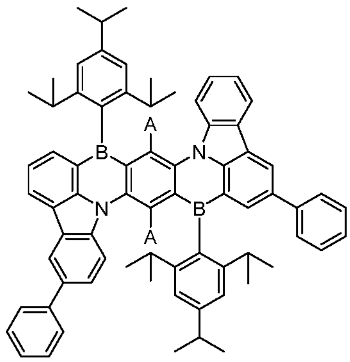

22a:A=A1
22b:A=A2
22c:A=A3
22d:A=A4
22e:A=A7
22f:A=A10
22g:A=A11
22h:A=A16
22i:A=A35
22j:A=A39
22k:A=A40

23a:A=A1
23b:A=A2
23c:A=A3
23d:A=A4
23e:A=A7
23f:A=A10
23g:A=A11
23h:A=A16
23i:A=A35
23j:A=A39
23k:A=A40

24a:A=A1
24b:A=A2
24c:A=A3
24d:A=A4
24e:A=A7
24f:A=A10
24g:A=A11
24h:A=A16
24i:A=A35
24j:A=A39
24k:A=A40

(continued)

| | | |
|---|---|---|
| 22l:A=A41 | 23l:A=A41 | 24l:A=A41 |
| 22m:A=A42 | 23m:A=A42 | 24m:A=A42 |

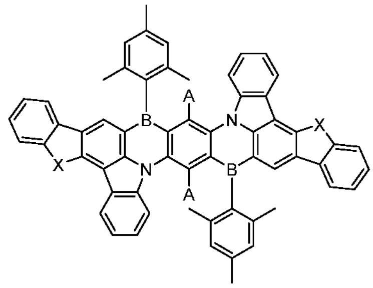
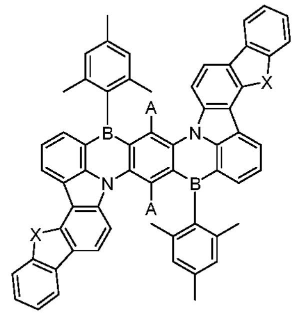
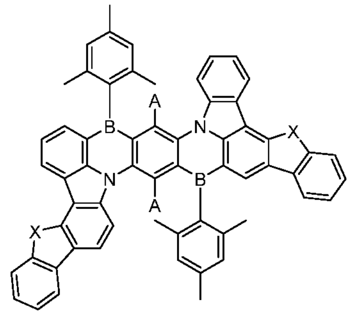

| | | |
|---|---|---|
| 25a:A=A1 | 26a:A=A1 | 27a:A=A1 |
| 25b:A=A2 | 26b:A=A2 | 27b:A=A2 |
| 25c:A=A3 | 26c:A=A3 | 27c:A=A3 |
| 25d:A=A4 | 26d:A=A4 | 27d:A=A4 |
| 25e:A=A7 | 26e:A=A7 | 27e:A=A7 |
| 25f:A=A10 | 26f:A=A10 | 27f:A=A10 |
| 25g:A=A11 | 26g:A=A11 | 27g:A=A11 |
| 25h:A=A16 | 26h:A=A16 | 27h:A=A16 |
| 25i:A=A35 | 26i:A=A35 | 27i:A=A35 |
| 25j:A=A39 | 26i:A=A39 | 27j:A=A39 |
| 25k:A=A40 | 26k:A=A40 | 27k:A=A40 |
| 25l:A=A41 | 26l:A=A41 | 27l:A=A41 |
| 25m:A=A42 | 26m:A=A42 | 27m:A=A42 |

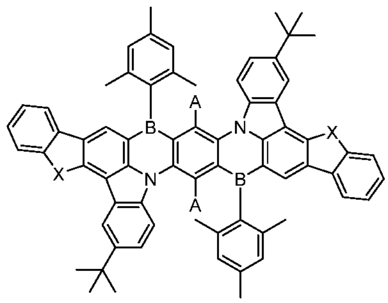
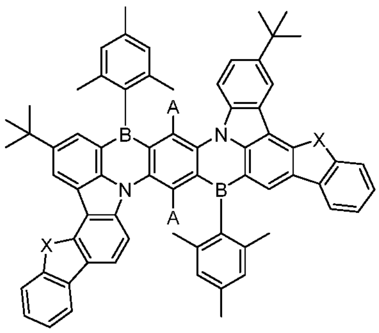
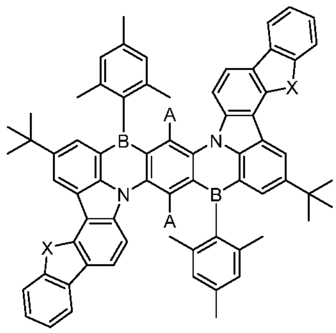

| | | |
|---|---|---|
| 28a:A=A1 | 29a:A=A1 | 30a:A=A1 |
| 28b:A=A2 | 29b:A=A2 | 30b:A=A2 |
| 28c:A=A3 | 29c:A=A3 | 30c:A=A3 |
| 28d:A=A4 | 29d:A=A4 | 30d:A=A4 |
| 28e:A=A7 | 29e:A=A7 | 30e:A=A7 |
| 28f:A=A10 | 29f:A=A10 | 30f:A=A10 |
| 28g:A=A11 | 29g:A=A11 | 30g:A=A11 |
| 28h:A=A16 | 29h:A=A16 | 30h:A=A16 |
| 28i:A=A35 | 29i:A=A35 | 30i:A=A35 |
| 28i:A=A39 | 29j:A=A39 | 30j:A=A39 |

(continued)

28k:A=A40 29k:A=A40 30k:A=A40
28l:A=A41 29l:A=A41 30l:A=A41
28m:A=A42 29m:A=A42 30m:A=A42

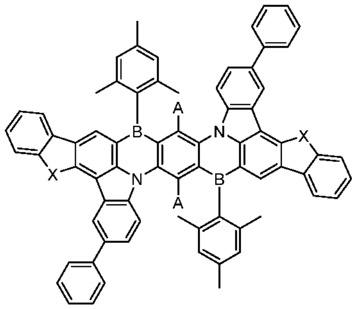
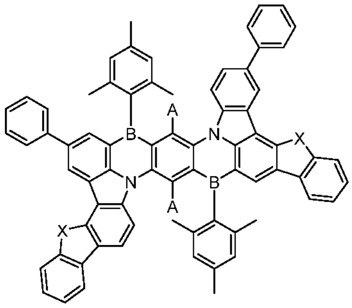
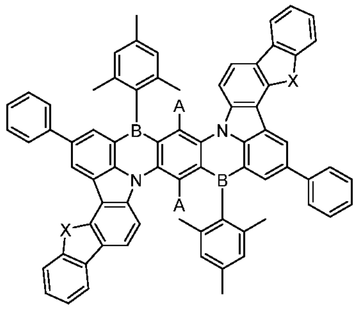

31a:A=A1 32a:A=A1 33a:A=A1
31b:A=A2 32b:A=A2 33b:A=A2
31c:A=A3 32c:A=A3 33c:A=A3
31d:A=A4 32d:A=A4 33d:A=A4
31e:A=A7 32e:A=A7 33e:A=A7
31f:A=A10 32f:A=A10 33f:A=A10
31g:A=A11 32g:A=A11 33g:A=A11
31h:A=A16 32h:A=A16 33h:A=A16
31i:A=A35 32i:A=A35 33i:A=A35
31j:A=A39 32j:A=A39 33j:A=A39
31k:A=A40 32k:A=A40 33k:A=A40
31l:A=A41 32l:A=A41 33l:A=A41
31m:A=A42 32m:A=A42 33m:A=A42

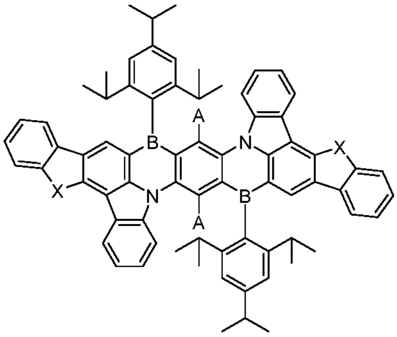
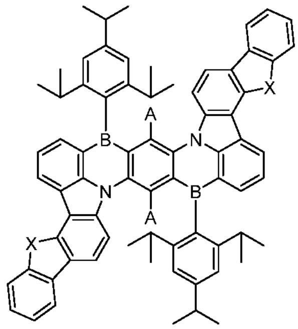
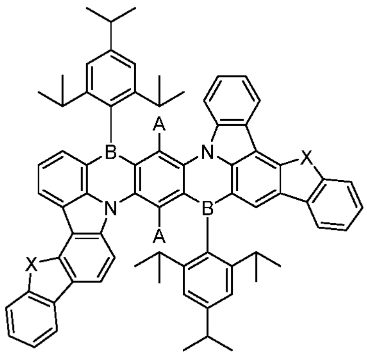

34a:A=A1 35a:A=A1 36a:A=A1
34b:A=A2 35b:A=A2 36b:A=A2
34c:A=A3 35c:A=A3 36c:A=A3
34d:A=A4 35d:A=A4 36d:A=A4
34e:A=A7 35e:A=A7 36e:A=A7
34f:A=A10 35f:A=A10 36f:A=A10
34g:A=A11 35g:A=A11 36g:A=A11
34h:A=A16 35h:A=A16 36h:A=A16
34i:A=A35 35i:A=A35 36i:A=A35

(continued)

| | | |
|---|---|---|
| 34j:A=A39 | 35j:A=A39 | 36j:A=A39 |
| 34k:A=A40 | 35k:A=A40 | 36k:A=A40 |
| 34l:A=A41 | 35i:A=A41 | 36l:A=A41 |
| 34m:A=A42 | 35m:A=A42 | 36m:A=A42 |

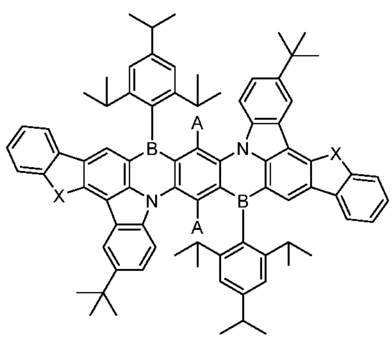
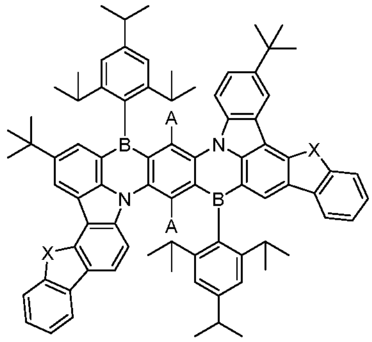
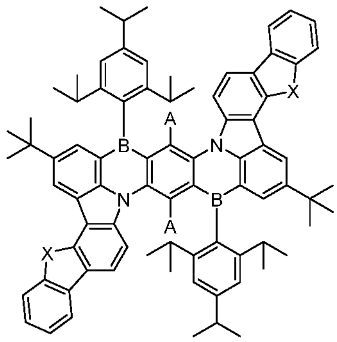

| | | |
|---|---|---|
| 37a:A=A1 | 38a:A=A1 | 39a:A=A1 |
| 37b:A=A2 | 38b:A=A2 | 39b:A=A2 |
| 37c:A=A3 | 38c:A=A3 | 39c:A=A3 |
| 37d:A=A4 | 38d:A=A4 | 39d:A=A4 |
| 37e:A=A7 | 38e:A=A7 | 39e:A=A7 |
| 37f:A=A10 | 38f:A=A10 | 39f:A=A10 |
| 37g:A=A11 | 38g:A=A11 | 39g:A=A11 |
| 37h:A=A16 | 38h:A=A16 | 39h:A=A16 |
| 37i:A=A35 | 38i:A=A35 | 39i:A=A35 |
| 37j:A=A39 | 38j:A=A39 | 39j:A=A39 |
| 37k:A=A40 | 38k:A=A40 | 39k:A=A40 |
| 37l:A=A41 | 38l:A=A41 | 39l:A=A41 |
| 37m:A=A42 | 38m:A=A42 | 39m:A=A42 |

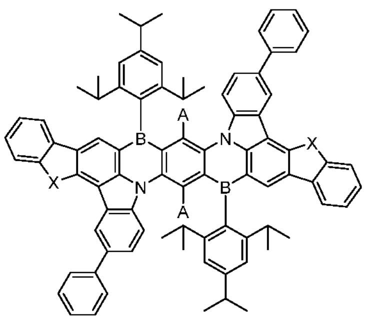
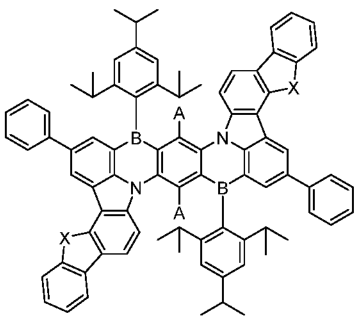
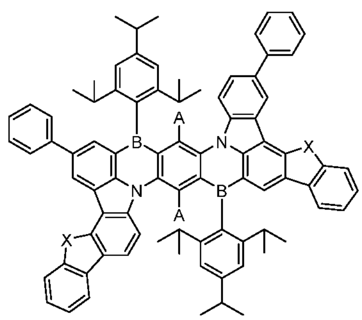

| | | |
|---|---|---|
| 40a:A=A1 | 41a:A=A1 | 42a:A=A1 |
| 40b:A=A2 | 41b:A=A2 | 42b:A=A2 |
| 40c:A=A3 | 41c:A=A3 | 42c:A=A3 |
| 40d:A=A4 | 41d:A=A4 | 42d:A=A4 |
| 40e:A=A7 | 41e:A=A7 | 42e:A=A7 |
| 40f:A=A10 | 41f:A=A10 | 42f:A=A10 |
| 40g:A=A11 | 41g:A=A11 | 42g:A=A11 |
| 40h:A=A16 | 41h:A=A16 | 42h:A=A16 |

(continued)

| | | |
|---|---|---|
| 40i:A=A35 | 41i:A=A35 | 42i:A=A35 |
| 40j:A=A39 | 41j:A=A39 | 42j:A=A39 |
| 40k:A=A40 | 41k:A=A40 | 42k:A=A40 |
| 40l:A=A41 | 41l:A=A41 | 42l:A=A41 |
| 40m:A=A42 | 41m:A=A42 | 42m:A=A42 |

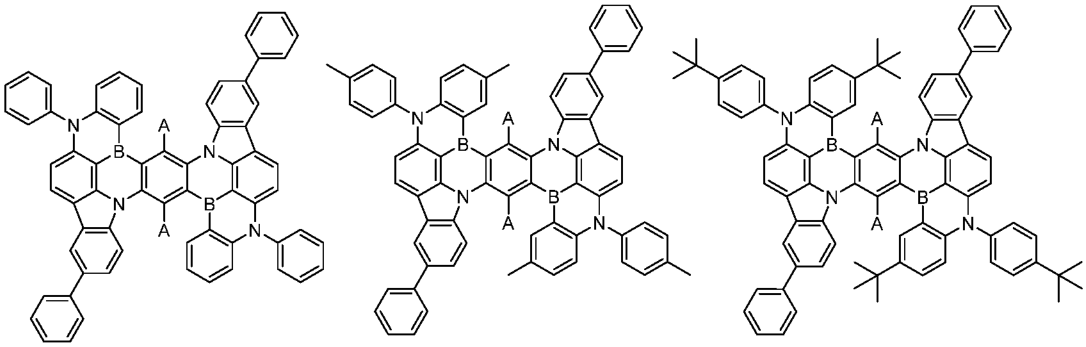

| | | |
|---|---|---|
| 43a:A=A1 | 44a:A=A1 | 45a:A=A1 |
| 43b:A=A2 | 44b:A=A2 | 45b:A=A2 |
| 43c:A=A3 | 44c:A=A3 | 45c:A=A3 |
| 43d:A=A4 | 44d:A=A4 | 45d:A=A4 |
| 43e:A=A7 | 44e:A=A7 | 45e:A=A7 |
| 43f:A=A10 | 44f:A=A10 | 45f:A=A10 |
| 43g:A=A11 | 44g:A=A11 | 45g:A=A11 |
| 43h:A=A16 | 44h:A=A16 | 45h:A=A16 |
| 43i:A=A35 | 44i:A=A35 | 45i:A=A35 |
| 43j:A=A39 | 44j:A=A39 | 45j:A=A39 |
| 43k:A=A40 | 44k:A=A40 | 45k:A=A40 |
| 43l:A=A41 | 44l:A=A41 | 45l:A=A41 |
| 43m:A=A42 | 44m:A=A42 | 45m:A=A42 |

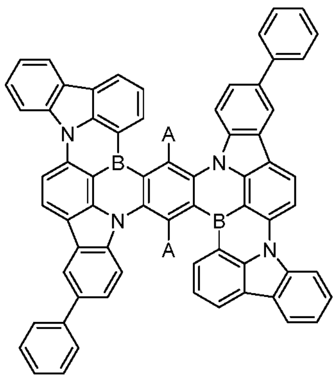

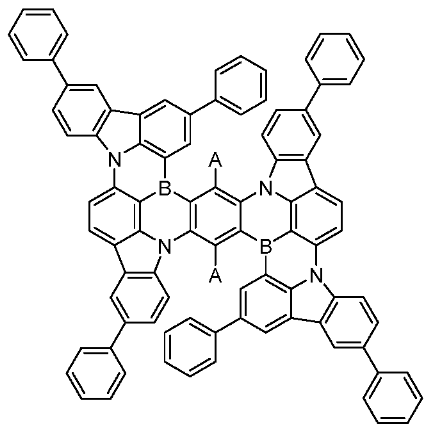

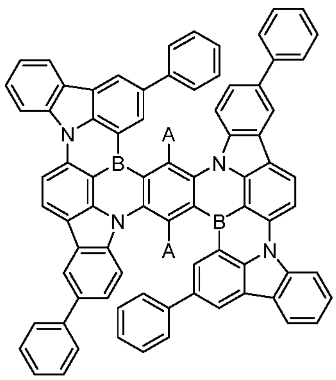

| | | |
|---|---|---|
| 46a:A=A1 | 47a:A=A1 | 48a:A=A1 |
| 46b:A=A2 | 47b:A=A2 | 48b:A=A2 |
| 46c:A=A3 | 47c:A=A3 | 48c:A=A3 |

(continued)

| | | |
|---|---|---|
| 46d:A=A4 | 47d:A=A4 | 48d:A=A4 |
| 46e:A=A7 | 47e:A=A7 | 48e:A=A7 |
| 46f:A=A10 | 47f:A=A10 | 48f:A=A10 |
| 46g:A=A11 | 47g:A=A11 | 48g:A=A11 |
| 46h:A=A16 | 47h:A=A16 | 48h:A=A16 |
| 46i:A=A35 | 47i:A=A35 | 48i:A=A35 |
| 46j:A=A39 | 47j:A=A39 | 48j:A=A39 |
| 46k:A=A40 | 47k:A=A40 | 48k:A=A40 |
| 46l:A=A41 | 47l:A=A41 | 48l:A=A41 |
| 46m:A=A42 | 47m:A=A42 | 48m:A=A42 |

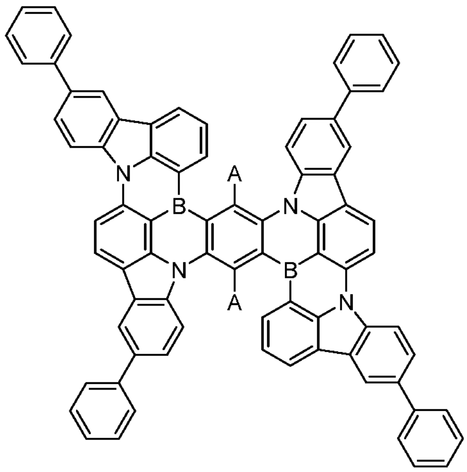

49a:A=A1
49b:A=A2
49c:A=A3
49d:A=A4
49e:A=A7
49f:A=A10
49g:A=A11
49h:A=A16
49i:A=A35
49j:A=A39
49k:A=A40
49l:A=A41
49m:A=A42

In one aspect of the invention, as for the compound represented by the formula (1), a compound having a rotationally symmetric structure is selected. In one aspect of the invention, as the compound represented by the formula (1), a compound having an axially symmetric structure is selected. In one aspect of the invention, as the compound represented by the formula (1), a compound having an asymmetric structure is selected.

Specific examples of a compound having an asymmetric skeleton will be given below. The compounds having asymmetric skeletons or the compounds having asymmetric structures, which may be used in the invention, are not construed as limiting to the following specific examples. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

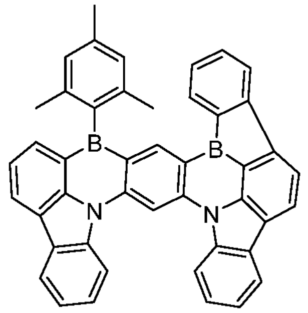
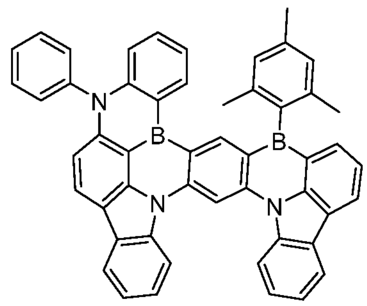
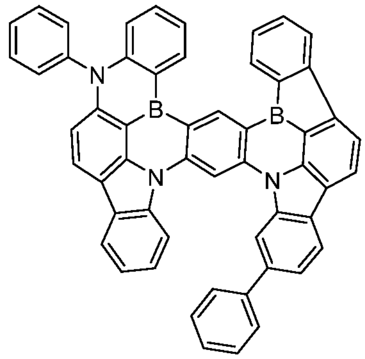
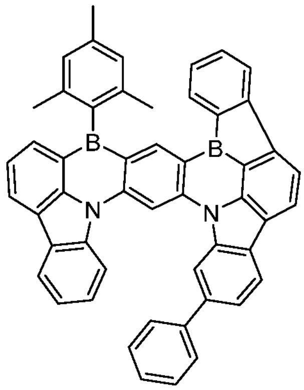
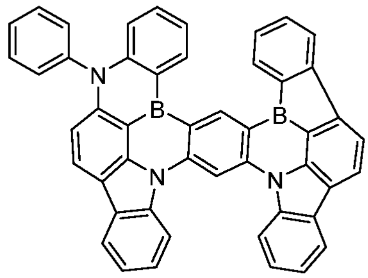
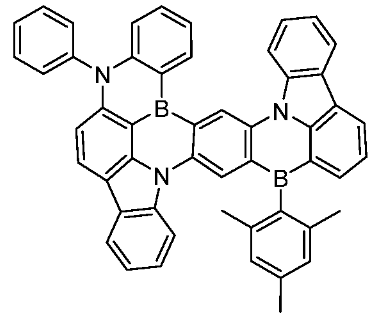
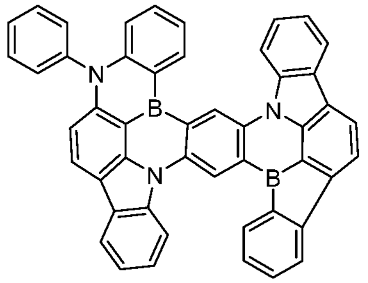
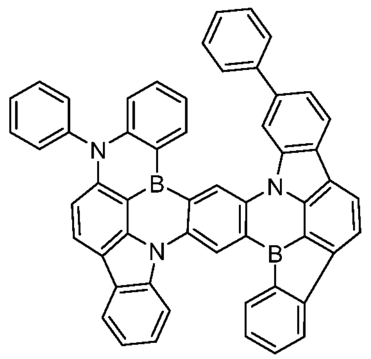
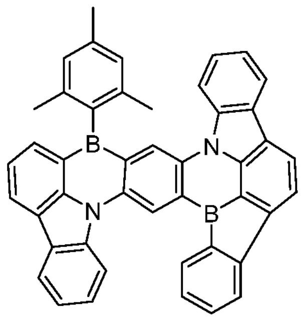
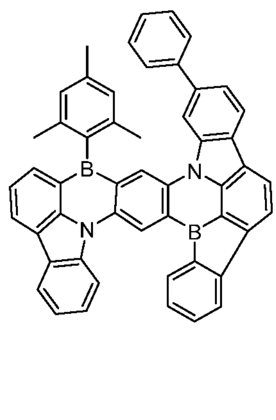
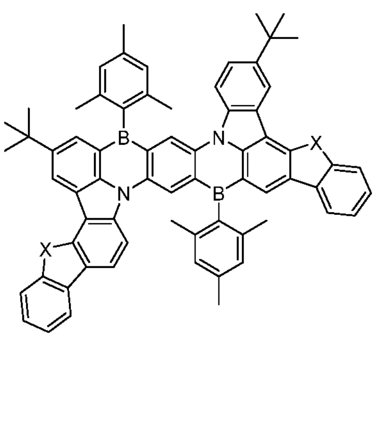
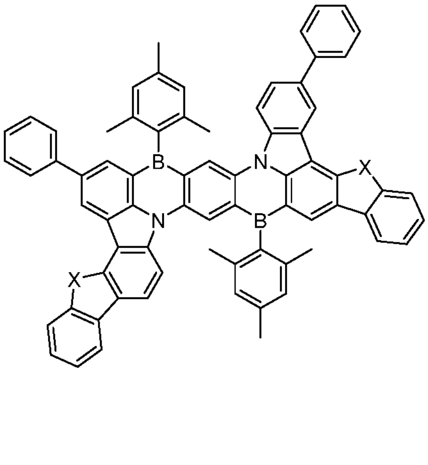

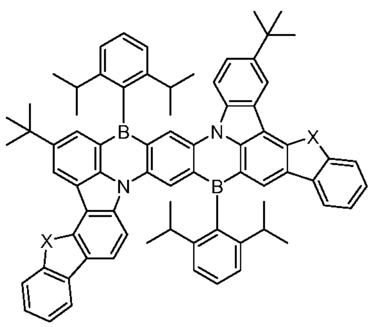

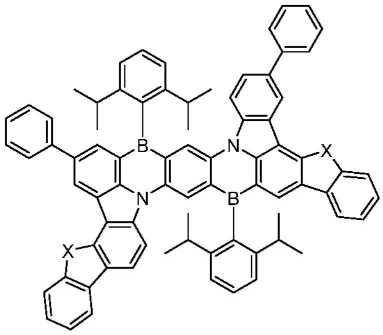

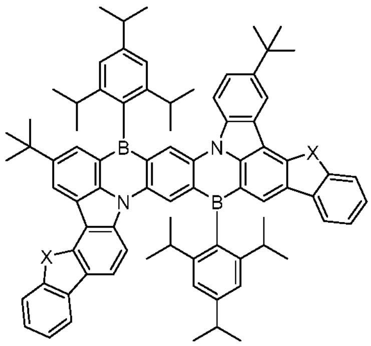

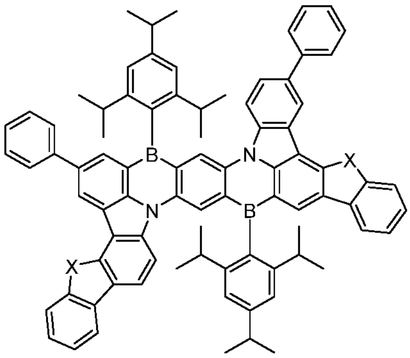

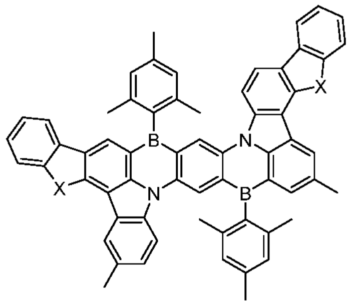

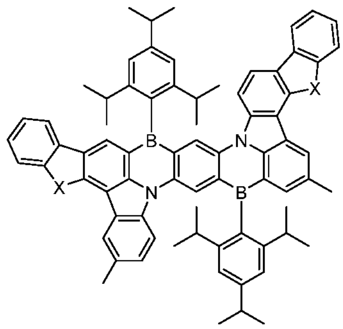

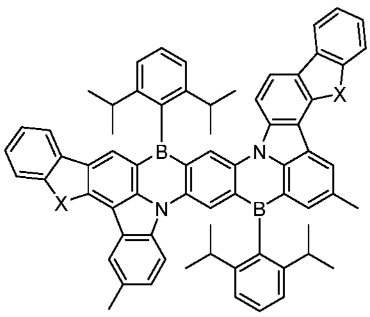

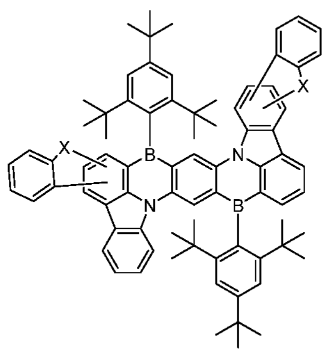

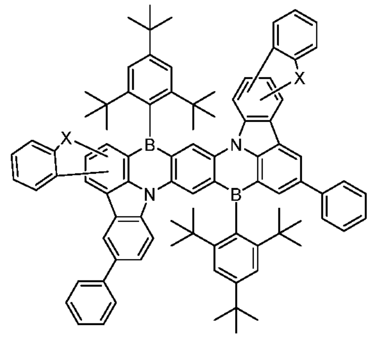

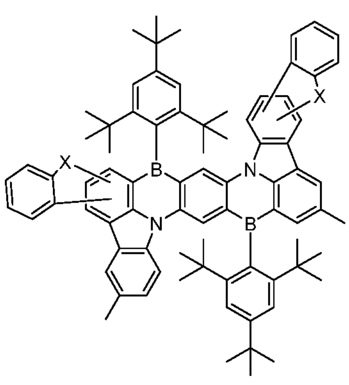

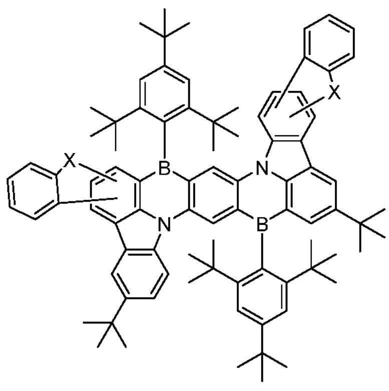

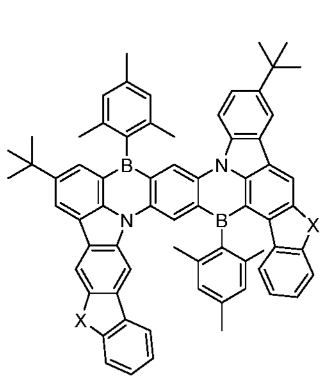

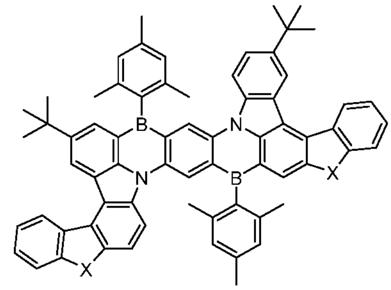

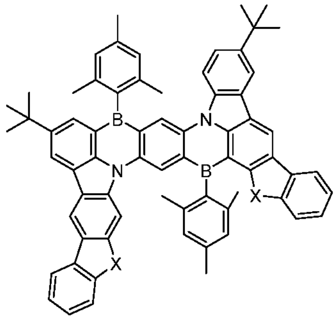

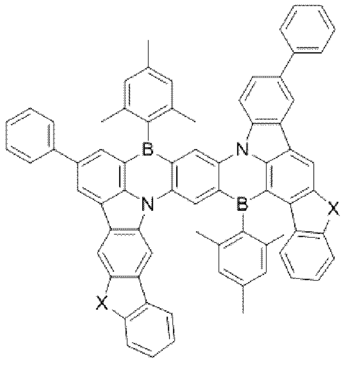

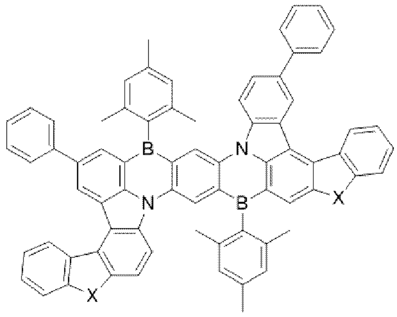

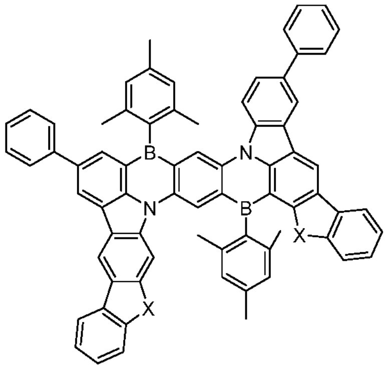

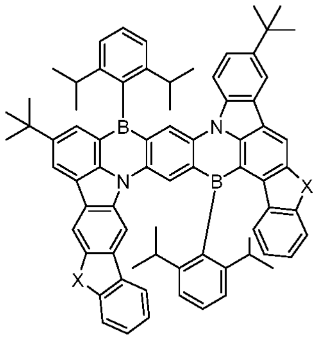

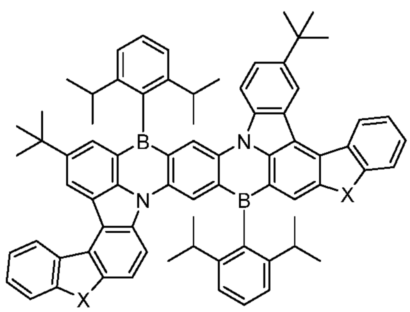

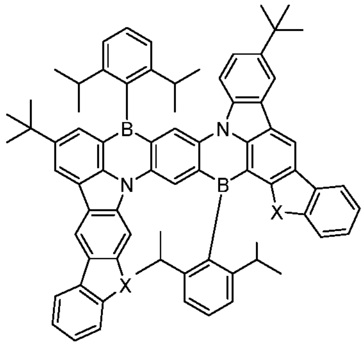

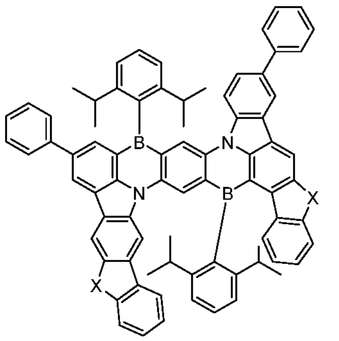

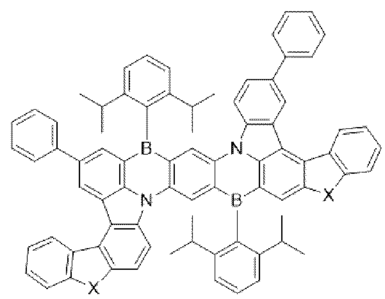

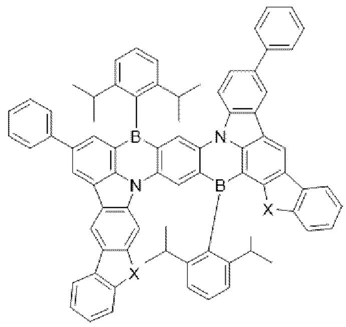

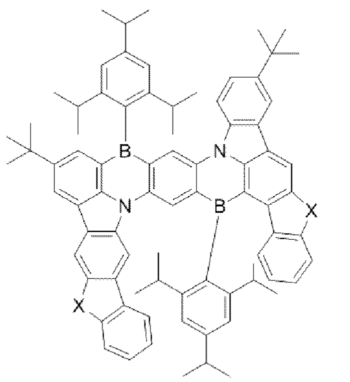

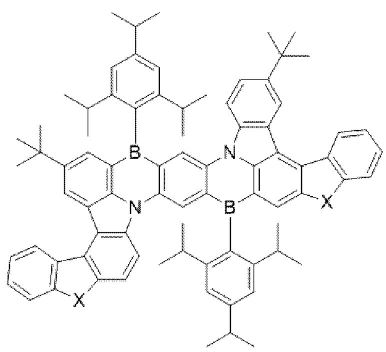
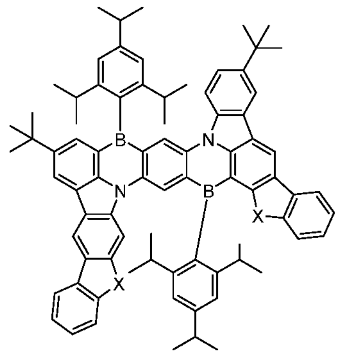
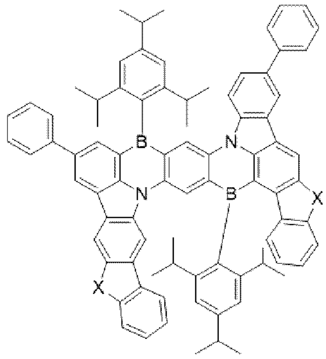

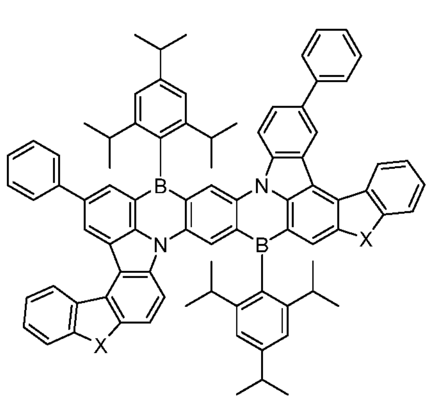
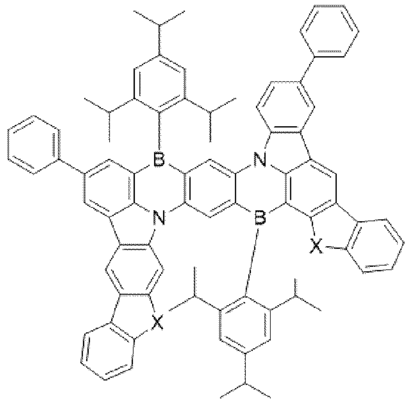

Hereinafter, specific examples of a compound that has a symmetric skeleton but has an asymmetric structure because a substituent is asymmetrically bonded will be given. The compounds having asymmetric structures, which may be used in the invention, are not construed as limiting to the following specific examples.

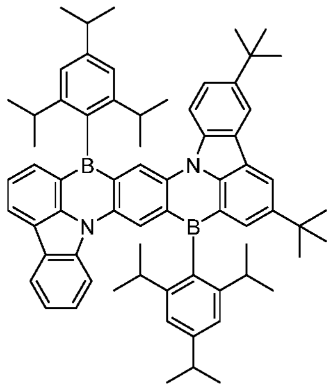
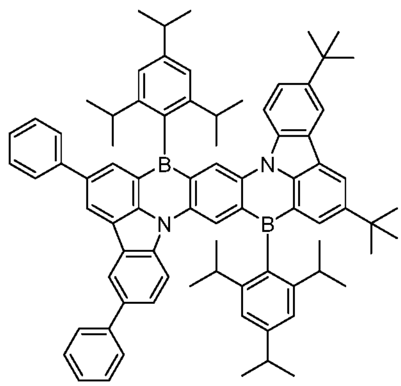
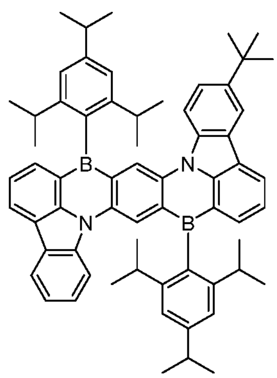

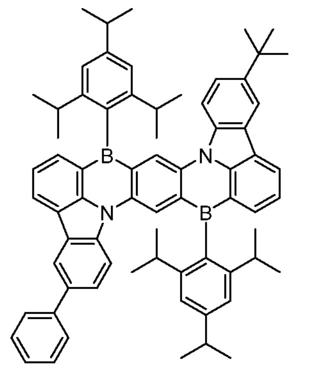
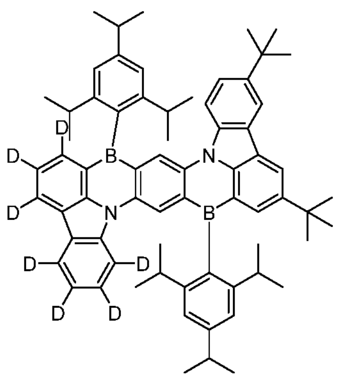
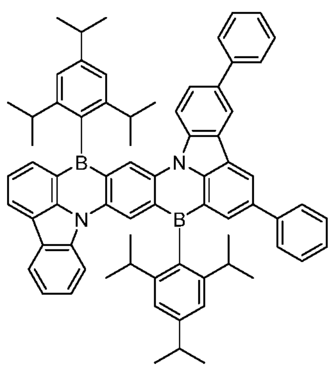

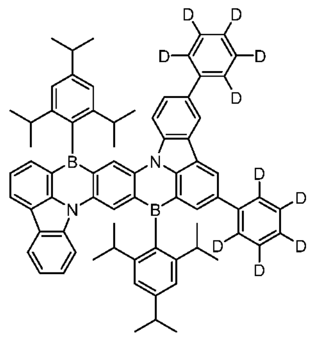
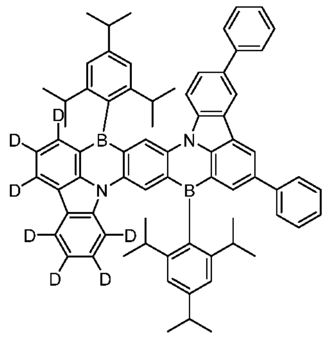
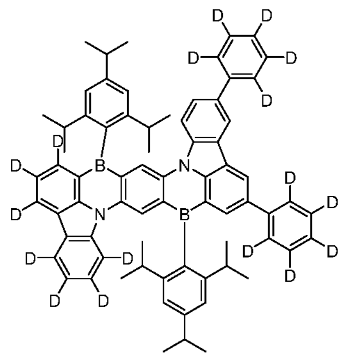

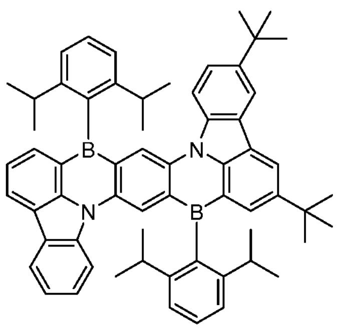
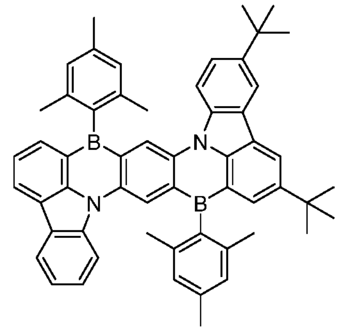
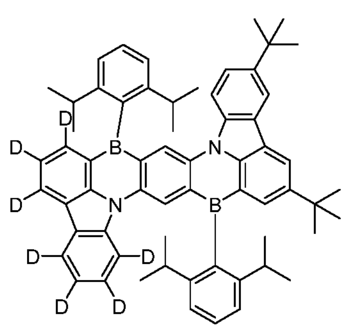

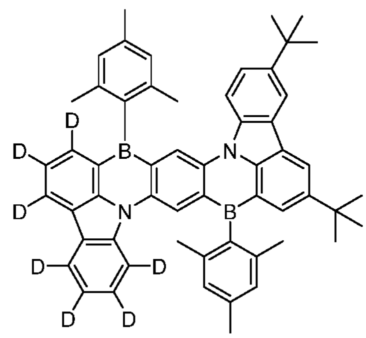

In one aspect of the invention, $R^3$ in the formula (1) is not a diaryl amino group (two aryl groups constituting the diaryl amino group may be bonded to each other). In a preferred aspect of the invention, $R^3$ in the formula (1) is a hydrogen atom, a deuterium atom, or an acceptor group (not a donor group).

In one aspect of the invention, at least one of n1 to n4 in the formula (1a) is 1 or more. In a preferred aspect of the invention, at least one of m1 and m2 in the formula (1a) is 1 or more. In a more preferable aspect of the invention, at least one of n1 to n4 in the formula (1a) is 1 or more, and moreover, at least one of m1 and m2 in the formula (1a) is 1 or more.

In one aspect of the invention, at least one of n5 to n8 in the formula (1b) is 1 or more. In a preferred aspect of the invention, at least one of m3 and m4 in the formula (1b) is 1 or more. In a more preferable aspect of the invention, at least one of n5 to n8 in the formula (1b) is 1 or more, and moreover, at least one of m3 and m4 in the formula (1a) is 1 or more.

When at least one of m1 and m2 is 1 or more, and at least one of m3 and m4 is 1 or more, it is preferable that at least one of $R^{41}$ and $R^{42}$ and at least one of $R^{43}$ and $R^{44}$ are alkyl groups which may be substituted with deuterium atoms. For example, all of $R^{41}$ to $R^{44}$ are alkyl groups which may be substituted with deuterium atoms. When at least one of n1 to n4 is 1 or more, and at least one of n5 to n8 is 1 or more, it is preferable that at least one of $Ar^1$ to $Ar^4$ and at least one of $Ar^5$ to $Ar^8$ are aryl groups which may be substituted with deuterium atoms or alkyl groups. For example, all of $Ar^1$ to $Ar^8$ are aryl groups which may be substituted with deuterium atoms or alkyl groups.

In one aspect of the invention, when $X^1$ in the formula (1) is a boron atom, and $R^8$, $R^{10}$, $R^{12}$, $R^{13}$, $R^{15}$, and $R^{17}$ are alkyl groups (or methyl groups), at least one of $R^1$ to R', $R^{18}$ to $R^{20}$, and $R^{13}$ to $R^{26}$ is a substituent, preferably a group of a substituent group E, and is, for example, an aryl group that may be substituted with a deuterium atom or an alkyl group. In one aspect of the invention, when $X^2$ in the formula (1) is a boron atom, and $R^8$, $R^{10}$, $R^{12}$, $R^{22}$, $R^{24}$, and

$R^{26}$ are alkyl groups (or methyl groups), at least one of $R^1$ to $R^7$, $R^{13}$ to $R^{16}$, and $R^{19}$ to $R^{21}$ is a substituent, preferably a group of a substituent group E, and is, for example, an aryl group that may be substituted with a deuterium atom or an alkyl group.

In one aspect of the invention, when $X^1$ in the formula (1) is a boron atom, and any one of sets of $R^8$ and $R^9$, and $R^9$ and $R^{10}$, and any one of sets of $R^{15}$ and $R^{16}$, and $R^{16}$ and $R^{17}$ are bonded to each other to form an aromatic ring (or a benzene ring), at least one of $R^1$ to $R^7$, $R^{18}$ to $R^{20}$, and $R^{13}$ to $R^{26}$ is a substituent, preferably a group of a substituent group E, and is, for example, an aryl group that may be substituted with a deuterium atom or an alkyl group. In one aspect of the invention, when $X^2$ in the formula (1) is a boron atom, and any one of sets of $R^8$ and $R^9$, and $R^9$ and $R^{10}$, and any one of sets of $R^{22}$ and $R^{23}$, and $R^{23}$ and $R^{24}$ are bonded to each other to form an aromatic ring (or a benzene ring), at least one of $R^1$ to $R^7$, $R^{13}$ to $R^{16}$, and $R^{19}$ to $R^{21}$ is a substituent, preferably a group of a substituent group E, and is, for example, an aryl group that may be substituted with a deuterium atom or an alkyl group.

In one aspect of the invention, $R^9$ and $R^{11}$ in the formula (1) are neither cyano groups nor alkyl groups. That is, $R^9$ and $R^{11}$ are hydrogen atoms, deuterium atoms, or substituents other than cyano groups and alkyl groups. In one aspect of the invention, $R^9$ and $R^{11}$ in the formula (1) are neither cyano groups nor tert-butyl groups.

In a preferred aspect of the invention, at least one of $R^8$ to $R^{12}$ in the formula (1) is a substituent.

In one aspect of the invention, $R^3$ in the formula (1) is not a substituted amino group or aryl group. In one aspect of the invention, $R^3$ in the formula (1) is not a substituted amino group or phenyl group. In one aspect of the invention, $R^3$ in the formula (1) is not a dimethyl amino group, a diphenyl amino group, or a phenyl group.

In a preferred aspect of the invention, at least one of $R^1$ to $R^{26}$ in the formula (1) is a substituent. More preferably, at least one of $R^1$ to $R^{26}$ is an alkyl group, and is, for example, an alkyl group having 1 to 4 carbon atoms.

The molecular weight of the compound represented by the formula (1) is preferably 1500 or less, more preferably 1200 or less, further preferably 1000 or less, still further preferably 900 or less, for example, when there is an intention to form and use a film of an organic layer containing the compound represented by the formula (1) through a vapor deposition method. The lower limit value of the molecular weight is the molecular weight of the smallest compound in the compound group represented by the formula (1). It is preferably 624 or more.

It is preferable that the compound represented by the formula (1) does not include a metal atom. The metal atom mentioned herein does not include a boron atom. For example, as the compound represented by the formula (1), a compound including an atom selected from the group consisting of a carbon atom, a hydrogen atom, a deuterium atom, a nitrogen atom, an oxygen atom, a sulfur atom, and a boron atom may be selected. For example, as the compound represented by the formula (1), a compound including an atom selected from the group consisting of a carbon atom, a hydrogen atom, a deuterium atom, a nitrogen atom, an oxygen atom, and a boron atom may be selected. For example, as the compound represented by the formula (1), a compound including an atom selected from the group consisting of a carbon atom, a hydrogen atom, a deuterium atom, a nitrogen atom, a sulfur atom, and a boron atom may be selected. For example, as the compound represented by the formula (1), a compound including an atom selected from the group consisting of a carbon atom, a hydrogen atom, a deuterium atom, a nitrogen atom, and a boron atom may be selected. For example, as the compound represented by the formula (1), a compound including an atom selected from the group consisting of a carbon atom, a hydrogen atom, a nitrogen atom, an oxygen atom, a sulfur atom, and a boron atom may be selected.

The compound represented by the formula (1) can be synthesized by combining existing reactions. For example, synthesis can be performed by using a ring-closure reaction, or using a substitution reaction.

In the present specification, the "alkyl group" may take any of linear, branched, and cyclic shapes. Further, two or more types of the linear portion, the cyclic portion, and the branched portion may be mixed. The number of carbon atoms of the alkyl group may be, for example, one or more, two or more, or four or more. Further, the number of carbon atoms may be 30 or less, 20 or less, 10 or less, 6 or less, or 4 or less. Specific examples of the alkyl group include a methyl group, an ethyl group, a n-propyl group, an isopropyl group, a n-butyl group, an isobutyl group, a tert-butyl group, a n-pentyl group, an isopentyl group, a n-hexyl group, an isohexyl group, a 2-ethylhexyl group, a n-heptyl group, an isoheptyl group, a n-octyl group, an isooctyl group, a n-nonyl group, an isononyl group, a n-decanyl group, an isodecanyl group, a cyclopentyl group, a cyclohexyl group, and a cycloheptyl group. The alkyl group as a substituent may be further substituted with an aryl group.

The "alkenyl group" may take any of linear, branched, and cyclic shapes. Further, two or more types of the linear portion, the cyclic portion, and the branched portion may be mixed. The number of carbon atoms of the alkenyl group may be, for example, two or more, or four or more. Further, the number of carbon atoms may be 30 or less, 20 or less, 10 or less, 6 or less, or 4 or less. Specific examples of the alkenyl group include an ethenyl group, a n-propenyl group, an isopropenyl group, a n-butenyl group, an isobutenyl group, a n-pentenyl group, an isopentenyl group, a n-hexenyl group, an isohexenyl group, and a 2-ethyl hexenyl group. The alkenyl group as a substituent may be further substituted with a substituent.

The "aryl group" and the "heteroaryl group" may be monocycles, or may be condensed rings in which two or more rings are condensed. In the case of the condensed ring, the number of rings for condensation is preferably two to

six, and, for example, may be selected from two to four. Specific examples of the ring include a benzene ring, a pyridine ring, a pyrimidine ring, a triazine ring, a naphthalene ring, an anthracene ring, a phenanthrene ring, a triphenylene ring, a quinoline ring, a pyrazine ring, a quinoxaline ring, and a naphthiridine ring, and these may be condensed to form a ring. Specific examples of the aryl group or the heteroaryl group include a phenyl group, a 1-naphthyl group, a 2-naphthyl group, a 1-anthrasenyl group, a 2-anthrasenyl group, a 9-anthrasenyl group, a 2-pyridyl group, a 3-pyridyl group, and a 4-pyridyl group. The number of ring skeleton forming atoms of the aryl group is preferably 6 to 40, more preferably 6 to 20, and may be selected in a range of 6 to 14, or selected in a range of 6 to 10. The number of ring skeleton forming atoms of the heteroaryl group is preferably 4 to 40, more preferably 5 to 20, and may be selected in a range of 5 to 14, or selected in a range of 5 to 10. The "arylene group" and the "heteroaryl group" may be those obtained by changing the valence in the descriptions for the aryl group and the heteroaryl group, from 1 to 2.

The "substituent group A" in the present specification means one group selected from the group consisting of a hydroxy group, a halogen atom (e.g., a fluorine atom, a chlorine atom, a bromine atom, an iodine atom), an alkyl group (e.g., 1 to 40 carbon atoms), an alkoxy group (e.g., 1 to 40 carbon atoms), an alkylthio group (e.g., 1 to 40 carbon atoms), an aryl group (e.g., 6 to 30 carbon atoms), an aryl oxy group (e.g., 6 to 30 carbon atoms), an arylthio group (e.g., 6 to 30 carbon atoms), a heteroaryl group (e.g., 5 to 30 ring skeleton forming atoms), a heteroaryl oxy group (e.g., 5 to 30 ring skeleton forming atoms), a heteroarylthio group (e.g., 5 to 30 ring skeleton forming atoms), an acyl group (e.g., 1 to 40 carbon atoms), an alkenyl group (e.g., 1 to 40 carbon atoms), an alkynyl group (e.g., 1 to 40 carbon atoms), an alkoxycarbonyl group (e.g., 1 to 40 carbon atoms), an aryl oxycarbonyl group (e.g., 1 to 40 carbon atoms), a heteroaryl oxycarbonyl group (e.g., 1 to 40 carbon atoms), a silyl group (e.g., a trialkyl silyl group having 1 to 40 carbon atoms) and a nitro group, or a group formed by combining two or more thereof.

The "substituent group B" in the present specification means one group selected from the group consisting of an alkyl group (e.g., 1 to 40 carbon atoms), an alkoxy group (e.g., 1 to 40 carbon atoms), an aryl group (e.g., 6 to 30 carbon atoms), an aryl oxy group (e.g., 6 to 30 carbon atoms), a heteroaryl group (e.g., 5 to 30 ring skeleton forming atoms), a heteroaryl oxy group (e.g., 5 to 30 ring skeleton forming atoms), and a diaryl aminoamino group (e.g., 0 to 20 carbon atoms), or a group formed by combining two or more thereof.

The "substituent group C" in the present specification means one group selected from the group consisting of an alkyl group (e.g., 1 to 20 carbon atoms), an aryl group (e.g., 6 to 22 carbon atoms), a heteroaryl group (e.g., 5 to 20 ring skeleton forming atoms), and a diaryl amino group (e.g., 12 to 20 carbon atoms), or a group formed by combining two or more thereof.

The "substituent group D" in the present specification means one group selected from the group consisting of an alkyl group (e.g., 1 to 20 carbon atoms), an aryl group (e.g., 6 to 22 carbon atoms) and a heteroaryl group (e.g., 5 to 20 ring skeleton forming atoms), or a group formed by combining two or more thereof.

The "substituent group E" in the present specification means one group selected from the group consisting of an alkyl group (e.g., 1 to 20 carbon atoms) and an aryl group (e.g., 6 to 22 carbon atoms), or a group formed by combining two or more thereof.

In the present specification, in the case of the description of "substituent" or "substituted or unsubstituted", the substituent may be selected from, for example, the substituent group A, may be selected from the substituent group B, may be selected from the substituent group C, may be selected from the substituent group D, or may be selected from the substituent group E.

[Compound represented by formula (2)]

Next, a compound represented by the following formula (2) will be described.

Formula (2)

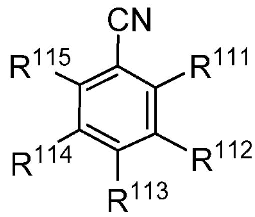

In the formula (2), one of $R^{112}$ and $R^{113}$ represents a cyano group or a substituted or unsubstituted triazinyl group.

In one aspect of the invention, $R^{112}$ is a cyano group. In one aspect of the invention, $R^{113}$ is a cyano group. In one aspect of the invention, $R^{112}$ is a substituted or unsubstituted triazinyl group. In one aspect of the invention, $R^{113}$ is a substituted or unsubstituted triazinyl group.

As the substituent of the triazinyl group, a group selected from any of substituent groups A to E may be employed, but one or more groups selected from the group consisting of an alkyl group, an aryl group, and a heteroaryl group are preferable, and a group of the substituent group E is more preferable. Examples of the substituent of the triazinyl group include an aryl group which may be substituted with one group selected from the group consisting of an alkyl group and an aryl group, or a group formed by combining two or more thereof. Examples of the substituent of the triazinyl group include a unsubstituted aryl group. As the triazinyl group, a 2,4,6-triazinyl group in which the 3-position and the 5-position may be substituted is preferable, a 2,4,6-triazinyl group in which both of the 3-position and the 5-position are substituted is more preferable, and a 2,4,6-triazinyl group in which both of the 3-position and the 5-position are substituted with a substituted or unsubstituted aryl group is further preferable.

In the formula (2), each of the other of $R^{112}$ and $R^{113}$ (that is, one which is not a cyano group or a substituted or unsubstituted triazinyl group), and $R^{111}$, $R^{114}$, and $R^{115}$ independently represents a hydrogen atom, a deuterium atom, a donor group having a substituted amino group, or a substituted or unsubstituted aryl group (excluding the donor group having a substituted amino group). Here, each of the other of $R^{112}$ and $R^{113}$, and at least one of $R^{111}$, $R^{114}$, and $R^{115}$ is independently a carbazole-9-yl group (the carbazole-9-yl group is at least substituted, or has a ring further condensed).

The donor group mentioned herein means a group having a negative Hammett $\sigma p$ value described above. In one aspect of the invention, the $\sigma p$ value of the donor group is -0.2 or less. In one aspect of the invention, the $\sigma p$ value of the donor group is -0.4 or less. The $\sigma p$ value of the donor group is -0.6 or less.

The donor group having a substituted amino group means a group having a substituted amino group in the donor group, may be a substituted amino group, or may be a group to which a substituted amino group is bonded. A typical donor group having a substituted amino group has a structure represented by the following formula (3).

Formula (3)

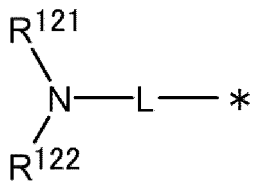

In the formula (3), each of $R^{121}$ and $R^{122}$ independently represents a substituted or unsubstituted alkyl group, a substituted or unsubstituted alkenyl group, a substituted or unsubstituted aryl group, or a substituted or unsubstituted heteroaryl group. $R^{121}$ and $R^{122}$ may be bonded to each other to form a ring structure. L represents a single bond, a substituted or unsubstituted arylene group, or a substituted or unsubstituted heteroarylene group. As the substituent, a group selected from any of the substituent groups A to E may be employed, but a substituted or unsubstituted alkyl group, a substituted or unsubstituted aryl group, or a substituted or unsubstituted heteroaryl group is preferable, and a group selected from the substituent group E is more preferable. When a plurality of substituents are introduced, the substituents may be the same or different. Further, the substituent which can be introduced into the arylene group or the heteroarylene group for L may be a group represented by the formula (3). * represents a bond position to a carbon atom (C) constituting the ring skeleton of the ring in the formula (2).

As the group represented by the formula (3), a group having a carbazole-9-yl structure is preferable. The carbazole-9-yl structure includes not only a substituted or unsubstituted carbazole-9-yl group (a ring may be further condensed), but also a group substituted with a substituted or unsubstituted carbazole-9-yl group (a ring may be further condensed). Further, the group may have two or more carbazole-9-yl structures.

The ring condensed in the carbazole-9-yl structure is one ring selected from the group consisting of an aromatic hydrocarbon ring, a hetero-aromatic ring, an aliphatic hydrocarbon ring, and a hetero-aliphatic ring, or a ring in which two or more thereof are condensed. In a case where two or more rings are condensed, two or more rings of the same type may be condensed, or two or more rings of different types may be condensed. Examples of the former include a naphthalene ring in which two benzene rings are condensed, and examples of the latter include a benzofuran ring in which a benzene ring and a furan ring are condensed. As the condensed ring, one ring selected from the group consisting of an aromatic hydrocarbon ring and a hetero-aromatic ring, or a ring in which two or more thereof are condensed is preferable.

Examples of the aromatic hydrocarbon ring include a benzene ring. The hetero-aromatic ring means a ring which includes hetero atoms as ring skeleton forming atoms and exhibits aromaticity, and is preferably 5 to 7-membered rings, and for example, a 5-membered ring or a 6-membered ring may be employed. In one aspect of the invention, a furan ring, a thiophene ring, and a pyrrole ring may be employed as the hetero-aromatic ring. Preferably, a substituent selected from the substituent group E is bonded to nitrogen atoms of the pyrrole ring, more preferably, an aryl group

which may be substituted with an alkyl group or an aryl group is bonded to the nitrogen atoms. As the hetero-aromatic ring, a furan ring and a thiophene ring are preferable. Examples of the aliphatic hydrocarbon ring include a cyclopentadiene ring.

In one aspect of the invention, the ring condensed in the carbazole-9-yl structure is selected from one ring selected from the group consisting of a benzene ring, a furan ring, a thiophene ring, a pyrrole ring, and a cyclopentadiene ring, or a ring in which two or more thereof are condensed. In a preferred aspect of the invention, the ring is selected from one ring selected from the group consisting of a benzene ring, a furan ring, a thiophene ring, and a pyrrole ring, or a ring in which two or more thereof are condensed. In a more preferred aspect of the invention, the ring is selected from one ring selected from the group consisting of a benzene ring, a furan ring, and a thiophene ring, or a ring in which two or more thereof are condensed.

In one aspect of the invention, the ring condensed in the carbazole-9-yl structure is a benzene ring, a naphthalene ring, a benzofuran ring, a benzothiophene ring, and an indole ring. In a preferred aspect of the invention, the ring condensed in the carbazole-9-yl structure is a benzene ring, a naphthalene ring, a benzofuran ring, or a benzothiophene ring. In a further preferred aspect of the invention, the ring condensed in the carbazole-9-yl structure is a benzene ring, a benzofuran ring, or a benzothiophene ring. In a particularly preferred aspect of the invention, the ring condensed in the carbazole-9-yl structure is a benzofuran ring or a benzothiophene ring. The benzofuran ring, the benzothiophene ring, the indole ring, and the indene ring mentioned herein are 5-membered rings and condensed with the benzene ring constituting the carbazole-9-yl structure.

In the carbazole-9-yl structure, the ring may be condensed at one to four locations, preferably, the ring is condensed at one location or two locations, more preferably, the ring is condensed at one location. In a case where the ring is condensed at two or more locations, the condensed rings may be the same or different from each other, and preferably the rings are the same.

The carbazole-9-yl structure in which the ring may be further condensed may be substituted with a substituent. Such a substituent, for example, can be selected from the substituent groups A to E, and is preferably selected from the substituent group E.

The donor group having a substituted amino group is preferably a substituted or unsubstituted carbazole-9-yl group (a ring may be further condensed). For a substituted or unsubstituted carbazole-9-yl group (a ring may be further condensed) mentioned herein, the description and the preferable range of the carbazole-9-yl structure can be referred to.

In the formula (2), each of the other of $R^{112}$ and $R^{113}$, and at least one of $R^{111}$, $R^{114}$, and $R^{115}$ is independently a carbazole-9-yl group (the carbazole-9-yl group mentioned herein is at least substituted, or has a ring further condensed). That is, at least one among donor groups that may be possessed by the other of $R^{112}$ and $R^{113}$, and $R^{111}$, $R^{114}$, and $R^{115}$ is a carbazole-9-yl group (the carbazole-9-yl group mentioned herein is at least substituted, or has a ring further condensed).

In one aspect of the invention, the carbazole-9-yl group mentioned herein is substituted, but does not have a ring further condensed. In one aspect of the invention, the carbazole-9-yl group mentioned herein is not substituted, but has a ring further condensed. In one aspect of the invention, the carbazole-9-yl group mentioned herein is substituted, and has a ring further condensed. For a case where the carbazole-9-yl group is substituted and a case where a ring is further condensed, corresponding descriptions on the carbazole-9-yl structure can be referred to.

In a preferred aspect of the invention, the other of $R^{112}$ and $R^{113}$, and at least one of $R^{111}$, $R^{114}$, and $R^{115}$ have a structure represented by the following formula (4).

Formula (4)

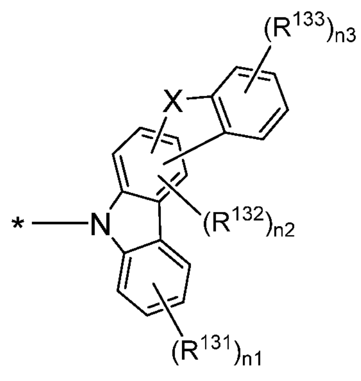

In the formula (4), X represents O, S, N($R^{134}$), or C($R^{135}$)($R^{136}$). X is preferably O, S, or N($R^{134}$), more preferably O or S. In a preferred aspect of the invention, X is O. In a preferred aspect of the invention, X is S. In the formula (4), a

benzene ring on the right top to which $(R^{133})_{n3}$ is bonded, and a benzene ring in the center to which $(R^{132})_{n2}$ is bonded are linked by a bond via X and a single bond, and a position relationship between of such two bonds is not limited. In the formula (4), the bond via X is described on the top, and the single bond is described on the bottom, but the formula (4) also includes a structure in which the bond via X is positioned on the bottom, and the single bond is positioned on the top.

In the formula (4), each of n1 and n3 independently represents any integer of 0 to 4, and is preferably 0 to 2, more preferably 0 or 1. n2 represents any integer of 0 to 2, and is preferably 0 or 1. n1+n2+n3 is any integer of 0 to 10, and for example, 0 to 4, and for example, 0 to 2. In one aspect of the invention, n1 is 1 to 4. In one aspect of the invention, n2 is 1 or 2. In one aspect of the invention, n3 is 1 to 4.

In the formula (4), each of $R^{131}$ to $R^{133}$ independently represents a deuterium atom or a substituent. The substituent, for example, can be selected from the substituent groups A to E, and is preferably selected from the substituent group E.

In the formula (4), $R^{131}$'s bonded to the adjacent ring skeleton forming carbon atoms, $R^{132}$'s bonded to the adjacent ring skeleton forming carbon atoms, and $R^{133}$'s bonded to the adjacent ring skeleton forming carbon atoms may be bonded to each other to form ring structures. For a ring structure to be formed, the description on the ring condensed in the carbazole-9-yl structure can be referred to. In one aspect of the invention, $R^{131}$s bonded to the adjacent ring skeleton forming carbon atoms are bonded to each other to form a ring structure, preferably, to form a benzofuro structure or a benzothieno structure. In one aspect of the invention, $R^{133}$'s bonded to the adjacent ring skeleton forming carbon atoms are bonded to each other to form a ring structure, preferably, to form a benzofuro structure or a benzothieno structure. In one aspect of the invention, $R^{131}$'s, $R^{132}$'s, and $R^{133}$'s are not bonded to each other to form ring structures. $R^{131}$ and $R^{132}$ and $R^{132}$ and $R^{133}$ are not bonded to each other to form ring structures.

**[0191]** * in the formula (4) represents a bond position to a benzene ring skeleton forming carbon atom in the formula (2).

Hereinafter, specific examples of the donor group that can be employed as the other of $R^{112}$ and $R^{113}$, and $R^{111}$, $R^{114}$, and $R^{115}$ will be illustrated. Here, the donor group that can be adopted in the invention is not construed as limiting to the following specific examples. In the following specific examples, * represents a bond position. The indication of a methyl group is omitted, and thus, for example, D2 has one methyl group.

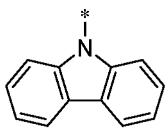
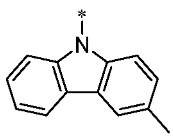
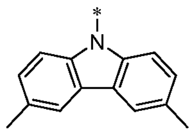
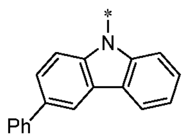
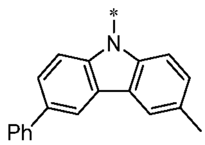

D1 D2 D3 D4 D5

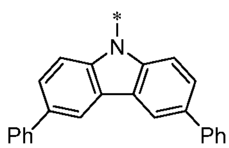
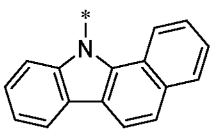
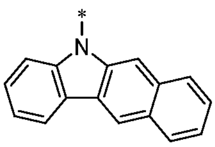
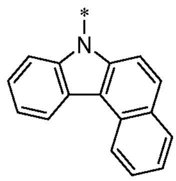
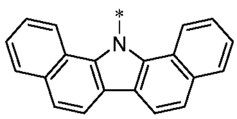

D6 D7 D8 D9 D10

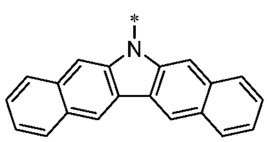
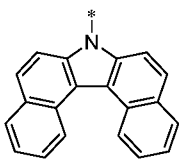
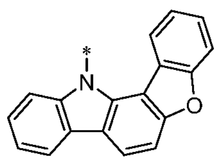
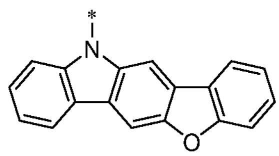

D11 D12 D13 D14

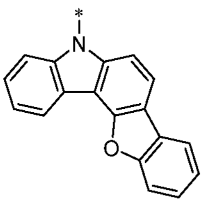
D15
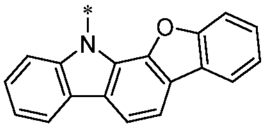
D16
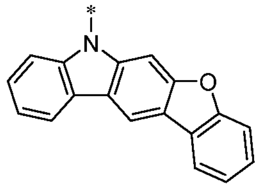
D17
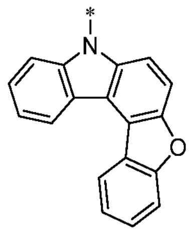
D18

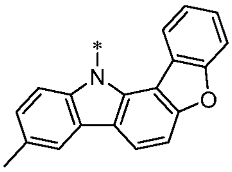
D19
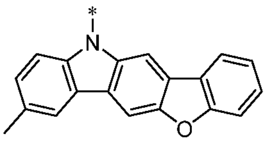
D20
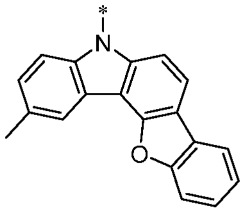
D21
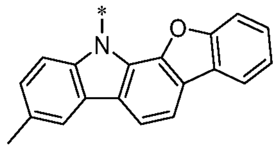
D22

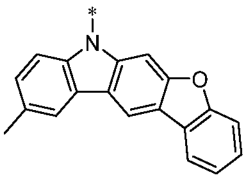
D23
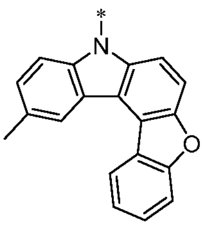
D24
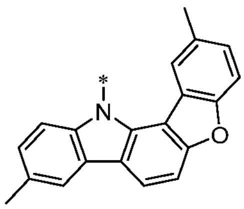
D25
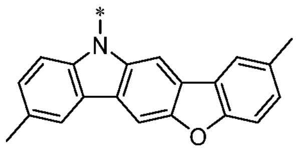
D26

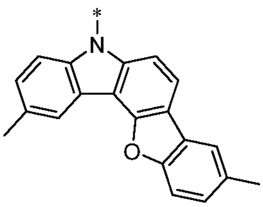
D27
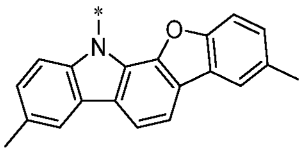
D28
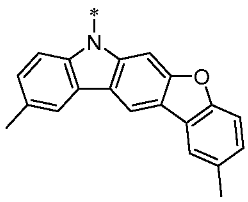
D29
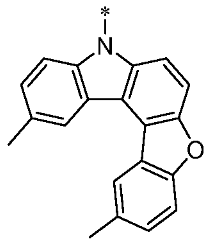
D30

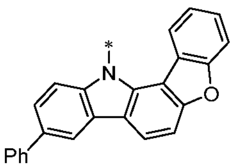
D31
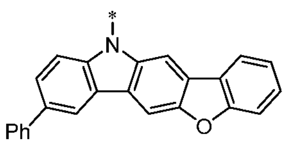
D32
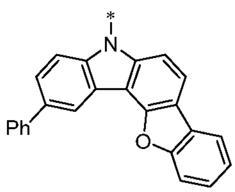
D33
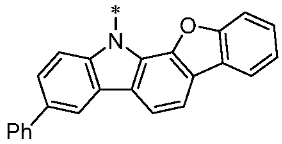
D34

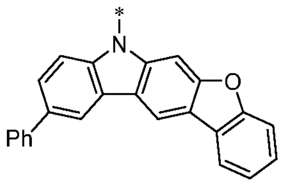
**D35**

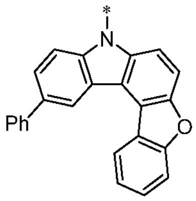
**D36**

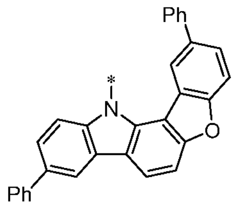
**D37**

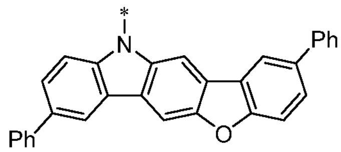
**D38**

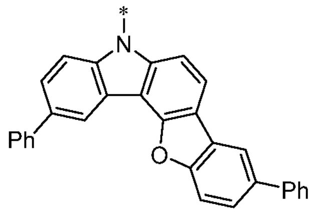
**D39**

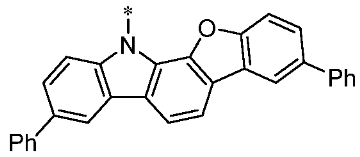
**D40**

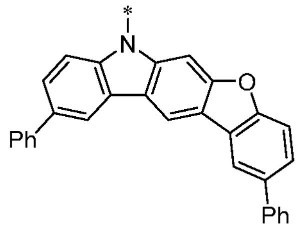
**D41**

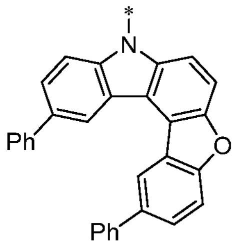
**D42**

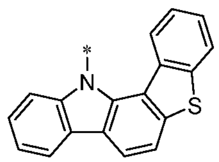
**D43**

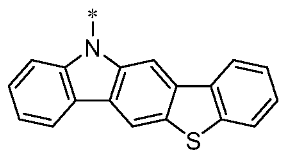
**D44**

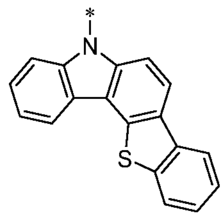
**D45**

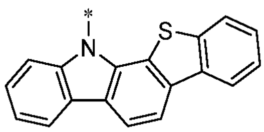
**D46**

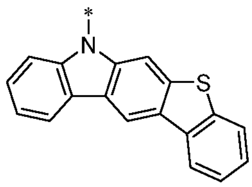
**D47**

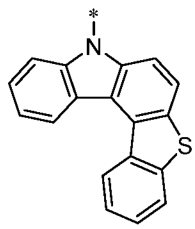
**D48**

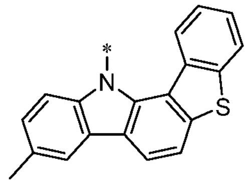
**D49**

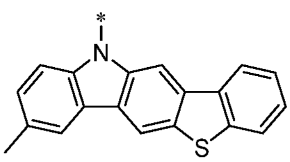
**D50**

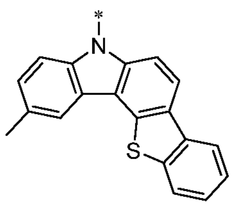
**D51**

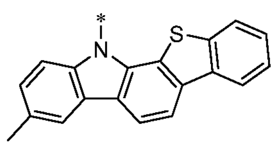
**D52**

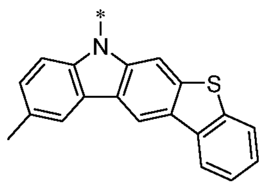
**D53**

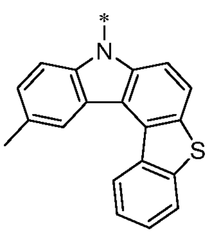
**D54**

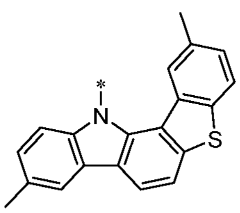
**D55**

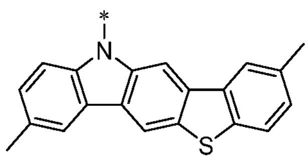
**D56**

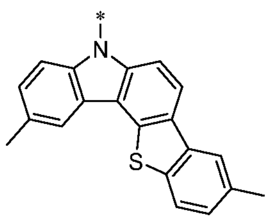
**D57**

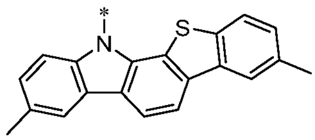
D58
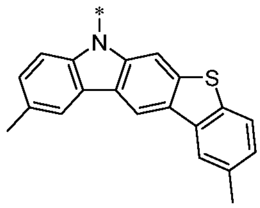
D59
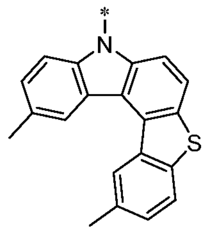
D60
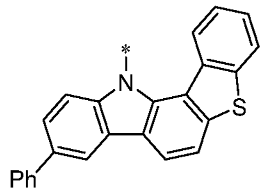
D61

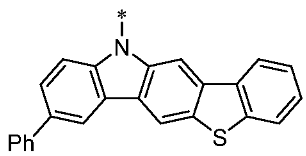
D62
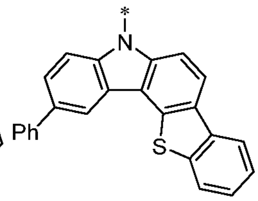
D63
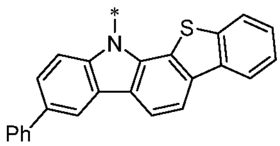
D64
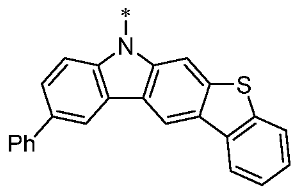
D65

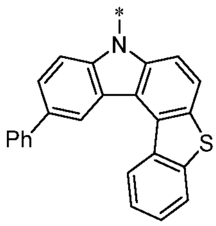
D66
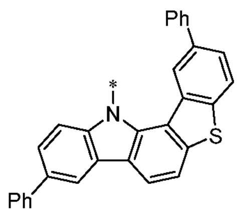
D67
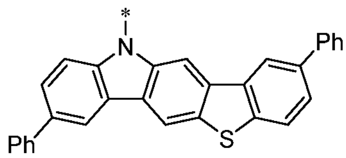
D68
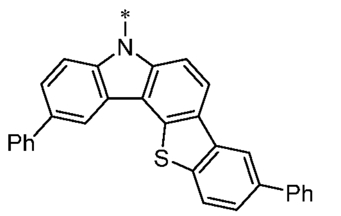
D69

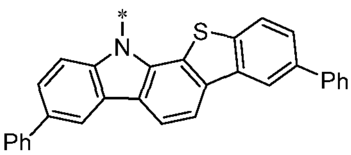
D70
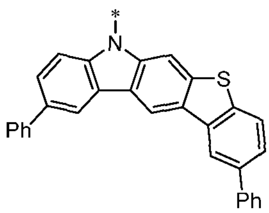
D71
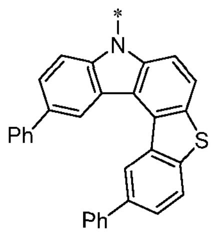
D72
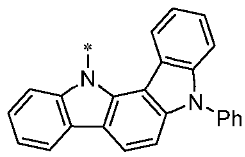
D73

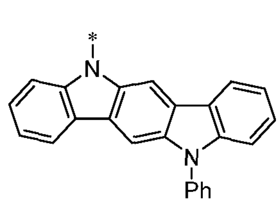
D74
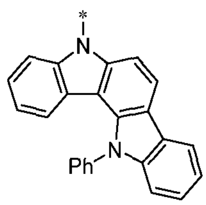
D75
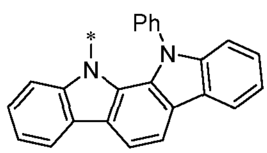
D76
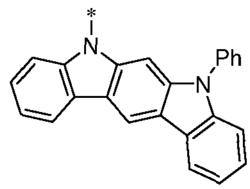
D77

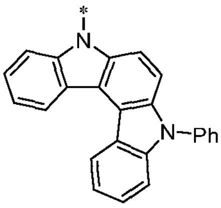
D78
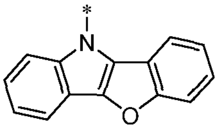
D79
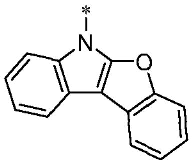
D80
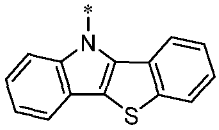
D81
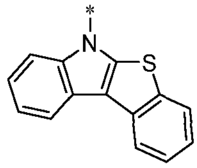
D82

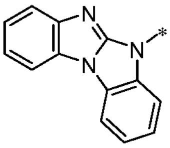
D83
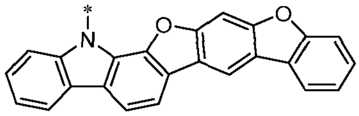
D84
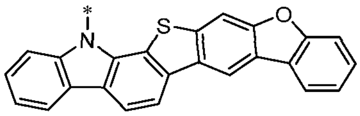
D85

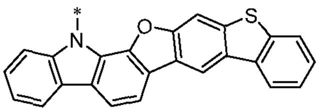
D86
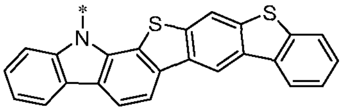
D87
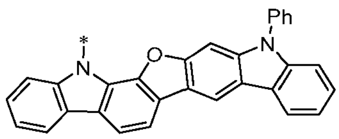
D88

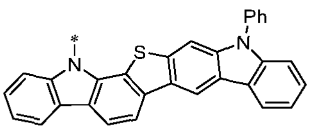
D89
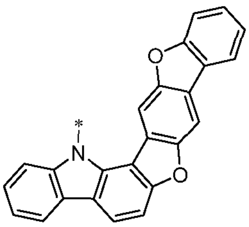
D90
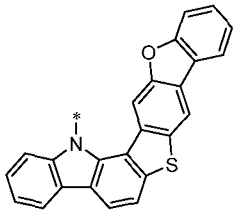
D91
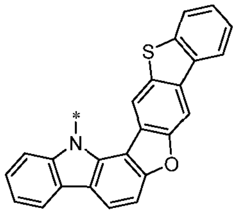
D92

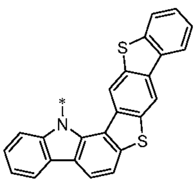
D93
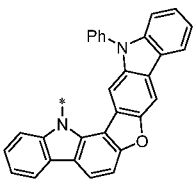
D94
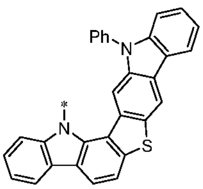
D95
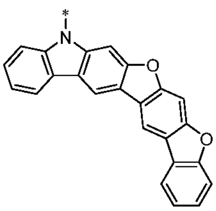
D96
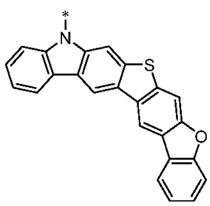
D97

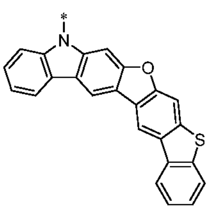
D98
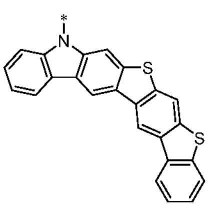
D99
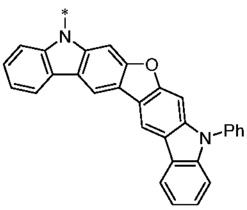
D100
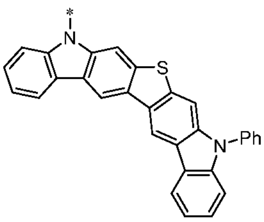
D101

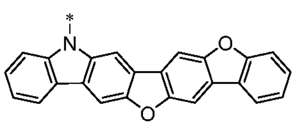 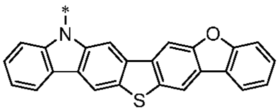 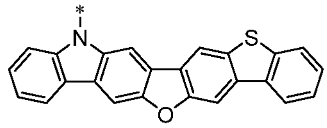

**D102** **D103** **D104**

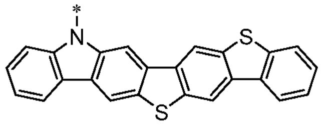 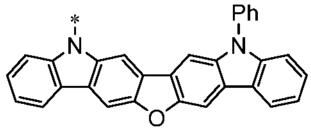 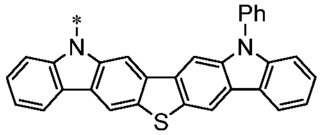

**D105** **D106** **D107**

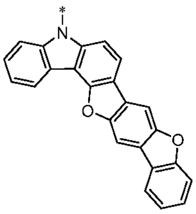 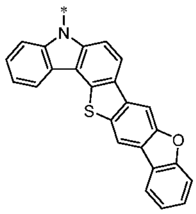 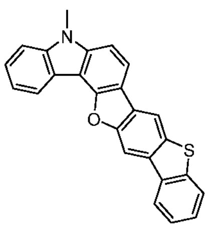 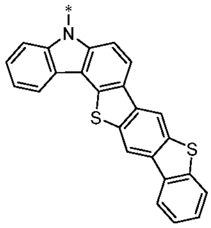 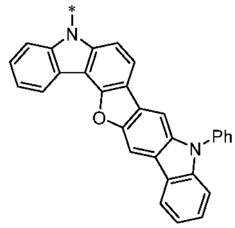

**D108** **D109** **D110** **D111** **D112**

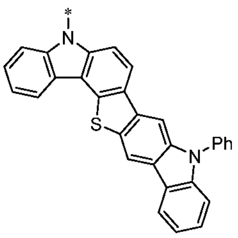 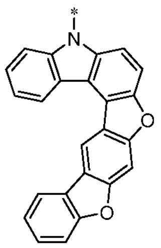 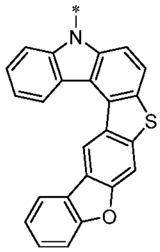 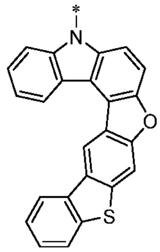 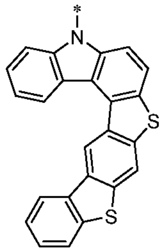 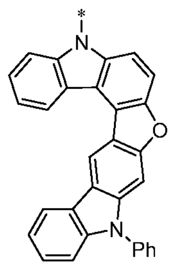

**D113** **D114** **D115** **D116** **D117** **D118**

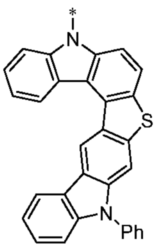 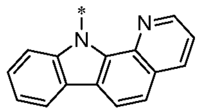 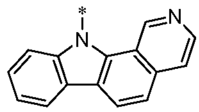 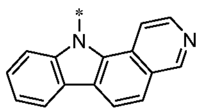 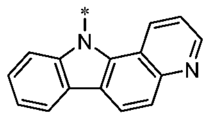

**D119** **D120** **D121** **D122** **D123**

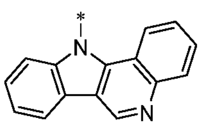 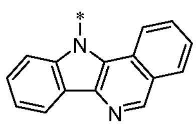 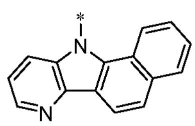 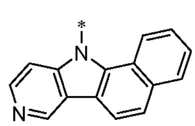 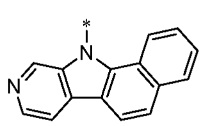

**D124** **D125** **D126** **D127** **D128**

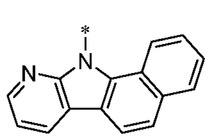 D129 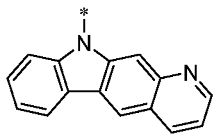 D130 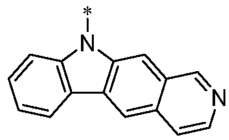 D131 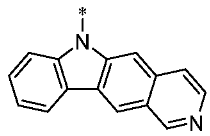 D132

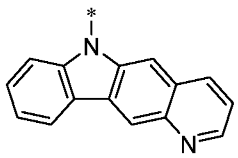 D133 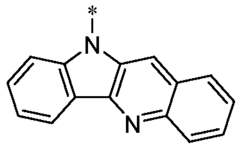 D134 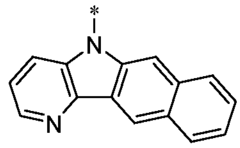 D135 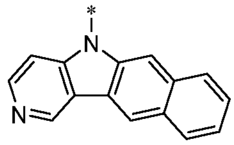 D136

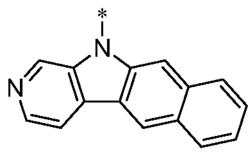 D137 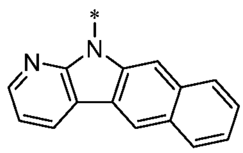 D138 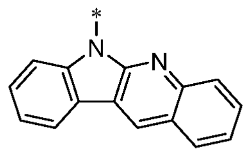 D139 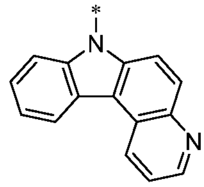 D140

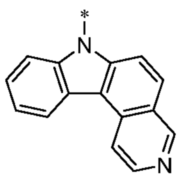 D141 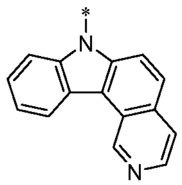 D142 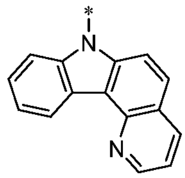 D143 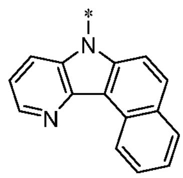 D144 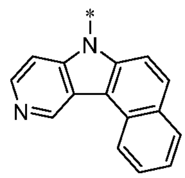 D145

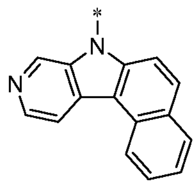 D146 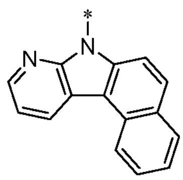 D147 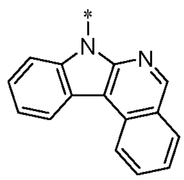 D148 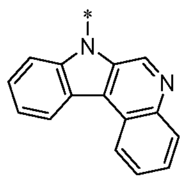 D149 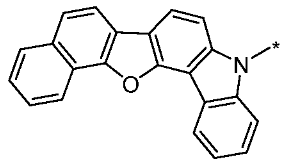 D150

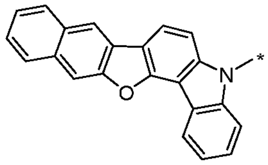 D151 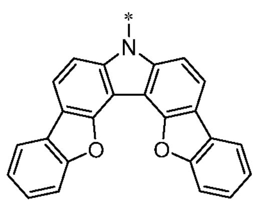 D152 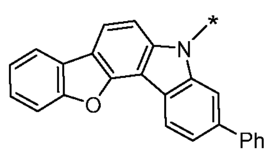 D153 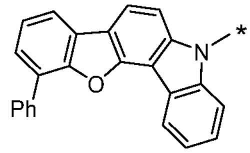 D154

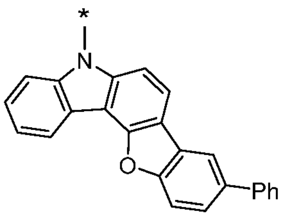 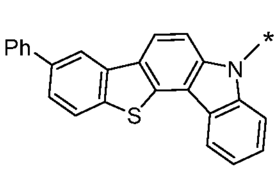 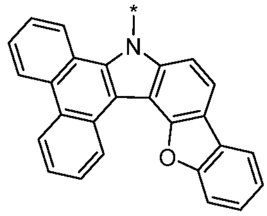 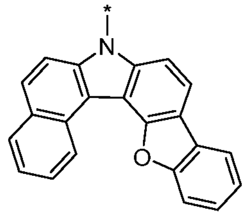

D155 D156 D157 D158

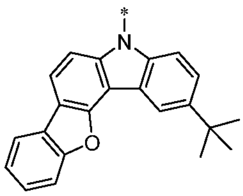 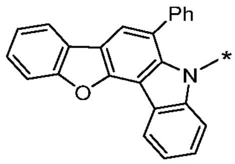 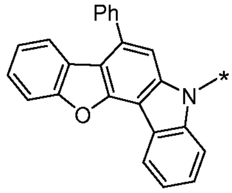 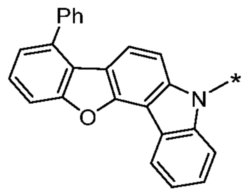

D159 D160 D161 D162

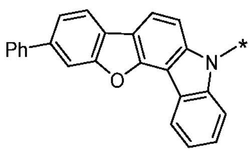 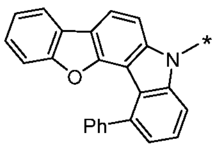 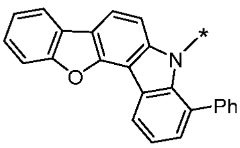 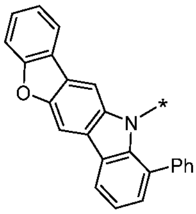 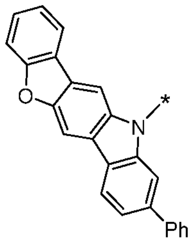

D163 D164 D165 D166 D167

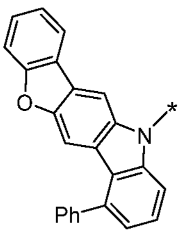 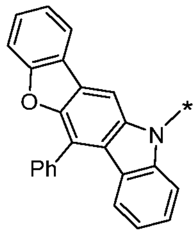 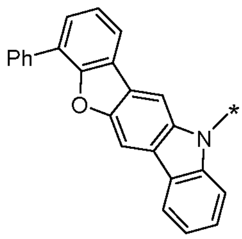 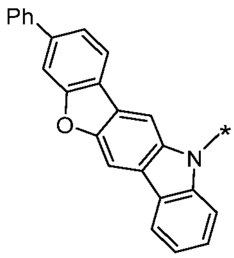 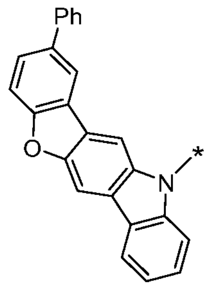

D168 D169 D170 D171 D172

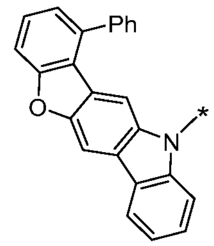 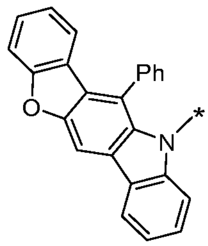 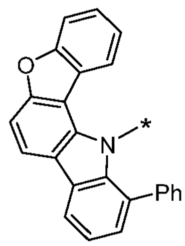 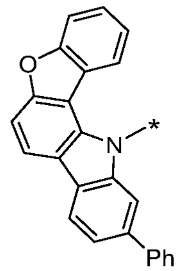

D173 D174 D175 D176

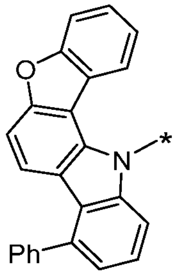 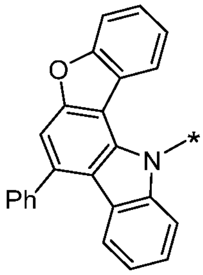 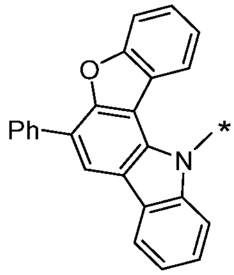 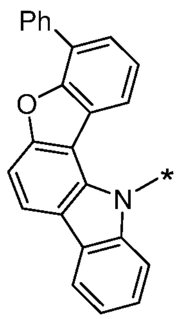

**D177** **D178** **D179** **D180**

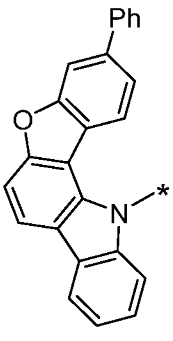 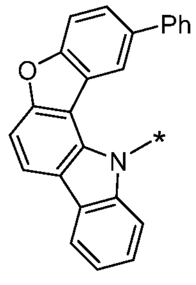 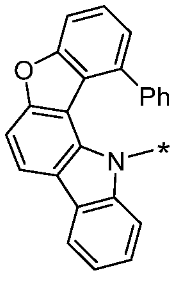 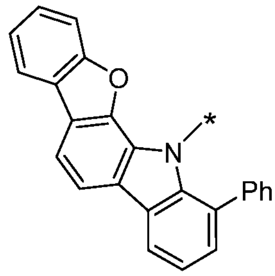

**D181** **D182** **D183** **D184**

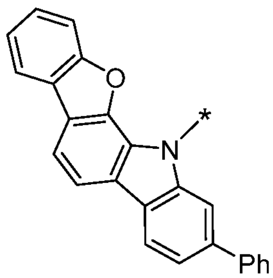 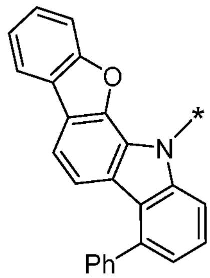 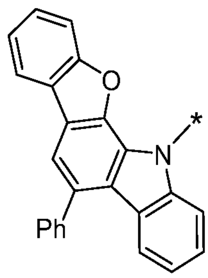 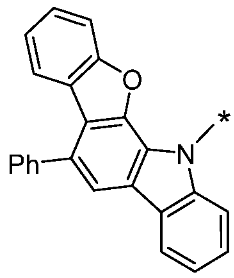

**D185** **D186** **D187** **D188**

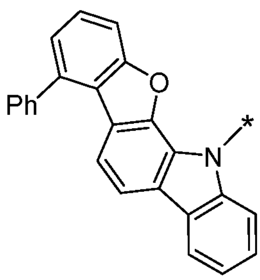 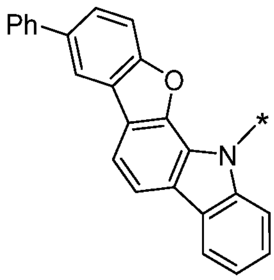 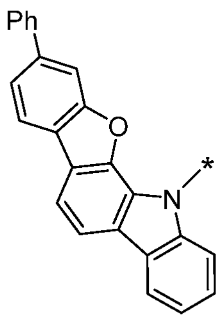 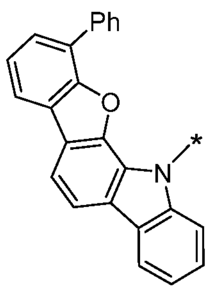

**D189** **D190** **D191** **D192**

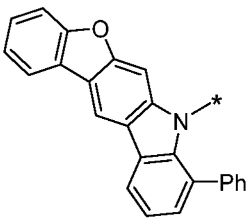
**D193**

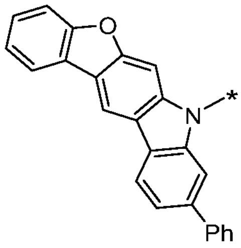
**D194**

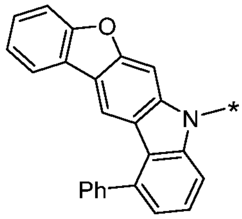
**D195**

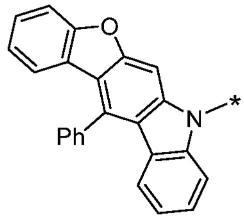
**D196**

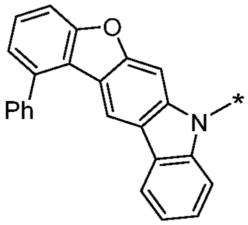
**D197**

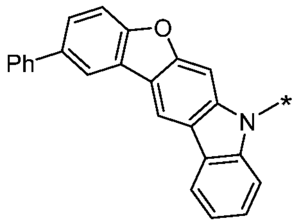
**D198**

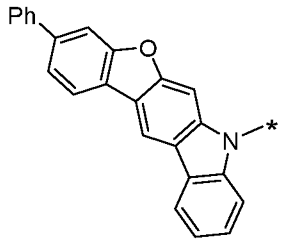
**D199**

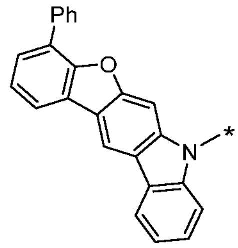
**D200**

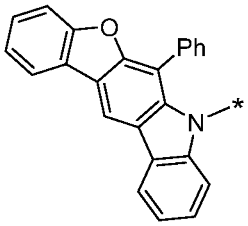
**D201**

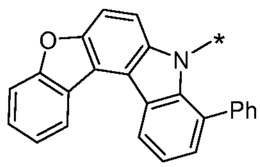
**D202**

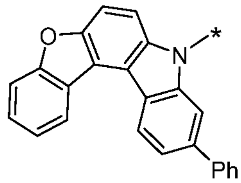
**D203**

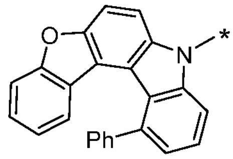
**D204**

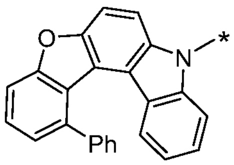
**D205**

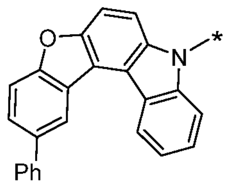
**D206**

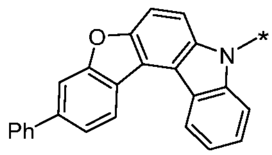
**D207**

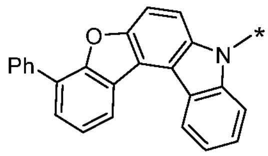
**D208**

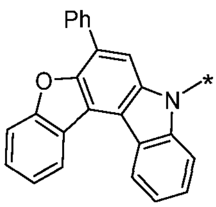
**D209**

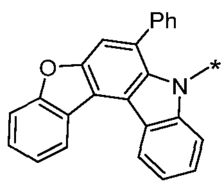
**D210**

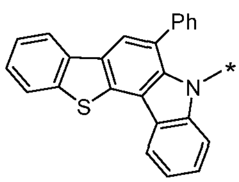
**D211**

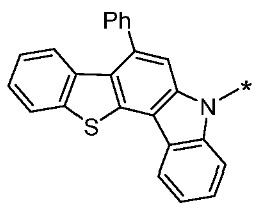
**D212**

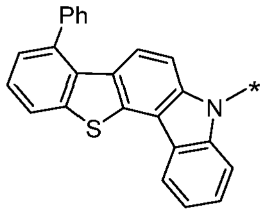
D213
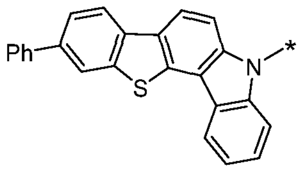
D214
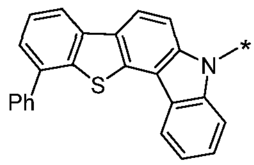
D215
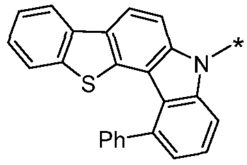
D216

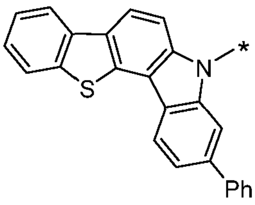
D217
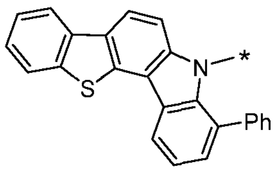
D218
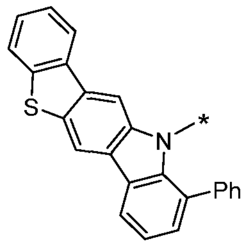
D219
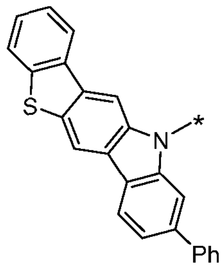
D220

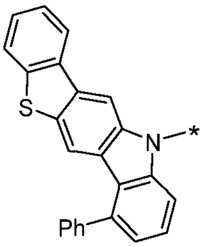
D221
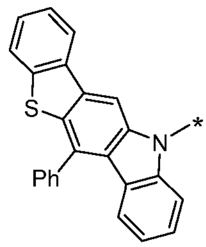
D222
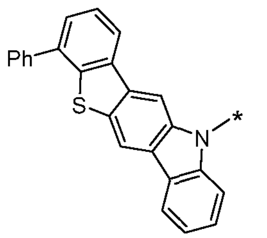
D223
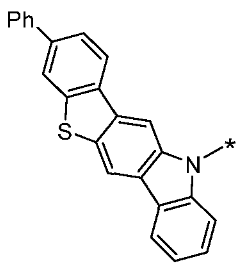
D224

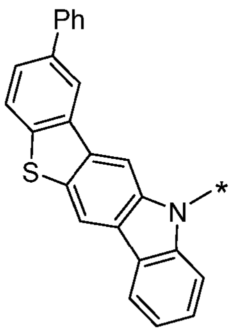
D225
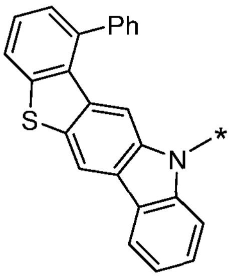
D226
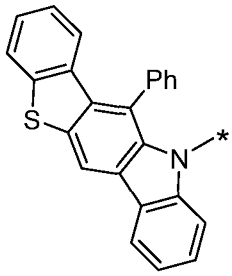
D227
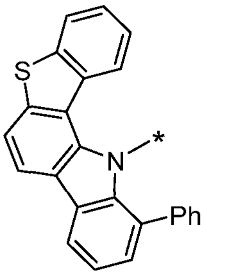
D228

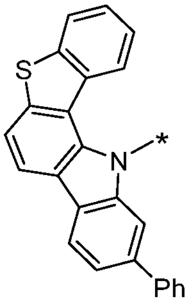
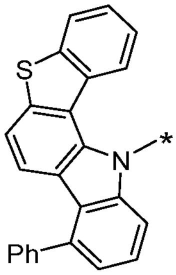
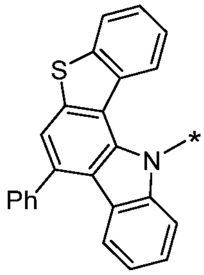
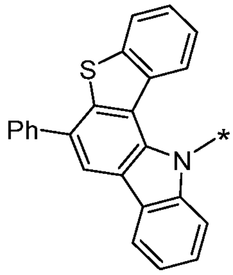

**D229** **D230** **D231** **D232**

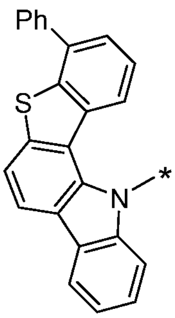
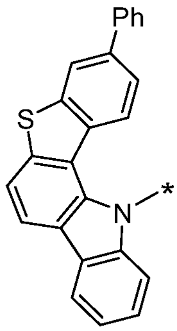
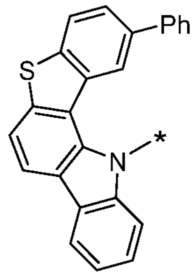
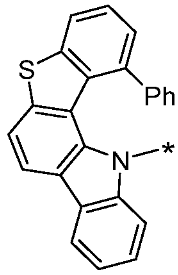

**D233** **D234** **D235** **D236**

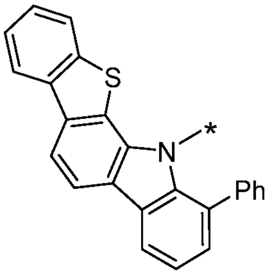
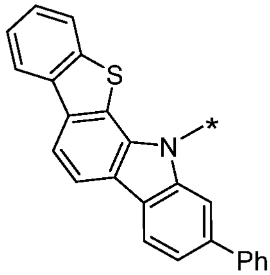
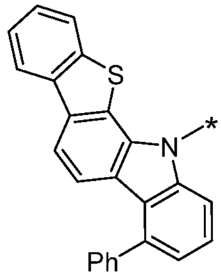
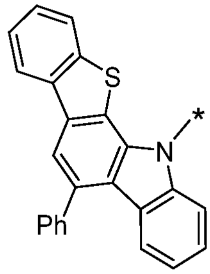

**D237** **D238** **D239** **D240**

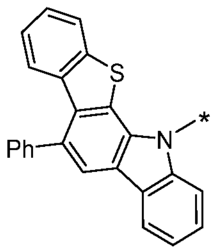
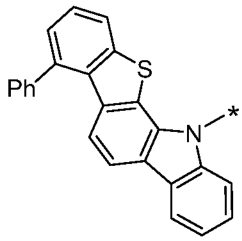
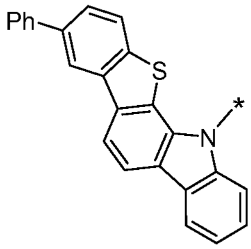
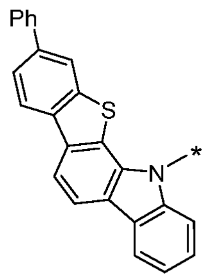

**D241** **D242** **D243** **D244**

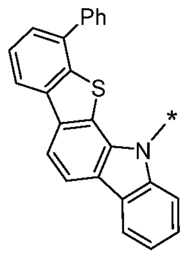
**D245**

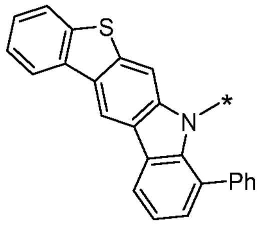
**D246**

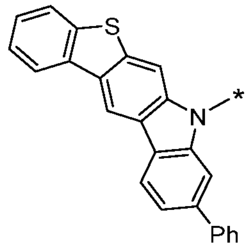
**D247**

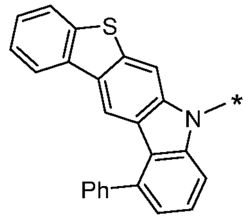
**D248**

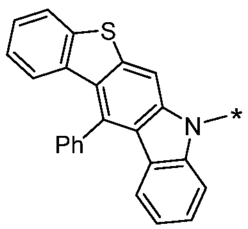
**D249**

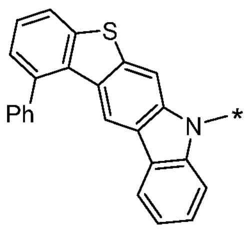
**D250**

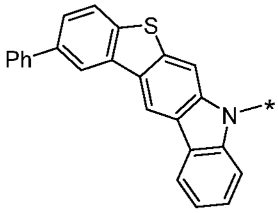
**D251**

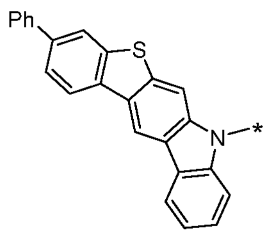
**D252**

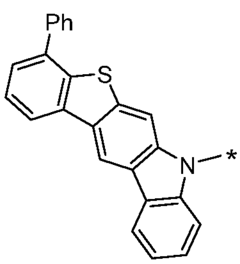
**D253**

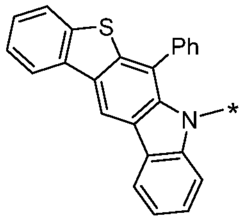
**D254**

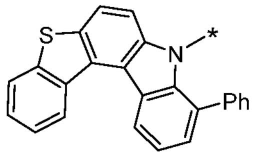
**D255**

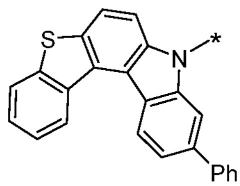
**D256**

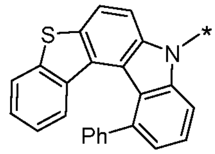
**D257**

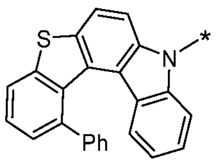
**D258**

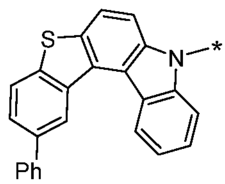
**D259**

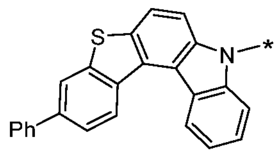
**D260**

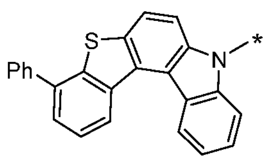
**D261**

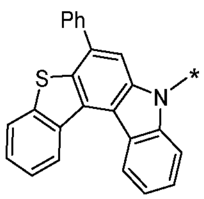
**D262**

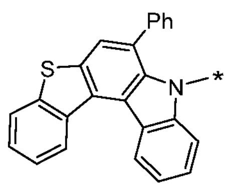
**D263**

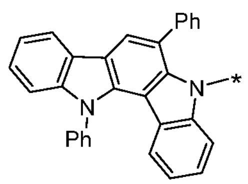
**D264**

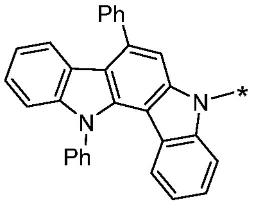
D265

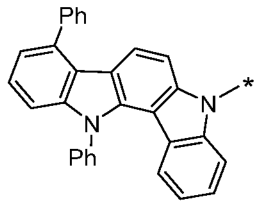
D266

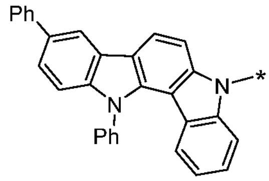
D267

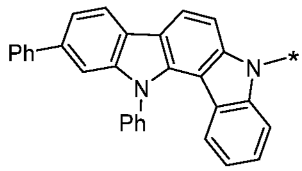
D268

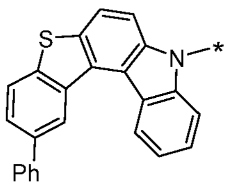
D269

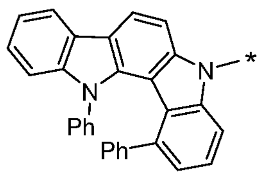
D270

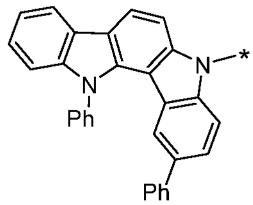
D271

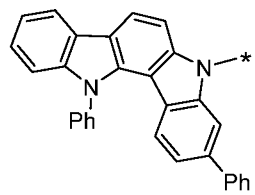
D272

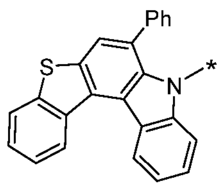
D273

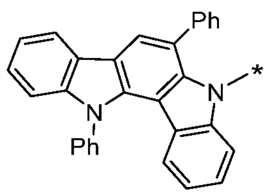
D274

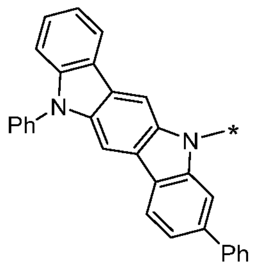
D275

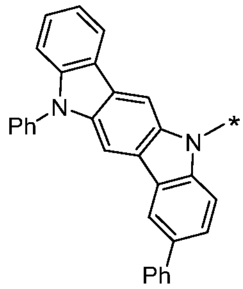
D276

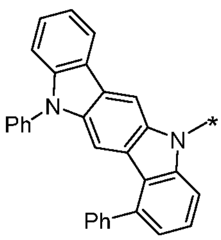
D277

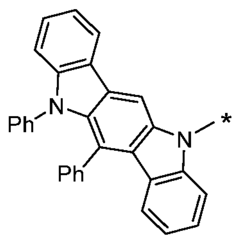
D278

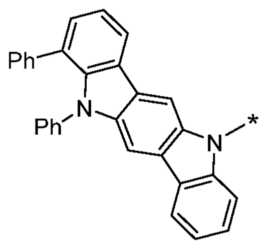
D279

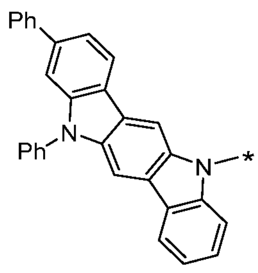
D280

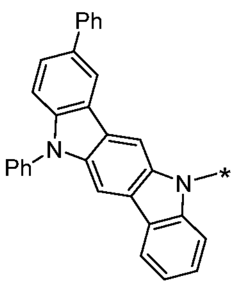
D281

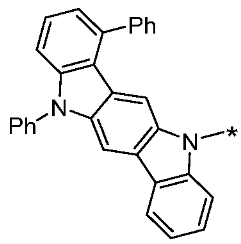
D282

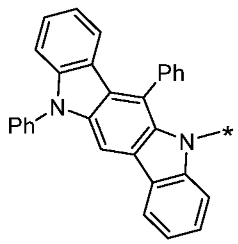
D283

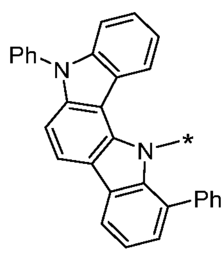
D284

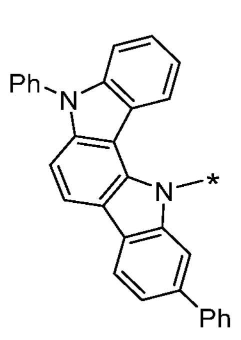

**D285**

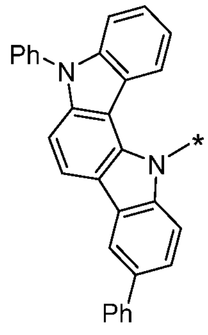

**D286**

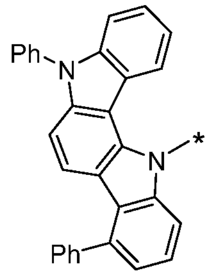

**D287**

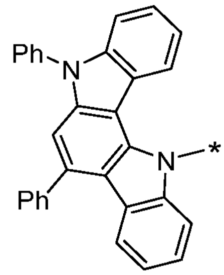

**D288**

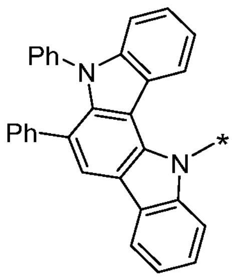

**D289**

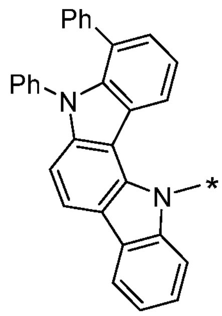

**D290**

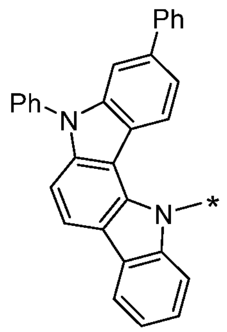

**D291**

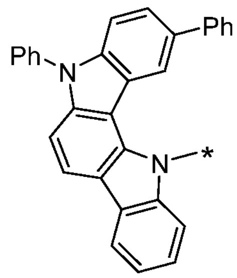

**D292**

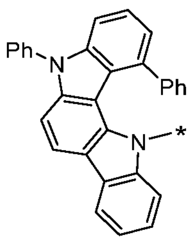

**D293**

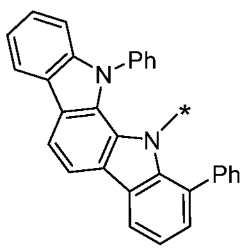

**D294**

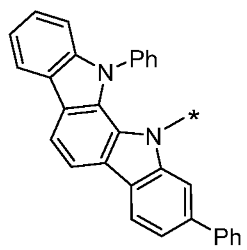

**D295**

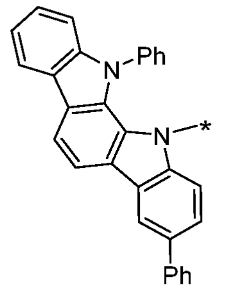

**D296**

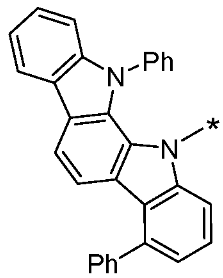

**D297**

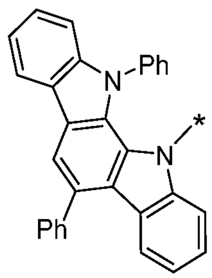

**D298**

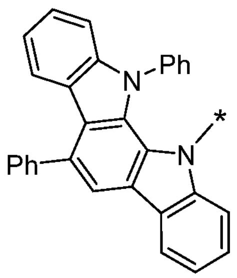

**D299**

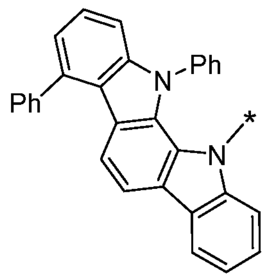

**D300**

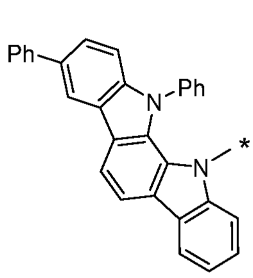

D301

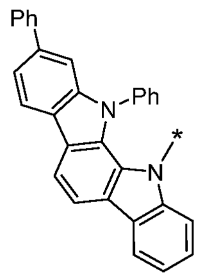

D302

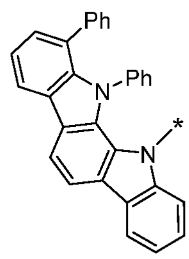

D303

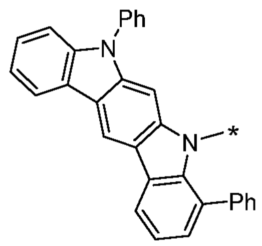

D304

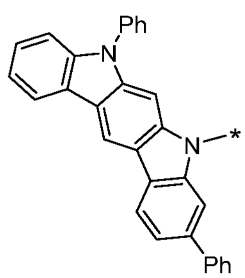

D305

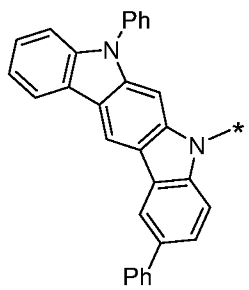

D306

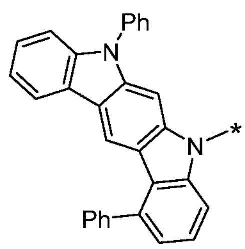

D307

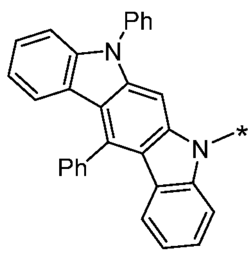

D308

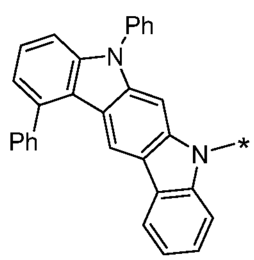

D309

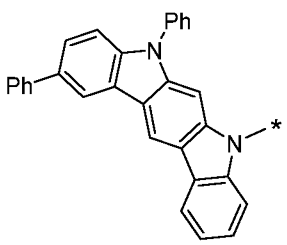

D310

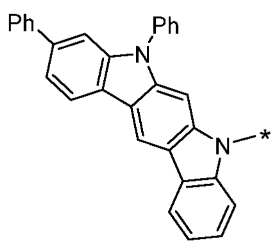

D311

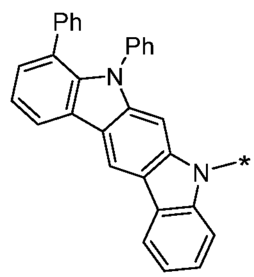

D312

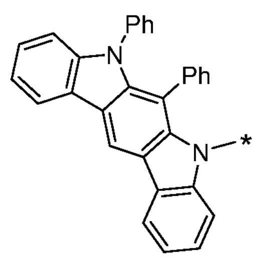

D313

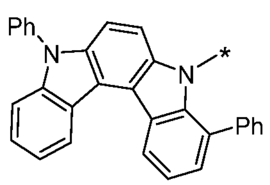

D314

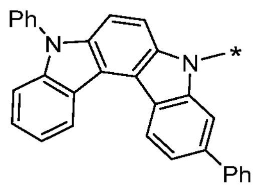

D315

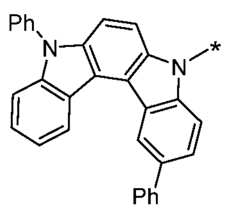

D316

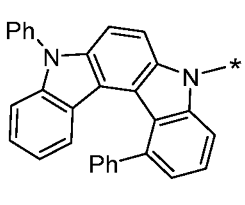

D317

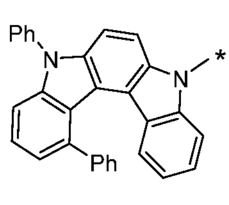

D318

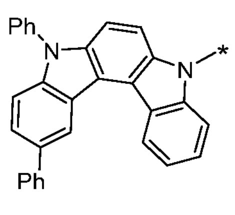

D319

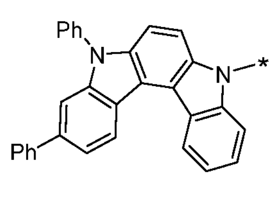

D320

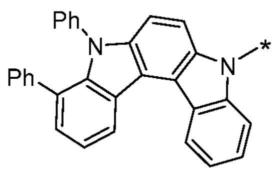 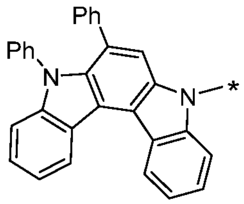 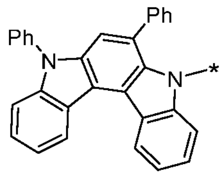 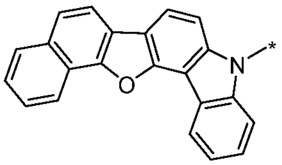

D321 D322 D323 D324

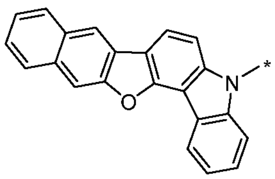 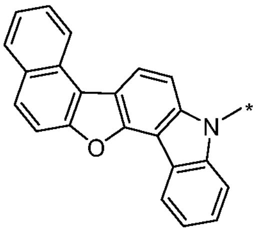 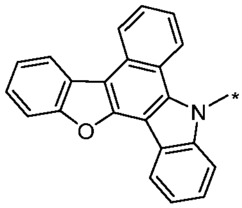 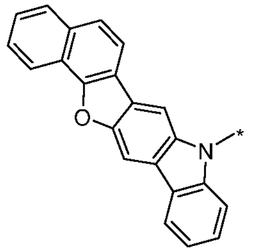

D325 D326 D327 D328

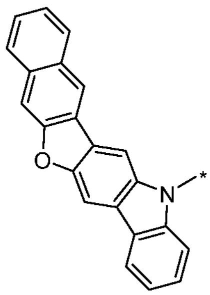 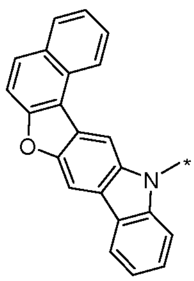 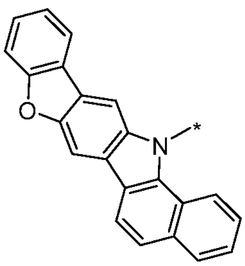 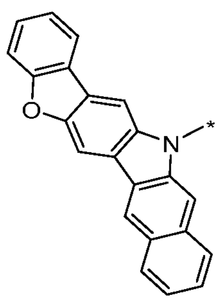 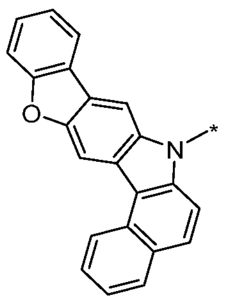

D329 D330 D331 D332 D333

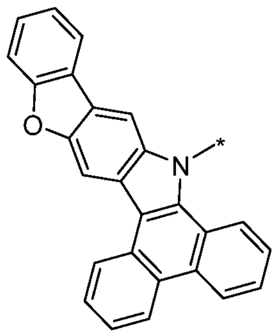 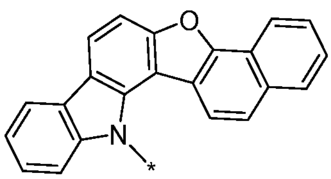 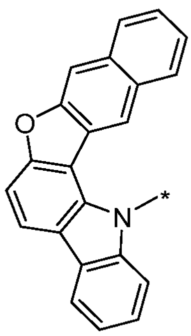 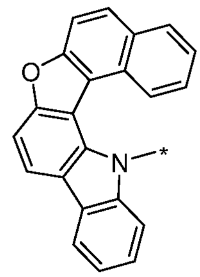

D334 D335 D336 D337

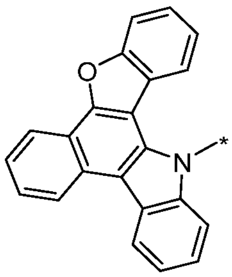 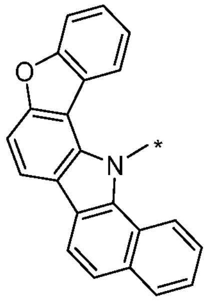 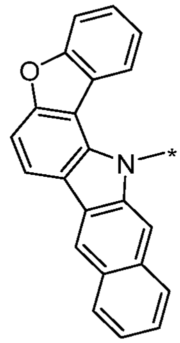 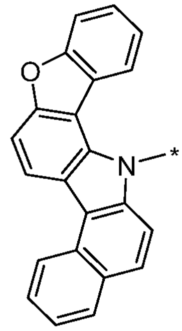

D338 D339 D340 D341

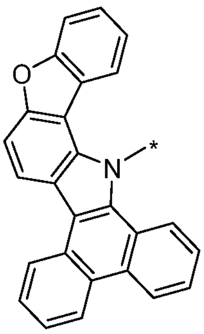 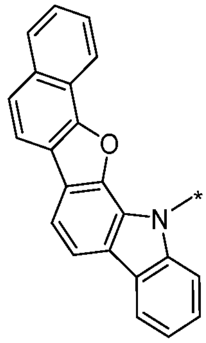 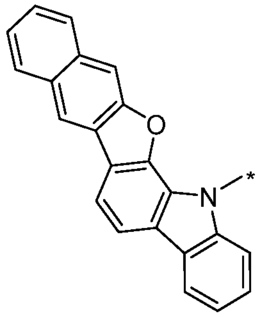 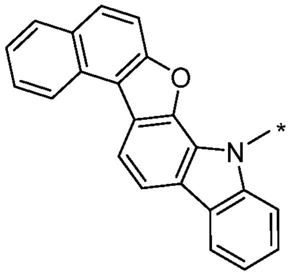

D342 D343 D344 D345

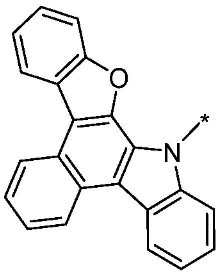 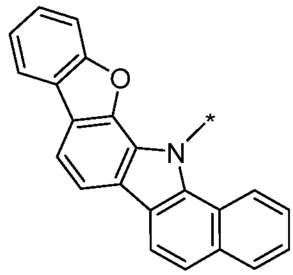 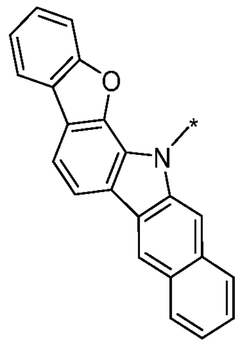 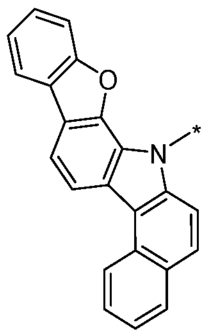

D346 D347 D348 D349

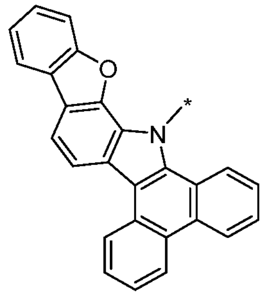 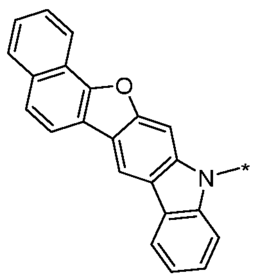 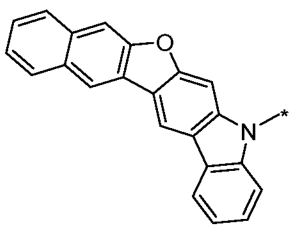 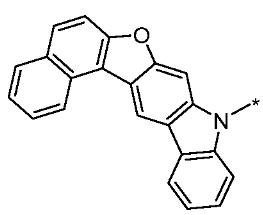

D350 D351 D352 D353

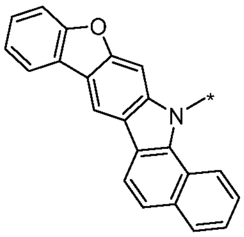
D354

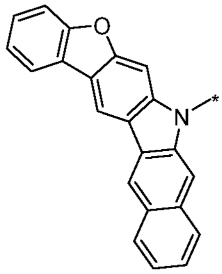
D355

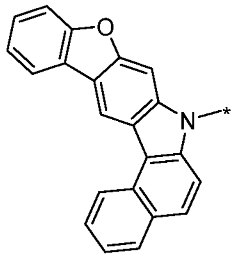
D356

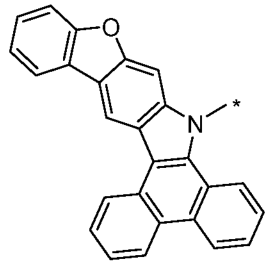
D357

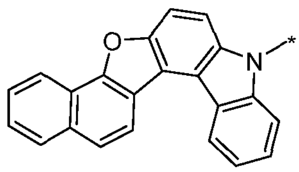
D358

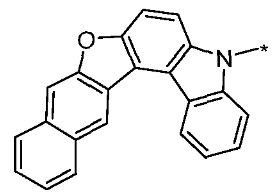
D359

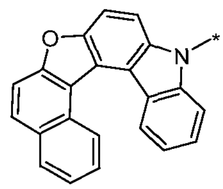
D360

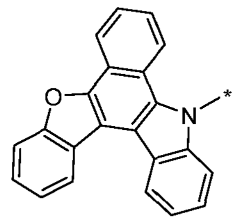
D361

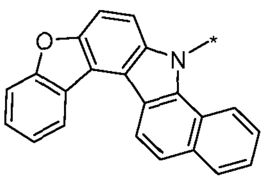
D362

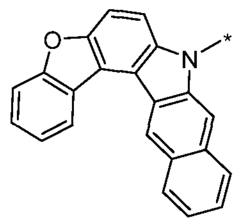
D363

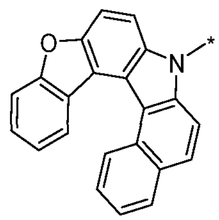
D364

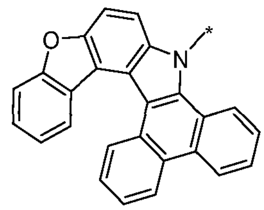
D365

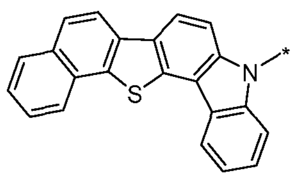
D366

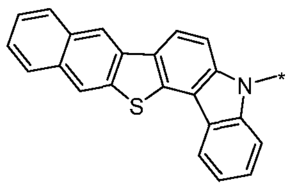
D367

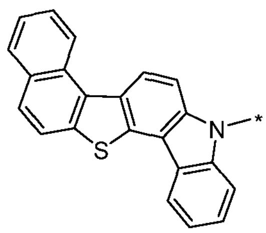
D368

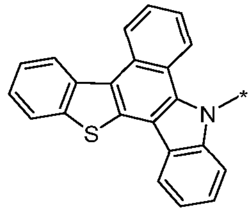
D369

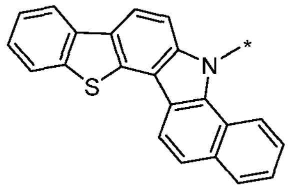
D370

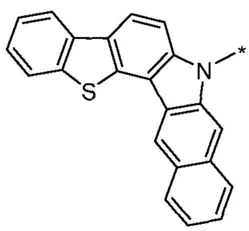
D371

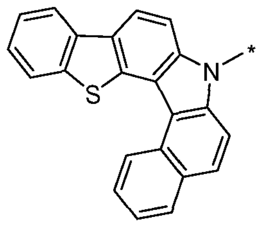
D372

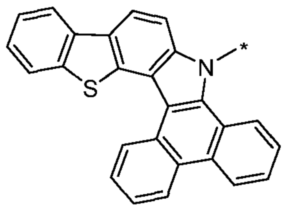
D373

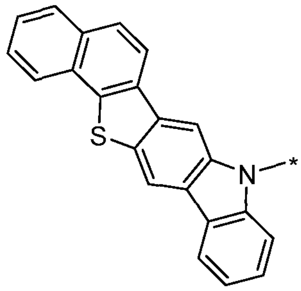
D374

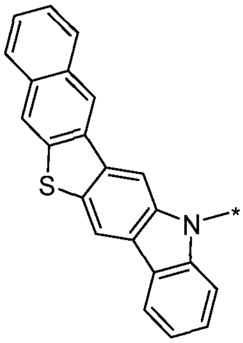
D375

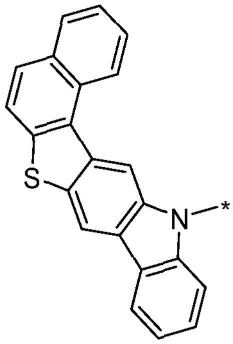
D376

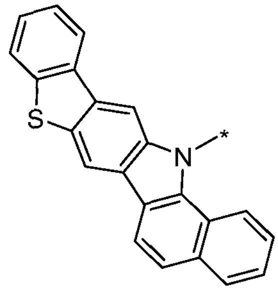
D377

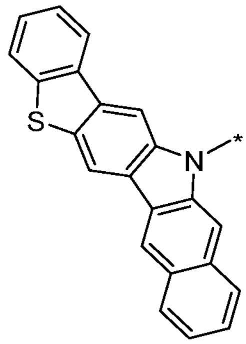
D378

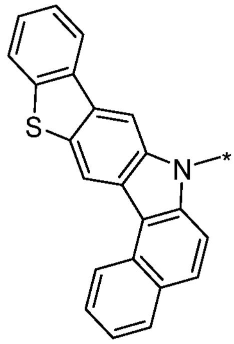
D379

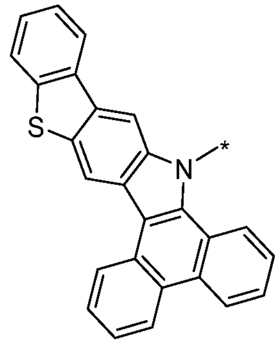
D380

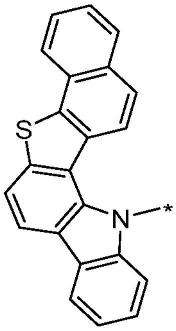
D381

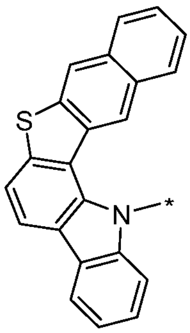
D382

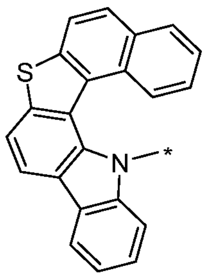
D383

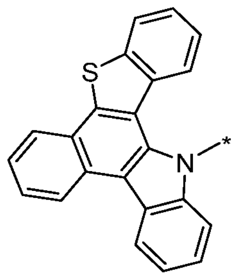
D384

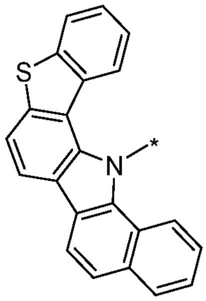
D385

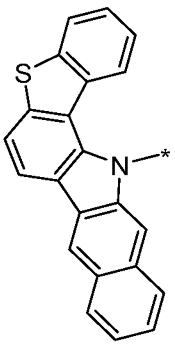
D386

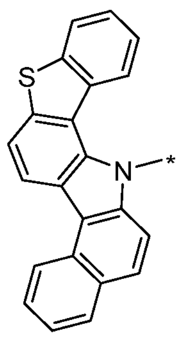
D387

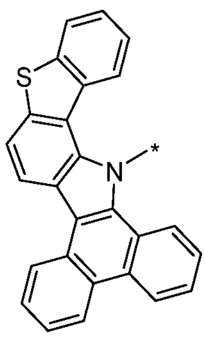
D388

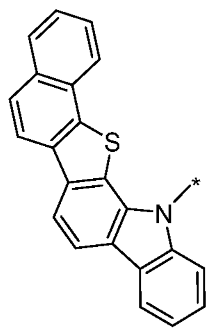
D389

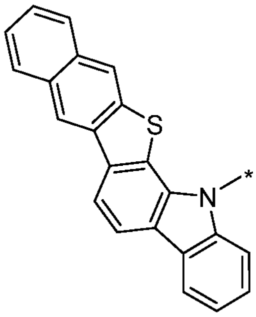
D390

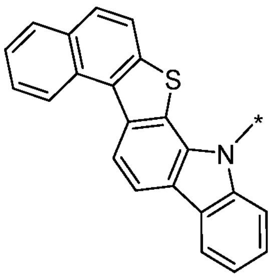
D391

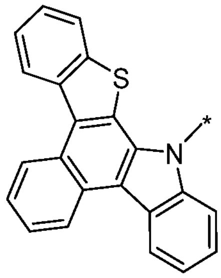
D392

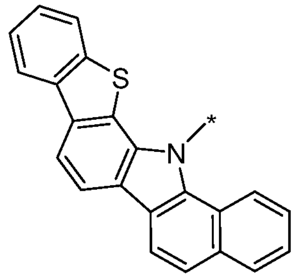
D393

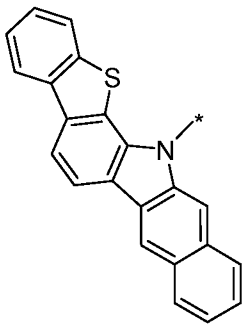
D394

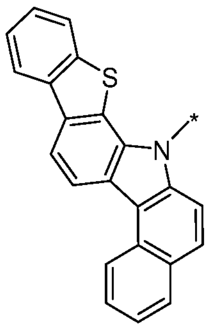
D395

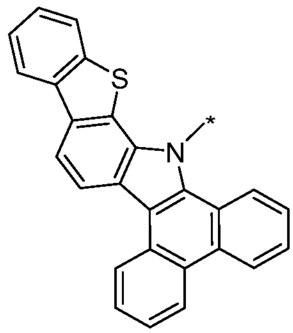
D396

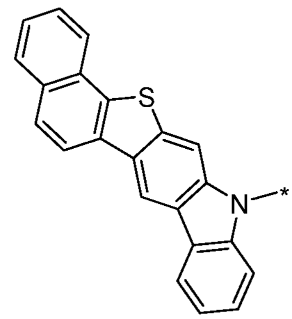
D397

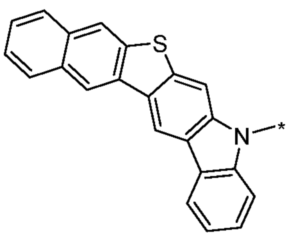
D398

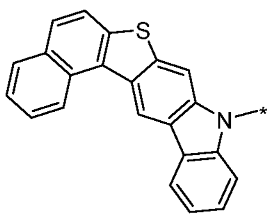
D399

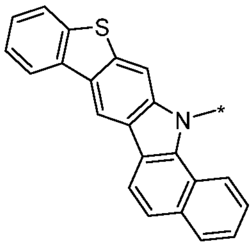
D400

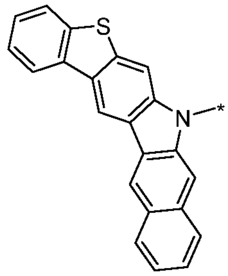
D401

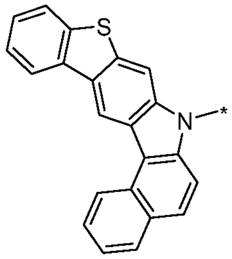
D402

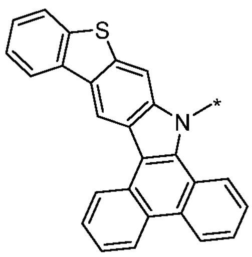
D403

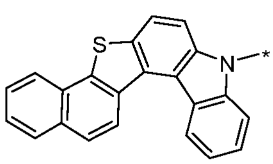
D404

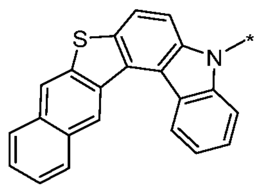
D405

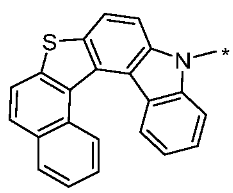

**D406**

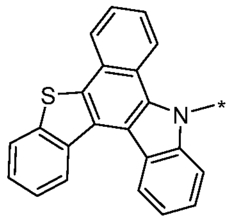

**D407**

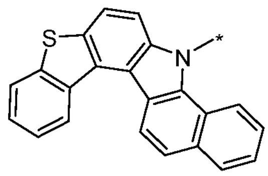

**D408**

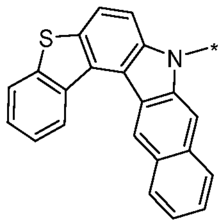

**D409**

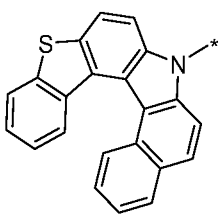

**D410**

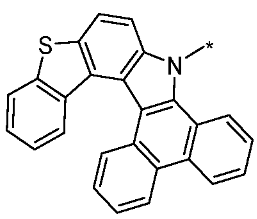

**D411**

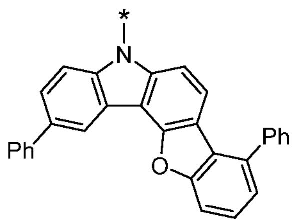

**D412**

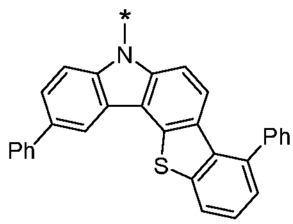

**D413**

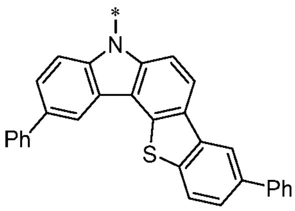

**D414**

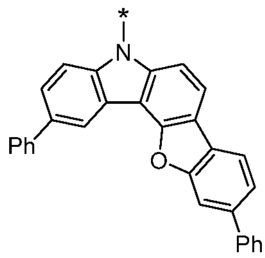

**D415**

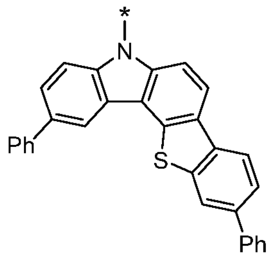

**D416**

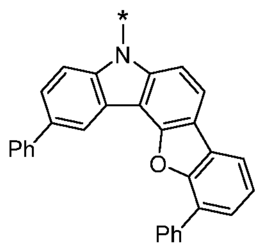

**D417**

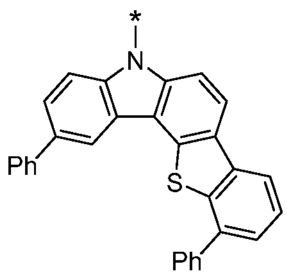

**D418**

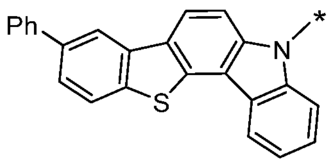

**D419**

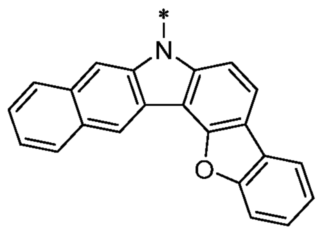

**D420**

D1 to D420 in which all of hydrogen atoms are substituted with a deuterium atom are disclosed as D421 to D840. D2 to 6, D19 to 42, D49 to D78, D100, D101, D106, D107, D112, D113, D118, D119, D153 toD156, D159 toD323, and D412 to D419 in which all of hydrogen atoms present in a phenyl group or an alkyl group as a substituent are substituted with a deuterium atom are disclosed as D841 to D1084 in this order.

In one aspect of the invention, the donor group is selected from the group consisting of D1 to D1084, and at least one donor group is selected from the group consisting of D2 to D1084. In one aspect of the invention, at least one donor group is selected from the group consisting of D13 to D78, D84 to D119, and D150 to D420.

In the formula (1), the other of $R^{112}$ and $R^{113}$, and $R^{111}$, $R^{114}$, and $R^{115}$ may be substituted or unsubstituted aryl groups. Here, the donor group having a substituted amino group is excluded from the substituted or unsubstituted aryl group mentioned herein. For the aryl group mentioned herein, the description on the aryl group in the formula (1) can be referred to. In a preferred aspect of the invention, the aryl group is a phenyl group. In one aspect of the invention, the aryl group is a 1-naphthyl group or a 2-naphthyl group. The substituent of the aryl group, for example, can be selected from the substituent groups A to E, and is preferably selected from the substituent group E.

Hereinafter, specific examples of the substituted or unsubstituted aryl group that may be possessed by the

other of $R^{112}$ and $R^{113}$, and $R^{111}$, $R^{114}$, and $R^{115}$ will be illustrated. Here, the substituted or unsubstituted aryl group that can be adopted in the invention is not construed as limiting to such specific examples. In the following specific examples, the indication of a methyl group is omitted. Therefore, for example, Ar4 is substituted with a methyl group, and Ar5 is substituted with an isopropyl group. * represents a bond position.

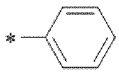 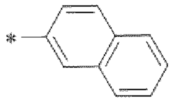 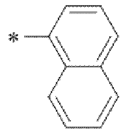 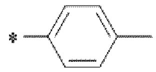 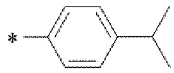

Ar1 Ar2 Ar3 Ar4 Ar5

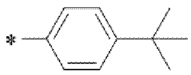 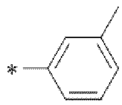 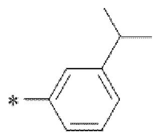 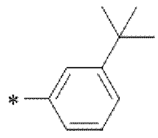 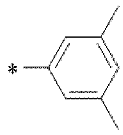

Ar6 Ar7 Ar8 Ar9 Ar10

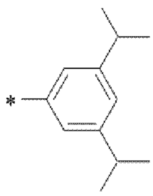 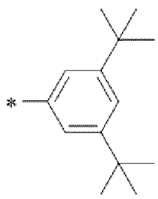 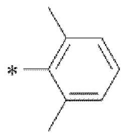 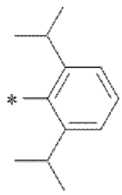 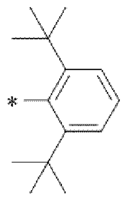

Ar11 Ar12 Ar13 Ar14 Ar15

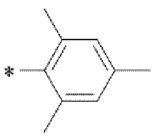 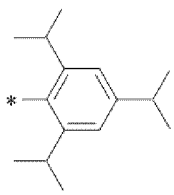 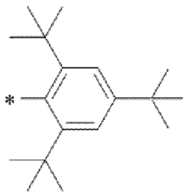 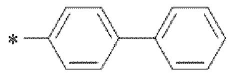 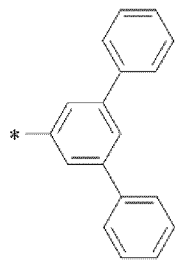

Ar16 Ar17 Ar18 Ar19 Ar20

Ar1 to Ar20 in all of hydrogen atoms are substituted with a deuterium atom are disclosed as Ar21 to Ar40 in this order. Ar4 to Ar20 in which all of hydrogen atoms present in a phenyl group or an alkyl group as a substituent are substituted with a deuterium atom are disclosed as Ar41 to Ar57 in this order.

In one aspect of the invention, the substituted or unsubstituted aryl group is selected from Ar1 to Ar57. In one aspect of the invention, the substituted or unsubstituted aryl group is selected from Ar1 to Ar3, Ar19 to Ar23, Ar39, Ar40, Ar56, and Ar57. In one aspect of the invention, the substituted or unsubstituted aryl group is Ar1 or Ar21. In one aspect of the invention, the substituted or unsubstituted aryl group is selected from Ar1, Ar4 to Ar18, Ar21, Ar24 to Ar38, and Ar41 to Ar55.

In the formula (1), the other of $R^{112}$ and $R^{113}$, and $R^{111}$, $R^{114}$, and $R^{115}$ preferably have 1 to 4 donor groups, preferably have 0 to 2 substituted or unsubstituted aryl group, and preferably have 0 to 2 hydrogen atoms or deuterium atoms. In one aspect of the invention, there are four donor groups. In one aspect of the invention, there are three donor groups, and one substituted or unsubstituted aryl group. In one aspect of the invention, there are three donor groups, and one hydrogen atom or deuterium atom. In one aspect of the invention, there are two donor groups, and two substituted or unsubstituted aryl groups. In one aspect of the invention, there are two donor groups, one substituted or unsubstituted aryl group, and one hydrogen atom or deuterium atom. In one aspect of the invention, there are two donor groups, and

two hydrogen atoms or deuterium atoms. In one aspect of the invention, there are one donor group, two substituted or unsubstituted aryl groups, and one hydrogen atom or deuterium atom. In one aspect of the invention, there are one donor group, one substituted or unsubstituted aryl group, and two hydrogen atoms or deuterium atoms. In one aspect of the invention, there are one donor group, and three hydrogen atoms or deuterium atoms.

In a preferred aspect of the invention, all of the donor groups have a carbazole-9-yl structure. In one aspect of the invention, some donor groups may not have a carbazole-9-yl structure. In one aspect of the invention, among the donor groups, there is one carbazole-9-yl group (the carbazole-9-yl group is at least substituted, or has a ring further condensed). In one aspect of the invention, when there are two or more donor groups, there are two carbazole-9-yl groups (the carbazole-9-yl group is at least substituted, or has a ring further condensed).

In a preferred aspect of the invention, $R^{112}$ is a cyano group or a substituted or unsubstituted triazinyl group, and $R^{113}$ to $R^{115}$ are donor groups or substituted or unsubstituted aryl groups. In one aspect of the invention, $R^{111}$ is a hydrogen atom or a deuterium atom. In one aspect of the invention, $R^{114}$ is a substituted or unsubstituted aryl group. In one aspect of the invention, $R^{113}$ to $R^{115}$ are donor groups.

Hereinafter, specific examples of the compound represented by the formula (2) will be given. Here, the compound represented by the formula (2) that can be adopted in the invention is not construed as limiting to such specific examples. In the following specific examples, D represents a deuterium atom, tBu represents a tertiary butyl group, and Ph represents a phenyl group. Further, the indication of a methyl group is omitted. Therefore, for example, Ar4 has a methyl group as a substituent.

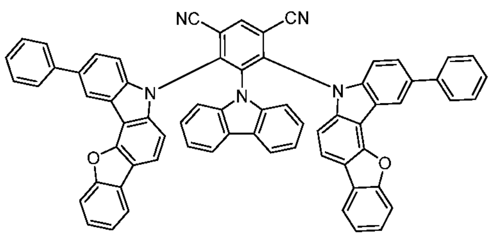

**T1**

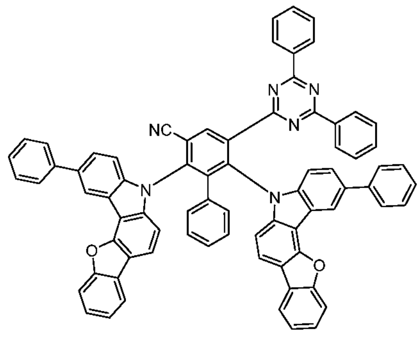

**T2**

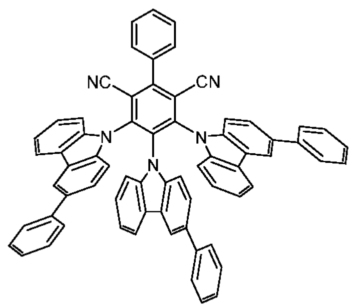

**T3**

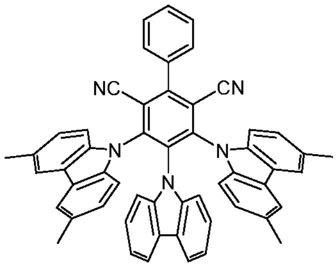

**T4**

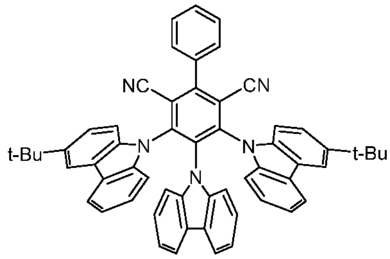

**T5**

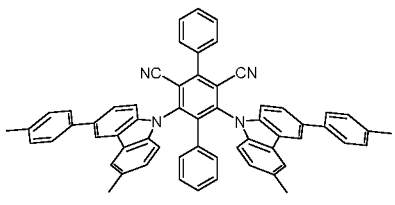

**T6**

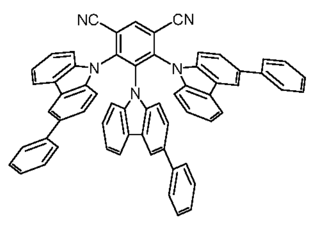

**T7**

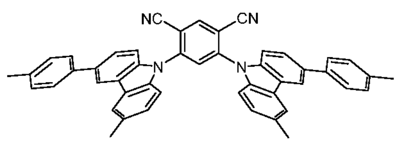

**T8**

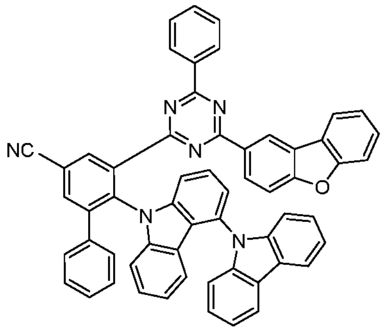

T9

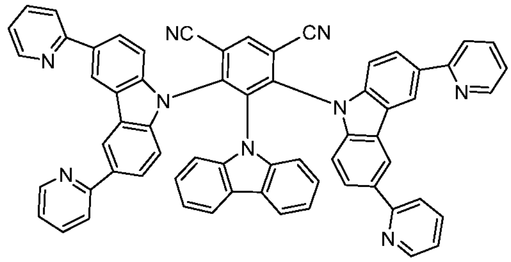

T10

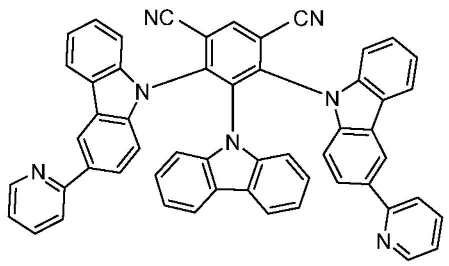

T11

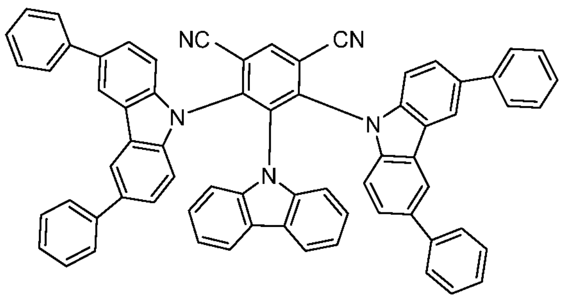

T12

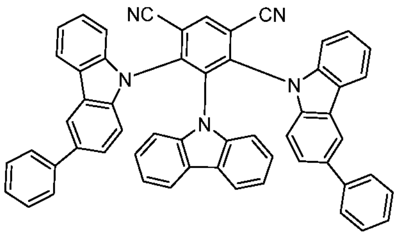

T13

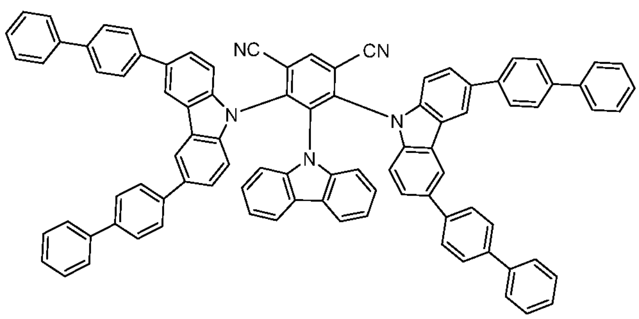

T14

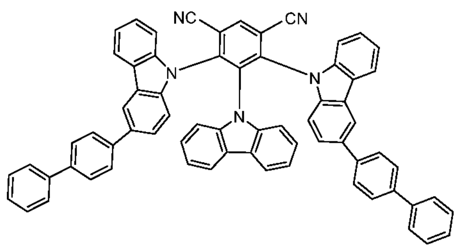

T15

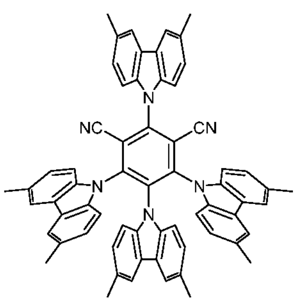

T16

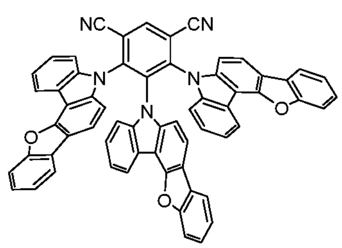

T17

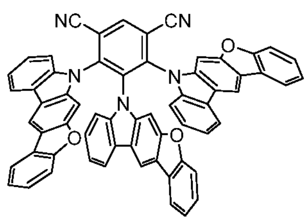

**T18**

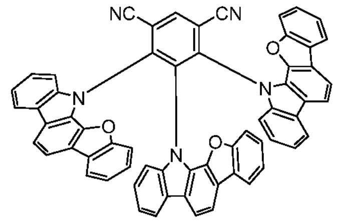

**T19**

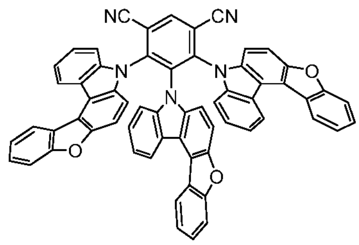

**T20**

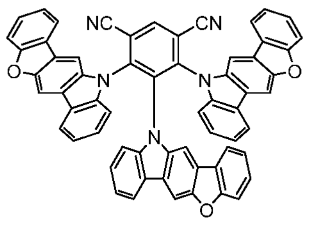

**T21**

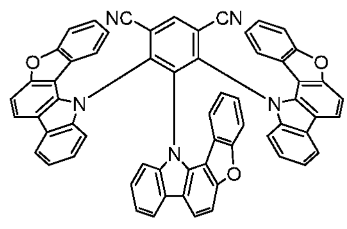

**T22**

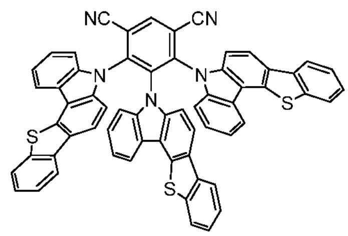

**T23**

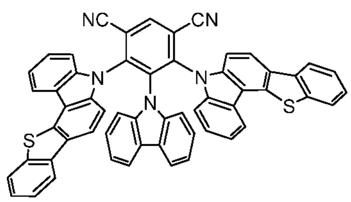

**T24**

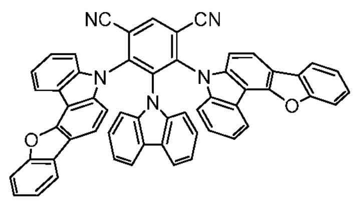

**T25**

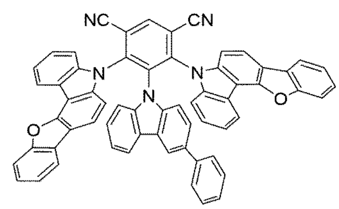

**T26**

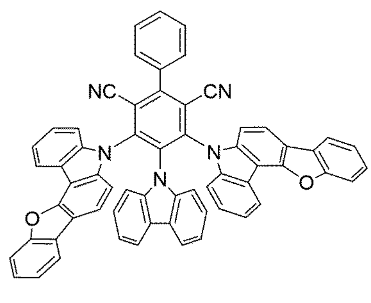

**T27**

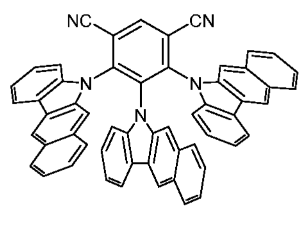

**T28**

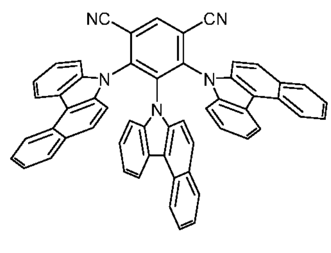

**T29**

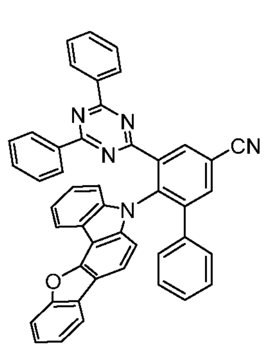

**T30**

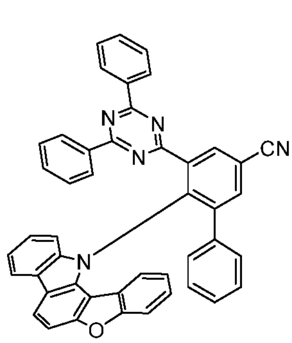

**T31**

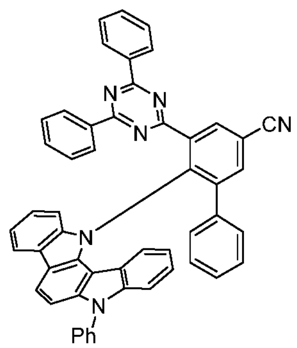

**T32**

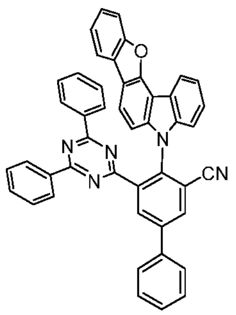

**T33**

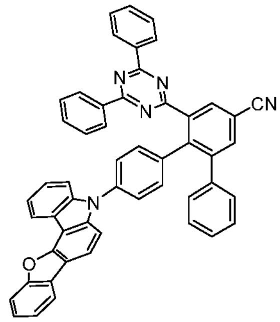

**T34**

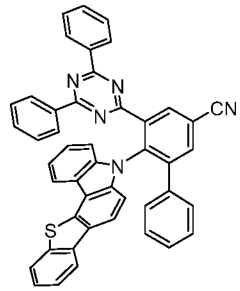

**T35**

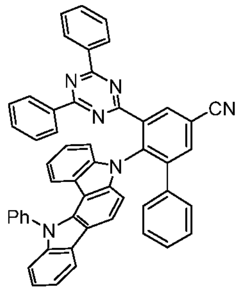

**T36**

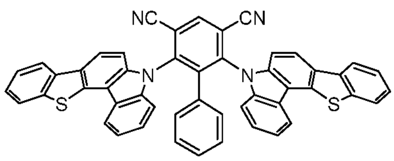

**T37**

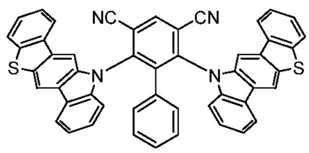

**T38**

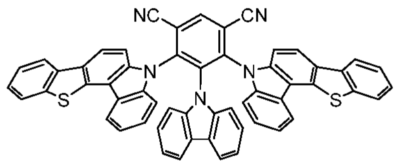

**T39**

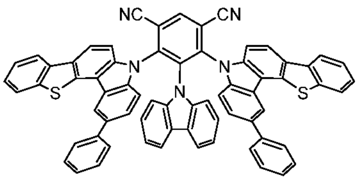

**T40**

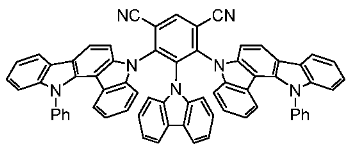

**T41**

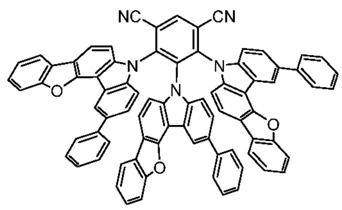

**T42**

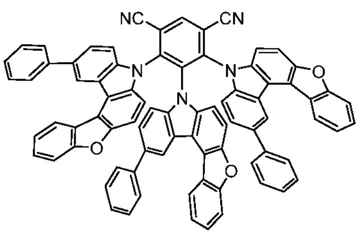

**T43**

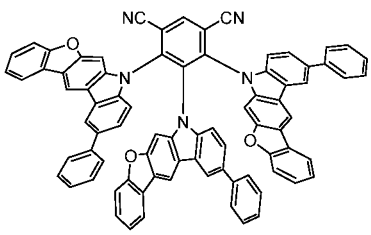

**T44**

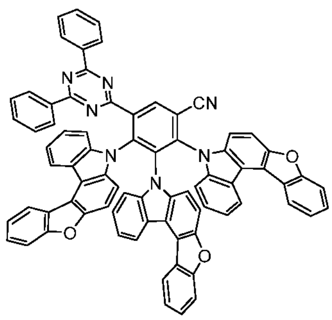

**T45**

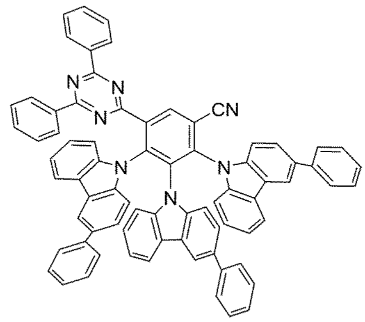

**T46**

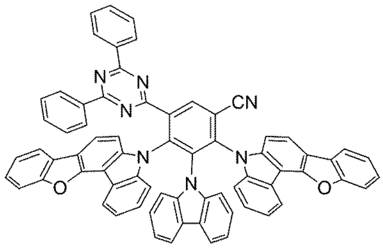

**T47**

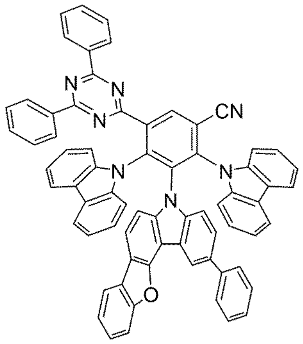

**T48**

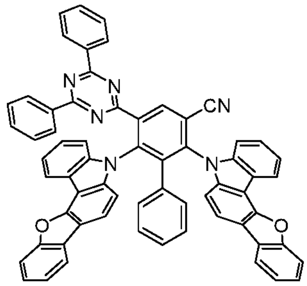

**T49**

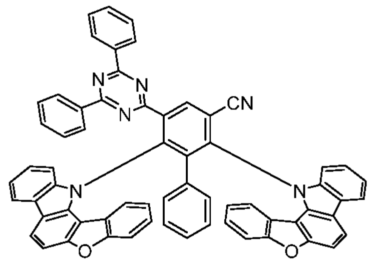

**T50**

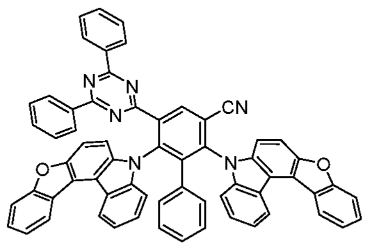

**T51**

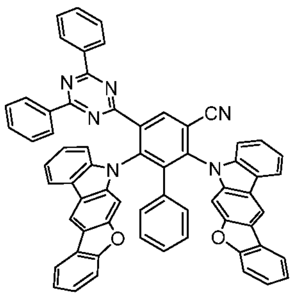

**T52**

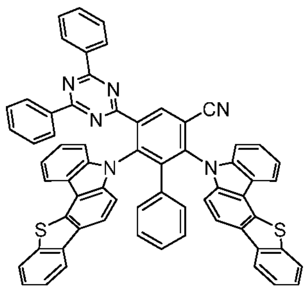

**T53**

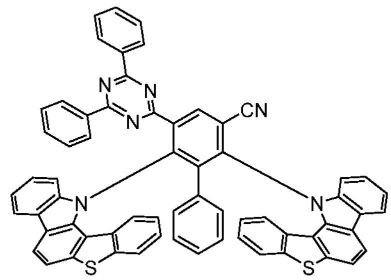

**T54**

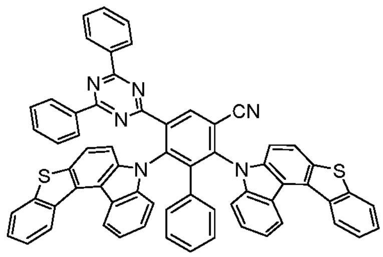

**T55**

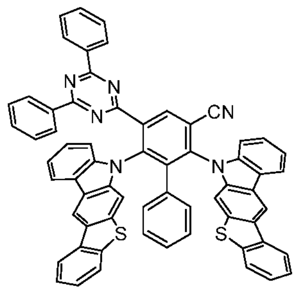

**T56**

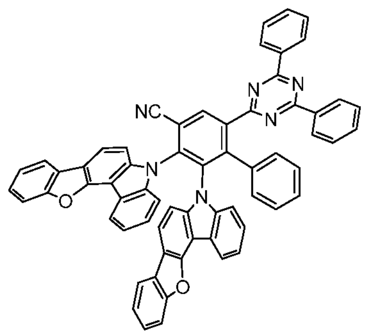

**T57**

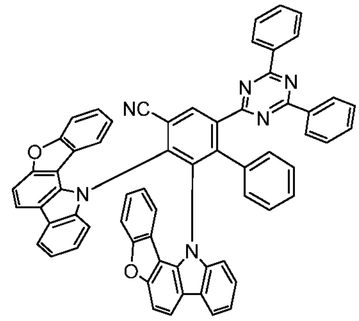

**T58**

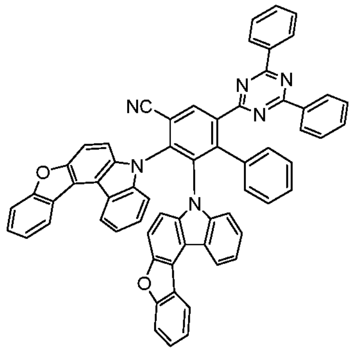

**T59**

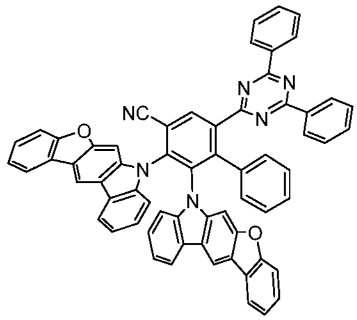

**T60**

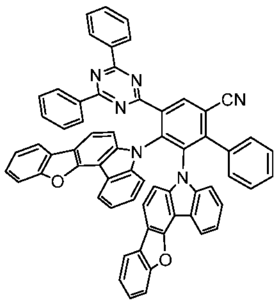

**T61**

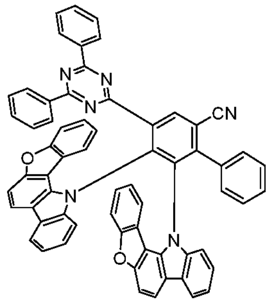

**T62**

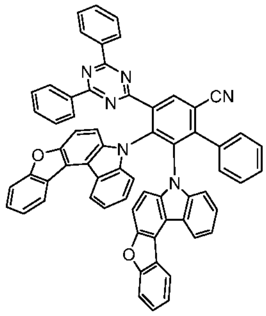

**T63**

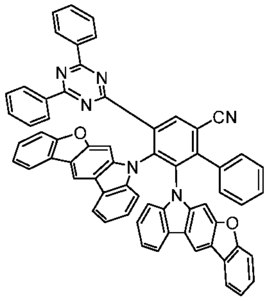

**T64**

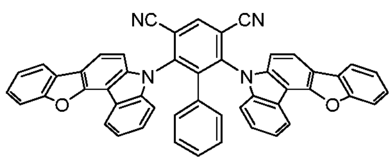

**T65**

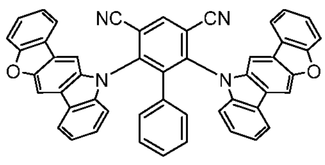

**T66**

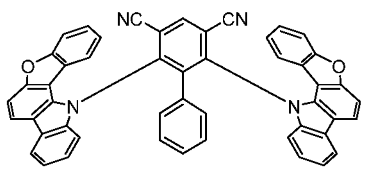

**T67**

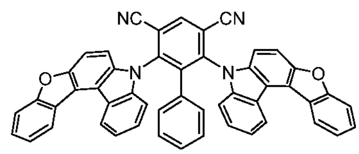

**T68**

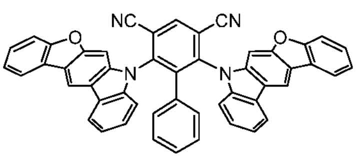

**T69**

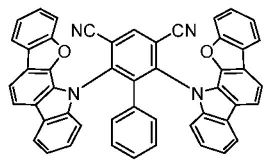

**T70**

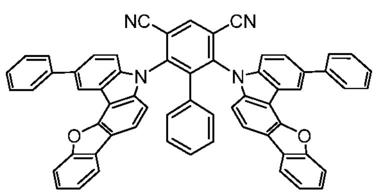

**T71**

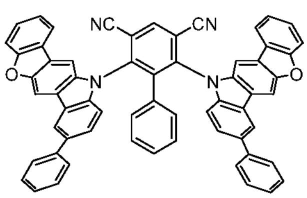

**T72**

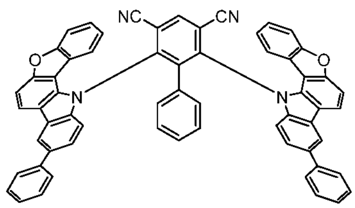

**T73**

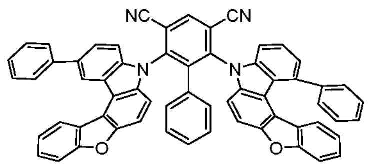

**T74**

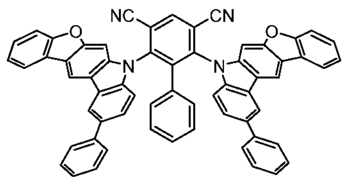

**T75**

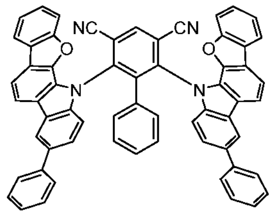

**T76**

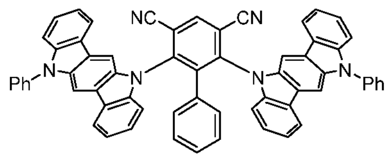

**T77**

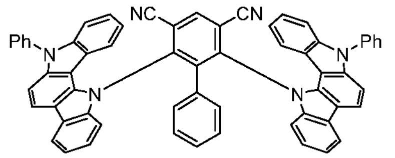

**T78**

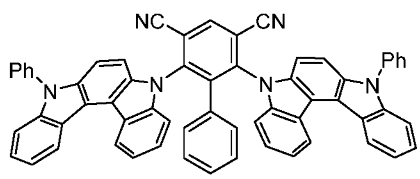

**T79**

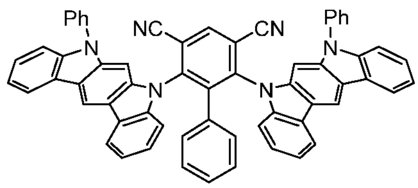

**T80**

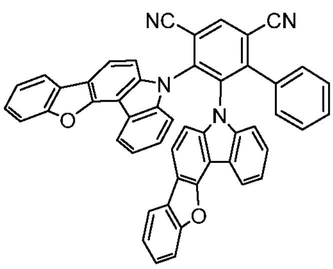

**T81**

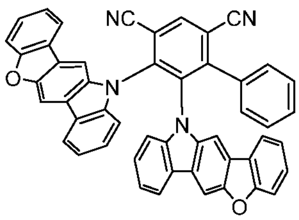

**T82**

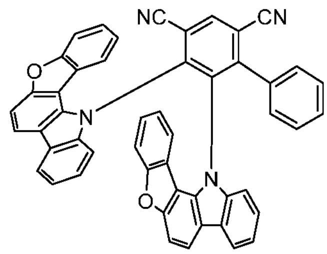

**T83**

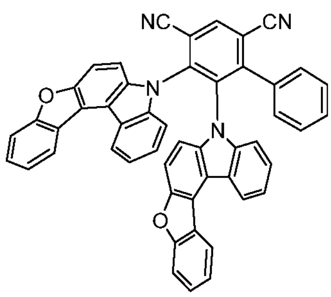

**T84**

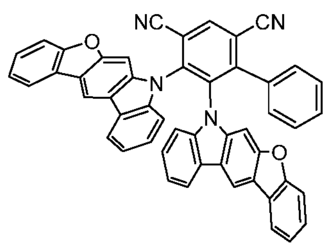

**T85**

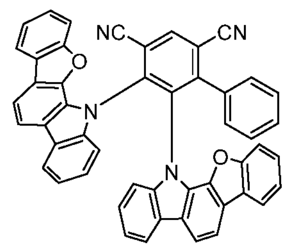

**T86**

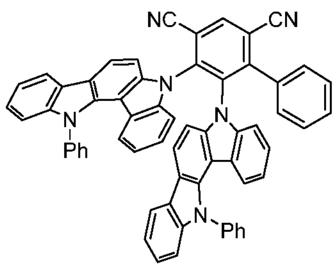

T87

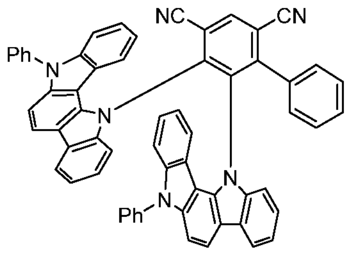

T88

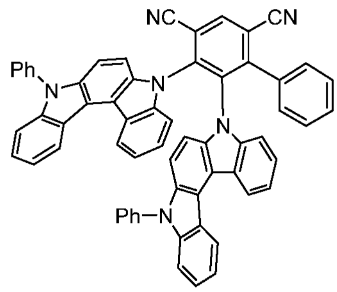

T89

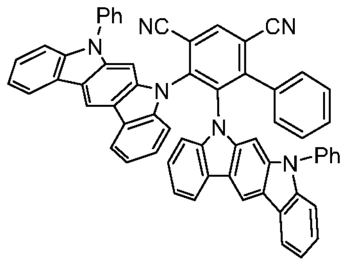

T90

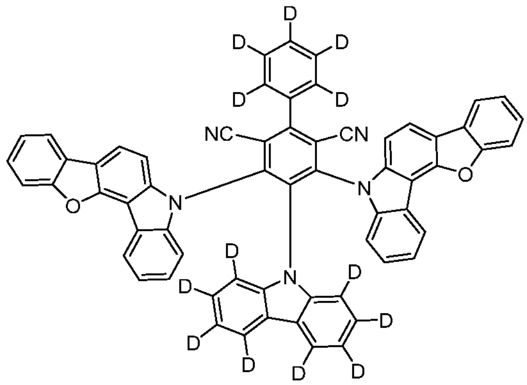

T91

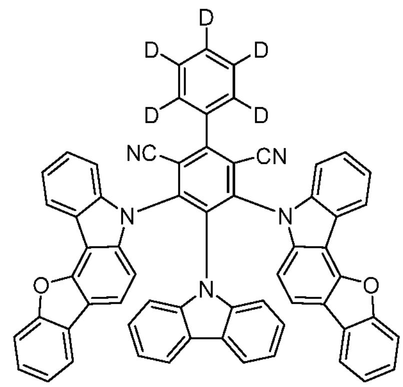

T92

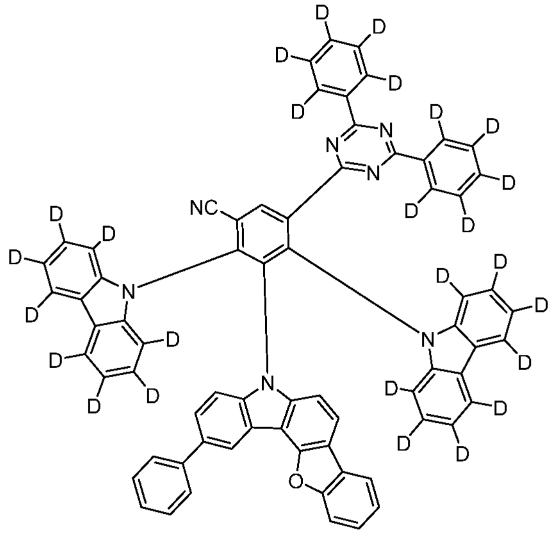

T93

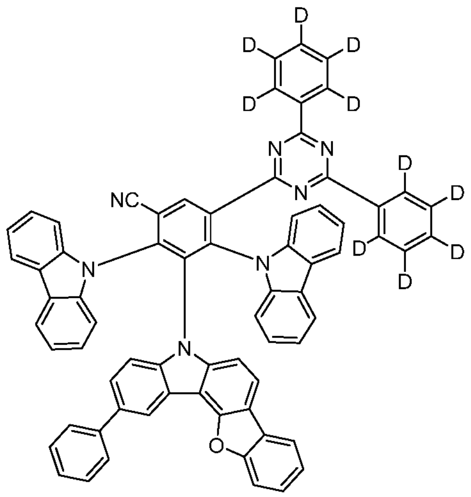

**T94**

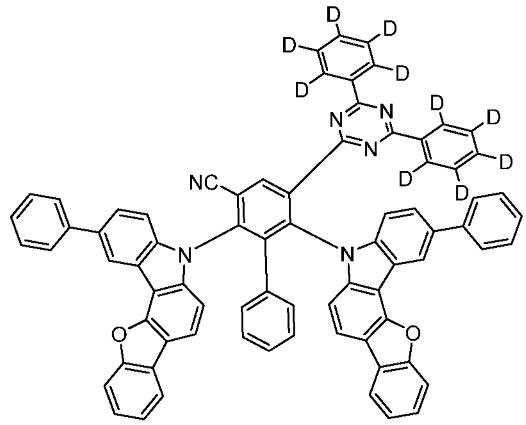

**T95**

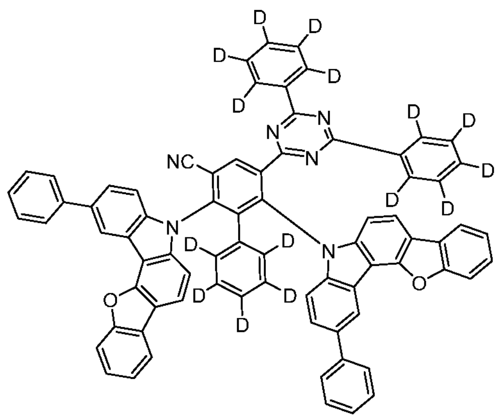

**T96**

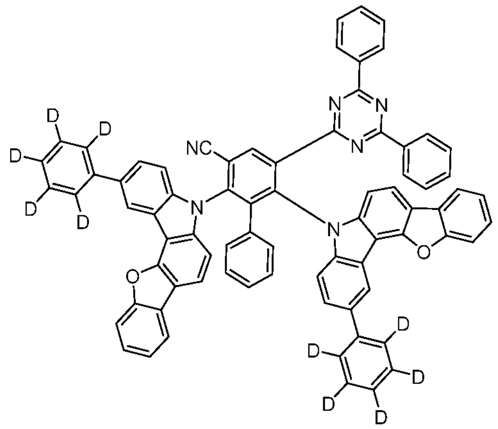

**T97**

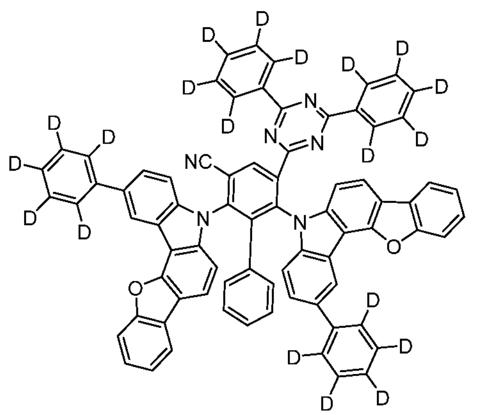

**T98**

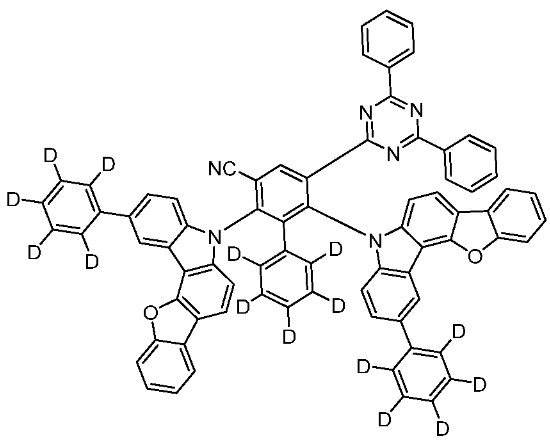

**T99**

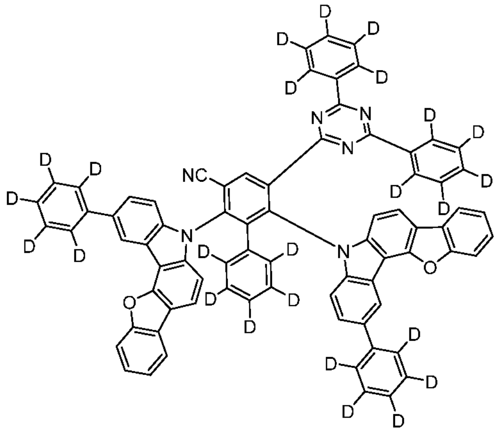

**T100**

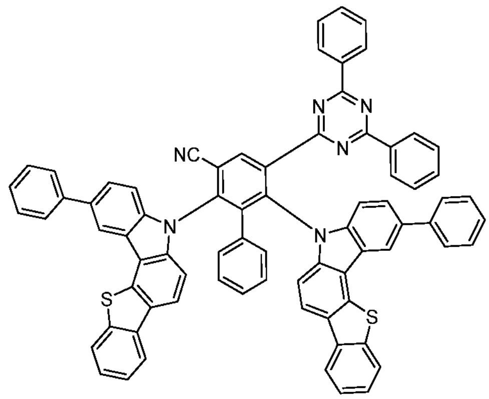

**T101**

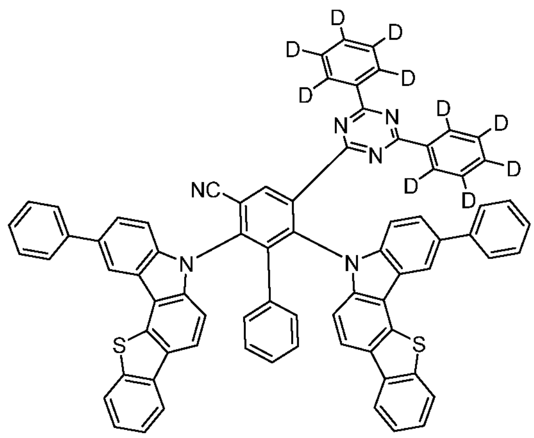

**T102**

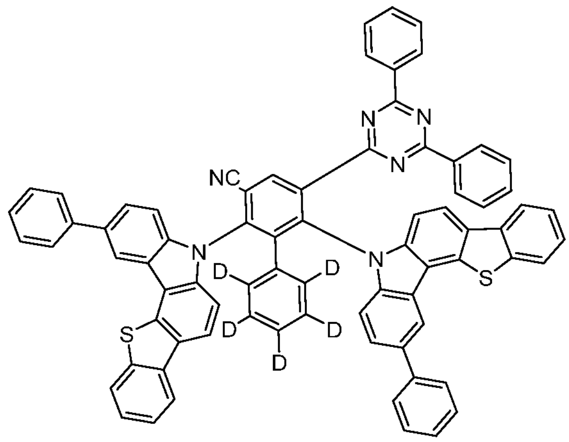

**T103**

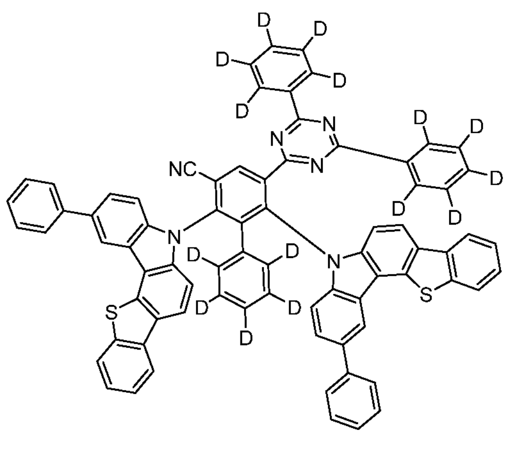

**T104**

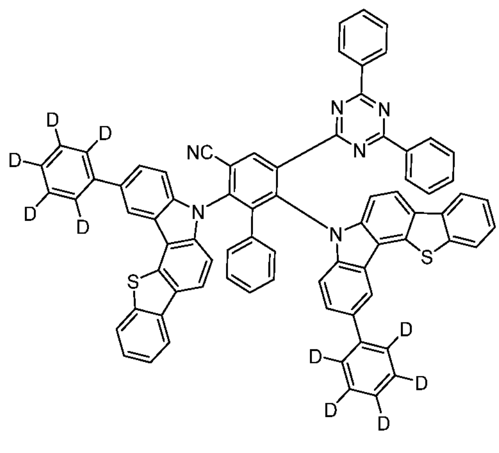

**T105**

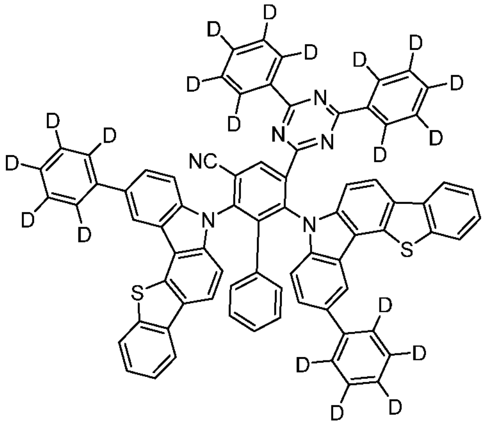

T106

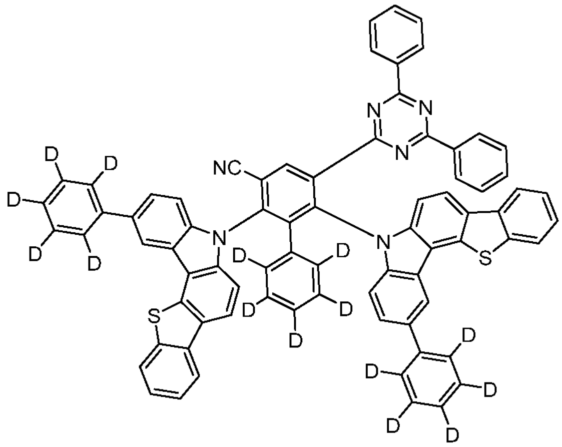

T107

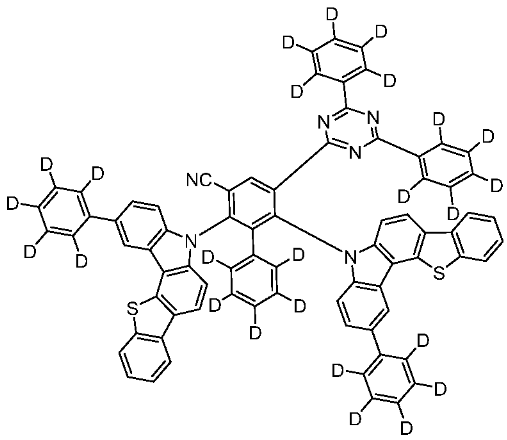

T108

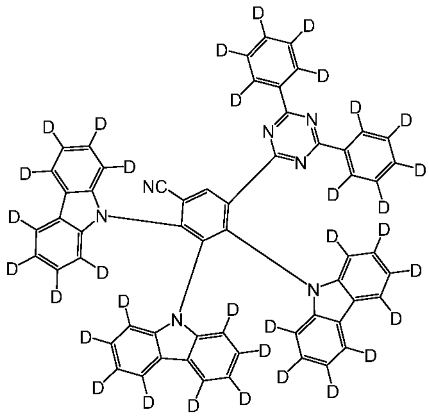

T109

The molecular weight of the compound represented by the formula (2) is preferably 1500 or less, more preferably 1200 or less, further preferably 1000 or less, still further preferably 900 or less, for example, when there is an intention to form and use a film of an organic layer containing the compound represented by the formula (2) through a vapor deposition method. The lower limit value of the molecular weight is the molecular weight of the smallest compound in the compound group represented by the formula (2).

It is preferable that the compound represented by the formula (2) does not include a metal atom. For example, as the compound represented by the formula (2), a compound including an atom selected from the group consisting of a carbon atom, a hydrogen atom, a deuterium atom, a nitrogen atom, an oxygen atom, and a sulfur atom may be selected. For example, as the compound represented by the formula (2), a compound including an atom selected from the group consisting of a carbon atom, a hydrogen atom, a deuterium atom, a nitrogen atom, and an oxygen atom may be selected. For example, as the compound represented by the formula (2), a compound including an atom selected from the group consisting of a carbon atom, a hydrogen atom, a deuterium atom, a nitrogen atom, and a sulfur atom may be selected. For example, as the compound represented by the formula (2), a compound including an atom selected from the group consisting of a carbon atom, a hydrogen atom, a deuterium atom, and a nitrogen atom may be selected.

[Film]

A film of the invention is a film containing the compound represented by the formula (1) and the compound represented by the formula (2).

The film of the invention may contain only the compound represented by the formula (1) and the compound represented by the formula (2), or may contain other materials. As the other materials, a compound having lowest excited singlet energy higher than that of the compound represented by the formula (1) or the compound represented by the formula (2) is preferably used. Such as compound can be used as a host material. The film of the invention may contain

two or more types of compounds represented by the formula (1). Further, the film of the invention may contain two or more types of compounds represented by the formula (2).

In some embodiments, the film of the invention does not contain a metal element. In some embodiments, the film of the invention can be made of a material composed of only atoms selected from the group consisting of a carbon atom, a hydrogen atom, a deuterium atom, a nitrogen atom, an oxygen atom, a sulfur atom, and a boron atom. Alternatively, the film of the invention may also be made of a material composed of only atoms selected from the group consisting of a carbon atom, a hydrogen atom, a deuterium atom, a nitrogen atom, an oxygen atom, and a boron atom.

It is preferable that the film of the invention contains more compound represented by the formula (2) than the compound represented by the formula (1). In one aspect of the invention, when the content of the compound represented by the formula (2) is 100 parts by weight, the content of the compound represented by the formula (1) is 0.1 to 50 parts by weight, preferably 0.5 to 20 parts by weight, and for example 1 to 10 parts by weight. When the film of the invention contains a host material, and the total amount of the host material, the compound represented by the formula (1), and the compound represented by the formula (2) is 100 parts by weight, the content of the host material is preferably 35 to 99 parts by weight, more preferably 45 to 95 parts by weight, and for example 50 to 90 parts by weight.

A method for manufacturing the film of the invention is not particularly limited. In some embodiments, the film of the invention can be formed in a wet process. In a wet process, a solution prepared by dissolving the compound of the invention is applied onto a surface, and then the solvent is removed to form a film. The wet process includes a spin coating method, a slit coating method, an ink jet method (a spraying method), a gravure printing method, an offset printing method and a flexographic printing method, which, however, are not limitative. In the wet process, an appropriate organic solvent capable of dissolving the compound of the formula (1) and the compound of the formula (2) is selected and used. In some embodiments, a substituent (for example, an alkyl group) capable of increasing the solubility in an organic solvent can be introduced into the compound to be contained in the light emitting material. Further, in some embodiments, a film containing the compound of the invention can be formed in a dry process. In some embodiments, a vacuum evaporation method is employable as a dry process, which, however, is not limitative. In the case where a vacuum evaporation method is employed, compounds to constitute a film can be co-evaporated from individual evaporation sources, or can be co-evaporated from a single evaporation source formed by mixing the compounds. In the case where a single evaporation source is used, a mixed powder prepared by mixing compound powders can be used, or a compression molded body prepared by compression-molding the mixed powder can be used, or a mixture prepared by heating and melting the constituent compounds and cooling the resulting melt can be used. In some embodiments, by co-evaporation under the condition where the evaporation rate (weight reduction rate) of the plural compounds contained in a single evaporation source is the same or is nearly the same, a film having a compositional ratio corresponding to the compositional ratio of the plural compounds contained in the evaporation source can be formed. When plural compounds are mixed in the same compositional ratio as the compositional ratio of the film to be formed to prepare an evaporation source, a film having a desired compositional ratio can be formed in a simplified manner. In some embodiments, the temperature at which the compounds to be co-evaporated has the same weight reduction ratio is specifically defined, and the temperature can be employed as the temperature of co-evaporation.

The thickness of the film of the invention can be suitably determined, depending on the purpose. For example, in a case where the film is used for light emission, the thickness can be within a range of 0.5 to 100 μm.

The film of the invention has high orientation of the constituent materials. In particular, the orientation of the compound represented by the formula (1) is high. The compound represented by the formula (1) is horizontally oriented on the film surface, and thus, has an advantage of high luminous efficiency. The orientation may be evaluated by an orientation value (S value). A larger negative value (a smaller numerical value) indicates that the orientation is higher. The orientation value (S value) may be determined by the method described in Scientific Reports 2017, 7, 8405. In the film of the invention, the orientation value of the compound represented by the formula (1) is preferably -0.35 or less, more preferably -0.38 or less, further preferably -0.41 or less.

In some embodiments, the film of the invention emits delayed fluorescence.

In some embodiments of the present disclosure, the film of the invention is, when excited thermally or by an electronic means, able to emit light in a UV region, light of blue, green, yellow, or orange in a visible region, in a red region (e.g., about 420 nm to about 500 nm, about 500 nm to about 600 nm, or about 600 nm to about 700 nm) or in a near IR region.

When a host material is used in the invention, it is preferable to select a host material from organic compounds having a hole transport function and an electron transport function. In some embodiments, a host material of a light-emitting layer is an organic compound that prevents the wavelength of emitted light from increasing. In some embodiments, the host material of the light-emitting layer is an organic compound having a high glass transition temperature.

In some embodiments, a host material is selected from the group consisting of the followings. Here, the host material that can be adopted in the invention is not construed as limiting to the following specific examples.

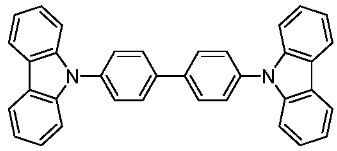
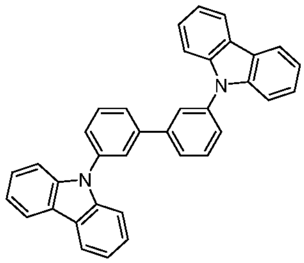
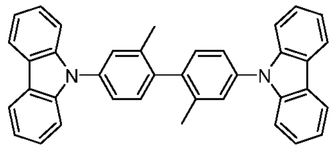

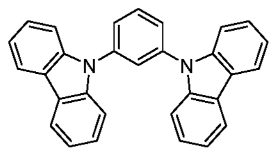
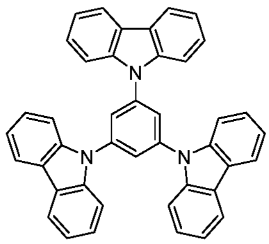
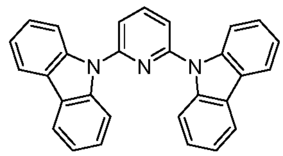

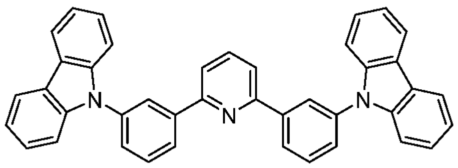
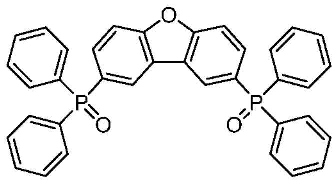

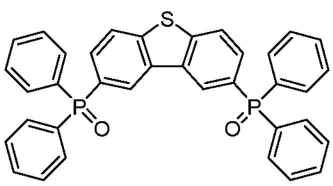
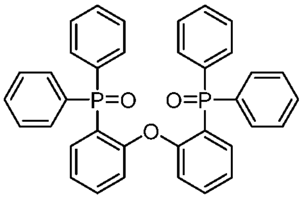
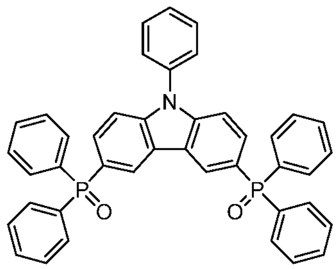

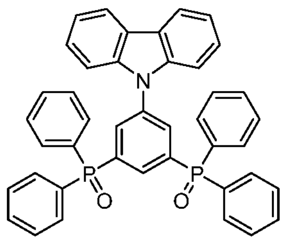
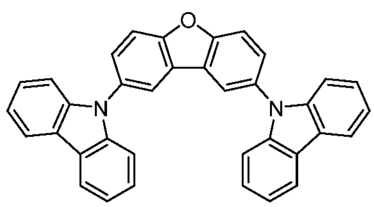
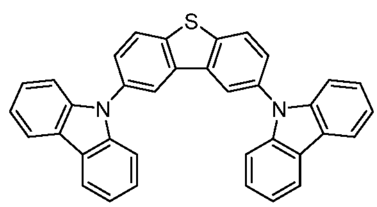

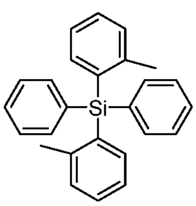
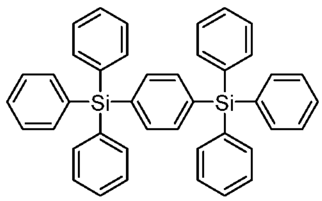
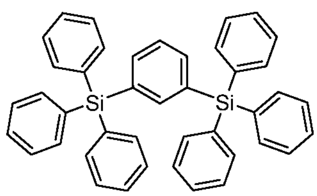
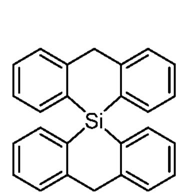

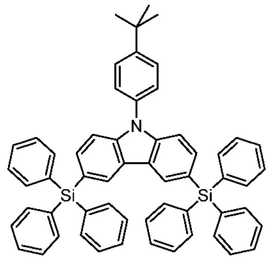
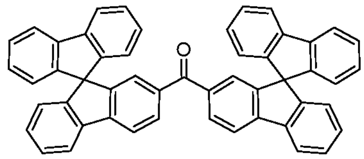
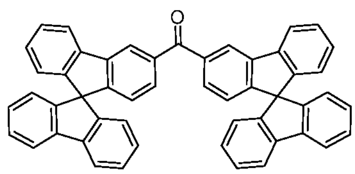

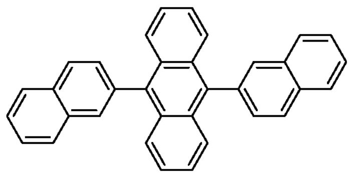
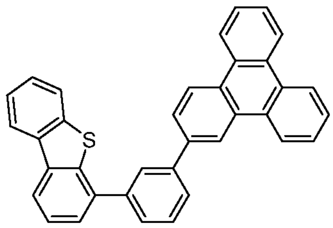
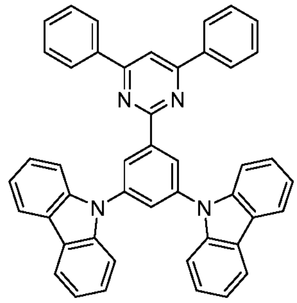

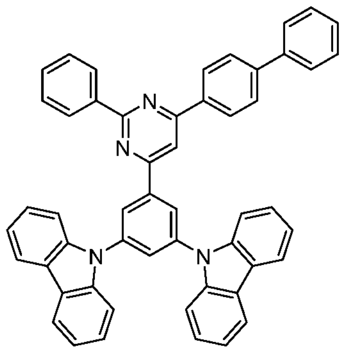
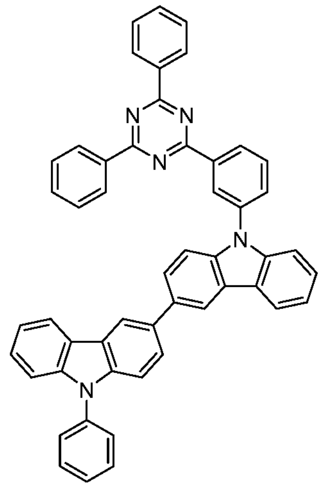
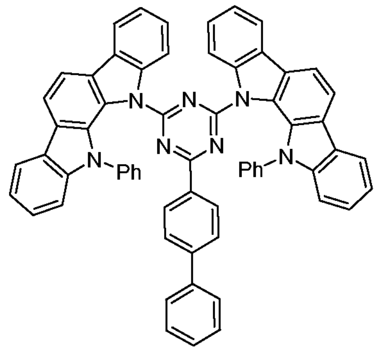

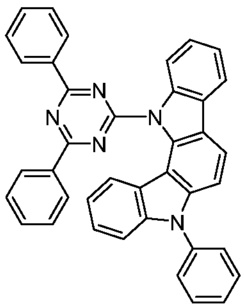
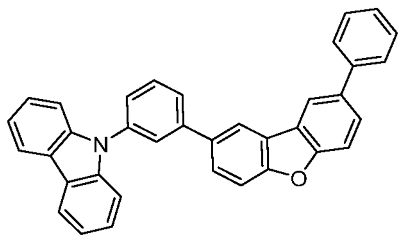
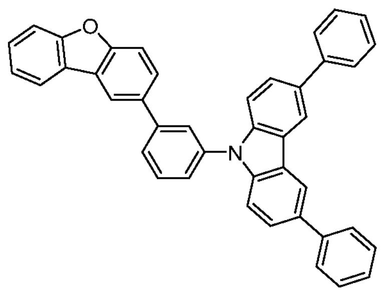

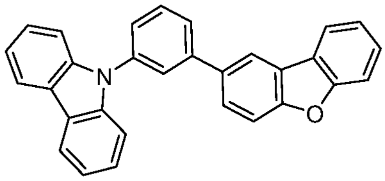

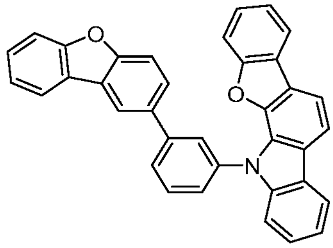

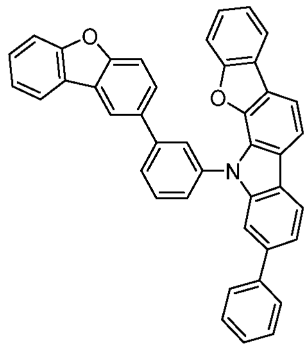

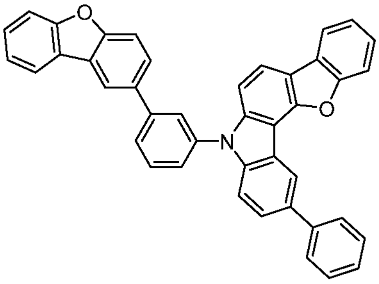

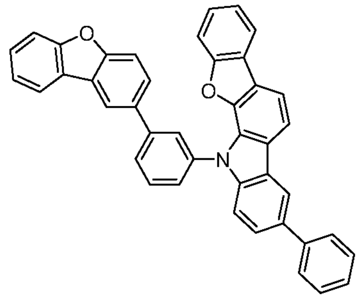

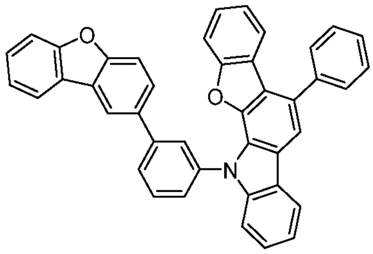

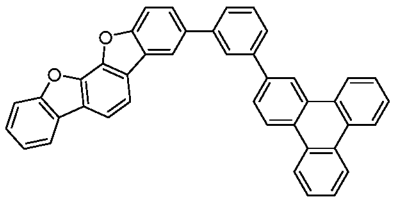

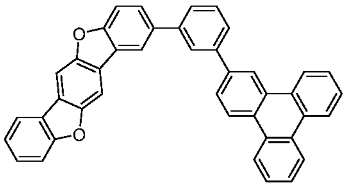

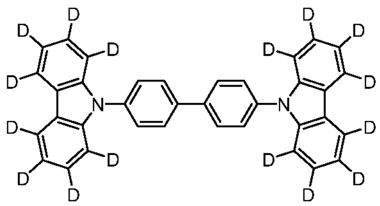

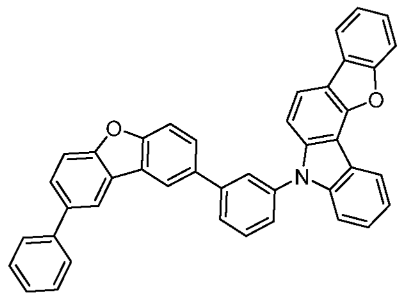

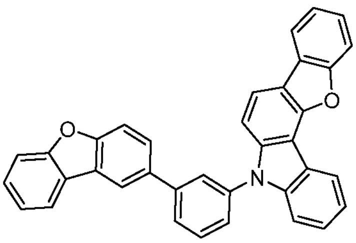

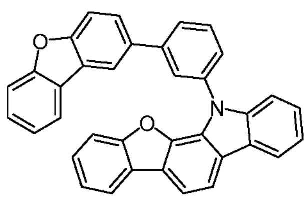

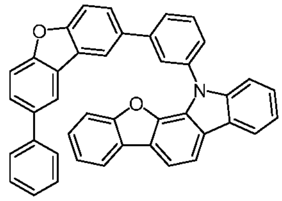

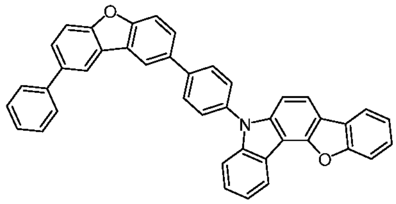

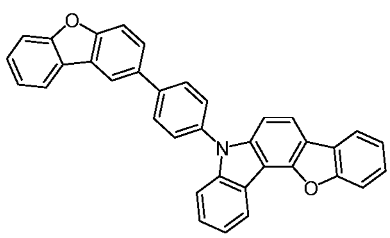

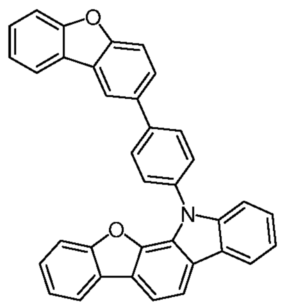 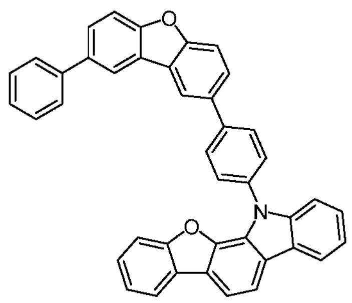

Further, a host material represented by the following formula can also be preferably employed.

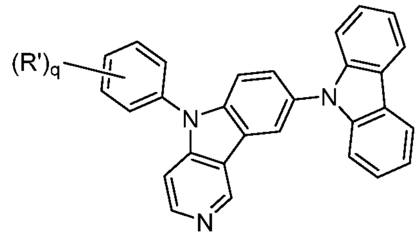

In the above formula, R' represents a substituted or unsubstituted aryl group or a substituted or unsubstituted heteroaryl group. For example, one group selected from the group consisting of a phenyl group, a pyridyl group, a dibenzofuryl group, and a dibenzothienyl group, or a group formed by combining two or more thereof can be exemplified. q represents any integer of 1 to 5, is preferably 1 or 2, more preferably 1.

Specific examples of the compound represented by the above formula will be exemplified below.

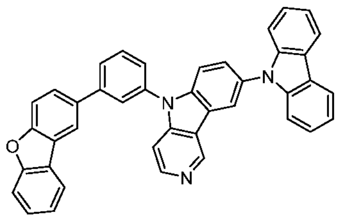 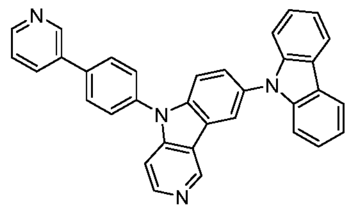 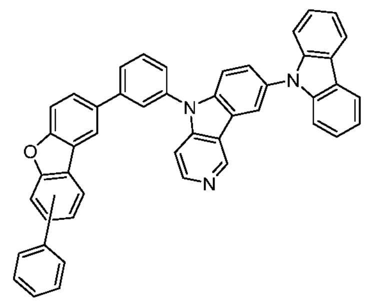

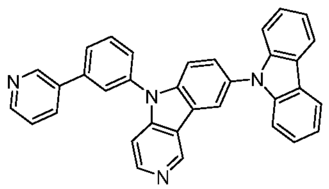 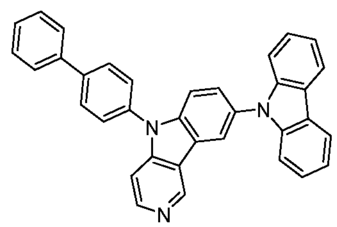

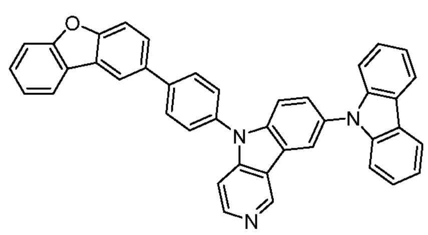

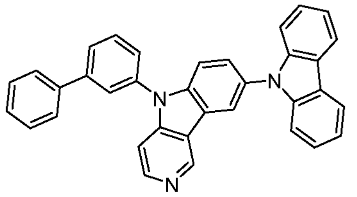

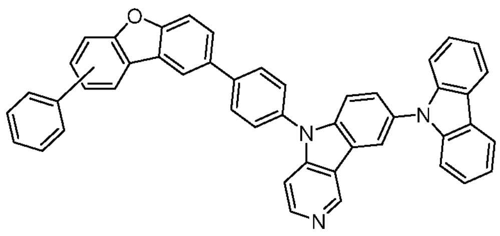

Further, a host material represented by the following formula can also be preferably employed.

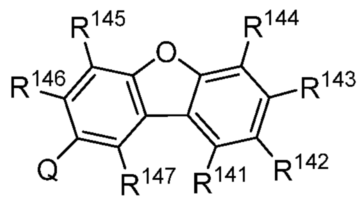

In the above formula, each of $R^{141}$ to $R^{147}$ independently represents a hydrogen atom, a deuterium atom, or a substituent, and at least one of $R^{141}$ to $R^{147}$ is a substituted or unsubstituted aryl group. Q represents a substituted or unsubstituted 12H-benzofurocarbazole-12-yl phenyl group.

In one aspect of the invention, each of $R^{141}$ to $R^{147}$ is independently a hydrogen atom, a deuterium atom, a substituted or unsubstituted alkyl group, or a substituted or unsubstituted aryl group. In one aspect of the invention, each of $R^{141}$ to $R^{147}$ is independently an aryl group that may be substituted with one atom or group selected from the group consisting of a hydrogen atom, a deuterium atom, or a deuterium atom, an alkyl group, and an aryl group, or a group formed by combining two or more thereof. In a preferred aspect of the invention, only one of $R^{141}$ to $R^{147}$ is an aryl group that may be substituted with one atom or group selected from the group consisting of a deuterium atom, an alkyl group, and an aryl group, or a group formed by combining two or more thereof, and each of the rest is independently a hydrogen atom or a deuterium atom. In a preferred aspect of the invention, one of $R^{141}$ to $R^{147}$ is selected from P1 to P14 described below.

In one aspect of the invention, Q is a 12H-benzofurocarbazole-12-yl phenyl group that may be substituted with one atom or group selected from the group consisting of a deuterium atom, an alkyl group, and an aryl group, or a group formed by combining two or more thereof. In one aspect of the invention, Q is a unsubstituted 12H-benzofurocarbazole-12-yl phenyl group. In a preferred aspect of the invention, Q is selected from Q1 to Q18 described below.

Hereinafter, specific examples of the compound represented by the above formula are shown in the format of a table. In Table 1, the structure of the compound is specified by representing Q and $R^{141}$ to $R^{147}$ of each of the compounds. For example, a compound 141-1-1 is a compound in which Q is Q1, $R^{141}$ is P1, and $R^{142}$ to $R^{147}$ are hydrogen atoms, and a compound 141-1-2 is a compound in which Q is Q1, $R^{141}$ is P2, and $R^{142}$ to $R^{147}$ are hydrogen atoms. In each row of Table 2, 14 types of structures are collectively specified in one row. For example, in the row of "141-1-1 to 141-1-14" in Table 2, compounds in which Q is Q1, $R^{141}$ is P1 to P14, and $R^{142}$ to $R^{147}$ are hydrogen atoms are collectively specified as a compound 141-1-1 to a compound 141-1-14 in this order. Here, the structures of the compound 141-1-1 to the compound 141-1-14 which are collectively indicated correspond to the structures of the compound 141-1-1 to the compound 141-1-14 which are specified in Table 1. In the row of "141-2-1 to 141-2-14" in Table 2, compounds in which Q is Q2, $R^{141}$ is P1 to P14, and $R^{142}$ to $R^{147}$ are hydrogen atoms are collectively specified as a compound 141-2-1 to a compound 141-2-14 in this order. For the subsequent rows, the structure of each compound is specified in the same manner. It is assumed that each compound specified in Table 2 is individually disclosed herein.

Table 2

| No. | Q | $R^{141}$ | $R^{142}$,$R^{143}$,$R^{144}$, $R^{145}$,$R^{146}$,$R^{147}$ |
|---|---|---|---|
| 141-1-1 | Q1 | P1 | H |
| 141-1-2 | Q1 | P2 | H |
| 141-1-3 | Q1 | P3 | H |
| 141-1-4 | Q1 | P4 | H |
| 141-1-5 | Q1 | P5 | H |

(continued)

| No. | Q | $R^{141}$ | $R^{142}$,$R^{143}$,$R^{144}$, $R^{145}$,$R^{146}$,$R^{147}$ |
|---|---|---|---|
| 141-1-6 | Q1 | P6 | H |
| 141-1-7 | Q1 | P7 | H |
| 141-1-8 | Q1 | P8 | H |
| 141-1-9 | Q1 | P9 | H |
| 141-1-10 | Q1 | P10 | H |
| 141-1-11 | Q1 | P11 | H |
| 141-1-12 | Q1 | P12 | H |
| 141-1-13 | Q1 | P13 | H |
| 141-1-14 | Q1 | P14 | H |
| 141-2-1 | Q2 | P1 | H |
| 141-2-2 | Q2 | P2 | H |
| 141-2-3 | Q2 | P3 | H |
| 141-2-4 | Q2 | P4 | H |
| 141-2-5 | Q2 | P5 | H |
| 141-2-6 | Q2 | P6 | H |
| 141-2-7 | Q2 | P7 | H |
| 141-2-8 | Q2 | P8 | H |
| 141-2-9 | Q2 | P9 | H |
| 141-2-10 | Q2 | P10 | H |
| 141-2-11 | Q2 | P11 | H |
| 141-2-12 | Q2 | P12 | H |
| 141-2-13 | Q2 | P13 | H |
| 141-2-14 | Q2 | P14 | H |
| 141-3-1 | Q3 | P1 | H |
| 141-3-2 | Q3 | P2 | H |
| 141-3-3 | Q3 | P3 | H |
| 141-3-4 | Q3 | P4 | H |
| 141-3-5 | Q3 | P5 | H |
| 141-3-6 | Q3 | P6 | H |
| 141-3-7 | Q3 | P7 | H |
| 141-3-8 | Q3 | P8 | H |
| 141-3-9 | Q3 | P9 | H |
| 141-3-10 | Q3 | P10 | H |
| 141-3-11 | Q3 | P11 | H |
| 141-3-12 | Q3 | P12 | H |
| 141-3-13 | Q3 | P13 | H |
| 141-3-14 | Q3 | P14 | H |

(continued)

| No. | Q | $R^{142}$ | $R^{141}$,$R^{143}$,$R^{144}$, $R^{145}$,$R^{146}$,$R^{147}$ |
|---|---|---|---|
| 142-1-1 | Q1 | P1 | H |
| 142-1-2 | Q1 | P2 | H |
| 142-1-3 | Q1 | P3 | H |
| 142-1-4 | Q1 | P4 | H |
| 142-1-5 | Q1 | P5 | H |
| 142-1-6 | Q1 | P6 | H |
| 142-1-7 | Q1 | P7 | H |
| 142-1-8 | Q1 | P8 | H |
| 142-1-9 | Q1 | P9 | H |
| 142-1-10 | Q1 | P10 | H |
| 142-1-11 | Q1 | P11 | H |
| 142-1-12 | Q1 | P12 | H |
| 142-1-13 | Q1 | P13 | H |
| 142-1-14 | Q1 | P14 | H |
| 142-2-1 | Q2 | P1 | H |
| 142-2-2 | Q2 | P2 | H |
| 142-2-3 | Q2 | P3 | H |
| 142-2-4 | Q2 | P4 | H |
| 142-2-5 | Q2 | P5 | H |
| 142-2-6 | Q2 | P6 | H |
| 142-2-7 | Q2 | P7 | H |
| 142-2-8 | Q2 | P8 | H |
| 142-2-9 | Q2 | P9 | H |
| 142-2-10 | Q2 | P10 | H |
| 142-2-11 | Q2 | P11 | H |
| 142-2-12 | Q2 | P12 | H |
| 142-2-13 | Q2 | P13 | H |
| 142-2-14 | Q2 | P14 | H |
| 142-3-1 | Q3 | P1 | H |
| 142-3-2 | Q3 | P2 | H |
| 142-3-3 | Q3 | P3 | H |
| 142-3-4 | Q3 | P4 | H |
| 142-3-5 | Q3 | P5 | H |
| 142-3-6 | Q3 | P6 | H |
| 142-3-7 | Q3 | P7 | H |
| 142-3-8 | Q3 | P8 | H |
| 142-3-9 | Q3 | P9 | H |
| 142-3-10 | Q3 | P10 | H |

(continued)

| No. | Q | $R^{142}$ | $R^{141}$,$R^{143}$,$R^{144}$, $R^{145}$,$R^{146}$,$R^{147}$ |
|---|---|---|---|
| 142-3-11 | Q3 | P11 | H |
| 142-3-12 | Q3 | P12 | H |
| 142-3-13 | Q3 | P13 | H |
| 142-3-14 | Q3 | P14 | H |

Table 3

| No. | Q | $R^{141}$ | $R^{142}$,$R^{143}$,$R^{144}$,$R^{145}$,$R^{146}$,$R^{147}$ |
|---|---|---|---|
| 141-1-1 ~ 141-1-14 | Q1 | P1~P14 | H |
| 141-2-1 ~ 141-2-14 | Q2 | P1~P14 | H |
| 141-3-1 ~ 141-3-14 | Q3 | P1~P14 | H |
| 141-4-1 ~ 141-4-1 4 | Q4 | P1~P14 | H |
| 141-5-1 ~ 141-5-14 | Q5 | P1~P14 | H |
| 141-6-1 ~ 141-6-14 | Q6 | P1~P14 | H |
| 141-7-1 ~ 141-7-14 | Q7 | P1~P14 | H |
| 141-8-1 ~ 141-8-14 | Q8 | P1~P14 | H |
| 141-9-1 ~ 141-9-14 | Q9 | P1~P14 | H |
| 141-10-1 ~ 141-10-14 | Q10 | P1~P14 | H |
| 141-11-1 ~ 141-11-14 | Q11 | P1~P14 | H |
| 141-12-1 ~ 141-12-14 | Q12 | P1~P14 | H |
| 141-13-1 ~ 141-13-14 | Q13 | P1~P14 | H |
| 141-14-1 ~ 141-14-14 | Q14 | P1~P14 | H |
| No. | Q | $R^{142}$ | $R^{141}$,$R^{143}$,$R^{144}$,$R^{145}$,$R^{146}$,$R^{147}$ |
| 142-1-1 ~ 142-1-14 | Q1 | P1~P14 | H |
| 142-2-1 ~ 142-2-14 | Q2 | P1~P14 | H |
| 142-3-1 ~ 142-3-14 | Q3 | P1~P14 | H |
| 142-4-1 ~ 142-4-14 | Q4 | P1~P14 | H |
| 142-5-1 ~ 142-5-14 | Q5 | P1~P14 | H |
| 142-6-1 ~ 142-6-14 | Q6 | P1~P14 | H |
| 142-7-1 ~ 142-7-14 | Q7 | P1~P14 | H |
| 142-8-1 ~ 142-8-14 | Q8 | P1~P14 | H |
| 142-9-1 ~ 142-9-14 | Q9 | P1~P14 | H |
| 142-10-1 ~ 142-10-14 | Q10 | P1~P14 | H |
| 142-11-1 ~ 142-11-14 | Q11 | P1~P14 | H |
| 142-12-1 ~ 142-12-14 | Q12 | P1~P14 | H |
| 142-13-1 ~ 142-13-14 | Q13 | P1~P14 | H |
| 142-14-1 ~ 142-14-14 | Q14 | P1~P14 | H |

(continued)

| No. | Q | $R^{143}$ | $R^{141}$,$R^{142}$,$R^{144}$,$R^{145}$,$R^{146}$,$R^{147}$ |
|---|---|---|---|
| 143-1-1 ~ 143-1-14 | Q1 | P1~P14 | H |
| 143-2-1 ~ 143-2-14 | Q2 | P1~P14 | H |
| 143-3-1 ~ 143-3-14 | Q3 | P1~P14 | H |
| 143-4-1 ~ 143-4-14 | Q4 | P1~P14 | H |
| 143-5-1 ~ 143-5-14 | Q5 | P1~P14 | H |
| 143-6-1 ~ 143-6-14 | Q6 | P1~P14 | H |
| 143-7-1 ~ 143-7-14 | Q7 | P1~P14 | H |
| 143-8-1 ~ 143-8-14 | Q8 | P1~P14 | H |
| 143-9-1 ~ 143-9-14 | Q9 | P1~P14 | H |
| 143-10-1 ~ 143-10-14 | Q10 | P1~P14 | H |
| 143-11-1 ~ 143-11-14 | Q11 | P1~P14 | H |
| 143-12-1 ~ 143-12-14 | Q12 | P1~P14 | H |
| 143-13-1 ~ 143-13-14 | Q13 | P1~P14 | H |
| 143-14-1 ~ 143-14-14 | Q14 | P1~P14 | H |
| No. | Q | $R^{144}$ | $R^{141}$,$R^{142}$,$R^{143}$,$R^{145}$,$R^{148}$,$R^{147}$ |
| 144-1-1 ~ 144-1-14 | Q1 | P1~P14 | H |
| 144-2-1 ~ 144-2-14 | Q2 | P1~P14 | H |
| 144-3-1 ~ 144-3-14 | Q3 | P1~P14 | H |
| 144-4-1 ~ 144-4-14 | Q4 | P1~P14 | H |
| 144-5-1 ~ 144-5-14 | Q5 | P1~P14 | H |
| 144-6-1 ~ 144-6-14 | Q6 | P1~P14 | H |
| 144-7-1 ~ 144-7-14 | Q7 | P1~P14 | H |
| 144-8-1 ~ 144-8-14 | Q8 | P1~P14 | H |
| 144-9-1 ~ 144-9-14 | Q9 | P1~P14 | H |
| 144-10-1 ~ 144-10-14 | Q10 | P1~P14 | H |
| 144-11-1 ~ 144-11-14 | Q11 | P1~P14 | H |
| 144-12-1 ~ 144-12-14 | Q12 | P1~P14 | H |
| 144-13-1 ~ 144-13-14 | Q13 | P1~P14 | H |
| 144-14-1 ~ 144-14-14 | Q14 | P1~P14 | H |
| No. | Q | $R^{145}$ | $R^{141}$,$R^{142}$,$R^{143}$,$R^{144}$,$R^{146}$,$R^{147}$ |
| 145-1-1 ~ 145-1-14 | Q1 | P1~P14 | H |
| 145-2-1 ~ 145-2-14 | Q2 | P1~P14 | H |
| 145-3-1 ~ 145-3-14 | Q3 | P1~P14 | H |
| 145-4-1 ~ 145-4-14 | Q4 | P1~P14 | H |
| 145-5-1 ~ 145-5-14 | Q5 | P1~P14 | H |
| 145-6-1 ~ 145-6-14 | Q6 | P1~P14 | H |
| 145-7-1 ~ 145-7-14 | Q7 | P1~P14 | H |

(continued)

| No. | Q | $R^{145}$ | $R^{141}$,$R^{142}$,$R^{143}$,$R^{144}$,$R^{146}$,$R^{147}$ |
|---|---|---|---|
| 145-8-1 ~ 145-8-14 | Q8 | P1~P14 | H |
| 145-9-1 ~ 145-9-14 | Q9 | P1~P14 | H |
| 145-10-1 ~ 145-10-14 | Q10 | P1~P14 | H |
| 145-11-1 ~ 145-11-14 | Q11 | P1~P14 | H |
| 145-12-1 ~ 145-12-14 | Q12 | P1~P14 | H |
| 145-13-1 ~ 145-13-14 | Q13 | P1~P14 | H |
| 145-14-1 ~ 145-14-14 | Q14 | P1~P14 | H |
| No. | Q | $R^{146}$ | $R^{141}$,$R^{142}$,$R^{143}$,$R^{144}$,$R^{145}$,$R^{147}$ |
| 146-1-1 ~ 146-1-14 | Q1 | P1~P14 | H |
| 146-2-1 ~ 146-2-14 | Q2 | P1~P14 | H |
| 146-3-1 ~ 146-3-14 | Q3 | P1~P14 | H |
| 146-4-1 ~ 146-4-14 | Q4 | P1~P14 | H |
| 146-5-1 ~ 146-5-14 | Q5 | P1~P14 | H |
| 146-6-1 ~ 146-6-14 | Q6 | P1~P14 | H |
| 146-7-1 ~ 146-7-14 | Q7 | P1~P14 | H |
| 146-8-1 ~ 146-8-14 | Q8 | P1~P14 | H |
| 146-9-1 ~ 146-9-14 | Q9 | P1~P14 | H |
| 146-10-1 ~ 146-10-14 | Q10 | P1~P14 | H |
| 146-11-1 ~ 146-11-14 | Q11 | P1~P14 | H |
| 146-12-1 ~ 146-12-14 | Q12 | P1~P14 | H |
| 146-13-1 ~ 146-13-14 | Q13 | P1~P14 | H |
| 146-14-1 ~ 146-14-14 | Q14 | P1~P14 | H |
| No. | Q | $R^{147}$ | $R^{141}$,$R^{142}$,$R^{143}$,$R^{144}$,$R^{145}$.$R^{146}$ |
| 147-1-1 ~ 147-1-14 | Q1 | P1~P14 | H |
| 147-2-1 ~ 147-2-14 | Q2 | P1~P14 | H |
| 147-3-1 ~ 147-3-14 | Q3 | P1~P14 | H |
| 147-4-1 ~ 147-4-14 | Q4 | P1~P14 | H |
| 147-5-1 ~ 147-5-14 | Q5 | P1~P14 | H |
| 147-6-1 ~ 147-6-14 | Q6 | P1~P14 | H |
| 147-7-1 ~ 147-7-14 | Q7 | P1~P14 | H |
| 147-8-1 ~ 147-8-14 | Q8 | P1~P14 | H |
| 147-9-1 ~ 147-9-14 | Q9 | P1~P14 | H |
| 147-10-1 ~ 147-10-14 | Q10 | P1~P14 | H |
| 147-11-1 ~ 147-11-14 | Q11 | P1~P14 | H |
| 147-12-1 ~ 147-12-14 | Q12 | P1~P14 | H |
| 147-13-1 ~ 147-13-14 | Q13 | P1~P14 | H |
| 147-14-1 ~ 147-14-14 | Q14 | P1~P14 | H |

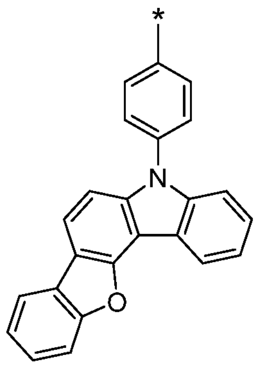

**Q1**

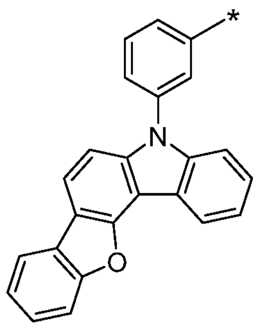

**Q2**

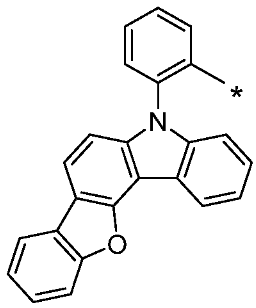

**Q3**

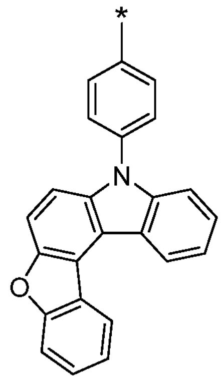

**Q4**

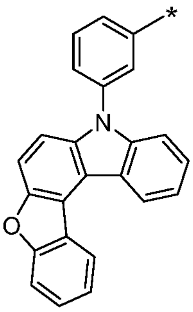

**Q5**

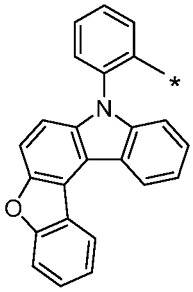

**Q6**

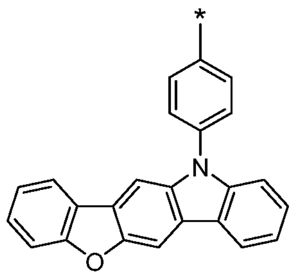

**Q7**

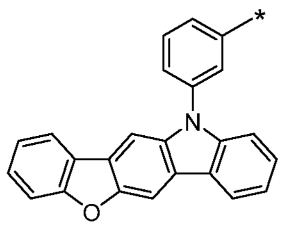

**Q8**

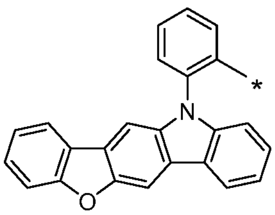

**Q9**

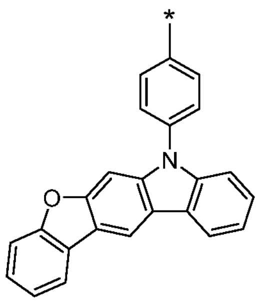

**Q10**

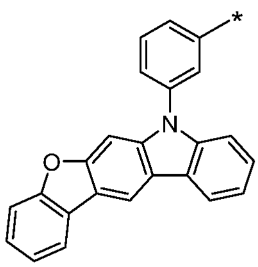

**Q11**

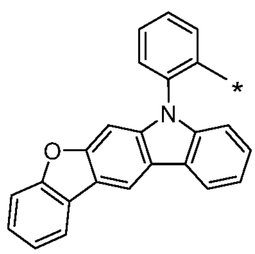

**Q12**

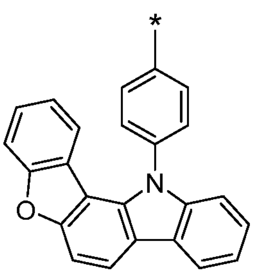

**Q13**

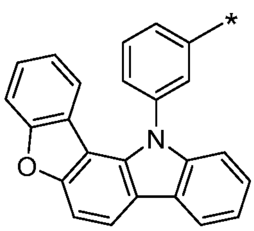

**Q14**

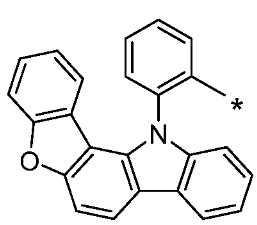

**Q15**

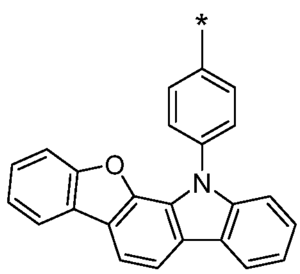

**Q16**

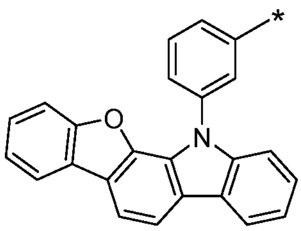
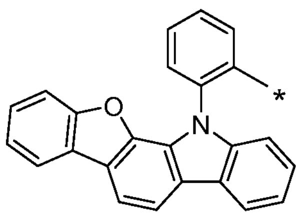

**Q17** **Q18**

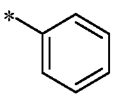
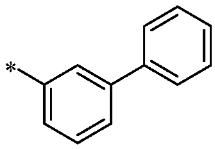
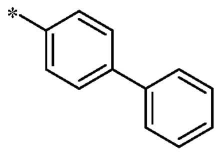
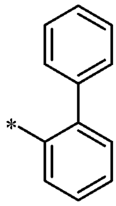
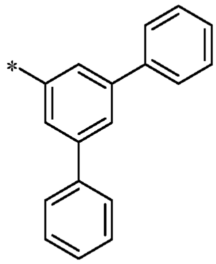

**P1** **P2** **P3** **P4** **P5**

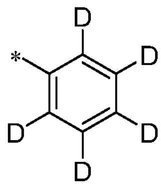
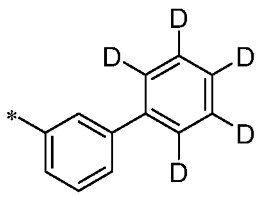
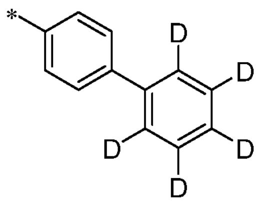
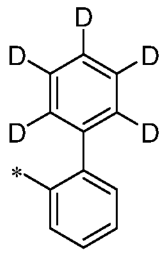

**P6** **P7** **P8** **P9**

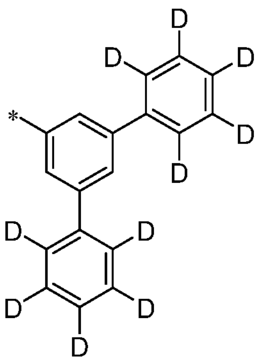
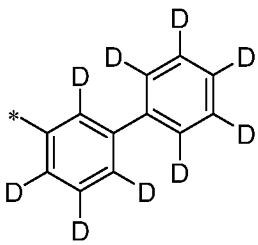
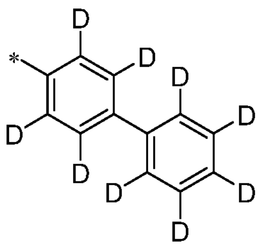
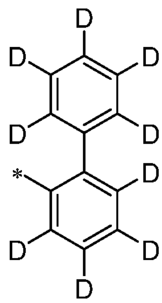

**P10** **P11** **P12** **P13**

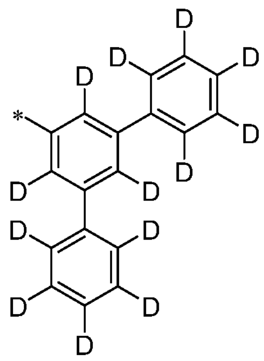

**P14**

[Organic light-emitting device]

An organic light-emitting device of the invention is an organic light-emitting device using the compound represented by the formula (1) and the compound represented by the formula (2).

In some embodiments, the organic light-emitting device EL device. In some embodiments, the light-emitting layer includes the compound represented by the formula (1) as a light-emitting material. In some embodiments, the organic light-emitting device is an organic photoluminescence device (organic PL device). In some embodiments, the organic light-emitting device is an organic electroluminescence device (organic EL device). In some embodiments, the compound represented by the formula (2) assists light emission of the light-emitting material represented by the formula (1) included in the light-emitting layer (as a so-called assist dopant). In some embodiments, the light-emitting layer is the film of the invention.

In some embodiments, the organic photoluminescence device includes at least one light-emitting layer. In some embodiments, the organic electroluminescence device includes at least an anode, a cathode, and an organic layer between the anode and the cathode. In some embodiments, the organic layer includes at least a light-emitting layer. In some embodiments, the organic layer includes only a light-emitting layer. In some embodiments, the organic layer includes one or more organic layers as well as the light-emitting layer. Examples of the organic layer include a hole transport layer, a hole injection layer, an electron barrier layer, a hole barrier layer, an electron injection layer, an electron transport layer and an exciton barrier layer. In some embodiments, the hole transport layer may be a hole injection transport layer having a hole injection function, and the electron transport layer may be an electron injection transport layer having an electron injection function.

In the following, the constituent members and the other layers than a light-emitting layer of the organic electroluminescent device are described.

Substrate:

In some embodiments, the organic electroluminescent device of the invention is supported by a substrate, wherein the substrate is not particularly limited and may be any of those that have been commonly used in an organic electroluminescent device, for example those formed of glass, transparent plastics, quartz, and silicon.

Anode:

In some embodiments, the anode of the organic electroluminescent device is made of a metal, an alloy, an electroconductive compound, or a combination thereof. In some embodiments, the metal, alloy, or electroconductive compound has a large work function (4 eV or more). In some embodiments, the metal is Au. In some embodiments, the electroconductive transparent material is selected from CuI, indium tin oxide (ITO), $SnO_2$, and ZnO. In some embodiments, an amorphous material capable of forming a transparent electroconductive film, such as IDIXO ($In_2O_3$-ZnO), is used. In some embodiments, the anode is a thin film. In some embodiments, the thin film is made by vapor deposition or sputtering. In some embodiments, the film is patterned by a photolithography method. In some embodiments, when the pattern may not require high accuracy (for example, approximately 100 μm or more), the pattern may be formed with a mask having a desired shape on vapor deposition or sputtering of the electrode material. In some embodiments, when a material can be applied as a coating material, such as an organic electroconductive compound, a wet film forming method, such as a printing method and a coating method is used. In some embodiments, when the emitted light goes through the anode, the anode has a transmittance of more than 10%, and the anode has a sheet resistance of several hundred Ohm per square or less. In some embodiments, the thickness of the anode is from 10 to 1,000 nm. In some

embodiments, the thickness of the anode is from 10 to 200 nm. In some embodiments, the thickness of the anode varies depending on the material used.

Cathode:

In some embodiments, the cathode is made of an electrode material such as a metal having a small work function (4 eV or less) (referred to as an electron injection metal), an alloy, an electroconductive compound, or a combination thereof. In some embodiments, the electrode material is selected from sodium, a sodium-potassium alloy, magnesium, lithium, a magnesium-cupper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth metal. In some embodiments, a mixture of an electron injection metal and a second metal that is a stable metal having a larger work function than the electron injection metal is used. In some embodiments, the mixture is selected from a magnesium-silver mixture, a magnesium-aluminum mixture, a magnesium-indium mixture, an aluminum-aluminum oxide ($Al_2O_3$) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per square or less. In some embodiments, the thickness of the cathode ranges from 10 nm to 5 $\mu$m. In some embodiments, the thickness of the cathode ranges from 50 to 200 nm. In some embodiments, for transmitting the emitted light, any one of the anode and the cathode of the organic electroluminescent device is transparent or translucent. In some embodiments, the transparent or translucent electroluminescent devices enhance the light emission luminance.

In some embodiments, the cathode is formed with an electroconductive transparent material, as described for the anode, to form a transparent or translucent cathode. In some embodiments, a device comprises an anode and a cathode, both being transparent or translucent.

Injection Layer:

An injection layer is a layer between the electrode and the organic layer. In some embodiments, the injection layer decreases the driving voltage and enhances the light emission luminance. In some embodiments, the injection layer includes a hole injection layer and an electron injection layer. The injection layer can be positioned between the anode and the light-emitting layer or the hole transport layer, and between the cathode and the light-emitting layer or the electron transport layer. In some embodiments, an injection layer is present. In some embodiments, no injection layer is present.

Preferred compound examples for use as a hole injection material are shown below.

MoOs,

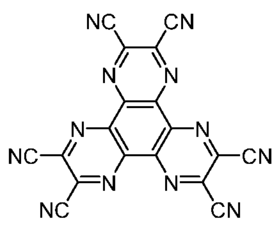
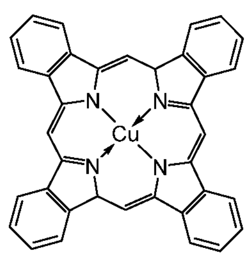
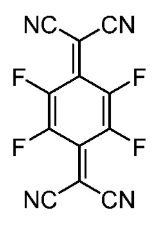
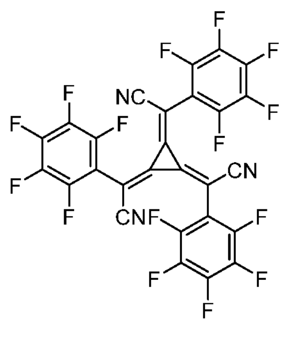

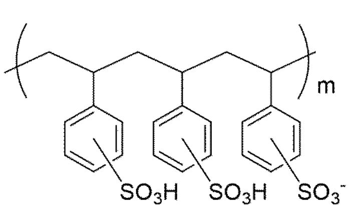
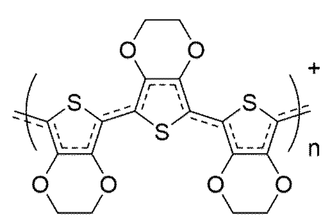

Next, preferred compound examples for use as an electron injection material are shown below.
LiF, CsF,

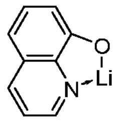

Barrier Layer:

A barrier layer is a layer capable of inhibiting charges (electrons or holes) and/or excitons present in the light-emitting layer from being diffused outside the light-emitting layer. In some embodiments, the electron barrier layer is between the light-emitting layer and the hole transport layer, and inhibits electrons from passing through the light-emitting layer toward the hole transport layer. In some embodiments, the hole barrier layer is between the light-emitting layer and the electron transport layer, and inhibits holes from passing through the light-emitting layer toward the electron transport layer. In some embodiments, the barrier layer inhibits excitons from being diffused outside the light-emitting layer. In some embodiments, the electron barrier layer and the hole barrier layer are exciton barrier layers. As used herein, the term "electron barrier layer" or "exciton barrier layer" includes a layer that has the functions of both electron barrier layer and of an exciton barrier layer.

Hole Barrier Layer:

A hole barrier layer acts as an electron transport layer. In some embodiments, the hole barrier layer inhibits holes from reaching the electron transport layer while transporting electrons. In some embodiments, the hole barrier layer enhances the recombination probability of electrons and holes in the light-emitting layer. The material for the hole barrier layer may be the same materials as the ones described for the electron transport layer.
Preferred compound examples for use for the hole barrier layer are shown below.

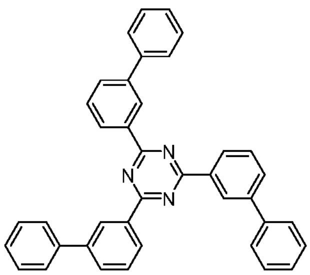

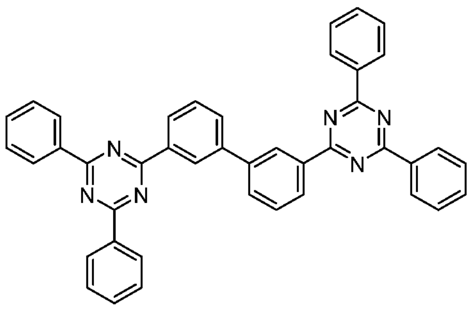

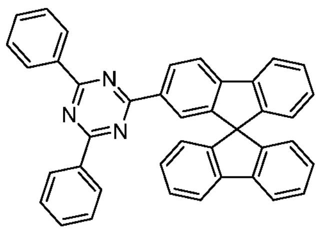

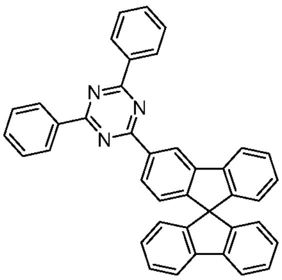

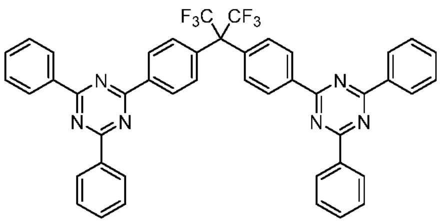

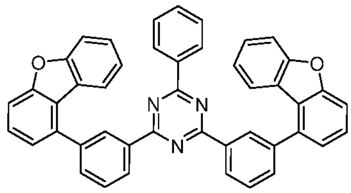

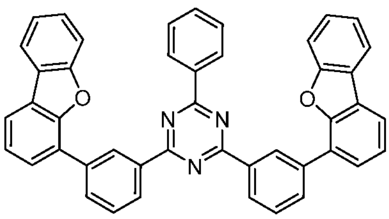

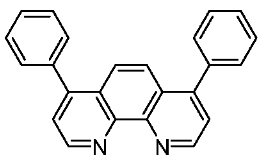

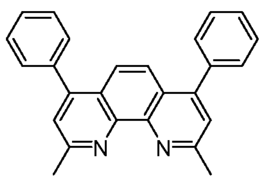

Electron Barrier Layer:

An electron barrier layer transports holes. In some embodiments, the electron barrier layer inhibits electrons from reaching the hole transport layer while transporting holes. In some embodiments, the electron barrier layer enhances the recombination probability of electrons and holes in the light-emitting layer. The materials for use for the electron barrier layer may be the same materials as those mentioned herein above for the hole transport layer.

As the compound for use as the electron barrier material, compounds represented by the following formula may be exemplified.

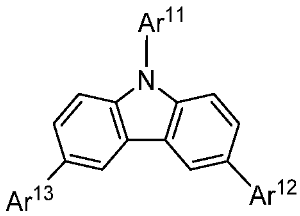

In the above formula, each of $Ar^{11}$ to $Ar^{13}$ independently represents a substituted or unsubstituted aryl group. For example, a substituted or unsubstituted phenyl group, a substituted or unsubstituted naphthalene-1-yl group, and a substituted or unsubstituted naphthalene-2-yl group can be exemplified. Examples of the substituent of the hydrogen atom of the aryl group include one atom or group selected from the group consisting of a deuterium atom, an alkyl group, and an aryl group, or a group formed by combining two or more thereof. Examples of the substituted aryl group include a 4-biphenylyl group, a 3-biphenylyl group, and a m-terphenyl-5'-yl group.

Specific examples of the compound represented by the above formula will be exemplified below.

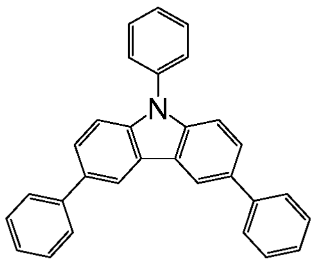
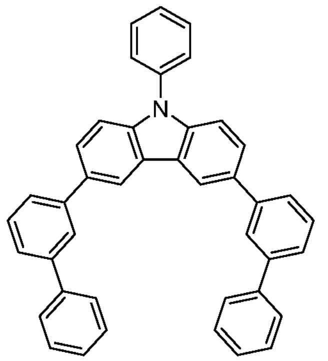
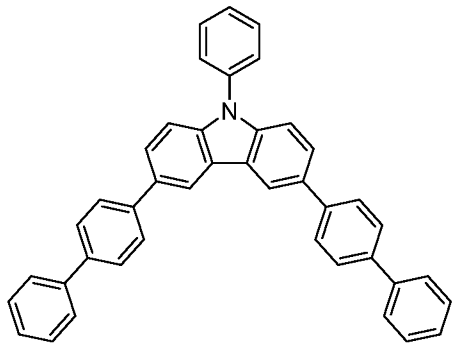

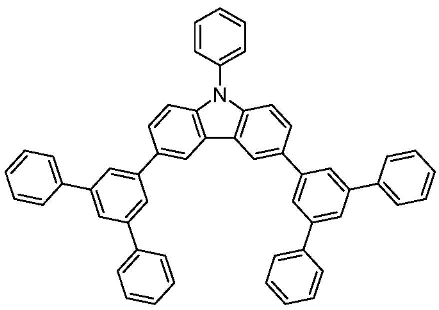
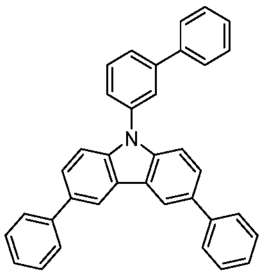
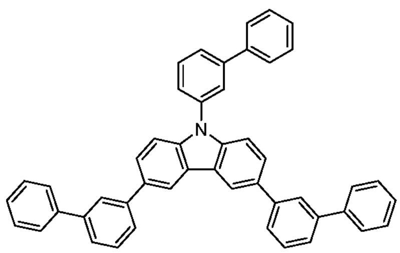

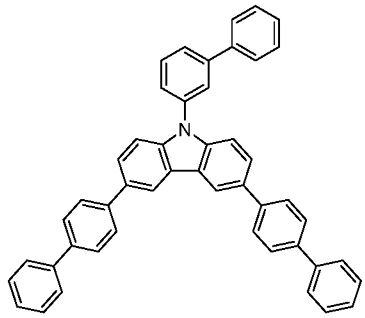

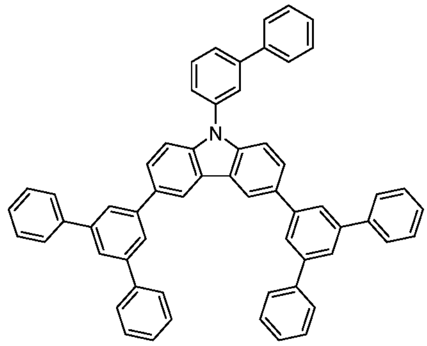

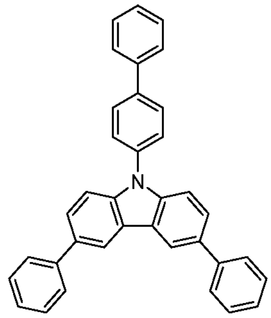

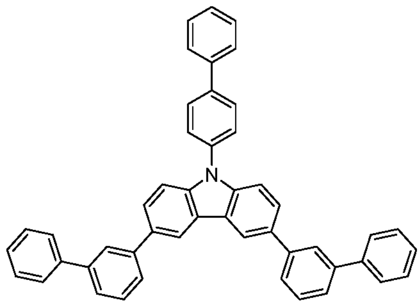

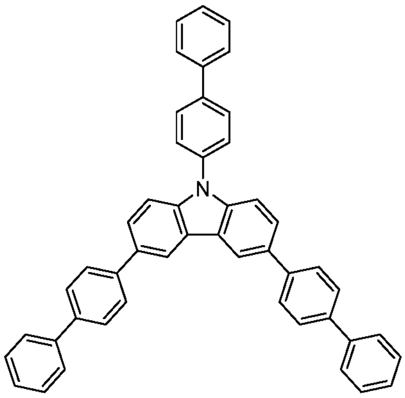

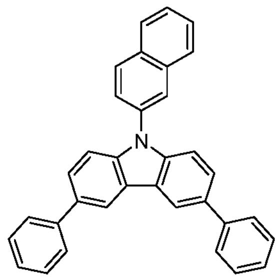

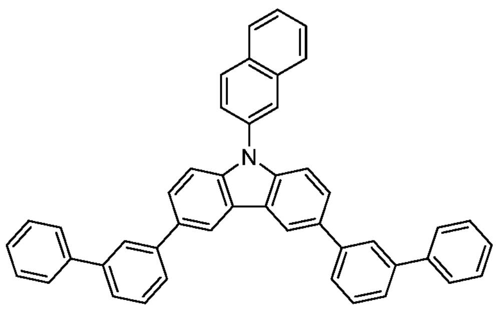

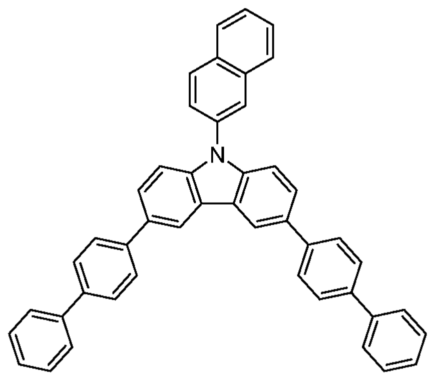

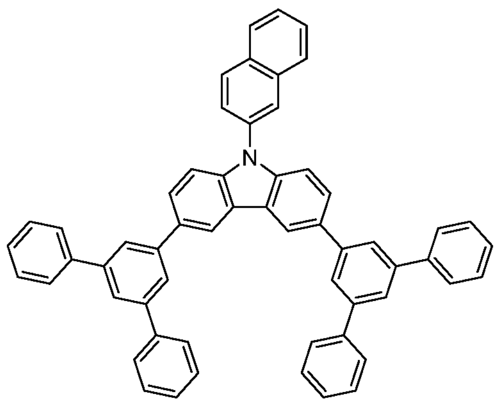

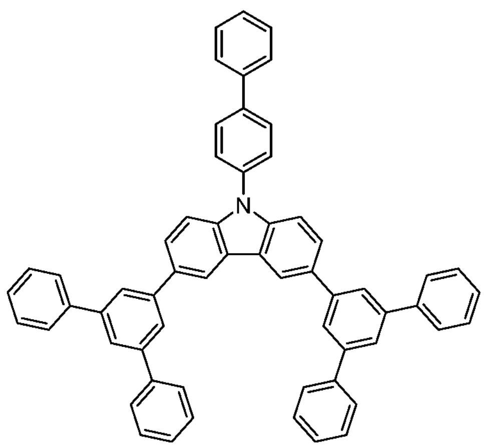

Preferred compound examples for use as the electron barrier material are shown below.

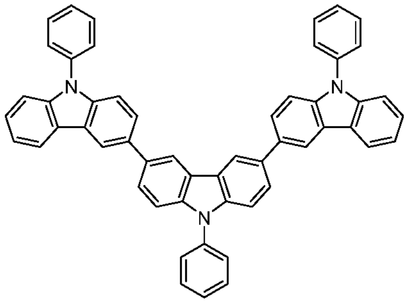

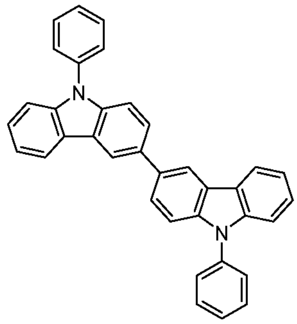

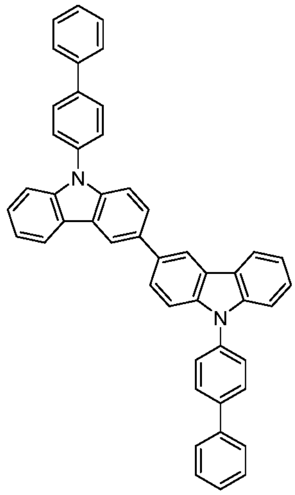

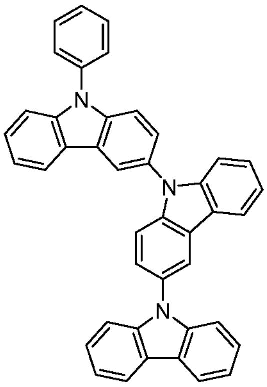

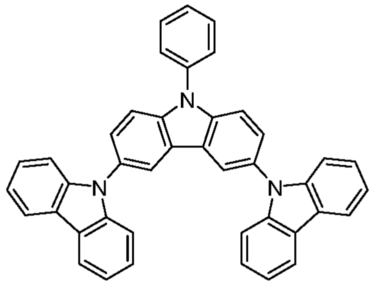

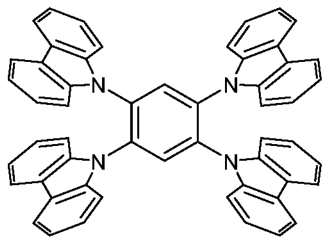

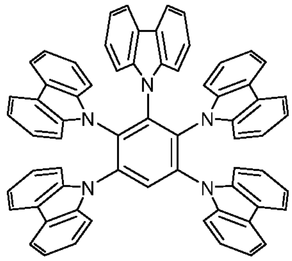

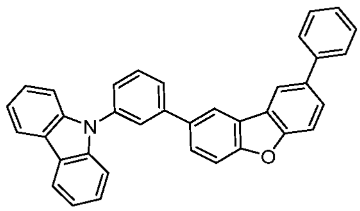

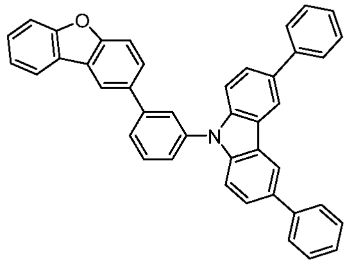

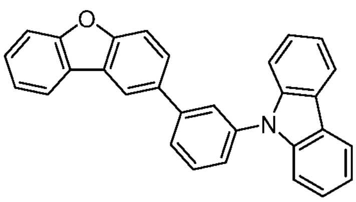

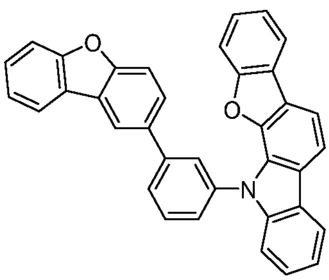

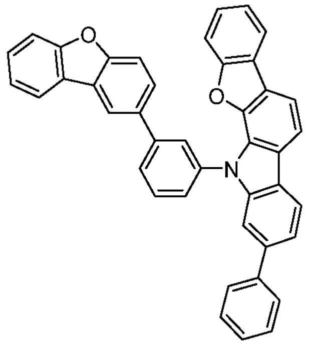

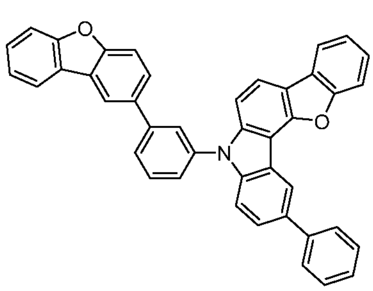

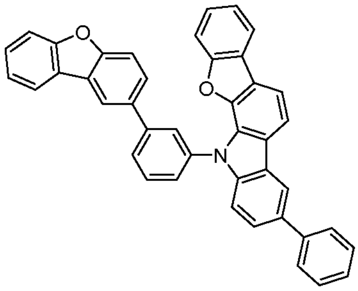

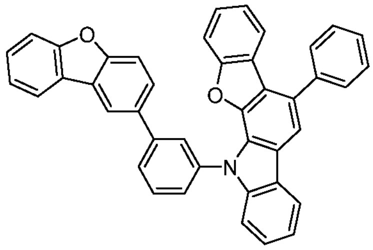

Exciton Barrier Layer:

An exciton barrier layer inhibits excitons generated through recombination of holes and electrons in the light-emitting layer from being diffused to the charge transport layer. In some embodiments, the exciton barrier layer enables effective confinement of excitons in the light-emitting layer. In some embodiments, the light emission efficiency of the device is enhanced. In some embodiments, the exciton barrier layer is adj acent to the light-emitting layer on any of the side of the anode and the side of the cathode, and on both the sides. In some embodiments, when the exciton barrier layer is on the side of the anode, the layer can be between the hole transport layer and the light-emitting layer and adjacent to the light-emitting layer. In some embodiments, when the exciton barrier layer is on the side of the cathode, the layer can be between the light-emitting layer and the cathode and adjacent to the light-emitting layer. In some embodiments, a hole injection layer, an electron barrier layer, or a similar layer is between the anode and the exciton barrier layer that is adjacent to the light-emitting layer on the side of the anode. In some embodiments, a hole injection layer, an electron barrier layer, a hole barrier layer, or a similar layer is between the cathode and the exciton barrier layer that is adjacent to the light-emitting layer on the side of the cathode. In some embodiments, the exciton barrier layer comprises excited singlet energy and excited triplet energy, at least one of which is higher than the excited singlet energy and the excited triplet energy of the light-emitting material, respectively.

Hole Transport Layer:

The hole transport layer comprises a hole transport material. In some embodiments, the hole transport layer is a single layer. In some embodiments, the hole transport layer comprises a plurality of layers.

In some embodiments, the hole transport material has one of injection or transport property of holes and barrier property of electrons. In some embodiments, the hole transport material is an organic material. In some embodiments, the hole transport material is an inorganic material. Examples of known hole transport materials that may be used herein include but are not limited to a triazole derivative, an oxadiazole derivative, an imidazole derivative, a carbazole derivative, an indolocarbazole derivative, a polyaryl alkane derivative, a pyrazoline derivative, a pyrazolone derivative, a phenylene diamine derivative, an arylamine derivative, an amino-substituted chalcone derivative, an oxazole derivative, a styryl anthracene derivative, a fluorenone derivative, a hydrazone derivative, a stilbene derivative, a silazane derivative, an aniline copolymer and an electroconductive polymer oligomer, particularly a thiophene oligomer, or a combination thereof. In some embodiments, the hole transport material is selected from a porphyrin compound, an aromatic tertiary amine compound, and a styryl amine compound. In some embodiments, the hole transport material is an aromatic tertiary amine compound. Preferred compound examples for use as the hole transport material are shown below.

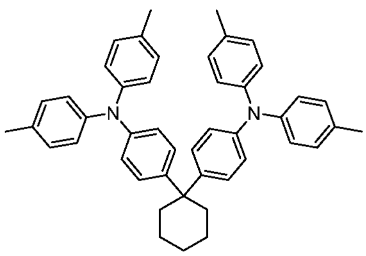

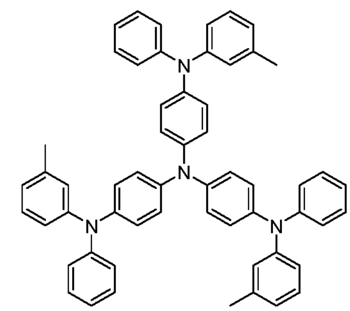

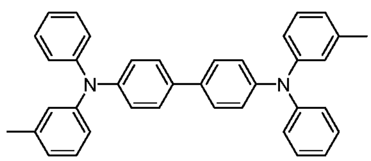

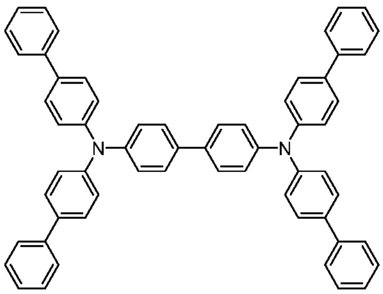

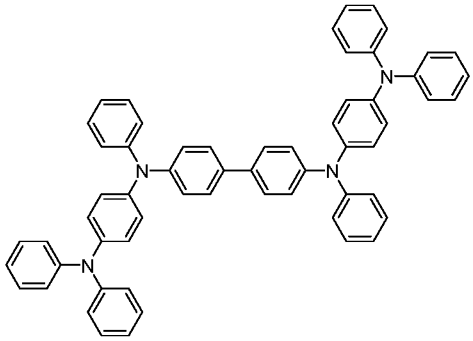

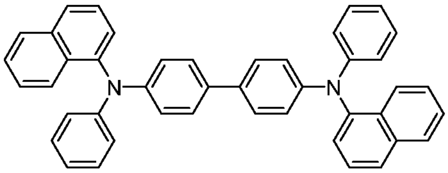

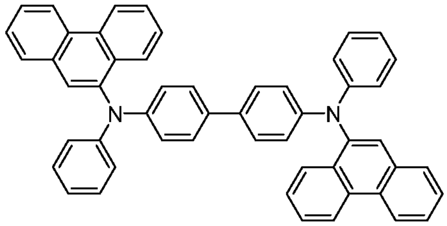

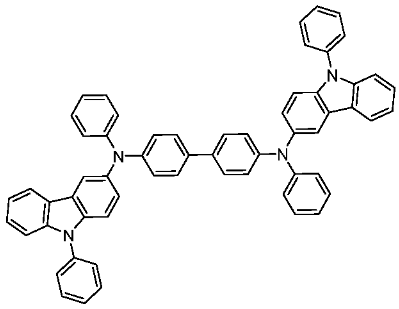

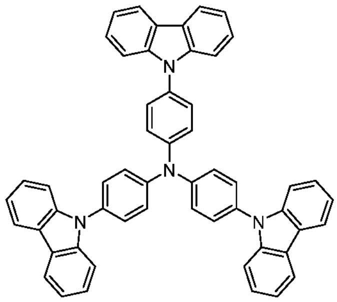

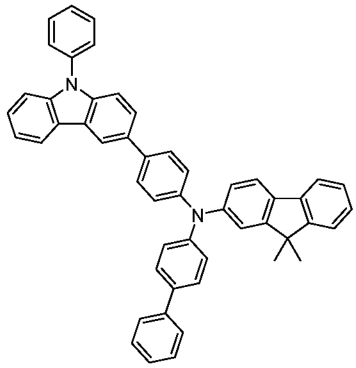

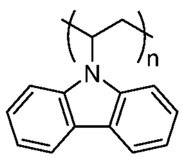

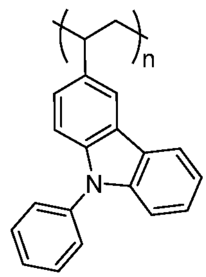

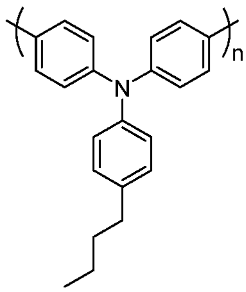

Electron Transport Layer:

The electron transport layer comprises an electron transport material. In some embodiments, the electron transport layer is a single layer. In some embodiments, the electron transport layer comprises a plurality of layers.

In some embodiments, the electron transport material needs only to have a function of transporting electrons, which are injected from the cathode, to the light-emitting layer. In some embodiments, the electron transport material also functions as a hole barrier material. Examples of the electron transport layer that may be used herein include but are not limited to a nitro-substituted fluorene derivative, a diphenyl quinone derivative, a thiopyran dioxide derivative, carbodiimide, a fluorenylidene methane derivative, anthraquinodimethane, an anthrone derivatives, an oxadiazole derivative, an azole derivative, an azine derivative, or a combination thereof, or a polymer thereof. In some embodiments, the electron transport material is a thiadiazole derivative, or a quinoxaline derivative. In some embodiments, the electron transport material is a polymer material. Preferred compound examples for use as the electron transport material are shown below.

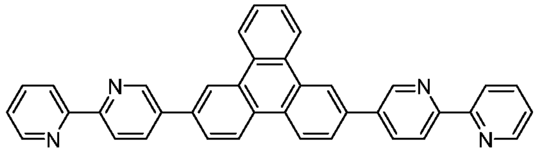
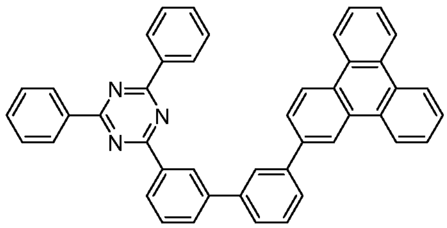

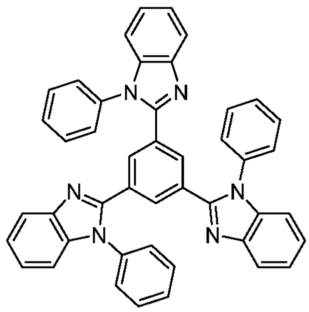
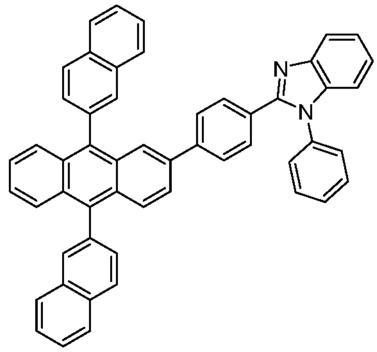
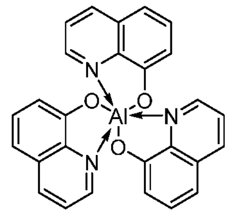

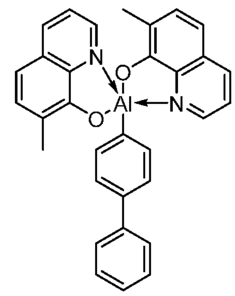
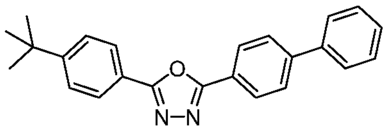
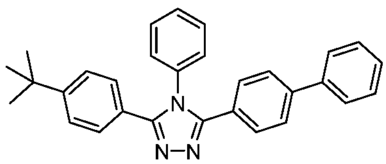

Preferred examples of compounds usable as materials that can be added to each organic layer are shown below. For example, the addition of a compound as a stabilizing material may be taken into consideration.

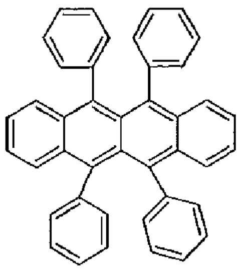
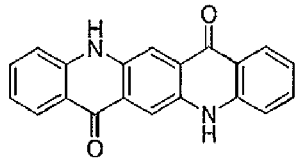
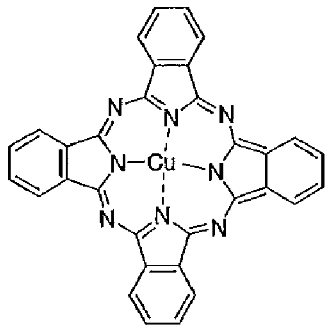

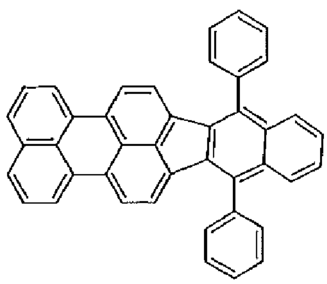
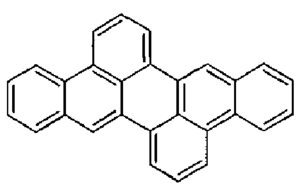

Herein under preferred materials for use in an organic electroluminescent device are specifically shown. However, the materials usable in the invention should not be limitatively interpreted by the following exemplary compounds. Compounds that are exemplified as materials having a specific function can also be used as materials having any other function.

Devices:

In some embodiments, the light-emitting layers are incorporated into a device. For example, the device includes, but is not limited to an OLED bulb, an OLED lamp, a television screen, a computer monitor, a mobile phone, and a tablet.

In some embodiments, an electronic device comprises an OLED comprising an anode, a cathode, and at least one organic layer comprising a light-emitting layer between the anode and the cathode.

In some embodiments, compositions described herein may be incorporated into various light-sensitive or light-activated devices, such as OLEDs or photovoltaic devices. In some embodiments, the composition may be useful in facilitating charge transfer or energy transfer within a device and/or as a hole transport material. The device may be, for example, an organic light-emitting diode (OLED), an organic integrated circuit (OIC), an organic field-effect transistor (O-FET), an organic thin-film transistor (O-TFT), an organic light-emitting transistor (O-LET), an organic solar cell (O-SC), an organic optical detector, an organic photoreceptor, an organic field-quench device (O-FQD), a light-emitting electrochemical cell (LEC) or an organic laser diode (O-laser).

Bulbs or Lamps:

In some embodiments, an electronic device comprises an OLED comprising an anode, a cathode, and at least one organic layer comprising a light-emitting layer between the anode and the cathode.

In some embodiments, a device comprises OLEDs that differ in color. In some embodiments, a device comprises an array comprising a combination of OLEDs. In some embodiments, the combination of OLEDs is a combination of three colors (e.g., RGB). In some embodiments, the combination of OLEDs is a combination of colors that are not red, green, or blue (for example, orange and yellow green). In some embodiments, the combination of OLEDs is a combination of two, four, or more colors.

In some embodiments, a device is an OLED light comprising,

a circuit board having a first side with a mounting surface and an opposing second side, and defining at least one aperture;
at least one OLED on the mounting surface, the at least one OLED configured to emanate light, comprising an anode, a cathode, and at least one organic layer comprising a light-emitting layer between the anode and the cathode;
a housing for the circuit board; and
at least one connector arranged at an end of the housing, the housing and the connector defining a package adapted for installation in a light fixture.

In some embodiments, the OLED light comprises a plurality of OLEDs mounted on a circuit board such that light emanates in a plurality of directions. In some embodiments, a portion of the light emanated in a first direction is deflected to emanate in a second direction. In some embodiments, a reflector is used to deflect the light emanated in a first direction.

Displays or Screens:

In some embodiments, the light-emitting layer in the invention can be used in a screen or a display. In some embodiments, the compounds in the invention are deposited onto a substrate using a process including, but not limited to, vacuum evaporation, deposition, vapor deposition, or chemical vapor deposition (CVD). In some embodiments, the substrate is a photoplate structure useful in a two-sided etch that provides a unique aspect ratio pixel. The screen (which may also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the close patterning of pixels needed for high definition displays while optimizing the chemical deposition onto a TFT backplane.

The internal patterning of the pixel allows the construction of a 3-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point, the entire pixel area is subjected to a similar etch rate but the depths are varying depending on the halftone pattern. Varying the size and spacing of the halftone pattern allows etching to be inhibited at different rates within the pixel

allowing for a localized deeper etch needed to create steep vertical bevels.

A preferred material for the deposition mask is invar. Invar is a metal alloy that is cold rolled into long thin sheet in a steel mill. Invar cannot be electrodeposited onto a rotating mandrel as the nickel mask. A preferred and more cost feasible method for forming the open areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etch. In further embodiments, a screen or display pattern is fabricated using plasma etching.

Methods of Manufacturing Devices:

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

In another aspect, provided herein is a method of manufacturing an organic light-emitting diode (OLED) display, the method comprising:

forming a barrier layer on a base substrate of a mother panel;
forming a plurality of display units in units of cell panels on the barrier layer;
forming an encapsulation layer on each of the display units of the cell panels; and
applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of the cell panel.

In some embodiments, the thin film transistor (TFT) layer EL device, a gate electrode, and a source/drain electrode. Each of the plurality of display units may include a thin film transistor (TFT) layer, a planarization film formed on the TFT layer, and a light-emitting unit formed on the planarization film, wherein the organic film applied to the interface portion is formed of a same material as a material of the planarization film and is formed at a same time as the planarization film is formed. In some embodiments, a light-emitting unit is connected to the TFT layer with a passivation layer and a planarization film therebetween and an encapsulation layer that covers and protects the light-emitting unit. In some embodiments of the method of manufacturing, the organic film contacts neither the display units nor the encapsulation layer.

Each of the organic film and the planarization film may include any one of polyimide and acryl. In some embodiments, the barrier layer may be an inorganic film. In some embodiments, the base substrate may be formed of polyimide. The method may further include, before the forming of the barrier layer on one surface of the base substrate formed of polyimide, attaching a carrier substrate formed of a glass material to another surface of the base substrate, and before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some embodiments, the planarization film and the organic film are simultaneously formed when the OLED display is manufactured. In some embodiments, the organic film may be formed on the edge portion of the barrier layer such that a portion of the organic film directly contacts the base substrate and a remaining portion of the organic film contacts the barrier layer while surrounding the edge portion of the barrier layer.

In some embodiments, the light-emitting layer includes a pixel electrode, a counter electrode, and an organic light-emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light-emitting layer emits

light, thereby forming an image. Hereinafter, an image forming unit including the TFT layer and the light-emitting unit is referred to as a display unit.

In some embodiments, the encapsulation layer that covers the display unit and prevents penetration of external moisture may be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In some embodiments, the OLED display is flexible and uses the soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In some embodiments, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further comprises cutting along the interface portion, wherein a groove is formed in the barrier layer, wherein at least a portion of the organic film is formed in the groove, and wherein the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, if the entire barrier layer is entirely exposed without the organic film, an impact generated when each of the cell panels is cut along the groove at the interface portion is transferred to the barrier layer, thereby increasing the risk of cracks. However, in some embodiments, since the groove at the interface portion between the barrier layers is covered with the organic film and the organic film absorbs an impact that would otherwise be transferred to the barrier layer, each of the cell panels may be softly cut and cracks may be prevented from occurring in the barrier layer. In some embodiments, the organic film covering the groove at the interface portion and the planarization film are spaced apart from each other. For example, if the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light-emitting unit, and the encapsulation layer is disposed on the display unit to cover the display unit. As such, once the mother panel is completely manufactured, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks may be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

## Examples

The features of the invention will be described more specifically with reference to Synthesis Examples and Examples given below. The materials, processes, procedures and the like shown below may be appropriately modified unless they deviate from the substance of the invention. Accordingly, the scope of the invention is not construed as being limited to the specific examples shown below. Herein under the light emission characteristics were evaluated using a source meter (available from Keithley Instruments Corporation: 2400 series), a semiconductor parameter analyzer (available from Agilent Corporation, E5273A), an optical power meter device (available from Newport Corporation, 1930C), an optical spectroscope (available from Ocean Optics Corporation, USB2000), a spectroradiometer (available

from Topcon Corporation, SR-3), and a streak camera (available from Hamamatsu Photonics K.K., Model C4334). The orientation value (S value) was measured using a molecular orientation characteristic measurement system (available from Hamamatsu Photonics K. K., C14234-01).

(Synthesis Example 1) Synthesis of Compound 1

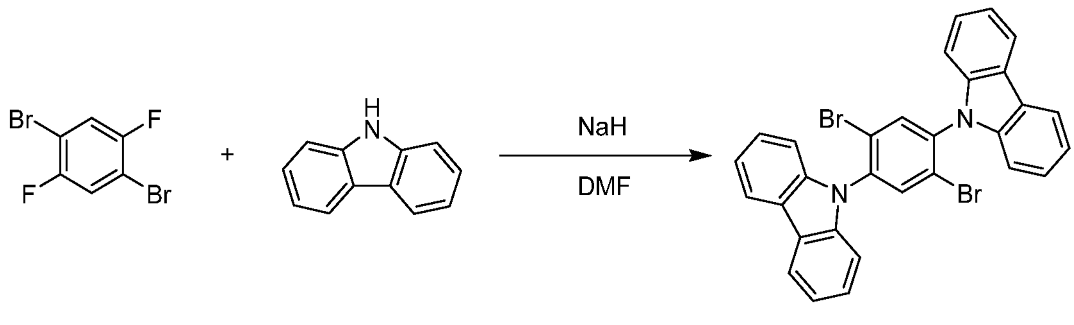


A

Under a nitrogen stream, carbazole (7.69 g, 46.0 mmol) was added to a N,N-dimethyl formamide solution (160 mL) of sodium hydride (1. 16 g, 29.0 mmol), followed by stirring at room temperature for 30 min. Then, 2,5-dibromo-1,4-difluorobenzene (5.00 g, 18.4 mmol) was added thereto, followed by stirring at 60°C for 16 h. This mixture was returned to room temperature, and water was added thereto. Then, the precipitated solid was filtered. This was purified with silica gel column chromatography (toluene), and the resultant solid was washed with acetonitrile to obtain Intermediate A (5.83 g, 10.3 mmol, yield 56%) as a white solid.

$^1$HNMR (400 MHz, $CDCl_3$, $\delta$): 8.19 (d, J = 8.0 Hz, 4H), 8.01 (s, 2H), 7.58-7.48 (m, 4H), 7.39-7.35 (m, 4H), 7.27-7.24 (m, 4H)

MS (ASAP): 567.01 ($M+H^+$). Calcd for. $C_{30}H_{18}Br_2N_2$ : 565.98

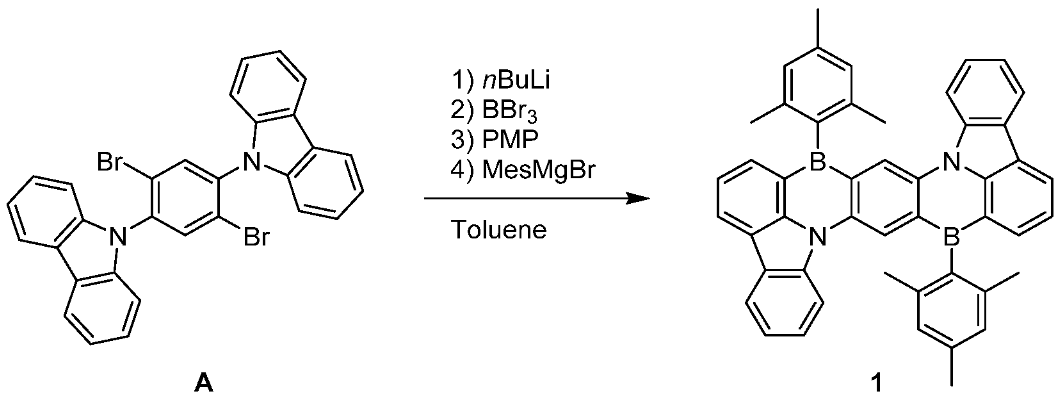


A 1

Under a nitrogen stream, to a toluene solution (100 mL) of the intermediate A (1.00 g, 1.80 mmol), n-butyl lithium (1.6 mol/L hexane solution, 4.5 mL, 7.19 mmol) was added at -30°C, followed by stirring at room temperature for 1 h. The reaction mixture was cooled to -30°C, and boron tribromide (0.991 g, 3.96 mmol) was added thereto, followed by stirring at room temperature for 30 min. To the reaction mixture, 1,2,2,6,6-pentamethyl piperidine (0.558 g, 3.60 mmol) was added, followed by stirring at 120°C for 17 h. The reaction mixture was cooled to room temperature, and 2-mesityl magnesium bromide (1.0 mol/L tetrahydrofuran solution, 5.4 mL, 5.40 mmol) was added thereto, followed by stirring at room temperature for 2.5 h. The solvent in the resultant reaction mixture was distilled off, and methanol was added, and then the precipitate was filtered. This solid was purified with silica gel column chromatography (toluene:hexane=4:6) to obtain Compound 1 as an orange solid (0.258 g, 0.388 mmol, yield 22%).

$^1$HNMR (400 MHz, $CDCl_3$, $\delta$): 9.26 (s, 2H), 8.52 (d, J = 6.8 Hz, 2H), 8.28-8.21 (m, 4H), 7.94-7.90 (m, 2H), 7.64-7.60 (m, 2H), 7.45-7.42 (m, 4H), 7.15-7.14 (m, 4H), 2.56 (s, 6H), 2.16 (s, 12H)

MS (ASAP): 664.23 ($M^+$). Calcd for. $C_{48}H_{38}B_2N_2$ : 664.32

(Synthesis Example 2) Synthesis of Compound 2

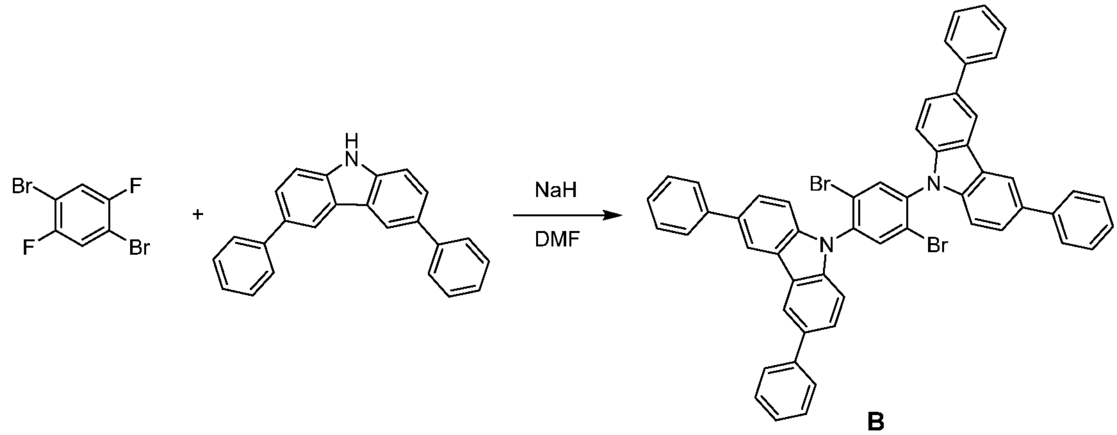


**B**

Under a nitrogen stream, 3,6-diphenyl carbazole (3.00 g, 9.39 mmol) was added to a N,N-dimethyl formamide solution (70 mL) of sodium hydride (0.376 g, 9.39 mmol), followed by stirring at room temperature for 30 min. Then, 2,5-dibromo-1,4-difluorobenzene (1.02 g, 3.76 mmol) was added thereto, followed by stirring at 60°C for 14 h. This mixture was returned to room temperature, and water and methanol were added thereto. Then, the precipitated solid was filtered. This solid was dissolved in hot toluene, and was filtered through a silica gel pad (toluene). Then, the solvent of the filtrate was distilled off. The resultant solid was washed with acetonitrile to obtain Intermediate B as a white solid (2.36 g, 2.71 mmol, yield 72%).
$^1$HNMR (400 MHz, $CDCl_3$, $\delta$): 8.46-8.44 (m, 4H), 8.10 (s, 2H), 7.79-7.75 (m, 12H), 7.54-7.49 (m, 8H), 7.41-7.35 (m, 8H)
MS (ASAP): 870.20 ($M^+$). Calcd for. $C_{54}H_{34}Br_2N_2$ : 870.11

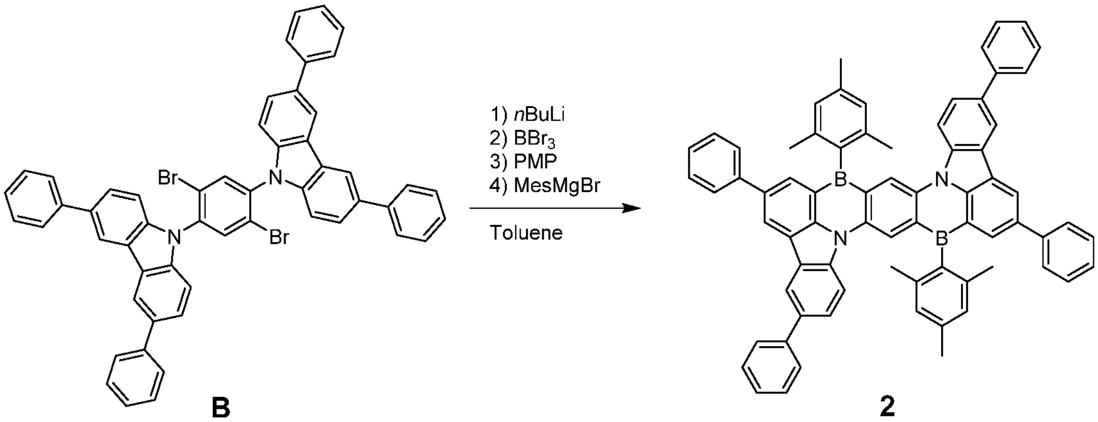


**B** **2**

Under a nitrogen stream, to a toluene solution (300 mL) of the intermediate B (1.00 g, 1.15 mmol), n-butyl lithium (1.6 mol/L hexane solution, 2.9 mL, 4.60 mmol) was added at -30°C, followed by stirring at room temperature for 1 h. The reaction mixture was cooled to -30°C, and boron tribromide (0.633 g, 2.53 mmol) was added thereto, followed by stirring at room temperature for 30 min. To the reaction mixture, 1,2,2,6,6-pentamethyl piperidine (0.357 g, 2.30 mmol) was added, followed by stirring at 120°C for 17 h. The reaction mixture was cooled to room temperature, and 2-mesityl magnesium bromide (tetrahydrofuran solution of 1.0 mol/L, 3.4 mL, 3.40 mmol) was added thereto, followed by stirring at room temperature for 4 h. The resultant reaction mixture was filtered through a silica pad (toluene), and the solvent of the filtrate was distilled off. Ethyl acetate was added to the resultant viscous body, and the precipitate was filtered to obtain Compound 2 as an orange solid (0.0710 g, 0.0732 mmol, yield 6%).
$^1$HNMR (400 MHz, $CDCl_3$, $\delta$): 9.29 (s, 2H), 8.81-8.79 (m, 2H), 8.53-8.52 (m, 2H), 8.46-8.45 (m, 2H), 7.82-7.78 (m, 8H), 7.59-7.36 (m, 14H), 7.20-7.18 (m, 4H), 2.59 (s, 6H), 2.22 (s, 12H)
MS (ASAP): 968.67 ($M^+$). Calcd for. $C_{72}H_{54}B_2N_2$ : 968.45

(Synthesis Example 3) Synthesis of Compound 3

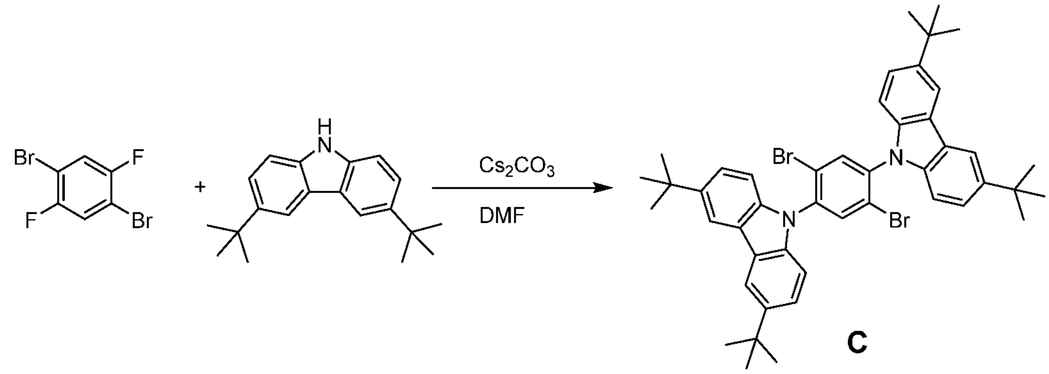


C

Intermediate C

Under a nitrogen stream, a N,N-dimethyl formamide solution (360 mL) of 3,6-ditert-butyl-9H-carbazole (29.2 g, 105 mmol), cesium carbonate (61.9 g, 190 mmol) and 2,5-dibromo-1,4-difluorobenzene (12.9 g, 47.5 mmol) was stirred at 120 °C for 17 h. This mixture was returned to room temperature, and water was added thereto, and then, the precipitated solid was filtered. This was purified with silica gel column chromatography (toluene), and the resultant solid was recrystallized by toluene/methanol to obtain Intermediate C as a white solid (17.5 g, 22.1 mmol, yield 47%).
$^1$HNMR (400 MHz, $CDCl_3$, δ): 8.2-8.17 (m, 4H), 7.93 (s, 2H), 7.55-7.52 (m, 4H), 7.17 (d, J = 8.4 Hz, 4 H), 1.49 (s, 36H)
MS (ASAP): 791.47 ($M+H^+$). Calcd for. $C_{46}H_{50}Br_2N_2$ : 790.23

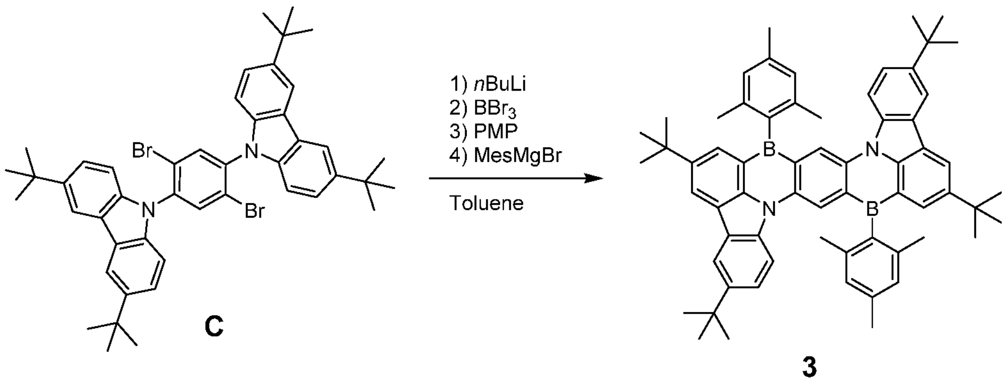


C

3

Compound 3

Under a nitrogen stream, to a toluene solution (100 mL) of the intermediate C (2.00 g, 2.52 mmol), n-BuLi (1.6 mol/L hexane solution, 4.7 mL, 7.56 mmol) was added at -30°C, followed by stirring at 50°C for 30 min. The reaction mixture was cooled to - 30°C, and boron tribromide (3.16 g, 12.6 mmol) was added thereto, followed by stirring at room temperature for 30 min. To the reaction mixture, 1,2,2,6,6-pentamethyl piperidine (1.96 g, 12.6 mmol) was added, followed by stirring at 130°C for 2 h. To the reaction mixture, 2-mesityl magnesium bromide (1.0 mol/L tetrahydrofuran solution, 25.2 mL, 25.2 mmol) was added and was stirred for 17 h while returned to room temperature. The solvent in the resultant reaction mixture was distilled off, and methanol was added, and then the precipitate was filtered. This solid was purified with silica gel column chromatography (toluene:hexane=l :9) to obtain Compound 3 as an orange solid (0.292 g, 0.328 mmol, yield 13%).
$^1$HNMR (400 MHz, $CDCl_3$, δ): 9.11 (s, 2H), 8.58 (d, J = 2.0 Hz, 2H), 8.25 (d, J = 2.0 Hz, 2H), 8.23 (d, J = 1.6 Hz, 2H), 7.75 (d, J = 9.2 Hz, 2H), 7.44-7.41 (m, 2H), 7.17 (s, 4H), 2.60 (s, 6H), 2.16 (s, 12H), 1.53-1.51 (m, 36H)
MS (ASAP): 888.85 ($M^+$). Calcd for. $C_{64}H_{70}B_2N_2$ : 888.57

(Synthesis Example 4) Synthesis of Compound 4

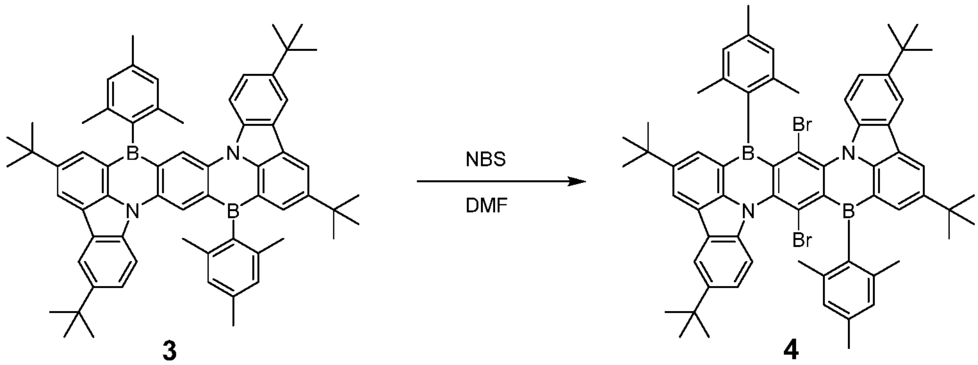


3

4

Compound 4

Under a nitrogen stream, a N, N-dimethyl formamide solution (20 mL) of Compound 3 (250 mg, 0.281 mmol) and N-bromosuccinimide (99.6 mg, 0.562 mmol) was stirred at room temperature for 16 h. To this mixture, water was added, and then the precipitated solid was filtered. This was purified with silica gel column chromatography to obtain Compound 4 as an orange solid.

(Synthesis Example 5) Synthesis of Compound 5

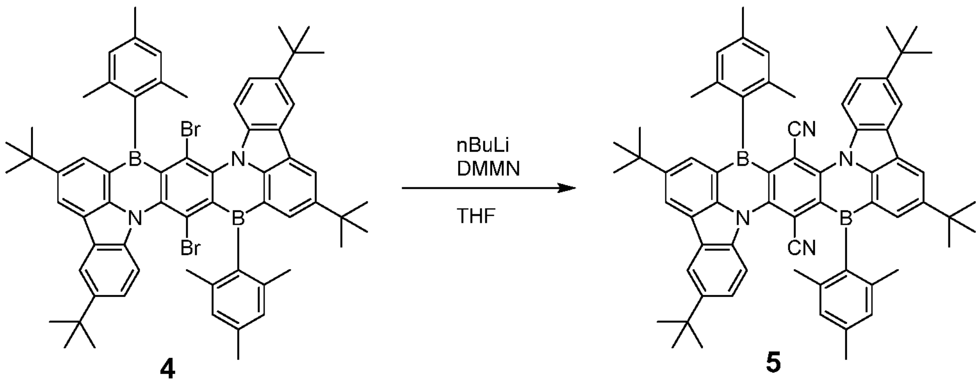


4

5

Compound 5

Under a nitrogen stream, n-BuLi (1.6 mol/L hexane solution, 0.13 mL, 0.201 mmol) was added to a tetrahydrofuran solution (10 mL) of Compound 4 (100 mg, 0.0955 mmol) at -30°C, followed by stirring at room temperature for 30 min. Dimethyl malononitrile (27.0 mg, 0.287 mmol) was added to the reaction mixture, followed by stirring at room temperature for 16 h. The solvent in the reaction mixture was distilled off, and then through purification with silica gel column chromatography, Compound 5 as a red solid was obtained.

(Synthesis example 6) Synthesis of Compound 6

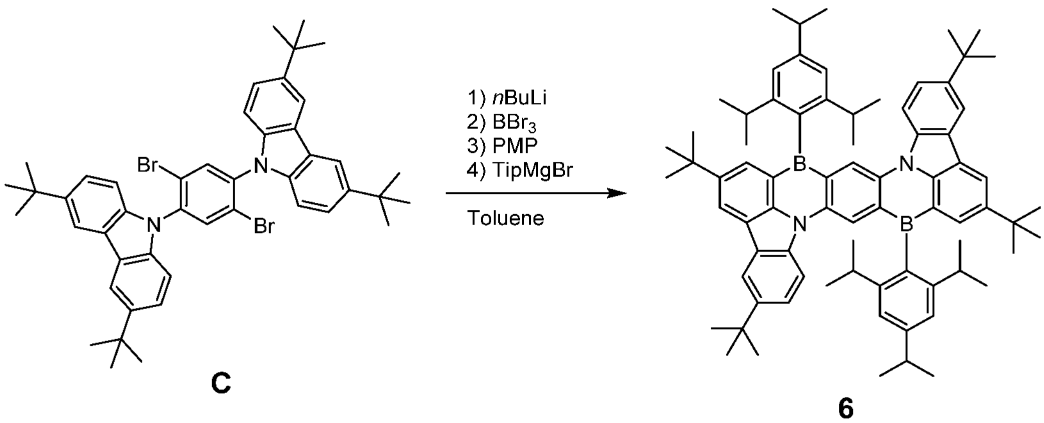


C

6

Compound 6

Under a nitrogen stream, to a toluene solution (100 mL) of the intermediate C (2.00 g, 2.53 mmol), n-BuLi (1.6 mol/L hexane solution, 4.7 mL, 7.56 mmol) was added at 0°C, followed by stirring at 50°C for 30 min. The reaction mixture was cooled to 0°C, and boron tribromide (3.16 g, 12.6 mmol) was added thereto, followed by stirring at room temperature for 30 min. To the reaction mixture, 1,2,2,6,6-pentamethyl piperidine (1.96 g, 12.6 mmol) was added, followed by stirring at 135°C for 2 h. To the reaction mixture, 2,4,6-triisopropyl magnesium bromide-lithium chloride complex (1.0 mol/L tetrahydrofuran solution, 25.2 mL, 25.2 mmol) was added and was stirred for 17 h while returned to room temperature. The solvent in the resultant reaction mixture was distilled off, and methanol was added, and then the precipitate was filtered. This solid was purified with silica gel column chromatography (toluene:hexane=1:49), and was recrystallized by toluene/methanol to obtain Compound 6 as an orange solid (0.140 g, 0.328 mmol, yield 5%).
$^1$HNMR (400 MHz, $CDCl_3$, $\delta$): 9.10 (s, 2H), 8.53 (d, J=1.6 Hz, 2H), 8.27 (d, J=1.6 Hz, 2H), 8.21 (d, J=1.6 Hz, 2H), 7.64 (d, J=8.8 Hz, 2H), 7.36 (dd, J=8.8, 1.6 Hz, 2H), 7.27 (s, 4H), 3.17 (sept, J=6.8 Hz, 2H), 2.55 (sept, J=6.8 Hz, 4H), 1.55 (d, J=6.8 Hz, 12H), 1.48 (s, 18H), 1.47 (s, 18H), 1.06 (d, J=6.8 Hz, 12H), 1.01 (d, J=6.8 Hz, 12H).
MS (MALDI): 1058.07 ($M^+$). Calcd for. $C_{76}H_{96}B_2N_2$ : 1058.78

(Synthesis example 7) Synthesis of Compounds 7 and 8

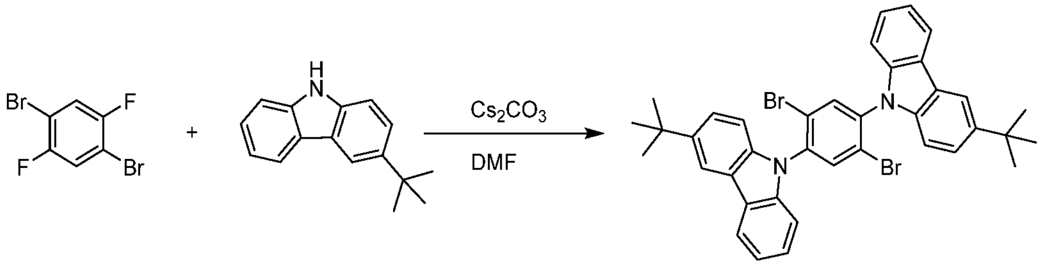


D

Intermediate D

Under a nitrogen stream, a N,N-dimethyl formamide solution (50 mL) of 3-tert-butyl-9H-carbazole (1.8 g, 8.06 mmol), potassium carbonate (1.78 g, 12.9 mmol), and 2,5-dibromo-1,4-difluorobenzene (0.876 g, 3.22 mmol) was stirred at 120°C for 17 h. This mixture was returned to room temperature, and water was added thereto, and then, the precipitated solid was filtered. This was purified with silica gel column chromatography (chloroform:hexane=1:4) to obtain Intermediate D as a white solid (0.44 g, 0.650 mmol, yield 20%).
$^1$HNMR (400 MHz, $CDCl_3$, $\delta$): 8.2-8.18 (m, 4H), 7.97 (s, 2H), 7.56 (d, J = 8.4 Hz, 2H), 7.48 (t, J = 8.4 Hz, 2H), 7.35 (t, J = 8.4 Hz, 2H), 7.25-7.18 (m, 6H), 1.49 (s, 18H)
MS (ASAP): 679.28 ($M+H^+$). Calcd for. $C_{38}H_{34}Br_2N_2$ : 678.11

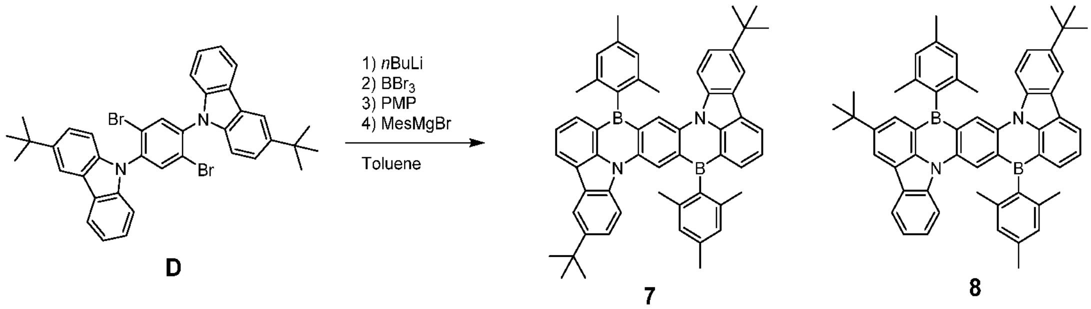


D 7 8

Compounds 7 and 8

Under a nitrogen stream, to a toluene solution (100 mL) of the intermediate D (1.90 g, 2.79 mmol), n-BuLi (1.6 mol/L hexane solution, 5.23 mL, 8.37 mmol) was added at -30°C, followed by stirring at 50 °C for 30 min. The reaction mixture was cooled to -30°C, and boron tribromide (3.49 g, 14.0 mmol) was added thereto, followed by stirring at room temperature for 30 min. To the reaction mixture, 1,2,2,6,6-pentamethyl piperidine (2.17 g, 14.0 mmol) was added, followed by stirring at 135°C for 2 h. To the reaction mixture, 2-mesityl magnesium bromide (1.0 mol/L tetrahydrofuran solution, 27.9 mL, 27.9 mmol) was added and was stirred for 17 h while returned to room temperature. The solvent in the resultant reaction mixture was distilled off, and methanol was added, and then the precipitate was filtered. This solid was purified with silica gel column chromatography (toluene:hexane=l :9) to obtain Compound 7 (0.243 g, 0.313 mmol, yield 11%) and Compound 8 (0.313 g, 0.403 mmol, yield 14%), as orange solids.

Compound 7

$^1H$ NMR (400 MHz, $CDCl_3$, δ): 9.21 (s, 2H), 8.52 (dd, J = 9.5, 2.0 Hz, 2H), 8.25 (d, J = 2.0 Hz, 2H), 8.20 (dd, J = 9.5, 2.0 Hz, 2H), 7.81 (d, J = 11.5 Hz, 2H), 7.60 (t, J = 9.5 Hz, 2H), 7.44 (dd, J= 11.5, 9.0 Hz, 2H), 7.16 (s, 4H), 2.57 (s, 6H), 2.15 (s, 12H), 1.51 (s, 18H)
MS (MALDI): 776.90 ($M^+$). Calcd for. $C_{56}H_{54}B_2N_2$ : 776.45

Compound 8

$^1H$ NMR (400 MHz, $CDCl_3$, δ): 9.21 (s, 1H), 9.16 (s, 1H), 8.58 (d, J = 2.0 Hz, 1H), 8.52 (d, J = 7.6 Hz, 1H), 8.3-8.24 (m, 3H), 8.19 (d, J = 7.2 Hz, 1H), 7.92-7.85 (m, 1H), 7.82-7.75 (m, 1H), 7.60 (t, J = 7.2 Hz, 1H), 7.47-7.39 (m, 3H), 7.19-7.13 (m, 4H), 2.6-2.55 (m, 6H), 2.17-2.14 (m, 12H), 1.51 (s, 18H)
MS (MALDI): 776.98 ($M^+$). Calcd for. $C_{56}H_{54}B_2N_2$ : 776.45

(Synthesis example 8) Synthesis of Compound 9

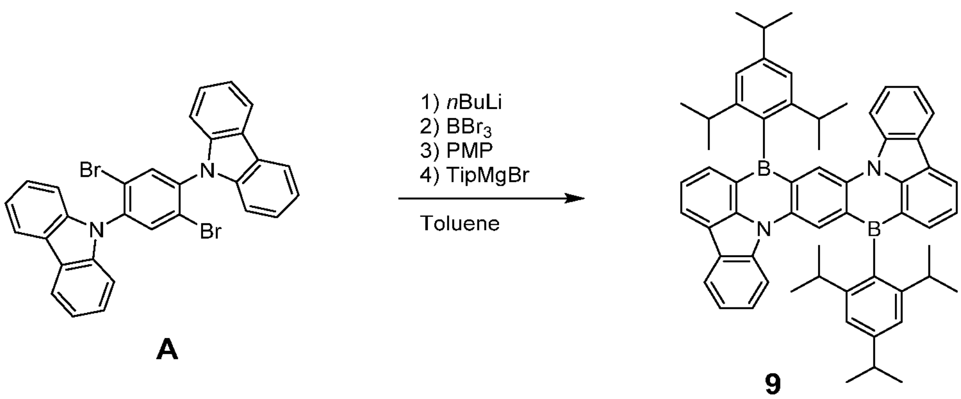


A 9

Compound 9

Under a nitrogen stream, to a toluene solution (100 mL) of the intermediate A (1.00 g, 1.77 mmol), n-BuLi (1.6

mol/L hexane solution, 3.3 mL, 5.30 mmol) was added at -30°C, followed by stirring at room temperature for 30 min. The reaction mixture was cooled to -30°C, and boron tribromide (2.21 g, 8.83 mmol) was added thereto, followed by stirring at room temperature for 30 min. To the reaction mixture, 1,2,2,6,6-pentamethyl piperidine (1.37 g, 8.83 mmol) was added, followed by stirring at 120°C for 15 h. The reaction mixture was returned to room temperature, and 2,4,6-triisopropyl magnesium bromide-lithium chloride complex (1.0 mol/L tetrahydrofuran solution, 17.7 mL, 17.7 mmol) was added thereto, followed by stirring at 120°C for 4 h. The resultant reaction mixture was filtered, and the solvent in the filtrate was distilled off. This residue was purified with silica gel column chromatography (toluene:hexane=15:85) to obtain Compound 9 as an orange solid (0.128 g, 0.154 mmol, yield 9%).
$^1$H NMR (400 MHz, $CDCl_3$, δ): 9.31 (s, 2H), 8.49 (d, J = 7.2 Hz, 2H), 8.28 (d, J = 7.2 Hz, 2H), 8.25 (d, J = 7.6 Hz, 2H), 7.85 (d, J = 8.4 Hz, 2H), 7.61 (t, J = 7.6 Hz, 2H), 7.41 (t, J = 7.6 Hz, 2H), 7.38-7.33 (m, 2H), 7.28 (s, 4H), 3.16 (sept, J = 6.8 Hz, 2H), 2.57 (sept, J = 6.8 Hz, 4H), 1.52 (d, J = 7.2 Hz, 12H), 1.09 (d, J = 6.8 Hz, 12H), 1.04 (d, J = 6.8 Hz, 12H)
MS (MALDI): 832.73 ($M^+$). Calcd for. $C_{60}H_{62}B_2N_2$ : 832.51

(Synthesis example 9) Synthesis of Compound 10

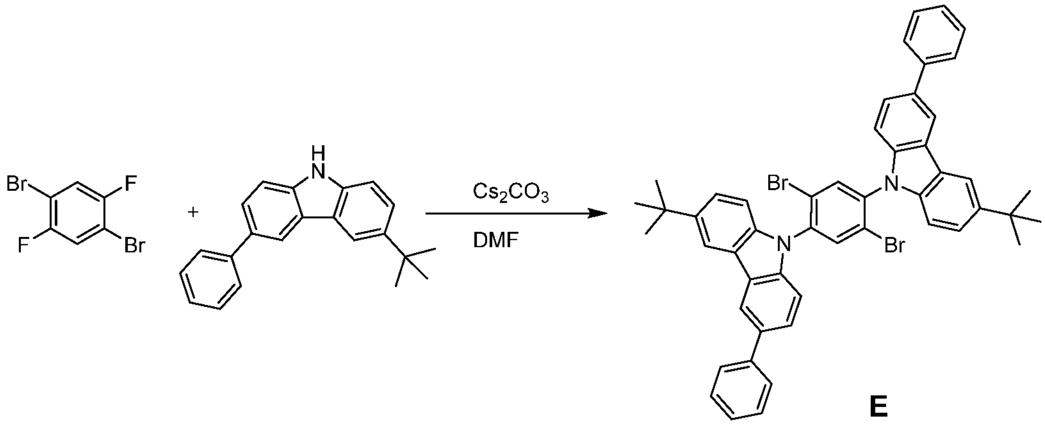

E

Intermediate E

Under a nitrogen stream, a N,N-dimethyl formamide solution (50 mL) of 3-tert-butyl-6-phenyl-9H-carbazole (2.70 g, 9.02 mmol), cesium carbonate (5.34 g, 16.4 mmol), and 2,5-dibromo-1,4-difluorobenzene (1.11 g, 4.10 mmol) was stirred at 120°C for 15 h. This mixture was returned to room temperature, and water was added thereto, and then, the precipitated solid was filtered. This was recrystallized by toluene to obtain Intermediate E as a white solid (3.06 g, 3.68 mmol, yield 90%).
$^1$HNMR (400 MHz, $CDCl_3$, δ): 8.40 (d, J = 2.0 Hz, 2H), 8.23 (d, J = 1.6 Hz, 2H), 8.02 (s, 2H), 7.79-7.76 (m, 4H), 7.73 (dd, J = 8.8, 1.6 Hz, 2H), 7.59 (dd, J = 8.4, 1.6 Hz, 2H), 7.53-7.48 (m, 4H), 7.41-7.35 (m, 2H), 7.31 (d, J = 7.6 Hz, 2H), 7.23 (d, J = 8.8 Hz, 2H), 1.50 (s, 18H)
MS (ASAP): 831.43 ($M+H^+$). Calcd for. $C_{50}H_{42}Br_2N_2$ : 830.17

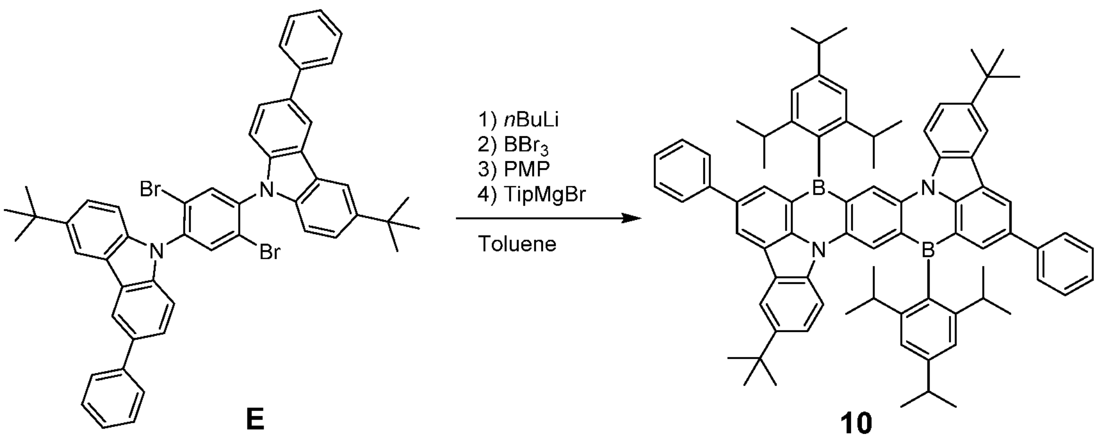

E
10

Compound 10

Under a nitrogen stream, to a toluene solution (100 mL) of the intermediate E (1.00 g, 1.21 mmol), n-BuLi (1.6 mol/L hexane solution, 2.27 mL, 3.63 mmol) was added at -30°C, followed by stirring at 50°C for 30 min. The reaction mixture was cooled to - 30°C, and boron tribromide (1.52 g, 6.05 mmol) was added thereto, followed by stirring at room temperature for 30 min. To the reaction mixture, 1,2,2,6,6-pentamethyl piperidine (0.939 g, 6.05 mmol) was added, followed by stirring at 135°C for 2 h. To the reaction mixture, 2,4,6-triisopropyl magnesium bromide-lithium chloride complex (1.0 mol/L tetrahydrofuran solution, 12.1 mL, 12.1 mmol) was added and was stirred for 17 h while returned to room temperature. The solvent in the resultant reaction mixture was distilled off, and methanol was added, and then the precipitate was filtered. This solid was purified with silica gel column chromatography (toluene:hexane= 1:9) and was recrystallized by toluene/methanol to obtain Compound 10 as an orange solid (0.364 g, 0.332 mmol, yield 27%).
$^1$HNMR (400 MHz, $CDCl_3$, δ): 9.24-9.19 (m, 2H), 8.62-8.60 (m, 2H), 8.50-8.47 (m, 2H), 8.35-8.32 (m, 2H), 7.85-7.69 (m, 6H), 7.64-7.60 (m, 2H), 7.56-7.46 (m, 4H), 7.45-7.35 (m, 2H), 7.31 (s, 4H), 3.23-3.16 (m, 2H), 2.65-2.56 (m, 4H), 1.60-1.55 (m, 18H), 1.51-1.48 (m, 12H), 1.14-1.02 (m, 24H)
MS (MALDI): 1098.09 ($M+H^+$). Calcd for. $C_{80}H_{86}B_2N_2$ : 1096.70

The compounds synthesized in Synthesis Examples were used for following purposes after sublimation purification.

(Example 1) Manufacturing of thin film

Host 1, a delayed fluorescence material described in Table 3, and a light-emitting material described in Table 3 were vapor-deposited on a quartz substrate by a vacuum evaporation method under a condition of a vacuum degree of less than $1\times10^{-3}$ Pa from different evaporation sources to form a thin film having a thickness of 100 nm. The contents of the Host 1, the delayed fluorescence material, and the light-emitting material were 64.5% by mass, 35.0% by mass, and 0.5% by mass in this order.

The results of measuring the orientation value S of the light-emitting material of the formed thin film are noted in Table 3.

(Example 2) Manufacturing and evaluation of organic electroluminescence device

On a glass substrate on which an anode made of indium tin oxide (ITO) was formed with a film thickness of 100 nm, each thin film was laminated through a vacuum evaporation method at a vacuum degree of $1\times10^{-5}$ Pa. First, HATCN was formed with a thickness of 5 nm on ITO, and NPD was formed with a thickness of 60 nm, and further EBL1 was formed with a thickness of 10 nm. Next, Host 1, a delayed fluorescence material described in Table 3, and a light-emitting material described in Table 3 were co-deposited from different evaporation sources to form a light-emitting layer with a thickness of 35 nm. The contents of the Host 1, the delayed fluorescence material, and the light-emitting material were 64.5% by mass, 35.0% by mass, and 0.5% by mass in this order. Next, SF3-TRZ was formed with a thickness of 10 nm, and then, Liq and SF3-TRZ were co-deposited from different evaporation sources to form a layer with a thickness of 30 nm. The contents of Liq and SF3-TRZ in this layer were 30% by mass and 70% by mass, respectively. Further, Liq was formed with a thickness of 2 nm, and then, aluminum (Al) was vapor-deposited with a thickness of 100 nm to form a cathode, and an organic electroluminescence device was obtained.

As a result of electrification to each organic electroluminescence device, light emission was observed from all devices. Among materials included in the light-emitting layer, the amount of light emitted from the light-emitting material was largest. The external quantum efficiency (EQE) of each organic electroluminescence device at 6.3 $mA/cm^2$ was measured, and the results are noted in Table 3. Further, time until luminescence intensity when starting the electrification was 95% was measured, and relative values when the time of Comparative Example 1 was set as 1 were noted in Table 3 (LT95). All the organic electroluminescence devices (devices 1 to 3) of the invention exhibited high EQE, and had long device lifetime and excellent durability.

Table 4

| | Light-emitting material | Delayed fluorescence material | S | EQE(%) | LT95 |
|---|---|---|---|---|---|
| Comparative Device 1 | Comparative Compound 1 | Comparative Compound T | -0.10 | 15 | 1 |
| Comparative Device 2 | Comparative Compound 1 | Compound T1 | -0.11 | 16 | 2.0 |
| Comparative Device 3 | Compound 3 | Comparative Compound T | -0.30 | 19 | 1.5 |
| Device 1 | Compound 3 | Compound T1 | -0.38 | 21 | 4.0 |

(continued)

| | Light-emitting material | Delayed fluorescence material | S | EQE(%) | LT95 |
|---|---|---|---|---|---|
| Device 2 | Compound 3 | Compound T2 | -0.41 | 21 | 3.0 |
| Device 3 | Compound 6 | Compound T1 | -0.39 | 21 | 2.0 |

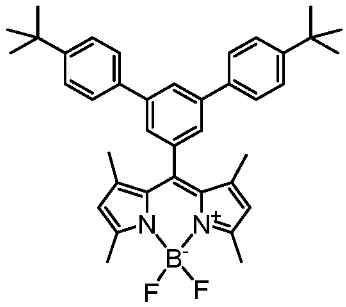

Comparative Compound 1

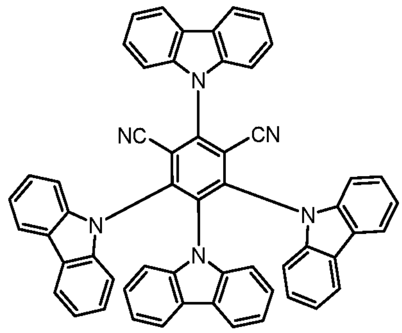

Comparative Compound T

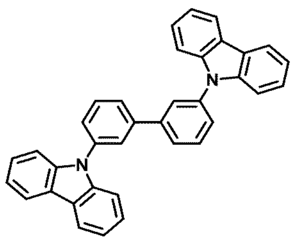

**mCBP**

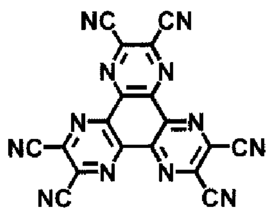

**HATCN**

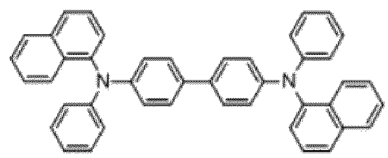

**NPD**

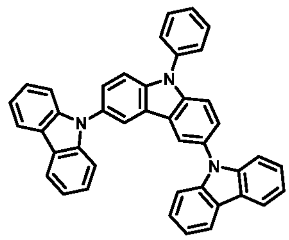

**EBL1**

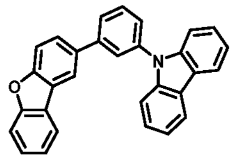

**Host1**

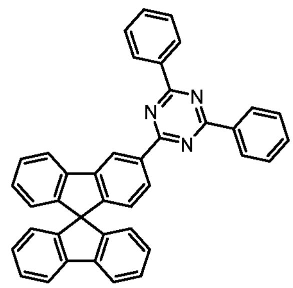

**SF3TRZ**

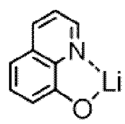

**Liq**

Industrial Applicability

In the film of the invention, the light-emitting material exhibits excellent orientation, and thus, the film can be preferably used in the organic light-emitting device. Further, the organic light-emitting device of the invention has high luminous efficiency, long device lifetime, and excellent durability, and thus, has high industrial applicability.

**Claims**

1. An organic light-emitting device comprising a compound represented by the following formula (1) and a compound represented by the following formula (2),

Formula (1)

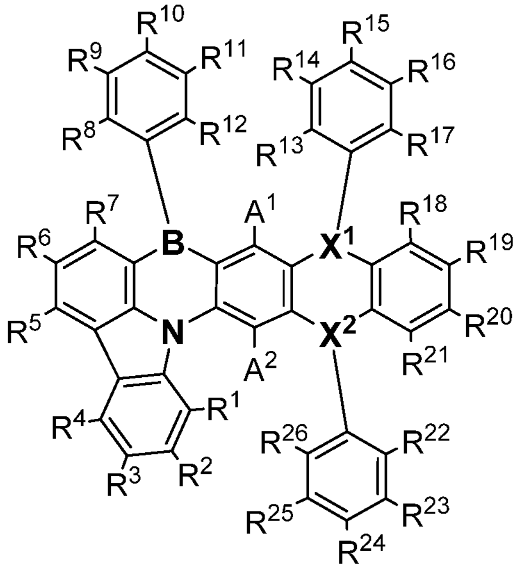

wherein in the formula (1), one of $X^1$ and $X^2$ is a nitrogen atom, and the other is a boron atom; each of $R^1$ to $R^{26}$, $A^1$, and $A^2$ independently represents a hydrogen atom, a deuterium atom, or a substituent; $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$ , $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ may be bonded to each other to form ring structures; here, when $X^1$ is a nitrogen atom, $R^{17}$ and $R^{18}$ are bonded to each other to form a single bond and to form a pyrrole ring, and when $X^2$ is a nitrogen atom, $R^{21}$ and $R^{22}$ are bonded to each other to form a single bond and to form a pyrrole ring; and here, when $X^1$ is a nitrogen atom, $R^7$ and $R^8$, and $R^{21}$ and $R^{22}$ are bonded via nitrogen atoms to form 6-membered rings, and $R^{17}$ and $R^{18}$ are bonded to each other to form a single bond, at least one of $R^1$ to $R^6$ is a substituted or unsubstituted aryl group, or an aromatic ring or a heteroaromatic ring is formed through bonding in any of $R^1$ and $R^2$, $R^2$ and $R^3$, $R^3$ and $R^4$, $R^4$ and $R^5$, and $R^5$ and $R^6$, and

Formula (2)

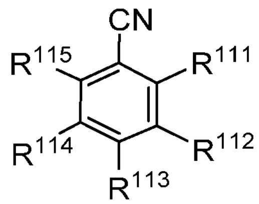

wherein in the formula (2), one of $R^{112}$ and $R^{113}$ represents a cyano group or a substituted or unsubstituted triazinyl group; each of the other of $R^{112}$ and $R^{113}$, and $R^{111}$, $R^{114}$, and $R^{115}$ independently represents a hydrogen atom, a deuterium atom, a donor group having a substituted amino group, or a substituted or unsubstituted aryl group excluding the donor group having a substituted amino group; and here, each of the other of $R^{112}$ and $R^{113}$, and at least one of $R^{111}$, $R^{114}$, and $R^{115}$ is independently a carbazole-9-yl group, provided that the carbazole-9-yl group is substituted or has a ring further condensed.

**2.** The organic light-emitting device according to claim 1, wherein both $R^3$ and $R^6$ in the formula (1) are substituents.

**3.** The organic light-emitting device according to claim 1 or 2, wherein both $R^8$ and $R^{12}$ in the formula (1) are substituents.

**4.** The organic light-emitting device according to any one of claims 1 to 3, wherein in the formula (1), $X^1$ is a nitrogen atom, and $X^2$ is a boron atom.

5. The organic light-emitting device according to any one of claims 1 to 4, wherein in the formula (1), $R^7$ and $R^8$, and $R^{17}$ and $R^{18}$ are bonded to each other to form -B($R^{32}$)-, and each of $R^{32}$'s independently represents a hydrogen atom, a deuterium atom, or a substituent.

6. The organic light-emitting device according to any one of claims 1 to 4, wherein $R^7$ and $R^8$, and $R^{17}$ and $R^{18}$ in the formula (1) are bonded to each other to form -CO-.

7. The organic light-emitting device according to any one of claims 1 to 4, wherein $R^7$ and $R^8$, and $R^{17}$ and $R^{18}$ in the formula (1) are bonded to each other to form -CS-.

8. The organic light-emitting device according to any one of claims 1 to 4, wherein in the formula (1), $R^7$ and $R^8$, and $R^{17}$ and $R^{18}$ are bonded to each other to form -N($R^{27}$)-, and each of $R^{27}$'s independently represents a hydrogen atom, a deuterium atom, or a substituent.

9. The organic light-emitting device according to any one of claims 1 to 8, wherein 1 to 6 sets among $R^1$ and $R^2$, $R^2$ and $R^3$, $R^3$ and $R^4$, $R^4$ and $R^5$, $R^5$ and $R^6$, $R^6$ and $R^7$, $R^8$ and $R^9$ , $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{18}$ and $R^{19}$, $R^{19}$ and $R^{20}$, $R^{20}$ and $R^{21}$, $R^{22}$ and $R^{23}$, $R^{23}$ and $R^{24}$, $R^{24}$ and $R^{25}$, and $R^{25}$ and $R^{26}$ in the formula (1) are bonded to each other to form benzofuran rings or benzothiophene rings.

10. The organic light-emitting device according to any one of claims 1 to 9, wherein the compound represented by the formula (1) has a rotationally symmetric structure.

11. The organic light-emitting device according to claim 1, wherein the compound represented by the formula (1) has any of following structures.

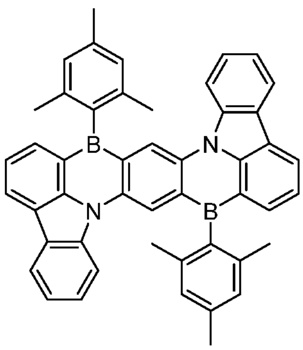

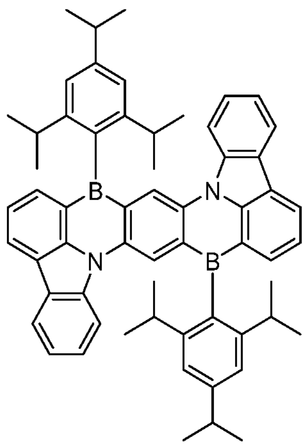

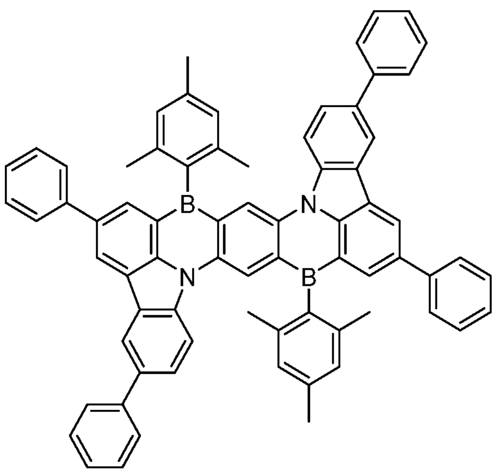

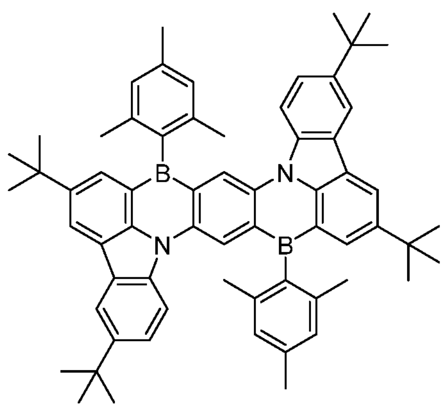

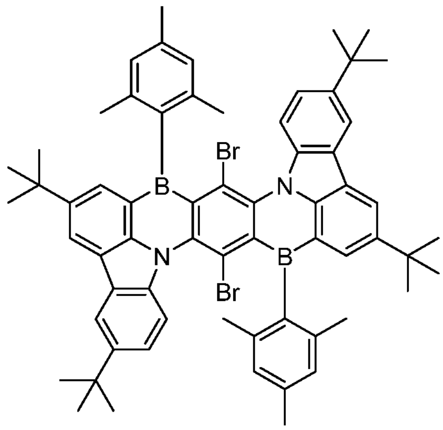

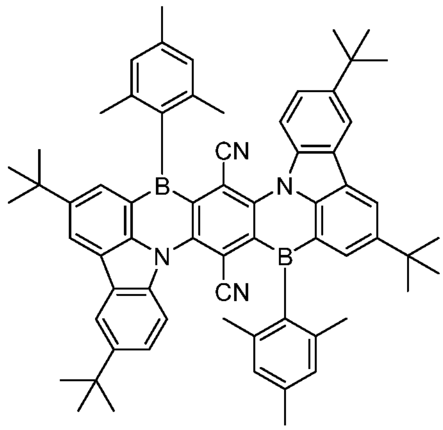

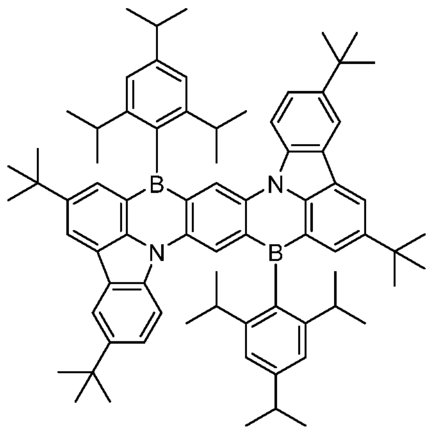

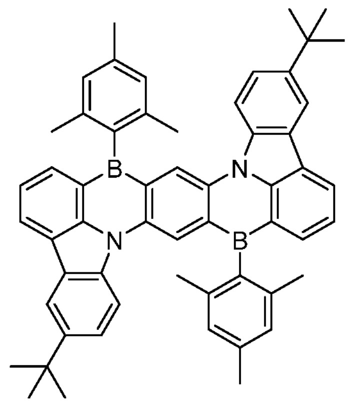

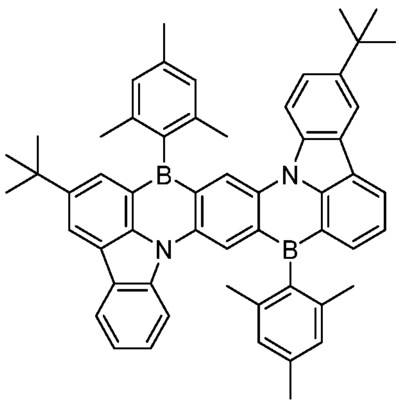

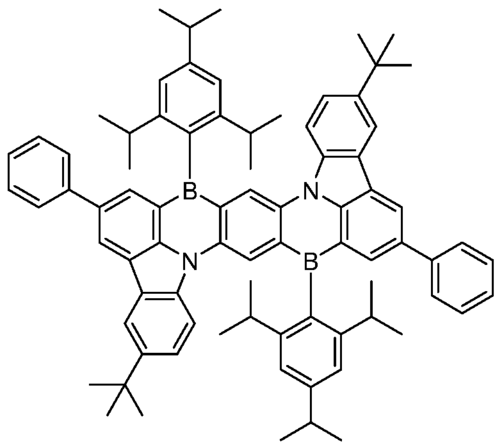

**12.** The organic light-emitting device according to any one of claims 1 to 11, wherein $R^{112}$ in the formula (2) is a cyano group or a substituted or unsubstituted triazinyl group.

**13.** The organic light-emitting device according to any one of claims 1 to 12, wherein the donor group in the formula (2) has a carbazole-9-yl structure.

**14.** A film comprising a compound represented by the following formula (1) and a compound represented by the following formula (2),

Formula (1)

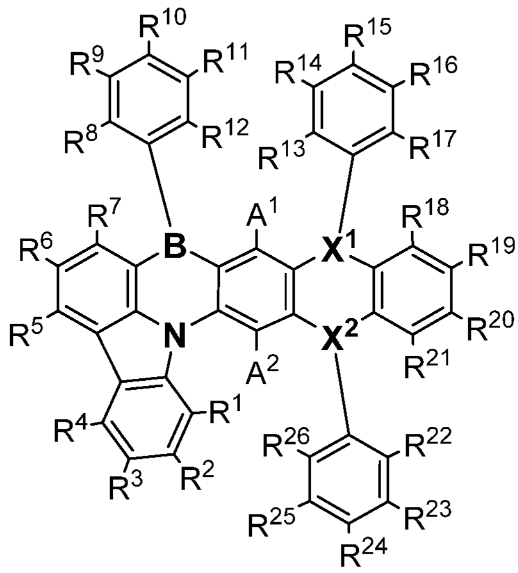

wherein in the formula (1), one of $X^1$ and $X^2$ is a nitrogen atom, and the other is a boron atom; each of $R^1$ to $R^{26}$, $A^1$, and $A^2$ independently represents a hydrogen atom, a deuterium atom, or a substituent; $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ may be bonded to each other to form ring structures; here, when $X^1$ is a nitrogen atom, $R^{17}$ and $R^{18}$ are bonded to each other to form a single bond and to form a pyrrole ring, and when $X^2$ is a nitrogen atom, $R^{21}$ and $R^{22}$ are bonded to each other to form a single bond and to form a pyrrole ring; here, when $X^1$ is a nitrogen atom, $R^7$ and $R^8$, and $R^{21}$ and $R^{22}$ are bonded via nitrogen atoms to form 6-membered rings, and $R^{17}$ and $R^{18}$ are bonded to each other to form a single bond, at least one of $R^1$ to $R^6$ is a substituted or unsubstituted aryl group, or an aromatic ring or a heteroaromatic ring is formed through bonding in any of $R^1$ and $R^2$, $R^2$ and $R^3$, $R^3$ and $R^4$, $R^4$ and $R^5$, and $R^5$ and $R^6$, and

Formula (2)

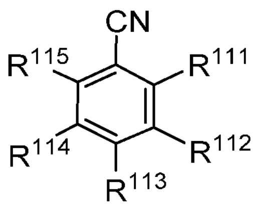

wherein in the formula (2), one of $R^{112}$ and $R^{113}$ represents a cyano group or a substituted or unsubstituted triazinyl group; each of the other of $R^{112}$ and $R^{113}$, and $R^{111}$, $R^{114}$, and $R^{115}$ independently represents a hydrogen atom, a deuterium atom, a donor group having a substituted amino group, or a substituted or unsubstituted aryl group excluding the donor group having a substituted amino group; and here, each of the other of $R^{112}$ and $R^{113}$, and at least one of $R^{111}$, $R^{114}$, and $R^{115}$ is independently a carbazole-9-yl group, provided that the carbazole-9-yl group is substituted or has a ring further condensed.

**15.** The film according to claim 14, wherein a content of the compound represented by the formula (1) is smaller than that of the compound represented by the formula (2).

**16.** The film according to claim 14 or 15, further comprising a host material having lowest excited singlet energy higher than that of the compound represented by the formula (1) and the compound represented by the formula (2).

## INTERNATIONAL SEARCH REPORT

International application No.
**PCT/JP2022/025188**

**A. CLASSIFICATION OF SUBJECT MATTER**

***H01L 51/50***(2006.01)i; ***C07D 209/86***(2006.01)i; ***C07F 5/02***(2006.01)i; ***C09K 11/06***(2006.01)i
FI: H05B33/14 B; C09K11/06 660; C07D209/86; C09K11/06 645; C09K11/06 635; C07F5/02 A CSP

According to International Patent Classification (IPC) or to both national classification and IPC

**B. FIELDS SEARCHED**

Minimum documentation searched (classification system followed by classification symbols)

H01L51/50; C07D209/86; C07F5/02; C09K11/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2020-132636 A (KWANSEI GAKUIN EDUCATIONAL FOUNDATION) 31 August 2020 (2020-08-31)<br>claims, paragraphs [0254], [0255], [0289]-[0375], [0615]-[0619], example 4 | 1-3, 8, 10, 12-16 |
| Y | | 1-3, 10, 12-16 |
| A | | 4-7, 9, 11 |
| Y | CN 110872316 A (TSINGHUA UNIVERSITY) 10 March 2020 (2020-03-10)<br>claims (in particular, claim 8), examples 2, 4, 6, 8, 10, 12, 14, 16 | 1-3, 10, 12-16 |
| A | CN 110407858 A (TSINGHUA UNIVERSITY) 05 November 2019 (2019-11-05)<br>claims (in particular, claim 4), examples | 1-16 |
| P, A | WO 2021/245221 A1 (CYNORA GMBH) 09 December 2021 (2021-12-09)<br>entire text (in particular, pp. 49-51) | 1-16 |
| P, A | CN 113402537 A (TSINGHUA UNIVERSITY) 17 September 2021 (2021-09-17)<br>claims (in particular, claim 8), example | 1-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 August 2022** | **16 August 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/025188**

**C. DOCUMENTS CONSIDERED TO BE RELEVANT**

| Category* | Citation of document, with indication, where appropriate, of the relevant passages | Relevant to claim No. |
|---|---|---|
| P, A | US 2022/0123228 A1 (SAMSUNG DISPLAY CO., LTD.) 21 April 2022 (2022-04-21) entire text, all drawings | 1-16 |

Form PCT/ISA/210 (second sheet) (January 2015)

**INTERNATIONAL SEARCH REPORT**
**Information on patent family members**

International application No.
**PCT/JP2022/025188**

| Patent document cited in search report | Publication date (day/month/year) | Patent family member(s) | Publication date (day/month/year) |
|---|---|---|---|
| JP 2020-132636 A | 31 August 2020 | KR 10-2020-0099107 A<br>CN 111560031 A | |
| CN 110872316 A | 10 March 2020 | WO 2021/008374 A1<br>claims (in particular, claim 8), examples 2, 4, 6, 8, 10, 12, 14, 16 | |
| CN 110407858 A | 05 November 2019 | WO 2021/008374 A1<br>claims (in particular, claim 4), examples | |
| WO 2021/245221 A1 | 09 December 2021 | (Family: none) | |
| CN 113402537 A | 17 September 2021 | (Family: none) | |
| US 2022/0123228 A1 | 21 April 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *Adv. Mater.,* 2016, vol. 28, 2777-2781
- *Angew. Chem. Int. Ed.,* 2018, vol. 57, 11316-11320
- **HANSCH, C.** *Chem. Rev.,* 1991, vol. 91, 165-195
- *Scientific Reports,* 2017, vol. 7, 8405